# EUROPEAN PATENT APPLICATION

(11) **EP 4 324 920 A1**
(43) Date of publication of application: **21.02.2024**
(21) Application number: 22788359.2
(22) Date of filing: 08.04.2022
(51) Int. Cl.: C12N 9/22, C12N 15/113, C12N 15/10, C12N 15/63

(54) **GENE EXPRESSION REGULATORY SYSTEM USING CRISPR SYSTEM**

(30) Priority: 16.04.2021 KR 20210050093
(71) Applicant: Genkore Inc, Yuseong-gu, Daejeon 34141 (KR)
(72) Inventor: KIM, Yong-Sam, Daejeon 34118 (KR); KIM, Do Yon, Daejeon 34141 (KR); CHIN, Hyun Jung, Daejeon 34141 (KR); JEONG, Dongmin, Daejeon 34141 (KR); KO, Jeong Heon, Daejeon 34141 (KR)
(74) Representative: Schön, Christoph
(86) International application number: PCT/KR2022/005135
(87) International publication number: WO 2022/220503

(57) **Abstract**

The present disclosure relates to a CRISPR regulatory system. More particularly, the present disclosure relates to a CRISPR regulatory system for effectively regulating expression of a target gene, comprising a Cas12f1 fusion protein and an engineered Cas12f1 guide RNA, and uses thereof. Also, the present disclosure relates to a method of regulating expression of a target gene by using a CRISPR regulatory system comprising a Cas12f1 fusion protein and an engineered Cas12f1 guide RNA.

## Description

### Technical Field

The present disclosure relates to a gene expression regulatory system using CRISPR technology. More particularly, the present disclosure relates to a gene expression regulatory system using CRISPR technology (hereinafter abbreviated as a CRISPR regulatory system) for effectively regulating expression of a target gene, the system comprising a Cas12f1 fusion protein and an engineered Cas12f1 guide RNA; and uses thereof.

### Background Art

The CRISPR regulatory system is a technology for regulating expression of a target gene by using a CRISPR/Cas system, and is currently being developed using the most studied CRISPR/Cas9 system. Generally, CRISPR regulatory systems are characterized by using a guide RNA and a dCas9 fusion protein comprising a domain that regulates transcription of a gene. Here, the CRISPR regulatory systems are divided into a CRISPR activation system and a CRISPR interference system depending on the type of a transcriptional regulatory domain fused to Cas9 protein. Such a CRISPR regulatory system can be used as an effective solution to regulate expression of a specific gene when the specific gene is overexpressed or underexpressed. However, dCas9 protein, which is mainly used in the CRISPR regulatory system, has a large size, and thus, it is difficult to make the dCas9 protein into a fusion protein for the CRISPR regulatory system and package the fusion protein into a vector such as AAV for delivery into a cell. To solve these problems, solutions are being sought through efforts such as development of a method in which the Cas9 protein is split to be delivered into a cell via multiple vectors and relatively small Cas proteins, and application thereof.

### Disclosure

### Technical Problem

An object of the present disclosure is to provide a gene expression regulatory composition for inhibiting expression of a target gene.

Another object of the present disclosure is to provide a method of inhibiting gene expression by using a gene expression regulatory composition for inhibiting expression of a target gene.

Yet another object of the present disclosure is to provide a gene expression regulatory composition for promoting expression of a target gene.

Still yet another object of the present disclosure is to provide a method of promoting gene expression by using a gene expression regulatory composition for promoting expression of a target gene.

### Technical Solution

To solve the above technical problems, the present disclosure provides a gene expression regulatory composition for inhibiting expression of a target gene, the composition comprising:
a transcriptional inhibitor Cas12f1 fusion protein or a nucleic acid encoding the transcriptional inhibitor Cas12f1 fusion protein, and
an engineered Cas12f1 guide RNA or a nucleic acid encoding the engineered Cas12f1 guide RNA,
wherein the transcriptional inhibitor Cas12f1 fusion protein comprises a dead Cas12f1 (dCas12f1) protein and a transcriptional inhibitor protein,
the dCas12f1 protein is a modified form of a wild-type Cas12f1 protein in which aspartic acid (D) which is the 326^{th} amino acid, glutamic acid (E) which is the 422^{nd} amino acid, arginine (R) which is the 490^{th} amino acid, or aspartic acid (D) which is the 510^{th} amino acid in the amino acid sequence constituting the wild-type Cas12f1 protein is substituted with alanine (A), glutamine (Q), leucine (L), tryptophan (W), or valine (V), and
the transcriptional inhibitor protein is a protein or peptide that inhibits or suppresses transcription of the gene, and
wherein the engineered Cas12f1 guide RNA comprises:
   an engineered scaffold region;
   a spacer; and
   a U-rich tail,
   wherein the engineered scaffold region, the spacer, and the U-rich tail are sequentially linked to each other in a 5' to 3' direction,
   the spacer comprises 10 nucleotides to 50 nucleotides, and has a sequence complementary to a target sequence in the gene,
   a sequence of the U-rich tail is represented by (UₐN)_{b}U_{c}, wherein N is one of adenosine (A), uridine (U), cytidine (C), and guanosine (G), and a, b, c are each an integer, with a being between 1 to 5 inclusive, and b being 0 or greater,
   a sequence of the engineered scaffold region is different from 5'-CUUCACUGAUAAAGUGGAGAACCGCUUCACCAAAAGCUGUCCCUUAGGGGAUUAGA ACUUGAGUGAAGGUGGGCUGCUUGCAUCAGCCUAAUGUCGAGAAGUGCUUUCUUC GGAAAGUAACCCUCGAAACCAAAUUCAUUUGAAAGAAUGAAGGAAUGCAAC-3' (SEQ ID NO: 7), and
   the sequence of the engineered scaffold region is such that the following sequences are sequentially linked in a 5' to 3' direction:
      a sequence selected from the group consisting of 5'-A-3', 5'-AA-3', 5'-GAA-3', 5'-AGAA-3', 5'-GAGAA-3', 5'-GGAGAA-3', 5'-UGGAGAA-3', 5'-GUGGAGAA-3', 5'-AGUGGAGAA-3', 5'-AAGUGGAGAA-3' (SEQ ID NO: 17),
      5'-AAAGUGGAGAA-3' (SEQ ID NO: 18),
      5'-UAAAGUGGAGAA-3' (SEQ ID NO: 19),
      5'-AUAAAGUGGAGAA-3' (SEQ ID NO: 20),
      5'-GAUAAAGUGGAGAA-3' (SEQ ID NO: 21),
      5'-UGAUAAAGUGGAGAA-3' (SEQ ID NO: 22),
      5'-CUGAUAAAGUGGAGAA-3' (SEQ ID NO: 23),
      5'-ACUGAUAAAGUGGAGAA-3' (SEQ ID NO: 24),
      5'-CACUGAUAAAGUGGAGAA-3' (SEQ ID NO: 25),
      5'-UCACUGAUAAAGUGGAGAA-3' (SEQ ID NO: 26),
      5'-UUCACUGAUAAAGUGGAGAA-3' (SEQ ID NO: 27), and
      5'-CUUCACUGAUAAAGUGGAGAA-3' (SEQ ID NO: 10);
      a sequence selected from the group consisting of 5'-G-3', 5'-UUAGG-3', 5'-CUUAGGG-3', 5'-CUUAGUGG-3', 5'-CCUUAGGUGG-3' (SEQ ID NO: 342), 5'-CCGUUAGGUGG-3' (SEQ ID NO: 343), 5'-CCGCUUAGGGUGG-3' (SEQ ID NO: 344),
      5'-CCGCUUUAGAGGUGG-3' (SEQ ID NO: 345),
      5'-CCGCUUUUAGAAGGUGG-3' (SEQ ID NO: 346),
      5'-CCGCUUCUUAGGAAGGUGG-3' (SEQ ID NO: 347),
      5'-CCGCUUCAUUAGUGAAGGUGG-3' (SEQ ID NO: 348),
      5'-CCGCUUCACUUAGGUGAAGGUGG-3' (SEQ ID NO: 349),
      5'-CCGCUUCACUUAGAGUGAAGGUGG-3' (SEQ ID NO: 350),
      5'-CCGCUUCACCUUAGGAGUGAAGGUGG-3' (SEQ ID NO: 351),
      5'-CCGCUUCACCAUUAGUGAGUGAAGGUGG-3' (SEQ ID NO: 352),
      5'-CCGCUUCACCAAUUAGUUGAGUGAAGGUGG-3' (SEQ ID NO: 353),
      5'-CCGCUUCACCAAAUUAGCUUGAGUGAAGGUGG-3' (SEQ ID NO: 354),
      5'-CCGCUUCACCAAAAUUAGACUUGAGUGAAGGUGG-3' (SEQ ID NO: 355),
      5'-CCGCUUCACCAAAAGUUAGAACUUGAGUGAAGGUGG-3' (SEQ ID NO: 356),
      5'-CCGCUUCACCAAAAGCUUAGGAACUUGAGUGAAGGUGG-3' (SEQ ID NO: 357),
      5'-CCGCUUCACCAAAAGCUUUAGAGAACUUGAGUGAAGGUGG-3' (SEQ ID NO: 358),
      5'-CCGCUUCACCAAAAGCUGUUAGUAGAACUUGAGUGAAGGUGG-3' (SEQ ID NO: 359),
      5'-CCGCUUCACCAAAAGCUGUUAGUUAGAACUUGAGUGAAGGUGG-3' (SEQ ID NO: 360),
      5'-CCGCUUCACCAAAAGCUGUUUAGAUUAGAACUUGAGUGAAGGUGG-3' (SEQ ID NO: 361),
      5'-CCGCUUCACCAAAAGCUGUCUUAGGAUUAGAACUUGAGUGAAGGUGG-3' (SEQ ID NO: 362), and 
5'-
CCGCUUCACCAAAAGCUGUCCCUUAGGGGAUUAGAACUUGAGUGAAGGUGG-3'
(SEQ ID NO: 11);
      a sequence selected from the group consisting of 5'-GCUGCUUGCAUCAGCCUAAUGUCGAGAAGUGCUUUCUUCGGAAAGUAACCCUCGA-3' (SEQ ID NO: 434), 
5'-
GCUGCUUGCAUCAGCCUAAUGUCGAGAAGUGCUUUUUCGAAAGUAACCCUCGA-3'
(SEQ ID NO: 435),
 
5'-
GCUGCUUGCAUCAGCCUAAUGUCGAGAAGUGCUUUUCGAAGUAACCCUCGA-3' (SEQ
ID NO: 436),
 5'- 
GCUGCUUGCAUCAGCCUAAUGUCGAGAAGUGCUCUUCGGAGUAACCCUCGA-3'
(SEQ ID NO: 437),
 
5'-GCUGCUUGCAUCAGCCUAAUGUCGAGAAGUGCUUUCGAGUAACCCUCGA-
3' (SEQ ID NO: 438),
      5'-GCUGCUUGCAUCAGCCUAAUGUCGAGAAGUGCUUCGGUAACCCUCGA-3' (SEQ ID NO: 439),
      5'-GCUGCUUGCAUCAGCCUAAUGUCGAGAAGUGUUCGUAACCCUCGA-3' (SEQ ID NO: 440),
      5'-GCUGCUUGCAUCAGCCUAAUGUCGAGAAGUUUCGAACCCUCGA-3' (SEQ ID NO: 441),
      5'-GCUGCUUGCAUCAGCCUAAUGUCGAGAAGUUCGACCCUCGA-3' (SEQ ID NO: 442),
      5'-GCUGCUUGCAUCAGCCUAAUGUCGAGAAGUUCGCCCUCGA-3' (SEQ ID NO: 443),
      5'-GCUGCUUGCAUCAGCCUAAUGUCGAGAAUUCGCCCUCGA-3' (SEQ ID NO: 444),
      5'-GCUGCUUGCAUCAGCCUAAUGUCGAGAAUUCGCCUCGA-3' (SEQ ID NO: 445),
      5'-GCUGCUUGCAUCAGCCUAAUGUCGAGAUUCGCCUCGA-3' (SEQ ID NO: 446),
      5'-GCUGCUUGCAUCAGCCUAAUGUCGAGUUCGCUCGA-3' (SEQ ID NO: 447), and 
5'-
GCUGCUUGCAUCAGCCUAAUGUCGAGAAGUGCUUUCUUCGGAAAGUAACCCUCGA-
3' (SEQ ID NO: 12);
      a sequence selected from 5'-AACAAAGAAAGGA-3' (SEQ ID NO: 111),
      5'-AACAAAUGAAAAGGA-3' (SEQ ID NO: 112),
      5'-AACAAAUUGAAAAAGGA-3' (SEQ ID NO: 113),
      5'-AACAAAUUCGAAAGAAGGA-3' (SEQ ID NO: 114),
      5'-AACAAAUUCAGAAAUGAAGGA-3' (SEQ ID NO: 115),
      5'-AACAAAUUCAUGAAAAUGAAGGA-3' (SEQ ID NO: 116),
      5'-AACAAAUUCAUUGAAAAAUGAAGGA-3' (SEQ ID NO: 117), and
      5'-AACAAAUUCAUUUGAAAGAAUGAAGGA-3' (SEQ ID NO: 118); and
      5'-AUGCAAC-3'.

To solve the above technical problems, the present disclosure provides a method of inhibiting expression of a target gene in a cell, the method comprising:
delivering, into a cell, a transcriptional inhibitor Cas12f1 fusion protein or a nucleic acid encoding the transcriptional inhibitor Cas12f1 fusion protein, and an engineered Cas12f1 guide RNA or a nucleic acid encoding the engineered Cas12f1 guide RNA,
which allows a CRISPR interference complex to be formed in the cell,
wherein the CRISPR interference complex is capable of suppressing transcription of the target gene,
the transcriptional inhibitor Cas12f1 fusion protein comprises a dead Cas12f1 (dCas12f1) protein and a transcriptional inhibitor protein,
the dCas12f1 protein is a modified form of a wild-type Cas12f1 protein in which aspartic acid (D) which is the 326^{th} amino acid, glutamic acid (E) which is the 422^{nd} amino acid, arginine (R) which is the 490^{th} amino acid, or aspartic acid (D) which is the 510^{th} amino acid with alanine (A) in the amino acid sequence constituting the wild-type Cas12f1 protein is substituted with glutamine (Q), leucine (L), tryptophan (W), or valine (V), and
the transcriptional inhibitor protein is a protein or peptide that inhibits or suppresses transcription of a gene, and
wherein the engineered Cas12f1 guide RNA comprises:
   an engineered scaffold region;
   a spacer; and
   a U-rich tail,
   wherein the engineered scaffold region, the spacer, and the U-rich tail are sequentially linked to each other in a 5' to 3' direction,
   the spacer comprises 10 to 50 nucleotides, and has a sequence complementary to a target sequence in the gene,
   a sequence of the U-rich tail is represented by (UₐN)_{b}U_{c}, wherein N is one of adenosine (A), uridine (U), cytidine (C), and guanosine (G), and a, b, c are each an integer, with a being between 1 to 5 inclusive, and b being 0 or greater,
   a sequence of the engineered scaffold region is different from 5'-CUUCACUGAUAAAGUGGAGAACCGCUUCACCAAAAGCUGUCCCUUAGGGGAUUAGA ACUUGAGUGAAGGUGGGCUGCUUGCAUCAGCCUAAUGUCGAGAAGUGCUUUUCUU CGGAAAGUAACCCUCGAAACCAAAUUCAUUUGAAAGAAUGAAGGAAUGCAAC-3' (SEQ ID NO: 7), and
   the sequence of the engineered scaffold region is such that the following sequences are sequentially linked to each other in a 5' to 3' direction:
      a sequence selected from the group consisting of 5'-A-3', 5'-AA-3', 5'-GAA-3', 5'-AGAA-3', 5'-GAGAA-3', 5'-GGAGAA-3', 5'-UGGAGAA-3', 5'-GUGGAGAA-3', 5'-AGUGGAGAA-3', 5'-AAGUGGAGAA-3' (SEQ ID NO: 17), 5'-AAAGUGGAGAA-3' (SEQ ID NO: 18), 5'-UAAAGUGGAGAA-3' (SEQ ID NO: 19), 5'-AUAAAGUGGAGAA-3' (SEQ ID NO: 20), 5'-GAUAAAGUGGAGAA-3' (SEQ ID NO: 21), 5'-UGAUAAAGUGGAGAA-3' (SEQ ID NO: 22), 5'-CUGAUAAAGUGGAGAA-3' (SEQ ID NO: 23), 5'-ACUGAUAAAGUGGAGAA-3' (SEQ ID NO: 24), 5'-CACUGAUAAAGUGGAGAA-3' (SEQ ID NO: 25), 5'-UCACUGAUAAAGUGGAGAA-3' (SEQ ID NO: 26), 5'-UUCACUGAUAAAGUGGAGAA-3' (SEQ ID NO: 27), and 5'-CUUCACUGAUAAAGUGGAGAA-3' (SEQ ID NO: 10);
      a sequence selected from the group consisting of 5'-G-3', 5'-UUAGG-3', 5'-CUUAGGG-3', 5'-CUUAGUGG-3', 5'-CCUUAGGUGG-3' (SEQ ID NO: 342), 5'-CCGUUAGGUGG-3' (SEQ ID NO: 343), 5'-CCGCUUAGGGUGG-3' (SEQ ID NO: 344),
      5'-CCGCUUUAGAGGUGG-3' (SEQ ID NO: 345),
      5'-CCGCUUUUAGAAGGUGG-3' (SEQ ID NO: 346),
      5'-CCGCUUCUUAGGAAGGUGG-3' (SEQ ID NO: 347),
      5'-CCGCUUCAUUAGUGAAGGUGG-3' (SEQ ID NO: 348),
      5'-CCGCUUCACUUAGGUGAAGGUGG-3' (SEQ ID NO: 349),
      5'-CCGCUUCACUUAGAGUGAAGGUGG-3' (SEQ ID NO: 350),
      5'-CCGCUUCACCUUAGGAGUGAAGGUGG-3' (SEQ ID NO: 351),
      5'-CCGCUUCACCAUUAGUGAGUGAAGGUGG-3' (SEQ ID NO: 352),
      5'-CCGCUUCACCAAUUAGUUGAGUGAAGGUGG-3' (SEQ ID NO: 353),
      5'-CCGCUUCACCAAAUUAGCUUGAGUGAAGGUGG-3' (SEQ ID NO: 354),
      5'-CCGCUUCACCAAAAUUAGACUUGAGUGAAGGUGG-3' (SEQ ID NO: 355),
      5'-CCGCUUCACCAAAAGUUAGAACUUGAGUGAAGGUGG-3' (SEQ ID NO: 356),
      5'-CCGCUUCACCAAAAGCUUAGGAACUUGAGUGAAGGUGG-3' (SEQ ID NO: 357),
      5'-CCGCUUCACCAAAAGCUUUAGAGAACUUGAGUGAAGGUGG-3' (SEQ ID NO: 358),
      5'-CCGCUUCACCAAAAGCUGUUAGUAGAACUUGAGUGAAGGUGG-3' (SEQ ID NO: 359),
      5'-CCGCUUCACCAAAAGCUGUUAGUUAGAACUUGAGUGAAGGUGG-3' (SEQ ID NO: 360),
      5'-CCGCUUCACCAAAAGCUGUUUAGAUUAGAACUUGAGUGAAGGUGG-3' (SEQ ID NO: 361),
      5'-CCGCUUCACCAAAAGCUGUCUUAGGAUUAGAACUUGAGUGAAGGUGG-3' (SEQ ID NO: 362), and 
5'-
CCGCUUCACCAAAAGCUGUCCCUUAGGGGAUUAGAACUUGAGUGAAGGUGG-3'
(SEQ ID NO: 11);
      a sequence selected from the group consisting of 5'-GCUGCUUGCAUCAGCCUAAUGUCGAGAAGUGCUUUCUUCGGAAAGUAACCCUCGA-3' (SEQ ID NO: 434), 
5'-
GCUGCUUGCAUCAGCCUAAUGUCGAGAAGUGCUUUUUCGAAAGUAACCCUCGA-3'
(SEQ ID NO: 435),
 
5'-
GCUGCUUGCAUCAGCCUAAUGUCGAGAAGUGCUUUUCGAAGUAACCCUCGA-3' (SEQ
ID NO: 436),
 
5'-
GCUGCUUGCAUCAGCCUAAUGUCGAGAAGUGCUCUUCGGAGUAACCCUCGA-3'
(SEQ ID NO: 437),
 
5'-GCUGCUUGCAUCAGCCUAAUGUCGAGAAGUGCUUUCGAGUAACCCUCGA-
3' (SEQ ID NO: 438),
      5'-GCUGCUUGCAUCAGCCUAAUGUCGAGAAGUGCUUCGGUAACCCUCGA-3' (SEQ ID NO: 439),
      5'-GCUGCUUGCAUCAGCCUAAUGUCGAGAAGUGUUCGUAACCCUCGA-3' (SEQ ID NO: 440),
      5'-GCUGCUUGCAUCAGCCUAAUGUCGAGAAGUUUCGAACCCUCGA-3' (SEQ ID NO: 441),
      5'-GCUGCUUGCAUCAGCCUAAUGUCGAGAAGUUCGACCCUCGA-3' (SEQ ID NO: 442),
      5'-GCUGCUUGCAUCAGCCUAAUGUCGAGAAGUUCGCCCUCGA-3' (SEQ ID NO: 443),
      5'-GCUGCUUGCAUCAGCCUAAUGUCGAGAAUUCGCCCUCGA-3' (SEQ ID NO: 444),
      5'-GCUGCUUGCAUCAGCCUAAUGUCGAGAAUUCGCCUCGA-3' (SEQ ID NO: 445),
      5'-GCUGCUUGCAUCAGCCUAAUGUCGAGAUUCGCCUCGA-3' (SEQ ID NO: 446),
      5'-GCUGCUUGCAUCAGCCUAAUGUCGAGUUCGCUCGA-3' (SEQ ID NO: 447), and 
5'-
GCUGCUUGCAUCAGCCUAAUGUCGAGAAGUGCUUUCUUCGGAAAGUAACCCUCGA-
3' (SEQ ID NO: 12);
      a sequence selected from 5'-AACAAAGAAAGGA-3' (SEQ ID NO: 111),
      5'-AACAAAUGAAAAGGA-3' (SEQ ID NO: 112),
      5'-AACAAAUUGAAAAAGGA-3' (SEQ ID NO: 113),
      5'-AACAAAUUCGAAAGAAGGA-3' (SEQ ID NO: 114),
      5'-AACAAAUUCAGAAAUGAAGGA-3' (SEQ ID NO: 115),
      5'-AACAAAUUCAUGAAAAUGAAGGA-3' (SEQ ID NO: 116),
      5'-AACAAAUUCAUUGAAAAAUGAAGGA-3' (SEQ ID NO: 117), and
      5'-AACAAAUUCAUUUGAAAGAAUGAAGGA-3' (SEQ ID NO: 118); and
      5'-AUGCAAC-3'.

To solve the above technical problem, the present disclosure provides a gene expression regulatory composition for promoting expression of a target gene, the composition comprising:
a transcriptional activator Cas12f1 fusion protein or a nucleic acid encoding the transcriptional activator Cas12f1 fusion protein; and
an engineered Cas12f1 guide RNA or a nucleic acid encoding the engineered Cas12f1 guide RNA,
wherein the transcriptional activator Cas12f1 fusion protein comprises a dead Cas12f1 (dCas12f1) protein and a transcriptional activator protein,
the dCas12f1 protein is a modified form of a wild-type Cas12f1 protein in which aspartic acid (D) which is the 326^{th} amino acid, glutamic acid (E) which is the 422^{nd} amino acid, arginine (R) which is the 490^{th} amino acid, or aspartic acid (D) which is the 510^{th} amino acid in the amino acid sequence constituting the wild-type Cas12f1 protein is substituted with alanine (A), glutamine (Q), leucine (L), tryptophan (W), or valine (V), and
the transcriptional activator protein is a DNA-binding protein or a DNA-binding peptide capable of binding to an enhancer or a promoter-proximal element, and
wherein the engineered Cas12f1 guide RNA comprises:
   an engineered scaffold region;
   a spacer; and
   a U-rich tail,
   wherein the engineered scaffold region, the spacer, and the U-rich tail are sequentially linked to each other in a 5' to 3' direction,
   the spacer comprises 10 to 50 nucleotides, and has a sequence complementary to a target sequence in the gene,
   a sequence of the U-rich tail is represented by (UₐN)_{b}U_{c}, wherein N is one of adenosine (A), uridine (U), cytidine (C), and guanosine (G), and a, b, c are each an integer, with a being between 1 to 5 inclusive, and b being 0 or greater,
   a sequence of the engineered scaffold region is different from 5'-CUUCACUGAUAAAGUGGAGAACCGCUUCACCAAAAGCUGUCCCUUAGGGGAUUAGA ACUUGAGUGAAGGUGGGCUGCUUGCAUCAGCCUAAUGUCGAGAAGUGCUUUUCUU CGGAAAGUAACCCUCGAAACCAAAUUCAUUUGAAAGAAUGAAGGAAUGCAAC-3' (SEQ ID NO: 7), and
   the sequence of the engineered scaffold region is such that the following sequences are sequentially linked to each other in a 5' to 3' direction:
      a sequence selected from the group consisting of 5'-A-3', 5'-AA-3', 5'-GAA-3', 5'-AGAA-3', 5'-GAGAA-3', 5'-GGAGAA-3', 5'-UGGAGAA-3', 5'-GUGGAGAA-3', 5'-AGUGGAGAA-3', 5'-AAGUGGAGAA-3' (SEQ ID NO: 17), 5'-AAAGUGGAGAA-3' (SEQ ID NO: 18), 5'-UAAAGUGGAGAA-3' (SEQ ID NO: 19), 5'-AUAAAGUGGAGAA-3' (SEQ ID NO: 20), 5'-GAUAAAGUGGAGAA-3' (SEQ ID NO: 21), 5'-UGAUAAAGUGGAGAA-3' (SEQ ID NO: 22), 5'-CUGAUAAAGUGGAGAA-3' (SEQ ID NO: 23), 5'-ACUGAUAAAGUGGAGAA-3' (SEQ ID NO: 24), 5'-CACUGAUAAAGUGGAGAA-3' (SEQ ID NO: 25), 5'-UCACUGAUAAAGUGGAGAA-3' (SEQ ID NO: 26), 5'-UUCACUGAUAAAGUGGAGAA-3' (SEQ ID NO: 27), and 5'-CUUCACUGAUAAAGUGGAGAA-3' (SEQ ID NO: 10);
      a sequence selected from the group consisting of 5'-G-3', 5'-UUAGG-3', 5'-CUUAGGG-3', 5'-CUUAGUGG-3', 5'-CCUUAGGUGG-3' (SEQ ID NO: 342), 5'-CCGUUAGGUGG-3' (SEQ ID NO: 343), 5'-CCGCUUAGGGUGG-3' (SEQ ID NO: 344),
      5'-CCGCUUUAGAGGUGG-3' (SEQ ID NO: 345),
      5'-CCGCUUUUAGAAGGUGG-3' (SEQ ID NO: 346),
      5'-CCGCUUCUUAGGAAGGUGG-3' (SEQ ID NO: 347),
      5'-CCGCUUCAUUAGUGAAGGUGG-3' (SEQ ID NO: 348),
      5'-CCGCUUCACUUAGGUGAAGGUGG-3' (SEQ ID NO: 349),
      5'-CCGCUUCACUUAGAGUGAAGGUGG-3' (SEQ ID NO: 350),
      5'-CCGCUUCACCUUAGGAGUGAAGGUGG-3' (SEQ ID NO: 351),
      5'-CCGCUUCACCAUUAGUGAGUGAAGGUGG-3' (SEQ ID NO: 352),
      5'-CCGCUUCACCAAUUAGUUGAGUGAAGGUGG-3' (SEQ ID NO: 353),
      5'-CCGCUUCACCAAAUUAGCUUGAGUGAAGGUGG-3' (SEQ ID NO: 354),
      5'-CCGCUUCACCAAAAUUAGACUUGAGUGAAGGUGG-3' (SEQ ID NO: 355),
      5'-CCGCUUCACCAAAAGUUAGAACUUGAGUGAAGGUGG-3' (SEQ ID NO: 356),
      5'-CCGCUUCACCAAAAGCUUAGGAACUUGAGUGAAGGUGG-3' (SEQ ID NO: 357),
      5'-CCGCUUCACCAAAAGCUUUAGAGAACUUGAGUGAAGGUGG-3' (SEQ ID NO: 358),
      5'-CCGCUUCACCAAAAGCUGUUAGUAGAACUUGAGUGAAGGUGG-3' (SEQ ID NO: 359),
      5'-CCGCUUCACCAAAAGCUGUUAGUUAGAACUUGAGUGAAGGUGG-3' (SEQ ID NO: 360),
      5'-CCGCUUCACCAAAAGCUGUUUAGAUUAGAACUUGAGUGAAGGUGG-3' (SEQ ID NO: 361),
      5'-CCGCUUCACCAAAAGCUGUCUUAGGAUUAGAACUUGAGUGAAGGUGG-3' (SEQ ID NO: 362), and 
5'-
CCGCUUCACCAAAAGCUGUCCCUUAGGGGAUUAGAACUUGAGUGAAGGUGG-3'
(SEQ ID NO: 11);
      a sequence selected from the group consisting of 5'-GCUGCUUGCAUCAGCCUAAUGUCGAGAAGUGCUUUCUUCGGAAAGUAACCCUCGA-3' (SEQ ID NO: 434), 
5'-
GCUGCUUGCAUCAGCCUAAUGUCGAGAAGUGCUUUUUCGAAAGUAACCCUCGA-3'
(SEQ ID NO: 435),
 
5'-
GCUGCUUGCAUCAGCCUAAUGUCGAGAAGUGCUUUUCGAAGUAACCCUCGA-3' (SEQ
ID NO: 436),
 
5'-
GCUGCUUGCAUCAGCCUAAUGUCGAGAAGUGCUCUUCGGAGUAACCCUCGA-3'
(SEQ ID NO: 437),
 
5'-GCUGCUUGCAUCAGCCUAAUGUCGAGAAGUGCUUUCGAGUAACCCUCGA-
3' (SEQ ID NO: 438),
      5'-GCUGCUUGCAUCAGCCUAAUGUCGAGAAGUGCUUCGGUAACCCUCGA-3' (SEQ ID NO: 439),
      5'-GCUGCUUGCAUCAGCCUAAUGUCGAGAAGUGUUCGUAACCCUCGA-3' (SEQ ID NO: 440),
      5'-GCUGCUUGCAUCAGCCUAAUGUCGAGAAGUUUCGAACCCUCGA-3' (SEQ ID NO: 441),
      5'-GCUGCUUGCAUCAGCCUAAUGUCGAGAAGUUCGACCCUCGA-3' (SEQ ID NO: 442),
      5'-GCUGCUUGCAUCAGCCUAAUGUCGAGAAGUUCGCCCUCGA-3' (SEQ ID NO: 443),
      5'-GCUGCUUGCAUCAGCCUAAUGUCGAGAAUUCGCCCUCGA-3' (SEQ ID NO: 444),
      5'-GCUGCUUGCAUCAGCCUAAUGUCGAGAAUUCGCCUCGA-3' (SEQ ID NO: 445),
      5'-GCUGCUUGCAUCAGCCUAAUGUCGAGAUUCGCCUCGA-3' (SEQ ID NO: 446),
      5'-GCUGCUUGCAUCAGCCUAAUGUCGAGUUCGCUCGA-3' (SEQ ID NO: 447), and 
5'-
GCUGCUUGCAUCAGCCUAAUGUCGAGAAGUGCUUUCUUCGGAAAGUAACCCUCGA-
3' (SEQ ID NO: 12);
      a sequence selected from 5'-AACAAAGAAAGGA-3' (SEQ ID NO: 111),
      5'-AACAAAUGAAAAGGA-3' (SEQ ID NO: 112),
      5'-AACAAAUUGAAAAAGGA-3' (SEQ ID NO: 113),
      5'-AACAAAUUCGAAAGAAGGA-3' (SEQ ID NO: 114),
      5'-AACAAAUUCAGAAAUGAAGGA-3' (SEQ ID NO: 115),
      5'-AACAAAUUCAUGAAAAUGAAGGA-3' (SEQ ID NO: 116),
      5'-AACAAAUUCAUUGAAAAAUGAAGGA-3' (SEQ ID NO: 117), and
      5'-AACAAAUUCAUUUGAAAGAAUGAAGGA-3' (SEQ ID NO: 118); and
      5'-AUGCAAC-3'.

To solve the above technical problems, the present disclosure provides a method of promoting expression of a target gene in a cell, the method comprising:
delivering, into a cell, a transcriptional activator Cas12f1 fusion protein or a nucleic acid encoding the transcriptional activator Cas12f1 fusion protein, and an engineered Cas12f1 guide RNA or a nucleic acid encoding the engineered Cas12f1 guide RNA,
which allows a CRISPR activation complex to be formed in the cell,
wherein the CRISPR activation complex is capable of suppressing transcription of the target gene,
the transcriptional activator Cas12f1 fusion protein comprises a dead Cas12f1 (dCas12f1) protein and a transcriptional activator protein,
the dCas12f1 protein is a modified form of a wild-type Cas12f1 protein in which aspartic acid (D) which is the 326^{th} amino acid, glutamic acid (E) which is the 422^{nd} amino acid, arginine (R) which is the 490^{th} amino acid, or aspartic acid (D) which is the 510^{th} amino acid in the amino acid sequence constituting the wild-type Cas12f1 protein is substituted with alanine (A), glutamine (Q), leucine (L), tryptophan (W), or valine (V), and
the transcriptional activator protein is a DNA-binding protein or peptide capable of binding to an enhancer or a promoter-proximal element, and
wherein the engineered Cas12f1 guide RNA comprises:
   an engineered scaffold region;
   a spacer; and
   a U-rich tail,
   wherein the engineered scaffold region, the spacer, and the U-rich tail are sequentially linked to each other in a 5' to 3' direction,
   the spacer comprises 10 to 50 nucleotides, and has a sequence complementary to a target sequence in the gene,
   a sequence of the U-rich tail is represented by (UₐN)_{b}U_{c}, wherein N is one of adenosine (A), uridine (U), cytidine (C), and guanosine (G), and a, b, c are each an integer, with a being between 1 to 5 inclusive, and b being 0 or greater,
   a sequence of the engineered scaffold region is different from 5'-CUUCACUGAUAAAGUGGAGAACCGCUUCACCAAAAGCUGUCCCUUAGGGGAUUAGA ACUUGAGUGAAGGUGGGCUGCUUGCAUCAGCCUAAUGUCGAGAAGUGCUUUUCUU CGGAAAGUAACCCUCGAAACCAAAUUCAUUUGAAAGAAUGAAGGAAUGCAAC-3' (SEQ ID NO: 7), and
   the sequence of the engineered scaffold region is such that the following sequences are sequentially linked to each other in a 5' to 3' direction:
   a sequence selected from the group consisting of 5'-A-3', 5'-AA-3', 5'-GAA-3', 5'-AGAA-3', 5'-GAGAA-3', 5'-GGAGAA-3', 5'-UGGAGAA-3', 5'-GUGGAGAA-3', 5'-AGUGGAGAA-3', 5'-AAGUGGAGAA-3' (SEQ ID NO: 17), 5'-AAAGUGGAGAA-3' (SEQ ID NO: 18), 5'-UAAAGUGGAGAA-3' (SEQ ID NO: 19), 5'-AUAAAGUGGAGAA-3' (SEQ ID NO: 20), 5'-GAUAAAGUGGAGAA-3' (SEQ ID NO: 21), 5'-UGAUAAAGUGGAGAA-3' (SEQ ID NO: 22), 5'-CUGAUAAAGUGGAGAA-3' (SEQ ID NO: 23), 5'-ACUGAUAAAGUGGAGAA-3' (SEQ ID NO: 24), 5'-CACUGAUAAAGUGGAGAA-3' (SEQ ID NO: 25), 5'-UCACUGAUAAAGUGGAGAA-3' (SEQ ID NO: 26), 5'-UUCACUGAUAAAGUGGAGAA-3' (SEQ ID NO: 27), and 5'-CUUCACUGAUAAAGUGGAGAA-3' (SEQ ID NO: 10);
   a sequence selected from the group consisting of 5'-G-3', 5'-UUAGG-3', 5'-CUUAGGG-3', 5'-CUUAGUGG-3', 5'-CCUUAGGUGG-3' (SEQ ID NO: 342), 5'-CCGUUAGGUGG-3' (SEQ ID NO: 343), 5'-CCGCUUAGGGUGG-3' (SEQ ID NO: 344),
   5'-CCGCUUUAGAGGUGG-3' (SEQ ID NO: 345),
   5'-CCGCUUUUAGAAGGUGG-3' (SEQ ID NO: 346),
   5'-CCGCUUCUUAGGAAGGUGG-3' (SEQ ID NO: 347),
   5'-CCGCUUCAUUAGUGAAGGUGG-3' (SEQ ID NO: 348),
   5'-CCGCUUCACUUAGGUGAAGGUGG-3' (SEQ ID NO: 349),
   5'-CCGCUUCACUUAGAGUGAAGGUGG-3' (SEQ ID NO: 350),
   5'-CCGCUUCACCUUAGGAGUGAAGGUGG-3' (SEQ ID NO: 351),
   5'-CCGCUUCACCAUUAGUGAGUGAAGGUGG-3' (SEQ ID NO: 352),
   5'-CCGCUUCACCAAUUAGUUGAGUGAAGGUGG-3' (SEQ ID NO: 353),
   5'-CCGCUUCACCAAAUUAGCUUGAGUGAAGGUGG-3' (SEQ ID NO: 354),
   5'-CCGCUUCACCAAAAUUAGACUUGAGUGAAGGUGG-3' (SEQ ID NO: 355),
   5'-CCGCUUCACCAAAAGUUAGAACUUGAGUGAAGGUGG-3' (SEQ ID NO: 356),
   5'-CCGCUUCACCAAAAGCUUAGGAACUUGAGUGAAGGUGG-3' (SEQ ID NO: 357),
   5'-CCGCUUCACCAAAAGCUUUAGAGAACUUGAGUGAAGGUGG-3' (SEQ ID NO: 358),
   5'-CCGCUUCACCAAAAGCUGUUAGUAGAACUUGAGUGAAGGUGG-3' (SEQ ID NO: 359),
   5'-CCGCUUCACCAAAAGCUGUUAGUUAGAACUUGAGUGAAGGUGG-3' (SEQ ID NO: 360),
   5'-CCGCUUCACCAAAAGCUGUUUAGAUUAGAACUUGAGUGAAGGUGG-3' (SEQ ID NO: 361),
   5'-CCGCUUCACCAAAAGCUGUCUUAGGAUUAGAACUUGAGUGAAGGUGG-3' (SEQ ID NO: 362), and 
5'-
CCGCUUCACCAAAAGCUGUCCCUUAGGGGAUUAGAACUUGAGUGAAGGUGG-3'
(SEQ ID NO: 11);
   a sequence selected from the group consisting of 5'-GCUGCUUGCAUCAGCCUAAUGUCGAGAAGUGCUUUCUUCGGAAAGUAACCCUCGA-3' (SEQ ID NO: 434), 
5'-
GCUGCUUGCAUCAGCCUAAUGUCGAGAAGUGCUUUUUCGAAAGUAACCCUCGA-3'
(SEQ ID NO: 435),
 
5'-
GCUGCUUGCAUCAGCCUAAUGUCGAGAAGUGCUUUUCGAAGUAACCCUCGA-3' (SEQ
ID NO: 436),
 5'- 
GCUGCUUGCAUCAGCCUAAUGUCGAGAAGUGCUCUUCGGAGUAACCCUCGA-3'
(SEQ ID NO: 437),
   5'-GCUGCUUGCAUCAGCCUAAUGUCGAGAAGUGCUUUCGAGUAACCCUCGA-3' (SEQ ID NO: 438),
   5'-GCUGCUUGCAUCAGCCUAAUGUCGAGAAGUGCUUCGGUAACCCUCGA-3' (SEQ ID NO: 439),
   5'-GCUGCUUGCAUCAGCCUAAUGUCGAGAAGUGUUCGUAACCCUCGA-3' (SEQ ID NO: 440),
   5'-GCUGCUUGCAUCAGCCUAAUGUCGAGAAGUUUCGAACCCUCGA-3' (SEQ ID NO: 441),
   5'-GCUGCUUGCAUCAGCCUAAUGUCGAGAAGUUCGACCCUCGA-3' (SEQ ID NO: 442),
   5'-GCUGCUUGCAUCAGCCUAAUGUCGAGAAGUUCGCCCUCGA-3' (SEQ ID NO: 443),
   5'-GCUGCUUGCAUCAGCCUAAUGUCGAGAAUUCGCCCUCGA-3' (SEQ ID NO: 444),
   5'-GCUGCUUGCAUCAGCCUAAUGUCGAGAAUUCGCCUCGA-3' (SEQ ID NO: 445),
   5'-GCUGCUUGCAUCAGCCUAAUGUCGAGAUUCGCCUCGA-3' (SEQ ID NO: 446),
   5'-GCUGCUUGCAUCAGCCUAAUGUCGAGUUCGCUCGA-3' (SEQ ID NO: 447), and 
5'-
GCUGCUUGCAUCAGCCUAAUGUCGAGAAGUGCUUUCUUCGGAAAGUAACCCUCGA-
3' (SEQ ID NO: 12);
   a sequence selected from 5'-AACAAAGAAAGGA-3' (SEQ ID NO: 111),
   5'-AACAAAUGAAAAGGA-3' (SEQ ID NO: 112),
   5'-AACAAAUUGAAAAAGGA-3' (SEQ ID NO: 113),
   5'-AACAAAUUCGAAAGAAGGA-3' (SEQ ID NO: 114),
   5'-AACAAAUUCAGAAAUGAAGGA-3' (SEQ ID NO: 115),
   5'-AACAAAUUCAUGAAAAUGAAGGA-3' (SEQ ID NO: 116),
   5'-AACAAAUUCAUUGAAAAAUGAAGGA-3' (SEQ ID NO: 117), and
   5'-AACAAAUUCAUUUGAAAGAAUGAAGGA-3' (SEQ ID NO: 118); and
   5'-AUGCAAC-3'.

### Advantageous Effects of Disclosure

Using the CRISPR expression regulatory system comprising a Cas12f1 fusion protein and an engineered Cas12f1 guide RNA, according to the present disclosure, it is possible to regulate expression of a target gene. Specifically, using the CRISPR expression regulatory system comprising a transcriptional inhibitor Cas12f1 fusion protein and an engineered Cas12f1 guide RNA, it is possible to inhibit expression of a target gene. Also, using the CRISPR expression regulatory system comprising a transcriptional activator Cas12f1 fusion protein and an engineered Cas12f1 guide RNA, it is possible to promote expression of a target gene.

### Brief Description of Drawings

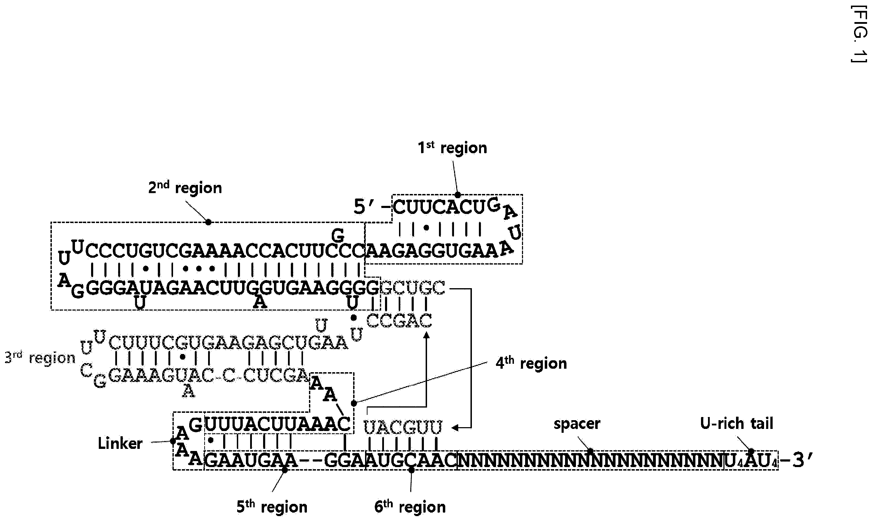

FIG. 1 is a schematic view illustrating the engineered Cas12f1 guide RNA disclosed herein.
FIGS. 2 to 5 are graphs showing average indel efficiency for Examples 1.1.1 to 1.1.13, which target DY2, among the examples disclosed in Experimental Example 2. Ex is an abbreviation for Example, Comp is an abbreviation for Comparative Example, and Control refers to a negative control.
FIGS. 6 to 9 are graphs showing average indel efficiency for Examples 1.2.1 to 1.2.13, which target DY10, among the examples disclosed in Experimental Example 2. Ex is an abbreviation for Example, Comp is an abbreviation for Comparative Example, and Control refers to a negative control.
FIGS. 10 to 13 are graphs showing average indel efficiency for Examples 1.3.1 to 1.3.13, which target Intergenic22, among the examples disclosed in Experimental Example 2. Ex is an abbreviation for Example, Comp is an abbreviation for Comparative Example, and Control refers to a negative control.
FIGS. 14 and 15 are graphs showing indel efficiency for Comparative Example 1.1.1, which targets DY2, and Comparative Example 1.2.1, which targets DY10, among the examples disclosed in Experimental Example 2, and their respective controls.
FIGS. 16 and 17 are graphs showing average indel efficiency for Examples 2.1.1 to 2.1.15, which target DY2, among the examples disclosed in Experimental Example 3.1. Ex is an abbreviation for Example.
FIGS. 18 and 19 are graphs showing average indel efficiency for Examples 2.2.1 to 2.2.15, which target DY10, among the examples disclosed in Experimental Example 3.1. Ex is an abbreviation for Example.
FIGS. 20 and 21 are graphs showing average indel efficiency for Examples 3.1.1 to 3.1.12, which target DY2, among the examples disclosed in Experimental Example 3.2. Ex is an abbreviation for Example.
FIGS. 22 and 23 are graphs showing average indel efficiency for Examples 3.2.1 to 3.2.12, which target DY10, among the examples disclosed in Experimental Example 3.2. Ex is an abbreviation for Example.
FIGS. 24 to 26 are graphs showing average indel efficiency for the respective examples disclosed in Experimental Example 4.1, and illustrate average indel efficiency for Examples 4.4.1 to 4.4.4, which target FUS, Examples 4.5.1 and 4.5.2, which target GAK, and Example 4.6.1, which targets MLH, respectively. Ex is an abbreviation for Example.
FIG. 27 illustrates results of a large-scale validation experiment of Experimental Example 5.
FIG. 28 illustrates results of an *in vitro* cleavage assay of Experimental Example 6.
FIG. 29 illustrates results obtained by identifying whether or not cleavage activity of a variety of dead Cas12f1 has been removed.
FIG. 30 schematically illustrates various module designs of a transcriptional inhibitor Cas12f1 fusion protein.
FIG. 31 illustrates results obtained by identifying effects of inhibiting expression of a target gene achieved in a case of using variously designed transcriptional inhibitor Cas12f1 fusion proteins.
FIG. 32 illustrates results obtained by identifying effects of inhibiting expression of a target gene achieved in a case of using variously designed transcriptional inhibitor Cas12f1 fusion proteins.
FIG. 33 illustrates results obtained by identifying effects of inhibiting expression of a target gene achieved in a case of using variously designed transcriptional inhibitor Cas12f1 fusion proteins.
FIG. 34 illustrates results obtained by identifying effects of inhibiting expression of a target gene achieved in a case of using variously designed transcriptional inhibitor Cas12f1 fusion proteins.
FIG. 35 illustrates results obtained by identifying effects of inhibiting expression of a target gene achieved in a case of using a transcriptional inhibitor Cas12f1 fusion protein containing KRAB.
FIG. 36 illustrates results obtained by identifying effects of inhibiting expression of a target gene achieved in a case of using transcriptional inhibitor Cas12f1 fusion proteins containing DNMT.
FIG. 37 illustrates results obtained by identifying effects of inhibiting expression of a target gene achieved in a case of using transcriptional inhibitor Cas12f1 fusion proteins containing KRAB and MeCP2.
FIG. 38 illustrates results obtained by identifying effects of inhibiting expression of a target gene achieved in a case of using transcriptional inhibitor Cas12f1 fusion proteins containing KRAB.
FIG. 39 illustrates results obtained by identifying effects of inhibiting expression of a target gene achieved in a case of using transcriptional inhibitor Cas12f1 fusion proteins containing KRAB and MeCP2.
FIG. 40 illustrates results obtained by identifying effects of inhibiting expression of a target gene achieved in a case of using transcriptional inhibitor Cas12f1 fusion proteins containing a plurality of KRABs.
FIG. 41 illustrates results obtained by identifying effects of inhibiting expression of a target gene achieved in a case of using transcriptional inhibitor Cas12f1 fusion proteins containing a plurality of KRABs and MeCP2.
FIG. 42 illustrates results obtained by identifying effects of inhibiting expression of a target gene achieved in a case of using transcriptional inhibitor Cas12f1 fusion proteins containing HDAC.
FIG. 43 illustrates results obtained by identifying effects of inhibiting expression of a target gene achieved in a case of using a transcriptional inhibitor Cas12f1 fusion protein containing KRAB and MeCP2.
FIG. 44 illustrates results obtained by identifying effects of inhibiting expression of a target gene achieved in a case of using transcriptional inhibitor Cas12f1 fusion proteins containing a plurality of KRABs and MeCP2.
FIG. 45 illustrates results obtained by identifying effects of inhibiting expression of a target gene achieved in a case of using transcriptional inhibitor Cas12f1 fusion proteins containing KRAB and MeCP2.
FIG. 46 illustrates results obtained by selecting targets located in the promoter of PCSK9 gene and identifying indel efficiency therefor to identify effects of inhibiting expression of the PCSK9 gene.
FIG. 47 illustrates results obtained by performing guide RNA optimization and spacer optimization to increase targeting efficiency.
FIG. 48 illustrates results obtained by performing guide RNA optimization and spacer optimization to increase targeting efficiency.
FIG. 49 illustrates results obtained by identifying indel efficiency for targets located in the promoter of PCSK9 gene using optimal guide RNA.
FIG. 50 illustrates results obtained by identifying inhibited expression of PCSK9 gene in HepG2 cells using variously designed CRISPR expression regulatory systems.
FIG. 51 illustrates results obtained by identifying inhibited expression of PCSK9 gene in Hep3B cells using variously designed CRISPR expression regulatory systems.
FIG. 52 illustrates results obtained by identifying inhibited expression of PCSK9 gene in Huh7 cells using variously designed CRISPR expression regulatory systems.
FIG. 53 illustrates results obtained by identifying inhibited expression of PCSK9 gene in HepG2 cells using variously designed CRISPR expression regulatory systems.
FIG. 54 is a schematic view of a transcriptional activator Cas12f1 fusion protein containing VP64.
FIG. 55 illustrates results obtained by identifying increased expression of OCT4 gene using CRISPR expression regulatory systems that comprise a transcriptional activator Cas12f1 fusion protein containing VP64.

### MODE OF DISCLOSURE

Definition of terms used herein is as follows.

### About

As used herein, the term "about" refers to an amount, level, value, number, frequency, percentage, dimension, size, amount, weight, or length that varies by approximately 30%, 25%, 20%, 25%, 10%, 9%, 8%, 7%, 6%, 5%, 4%, 3%, 2%, or 1% with respect to a reference amount, level, value, number, frequency, percentage, dimension, size, amount, weight or length.

### A,T, C, G, and U

As used herein, the symbols A, T, C, G, and U have the same meanings as commonly understood by those skilled in the art to which the disclosure belongs. It may be properly interpreted as a base, a nucleoside, or a nucleotide in DNA or RNA depending on the context and description. For example, in a case where the symbols mean bases, they may be interpreted as adenine (A), thymine (T), cytosine (C), guanine (G), or uracil (U), respectively; in a case where the symbols mean nucleosides, they may be interpreted as adenosine (A), thymidine (T), cytidine (C), guanosine (G), or uridine (U), respectively; and in a case where the symbols mean nucleotides, they may be interpreted to mean nucleotides including the respective nucleosides.

### Operably linked

As used herein, the term "operably linked" means that, in gene expression technology, a particular component is linked to another component so that the particular component can perform its intended function. For example, in a case where a promoter sequence is operably linked to a coding sequence, it means that the promoter is linked thereto so as to affect transcription and/or expression of the coding sequence in a cell. In addition, the term includes all meanings recognized by those of ordinary skill in the art and may be appropriately interpreted depending on the context.

### Target gene or target nucleic acid

As used herein, "target gene" or "target nucleic acid" basically means a gene or nucleic acid in a cell which becomes a target for regulation of gene expression. The target gene or target nucleic acid may be used interchangeably and may refer to the same target. Unless otherwise described, the target gene or target nucleic acid may refer to an endogenous gene or nucleic acid found in the cell, or an exogenous gene or nucleic acid, and is not limited to particular embodiments as long as it can be a target for regulation of gene expression. The target gene or target nucleic acid may be single-stranded DNA, double-stranded DNA, and/or RNA. In addition, the term includes all meanings recognized by those of ordinary skill in the art, and may be appropriately interpreted depending on the context.

### Target Sequence

As used herein, "target sequence" refers to a particular sequence recognized by a CRISPR activation complex or a CRISPR interference complex to regulate expression of a target gene or a target nucleic acid. The target sequence may be appropriately selected depending on the purpose. Specifically, the "target sequence" is a sequence included in the target gene or target nucleic acid sequence, and refers to a sequence having complementarity with a spacer sequence included in the guide RNA or the engineered guide RNA provided herein. In general, the spacer sequence is determined in consideration of a sequence of a target gene or target nucleic acid and a PAM sequence recognized by a Cas12f1 fusion protein. The target sequence may refer only to a particular strand complementarily binding to a guide RNA of a CRISPR activation complex or a CRISPR interference complex, or may refer to an entire target double strand including the particular strand. The term may be interpreted appropriately depending on the context. In addition, the term includes all meanings recognized by those of ordinary skill in the art and may be appropriately interpreted depending on the context.

### Vector

As used herein, unless otherwise specified, the "vector" refers collectively to any material capable of transporting a genetic material into a cell. For example, a vector may be a DNA molecule including a genetic material of interest, for example, a nucleic acid encoding a Cas12f1 fusion protein of the CRISPR expression regulatory system, and/or a nucleic acid encoding a guide RNA, but the disclosure is not limited thereto. The term includes all meanings that can be recognized by those of ordinary skill in the art, and may be appropriately interpreted depending on the context.

### Naturally occurring

As used herein, the term "naturally occurring" refers to an object that is found in nature and is not artificially modified. The term is used to distinguish it from an "engineered object" obtained by artificial modification. The "naturally occurring" gene, nucleic acid, DNA, RNA, and the like are used as concepts that encompass all genes, nucleic acids, DNA, and RNA in wild type and mature form (active form). The term includes all meanings recognized by those of ordinary skill in the art and should be appropriately interpreted depending on the context.

### Engineered

As used herein, the term "engineered" is used to distinguish it from a material, a molecule, or the like whose configuration already exists in nature, and refers tp a material, a molecule or the like which has undergone artificial modification. For example, the "engineered guide RNA" refers to a guide RNA obtained by applying artificial modification to the configuration of a naturally occurring guide RNA. In addition, the term includes all meanings recognized by those of ordinary skill in the art and may be appropriately interpreted depending on the context.

### Nuclear localization sequence or signal (NLS)

The term "NLS" as used herein refers to a peptide of a certain length or a sequence thereof that is attached to a substance to be transported into the cell nucleus by nuclear transport and acts as a type of "tag."

Specifically, the NLS may be, but is not limited to, an NLS sequence derived from: the NLS of an SV40 virus large T-antigen having the amino acid sequence PKKKRKV (SEQ ID NO: 278); the NLS from a nucleoplasmin (for example, the nucleoplasmin bipartite NLS having the sequence KRPAATKKAGQAKKKK (SEQ ID NO: 279)); the c-myc NLS having the amino acid sequence PAAKRVKLD (SEQ ID NO: 280) or RQRRNELKRSP (SEQ ID NO: 281); the hRNPA1 M9 NLS having the sequence NQSSNFGPMKGGNFGGRSSGPYGGGGQYFAKPRNQGGY (SEQ ID NO: 282); the sequence RMRIZFKNKGKDTAELRRRRVEVSVELRKAKKDEQILKRRNV (SEQ ID NO: 283) of an IBB domain from importin alpha; the sequences VSRKRPRP (SEQ ID NO: 284) and PPKKARED (SEQ ID NO: 285) of myoma T protein; the sequence PQPKKKPL (SEQ ID NO: 286) of human p53; the sequence SALIKKKKKMAP (SEQ ID NO: 287) of mouse c-abl IV; the sequences DRLRR (SEQ ID NO: 288) and PKQKKRK (SEQ ID NO: 289) of influenza virus NS1; the sequence RKLKKKIKKL (SEQ ID NO: 290) of hepatitis virus delta antigen; the sequence REKKKFLKRR (SEQ ID NO: 291) of mouse Mx1 protein; the sequence KRKGDEVDGVDEVAKKKSKK (SEQ ID NO: 292) of human poly(ADP-ribose) polymerase; or the sequence RKCLQAGMNLEARKTKK (SEQ ID NO: 293) of steroid hormone receptor (human) glucocorticoid. As used herein, the term "NLS" includes all meanings recognized by those of ordinary skill in the art and may be appropriately interpreted depending on the context.

### Nuclear export sequence or signal (NES)

The term "NES" as used herein refers to a peptide of a certain length or a sequence thereof that is attached to a substance to be transported outside the nucleus by nuclear transport and acts as a type of "tag." As used herein, the term "NES" includes all meanings recognized by those of ordinary skill in the art and may be appropriately interpreted depending on the context.

### Tag

As used herein, the term "tag" refers collectively to a functional domain added to facilitate tracking and/or separation and purification of a peptide or protein. Specifically, the tag includes, but is not limited to: tag proteins such as a histidine (His) tag, a V5 tag, a FLAG tag, an influenza hemagglutinin (HA) tag, a Myc tag, a VSV-G tag, and a thioredoxin (Trx) tag; autofluorescent proteins such as a green fluorescent protein (GFP), a yellow fluorescent protein (YFP), a cyan fluorescent protein (CFP), a blue fluorescent protein (BFP), HcRED, and DsRed; and reporter genes such as a glutathione-S-transferase (GST), a horseradish peroxidase (HRP), a chloramphenicol acetyltransferase (CAT) beta-galactosidase, a betaglucuronidase, and a luciferase. As used herein, the term "tag" includes all meanings recognized by those of ordinary skill in the art and may be appropriately interpreted depending on the context.

Unless otherwise defined, all technical and scientific terms used herein have the same meaning as commonly understood by those of ordinary skill in the art to which the present disclosure belongs. Although methods and materials similar or equivalent to those described herein may be used in practice or experimentation of the present disclosure, suitable methods and materials are described below. All publications, patents, and other references mentioned herein are incorporated by reference in their entirety. Additionally, the materials, methods, and examples are illustrative only and not intended to limit the present disclosure.

Hereinafter, the present disclosure will be described.

### Background art - CRISPR/Cas12f1 system

### CRISPR/Cas12f1 system

A CRISPR/Cas12f system belongs to a V-F subtype among type V CRISPR/Cas systems, which is further divided into V-F1 to V-F3 variants. The CRISPR/Cas12f system includes a CRISPR/Cas14 system comprising Cas14a, Cas14b, and Cas14c variants among the effector proteins named Cas14 in a previous study (Harrington et al., Programmed DNA destruction by miniature CRISPR-Cas14 enzymes, Science 362, 839-842 (2018)). Among them, the CRISPR/Cas14a system comprising a Cas14a effector protein is classified as a CRISPR/Cas12f1 system (Makarova et al., Nature Reviews, Microbiology volume 18, 67 (2020)). Recent previous studies (Takeda et al., Structure of the miniature type V-F CRISPR-Cas effector enzyme, Molecular Cell 81, 1-13 (2021), Xiao et al., Structural basis for the dimerization-dependent CRISPR-Cas12f nuclease, bioRxiv (2020)) and the like have revealed a structure of the CRISPR/Cas12f1 complex.

### CRISPR regulatory system using CRISPR/Cas12f1 system

The CRISPR/Cas12f1 system is characterized in that a size of the Cas12f1 protein is significantly smaller than a CRISPR/Cas9 system. This characteristic makes it possible to solve the difficulty of developing a fusion protein, which is caused by sizes of the most previously studied Cas nucleases, the difficulty of loading the resulting system into adenoassociated virus (AAV), and the consequent difficulty of applying it as a therapeutic agent. However, despite these advantages, as revealed in previous studies (Harrington et al., Science 362, 839-842 (2018), Tautvydas Karvelis et al., Nucleic Acids Research 48, 5016-5023 (2020)), the CRISPR/Cas12f1 system shows no cleavage activity or shows cleavage activity with extremely low efficiency on double-stranded DNA in a cell, which limits its active application to gene editing. However, to overcome such limitation, the present inventors have recently developed an engineered Cas12f1 guide RNA to increase intracellular gene editing activity of the CRISPR/Cas12f1 system.

Thus, in a case of using an engineered guide RNA with improved target specificity and a Cas12f1 protein with a small size, it is expected that the CRISPR regulatory system can be more efficiently used for which difficulties have existed in efficient use because a large size of conventional Cas9 makes it difficult to develop a fusion protein and package the resulting system into an AAV vector.

Hereinafter, the CRISPR regulatory system using the CRISPR/Cas12f1 system will be described in detail.

### <CRISPR regulatory system>

In the present disclosure, there is provided a CRISPR regulatory system using a CRISPR/Cas12f1 system. More specifically, the CRISPR regulatory system comprises an engineered Cas12f1 guide RNA and a Cas12f1 fusion protein. Here, the CRISPR regulatory system can be divided into a CRISPR activation system and a CRISPR interference system depending on the Cas12f1 fusion protein. The CRISPR activation system serves to increase or promote expression of a target whose expression is to be regulated, that is, a target gene. In contrast, the CRISPR interference system serves to inhibit or suppress expression of a target gene. This effect is achieved by an expression regulatory domain included in the Cas12f1 fusion protein. Thus, an effect of the CRISPR regulatory system varies depending on whether the expression regulatory domain is a transcriptional activator protein or a transcriptional inhibitor protein.

The CRIPSR regulatory system comprises a Cas12f1 fusion protein and an engineered Cas12f1 guide RNA. Here, the Cas12f1 fusion protein comprises a modified Cas12f1 protein and an expression regulatory domain. An effect of the CRIPSR regulatory system may vary depending on the type of the expression regulatory domain.

In an embodiment, the CRIPSR regulatory system may be a CRISPR interference system for inhibiting or suppressing expression of a target gene. The CRISPR interference system comprises a transcriptional inhibitor Cas12f1 fusion protein and an engineered Cas12f1 guide RNA. Here, the transcriptional inhibitor Cas12f1 fusion protein comprises a modified Cas12f1 protein and a transcriptional inhibitor protein.

In an embodiment, the CRIPSR regulatory system may be a CRISPR activation system for increasing or promoting expression of a target gene. The CRISPR activation system comprises a transcriptional activator Cas12f1 fusion protein and an engineered Cas12f1 guide RNA. Here, the transcriptional activator Cas12f1 fusion protein comprises a modified Cas12f1 protein and a transcriptional activator protein.

Hereinafter, each component will be described in detail.

### 1. Expression regulatory protein - Cas12f1 fusion protein

The CRISPR regulatory system provided herein comprises a Cas12f1 fusion protein. The Cas12f1 fusion protein serves as an expression regulatory protein that regulates expression of a target gene. Basically, the Cas12f1 fusion protein comprises a modified Cas12f1 protein and an expression regulatory domain. The CRISPR regulatory system may increase or enhance, or inhibit or suppress expression of a target gene depending on the expression regulatory domain of the Cas12f1 fusion protein. In addition, efficiency of the CRISPR regulatory system may vary depending on the type, number, combination, and fusion location of expression regulatory domain(s) included in the Cas12f1 fusion protein.

In the present disclosure, there is provided a Cas12f1 fusion protein for a CRISPR regulatory system. The Cas12f1 fusion protein comprises a modified Cas12f1 protein and an expression regulatory domain. The modified Cas12f1 protein is a Cas12f1 variant obtained by modifying at least a portion of the sequence of the wild-type Cas12f1 protein, and the Cas12f1 variant has an altered function as compared with the wild-type Cas12f1 protein due to the modification. The modified Cas12f1 protein is characterized by having an altered function such that it cannot cleave all double strands of a target nucleic acid or target gene. The expression regulatory domain is characterized by being a protein that activates or inhibits transcription of a target gene.

In an embodiment, the Cas12f1 fusion protein may comprise a modified Cas12f1 protein and a transcriptional activator protein. Here, the CRISPR regulatory system comprising the Cas12f1 fusion protein is characterized by increasing or enhancing expression of a target gene.

In another embodiment, the Cas12f1 fusion protein may comprise a modified Cas12f1 protein and a transcriptional inhibitor protein. Here, the CRISPR regulatory system comprising the Cas12f1 fusion protein is characterized by inhibiting or suppressing expression of a target gene.

### Characteristic 1 of Cas12f1 fusion protein - Inclusion of modified Cas12f1 protein

The Cas12f1 fusion protein provided herein is characterized by comprising a modified Cas12f1 protein whose function is altered such that it cannot cleave double strands of a target nucleic acid or target gene. The modified Cas12f1 protein is characterized in that it is obtained by modifying at least one amino acid in the amino acid sequence of the wild-type Cas12f1 protein.

### Characteristic 2 of Cas12f1 fusion protein - Inclusion of expression regulatory domain

The Cas12f1 fusion protein provided herein is characterized by comprising, as an expression regulatory domain, a protein that activates or inhibits transcription of a target gene. The expression regulatory domain is characterized by being a transcriptional activator protein or a transcriptional inhibitor protein.

In an embodiment, the transcriptional activator protein may be VP64, Sun Tag, VPR (VP64, p65, Rta), or TV (TAL, VP64).

In another embodiment, the transcriptional inhibitor protein may be KRAB, DNMT, MeCP2, HDAC, LSD, SRDX SALL1, or SDS3.

### Characteristic 3 of Cas12f1 fusion protein - Modularization being done in various ways

The Cas12f1 fusion protein provided herein may comprise two or more expression regulatory domains, in which the type, number, combination, and fusion location of the expression regulatory domains can be designed in various ways. The fact that modularization of the Cas12f1 fusion protein is done in various ways enables development of a more effective CRISPR regulatory system by taking advantage of a small size of the Cas12f1 protein. Efficiency of the CRISPR regulatory system may vary depending on the modularization done in various ways, and such modularization makes it possible to design a CRISPR regulatory system comprising an optimal Cas12f1 fusion protein depending on the target gene.

In an embodiment, the Cas12f1 fusion protein may comprise a modified Cas12f1 protein and at least two transcriptional inhibitor proteins. Here, the at least two transcriptional inhibitor proteins may be different proteins. Here, all of the different transcriptional inhibitor proteins may be located at the N-terminus of the modified Cas12f1 protein. Alternatively, all of the different transcriptional inhibitor proteins may be located at the C-terminus of the modified Cas12f1 protein. Alternatively, each of the different transcriptional inhibitor proteins may be located at the N-terminus or the C-terminus of the modified Cas12f1 protein.

### Characteristic 4 of Cas12f1 fusion protein - Inclusion of linker

The Cas12f1 fusion protein provided herein is characterized by comprising a linker that links the modified Cas12f1 protein with the expression regulatory domain. Here, the linker is characterized by being an amino acid sequence that does not affect functions and structures of the modified Cas12f1 protein and the expression regulatory domain.

### Effect of Cas12f1 fusion protein

In a case where the Cas12f1 fusion protein provided herein is used in a CRISPR/Cas12f1 system, unlike a case where the wild-type Cas12f1 protein is used, an effect of increasing or inhibiting expression of a target gene occurs without cleavage of double strands of the target gene. Conventional CRISPR/Cas12f1 systems are used in the field of gene editing technology (knock-out of a target gene, knock-in of a target gene, and the like). In contrast, the CRISPR/Cas12f1 system using the Cas12f1 fusion protein, that is, the CRISPR regulatory system can regulate expression of a target gene without separate gene editing (nucleic acid modification caused by double-strand cleavage, and the like), and thus can be used in various techniques for regulating gene expression.

### Use of Cas12f1 fusion protein

The Cas12f1 fusion protein provided herein may be used for regulating gene expression together with an engineered Cas12f1 guide RNA. In addition, the engineered Cas12f1 guide RNA may be used for preparing a gene expression regulatory composition.

Hereinafter, the configuration and various embodiments of the Cas12f1 fusion protein will be described.

### 1) Modified Cas12f1 protein

### Modified Cas12f1 protein - Overview

The Cas12f1 fusion protein for the CRISPR regulatory system provided herein comprises a modified Cas12f1 protein. Basically, the modified Cas12f1 protein may be obtained by modifying at least one amino acid in the amino acid sequence of the wild-type Cas12f1 protein that exists in nature. The sequence encoding the modified Cas12f1 protein may be a human codon-optimized Cas12f1 sequence for the modified Cas12f1 protein. In addition, the modified Cas12f1 protein has an altered function as compared with the wild-type Cas12f1 protein that exists in nature. Specifically, the modified Cas12f1 protein does not have a function to cleave double strands of a target nucleic acid or target gene unlike the wild-type Cas12f1 protein. Hereinafter, the modified Cas12f1 protein is referred to as a "dead Cas12f1 protein (dCas12f1 protein)," and these terms are used interchangeably. Unless otherwise specified, the "modified Cas12f1 protein" as used herein refers to a dCas12f1 protein incapable of cleaving double strands of a target nucleic acid or target gene.

### Modified Cas12f1 protein - Wild-type Cas12f1 protein

The Cas12f1 fusion protein provided herein comprises a modified Cas12f1 protein. Here, the modified Cas12f1 protein may be obtained by modifying at least one amino acid in the amino acid sequence of the wild-type Cas12f1 protein.

In an embodiment, the wild-type Cas12f1 protein may be derived from the Cas14 family (Harrington et al., Programmed DNA destruction by miniature CRISPR-Cas14 enzymes, Science 362, 839-842 (2018)). In an embodiment, the wild-type Cas12f1 protein may be a Cas14a protein derived from an uncultured archaeon (Harrington et al., Programmed DNA destruction by miniature CRISPR-Cas14 enzymes, Science 362, 839-842 (2018)). In an embodiment, the wild-type Cas12f1 protein may be a wild-type Cas14a1 protein. In an embodiment, the wild-type Cas12f1 protein may have the amino acid sequence set forth in SEQ ID NO: 260.

### dCas12f1 protein - Altering function of wild-type Cas12f1 protein

The dCas12f1 protein included in the Cas12f1 fusion protein provided herein may be obtained by altering or removing a function of a wild-type Cas12f1 protein.

In an embodiment, the dCas12f1 protein may be a modified form of the wild-type Cas12f1 protein which cannot cleave all double strands of a target nucleic acid or target gene.

### dCas12f1 protein - Modified amino acid sequence

The dCas12f1 protein included in the Cas12f1 fusion protein provided herein may be a modified form of the wild-type Cas12f1 protein in which at least one amino acid in the amino acid sequence constituting the wild-type Cas12f1 protein is substituted with another amino acid. Thus, the dCas12f1 protein may have at least one different amino acid as compared with the wild-type Cas12f1 protein. The dCas12f1 protein may be a modified form of the wild-type Cas12f1 protein in which at least one of arginine (R) which is the 490^{th} amino acid, aspartic acid (D) which is the 510^{th} amino acid, glutamic acid (E) which is the 422^{nd} amino acid, and aspartic acid (D) which is the 326^{th} amino acid in the amino acid sequence constituting the wild-type Cas12f1 protein is substituted with another amino acid.

### Embodiments of dCas12f1 protein

In an embodiment, the dCas12f1 protein may be a modified form of the wild-type Cas12f1 protein in which arginine (R) which is the 490^{th} amino acid in the amino acid sequence constituting the wild-type Cas12f1 protein is substituted with another amino acid. Here, the other amino acid may be, but is not limited to, alanine (A), glutamine (Q), leucine (L), or tryptophan (W).

In an embodiment, the dCas12f1 protein may be a modified form of the wild-type Cas12f1 protein in which aspartic acid (D) which is the 510^{th} amino acid in the amino acid sequence constituting the wild-type Cas12f1 protein is substituted with another amino acid. Here, the other amino acid may be, but is not limited to, alanine (A), leucine (L), or valine (V).

In an embodiment, the dCas12f1 protein may be a modified form of the wild-type Cas12f1 protein in which glutamic acid (E) which is the 422^{nd} amino acid in the amino acid sequence constituting the wild-type Cas12f1 protein is substituted with another amino acid. Here, the other amino acid may be, but is not limited to, alanine (A).

In an embodiment, the dCas12f1 protein may be a modified form of the wild-type Cas12f1 protein in which aspartic acid (D) which is the 326^{th} amino acid in the amino acid sequence constituting the wild-type Cas12f1 protein is substituted with another amino acid. Here, the other amino acid may be, but is not limited to, alanine (A).

### Example 1 of dCas12f1 protein

In an embodiment, the dCas12f1 protein may be a modified form of the wild-type Cas12f1 protein in which arginine (R) which is the 490^{th} amino acid in the amino acid sequence constituting the wild-type Cas12f1 protein is substituted with alanine (A). Here, the dCas12f1 protein may have alanine at position 490, as compared with the amino acid sequence of the wild-type Cas12f1 protein. The dCas12f1 protein may have the amino acid sequence set forth in SEQ ID NO: 261. The dCas12f1 protein may be expressed as "R490A dCas12f1 protein" or "dCas12f1 R490A protein."

In an embodiment, the dCas12f1 protein may be a modified form of the wild-type Cas12f1 protein in which arginine (R) which is the 490^{th} amino acid in the amino acid sequence constituting the wild-type Cas12f1 protein is substituted with glutamine (Q). Here, the dCas12f1 protein may have glutamine at position 490, as compared with the amino acid sequence of the wild-type Cas12f1 protein. The dCas12f1 protein may have the amino acid sequence set forth in SEQ ID NO: 262. The dCas12f1 protein may be expressed as "R490Q dCas12f1 protein" or "dCas12f1 R490Q protein."

In an embodiment, the dCas12f1 protein may be a modified form of the wild-type Cas12f1 protein in which arginine (R) which is the 490^{th} amino acid in the amino acid sequence constituting the wild-type Cas12f1 protein is substituted with leucine (L). Here, the dCas12f1 protein may have leucine at position 490, as compared with the amino acid sequence of the wild-type Cas12f1 protein. The dCas12f1 protein may have the amino acid sequence set forth in SEQ ID NO: 264. The dCas12f1 protein may be expressed as "R490L dCas12f1 protein" or "dCas12f1 R490L protein."

In an embodiment, the dCas12f1 protein may be a modified form of the wild-type Cas12f1 protein in which arginine (R) which is the 490^{th} amino acid in the amino acid sequence constituting the wild-type Cas12f1 protein is substituted with tryptophan(W). Here, the dCas12f1 protein may have tryptophan at position 490, as compared with the amino acid sequence of the wild-type Cas12f1 protein. The dCas12f1 protein may have the amino acid sequence set forth in SEQ ID NO: 265. The dCas12f1 protein may be expressed as "R490W dCas12f1 protein" or "dCas12f1 R490W protein."

### Example 2 of dCas12f1 protein

In an embodiment, the dCas12f1 protein may be a modified form of the wild-type Cas12f1 protein in which aspartic acid (D) which is the 510^{th} amino acid in the amino acid sequence constituting the wild-type Cas12f1 protein is substituted with alanine (A). Here, the dCas12f1 protein may have alanine at position 510, as compared with the amino acid sequence of the wild-type Cas12f1 protein. The dCas12f1 protein may have the amino acid sequence set forth in SEQ ID NO: 266. The dCas12f1 protein may be expressed as "D510A dCas12f1 protein" or "dCas12f1 D510A protein."

In an embodiment, the dCas12f1 protein may be a modified form of the wild-type Cas12f1 protein in which aspartic acid (D) which is the 510^{th} amino acid in the amino acid sequence constituting the wild-type Cas12f1 protein is substituted with leucine (L). Here, the dCas12f1 protein may have leucine at position 510, as compared with the amino acid sequence of the wild-type Cas12f1 protein. The dCas12f1 protein may have the amino acid sequence set forth in SEQ ID NO: 267. The dCas12f1 protein may be expressed as "D510L dCas12f1 protein" or "dCas12f1 D510L protein."

In an embodiment, the dCas12f1 protein may be a modified form of the wild-type Cas12f1 protein in which aspartic acid (D) which is the 510^{th} amino acid in the amino acid sequence constituting the wild-type Cas12f1 protein is substituted with valine (V). Here, the dCas12f1 protein may have valine at position 510, as compared with the amino acid sequence of the wild-type Cas12f1 protein. The dCas12f1 protein may have the amino acid sequence set forth in SEQ ID NO: 268. The dCas12f1 protein may be expressed as "D510V dCas12f1 protein" or "dCas12f1 D510V protein."

### Example 3 of dCas12f1 protein

In an embodiment, the dCas12f1 protein may be a modified form of the wild-type Cas12f1 protein in which glutamic acid (E) which is the 422^{nd} amino acid in the amino acid sequence constituting the wild-type Cas12f1 protein is substituted with alanine (A). Here, the dCas12f1 protein may have alanine at position 422, as compared with the amino acid sequence of the wild-type Cas12f1 protein. The dCas12f1 protein may have the amino acid sequence set forth in SEQ ID NO: 269. The dCas12f1 protein may be expressed as "E422A dCas12f1 protein" or "dCas12f1 E422A protein."

### Example 4 of dCas12f1 protein

In an embodiment, the dCas12f1 protein may be a modified form of the wild-type Cas12f1 protein in which aspartic acid (D) which is the 326^{th} amino acid in the amino acid sequence constituting the wild-type Cas12f1 protein is substituted with alanine (A). Here, the dCas12f1 protein may have alanine at position 326, as compared with the amino acid sequence of the wild-type Cas12f1 protein. The dCas12f1 protein may have the amino acid sequence set forth in SEQ ID NO: 271. The dCas12f1 protein may be expressed as "D326A dCas12f1 protein" or "dCas12f1 D326A protein."

### 2) Expression regulatory domain

### Expression regulatory domain - Overview

The Cas12f1 fusion protein for the CRISPR regulatory system provided herein comprises an expression regulatory domain. The expression regulatory domain may be a protein that activates or inhibits transcription of a target gene, that is, a transcriptional activator protein or a transcriptional inhibitor protein. In a case where the expression regulatory domain is a transcriptional activator protein, the Cas12f1 fusion protein comprising the transcriptional activator protein may be used in a CRISPR regulatory system for increasing or promoting expression of a target gene. Alternatively, in a case where the expression regulatory domain is a transcriptional inhibitor protein, the Cas12f1 fusion protein comprising the transcriptional inhibitor protein may be used in a CRISPR regulatory system for inhibiting or suppressing expression of a target gene.

### Type 1 of expression regulatory domain - Transcriptional activator protein

In an embodiment, the expression regulatory domain may be a transcriptional activator protein. The transcriptional activator protein may be a protein that serves to activate or promote transcription of a target gene. The transcriptional activator protein may be a DNA-binding protein capable of binding to an enhancer or a promoter-proximal element of a target gene. The transcriptional activator protein may bind to a regulatory DNA site located near a promoter of a target gene and facilitate, through protein-protein interaction, binding of general transcription machinery (RNA polymerase, common transcription factors, and the like) to the promoter, thereby promoting transcription of the gene. Alternatively, the transcriptional activator protein may cause RNA polymerase to move from the promoter and proceed with synthesis along DNA, thereby promoting transcription of the gene.

In an embodiment, the transcriptional activator protein may be VP64.

### Type 2 of expression regulatory domain - Transcriptional inhibitor protein

In an embodiment, the expression regulatory domain may be a transcriptional inhibitor protein. The transcriptional inhibitor protein may be a protein that serves to inhibit or suppress transcription of a target gene. The transcriptional inhibitor protein may be a DNA-binding protein or peptide that binds to an operator or silencer of a target gene to inhibit or suppress expression of the target gene. Here, the transcriptional inhibitor protein may block RNA polymerase from being attached to a promoter of a gene, thereby inhibiting or suppressing transcription of the gene. Alternatively, the transcriptional inhibitor protein may be a protein or peptide that inhibits or suppresses transcription of a gene by inducing a structural change in chromatin of the gene. Here, the structural change in chromatin may be caused by methylation, demethylation, acetylation, deacetylation, or the like.

In an embodiment, the transcriptional inhibitor protein may be KRAB, DNMT, MeCP2, LSD, or HDAC. Here, the DNMT may be DNMT1, TRDMT1, or DNMT3. Here, the HDAC may be H DAC 1, HDAC2, HDAC3, HDAC4, HDAC5, H DAC6, HDAC7, HDAC8, HDAC9, HDAC10, or HDAC11.

### Expression regulatory domain - Number on Cas12f1 fusion protein

A Cas12f1 fusion protein may comprise at least one expression regulatory domain. Here, the expression regulatory domain may be a transcriptional activator protein or a transcriptional inhibitor protein. Alternatively, a Cas12f1 fusion protein may comprise a plurality of expression regulatory domains. Here, all of the plurality of expression regulatory domains are domains having the same function, and may be proteins that function to promote transcriptional activity or inhibit transcriptional activity. That is, all of the plurality of expression regulatory domains may be transcriptional activator proteins or transcriptional inhibitor proteins. However, the plurality of expression regulatory domains only have the same function and do not necessarily have to be the same protein. For example, in a case where all of the plurality of expression regulatory domains are transcriptional inhibitor proteins, there may be a plurality of the same type of transcriptional inhibitor proteins or a plurality of several types of transcriptional inhibitor proteins.

### Expression regulatory protein - Location on Cas12f1 fusion protein

The expression regulatory domain may be located at the N-terminus and/or C-terminus of the modified Cas12f1 protein, that is, the dCas12f1 protein, included in the Cas12f1 fusion protein. In a case where two or more expression regulatory domains are included in the Cas12f1 fusion protein, all of the expression regulatory domains may be located at the N-terminus or the C-terminus of the dCas12f1 protein, or some of the expression regulatory domains may be located at the N-terminus of the dCas12f1 protein and the other expression regulatory domain(s) may be located at the C-terminus of the dCas12f1 protein.

In an embodiment, in a case where two expression regulatory domains are included in the Cas12f1 fusion protein, all of the two expression regulatory domains may be located at the N-terminus of the dCas12f1 protein. Alternatively, all of the two expression regulatory domains may be located at the C-terminus of the dCas12f1 protein. Alternatively, one of the two expression regulatory domains may be located at the C-terminus of the dCas12f1 protein, or the other expression regulatory domain may be located at the N-terminus of the dCas12f1 protein.

In an embodiment, in a case where three expression regulatory domains are included in the Cas12f1 fusion protein, all of the three expression regulatory domains may be located at the N-terminus of the dCas12f1 protein. Alternatively, all of the three expression regulatory domains may be located at the C-terminus of the dCas12f1 protein. Alternatively, two of the three expression regulatory domains may be located at the C-terminus of the dCas12f1 protein, or the other expression regulatory domain may be located at the N-terminus of the dCas12f1 protein. Alternatively, one of the three expression regulatory domains may be located at the C-terminus of the dCas12f1 protein, or the other two expression regulatory domains may be located at the N-terminus of the dCas12f1 protein.

### Exemplary expression regulatory domain

In an embodiment, the expression regulatory domain may be a transcriptional activator protein. Here, the transcriptional activator protein may be VP64. The expression regulatory domain may have the amino acid sequence set forth in SEQ ID NO: 272.

In an embodiment, the expression regulatory domain may be a transcriptional inhibitor protein. Here, the transcriptional inhibitor protein may be KRAB. The expression regulatory domain may have the amino acid sequence set forth in SEQ ID NO: 274.

In an embodiment, the expression regulatory domain may be a transcriptional inhibitor protein. Here, the transcriptional inhibitor protein may be MeCP2. The expression regulatory domain may have the amino acid sequence set forth in SEQ ID NO: 275.

In an embodiment, the expression regulatory domain may be a transcriptional inhibitor protein. Here, the transcriptional inhibitor protein may be DNMT3. The expression regulatory domain may have the amino acid sequence set forth in SEQ ID NO: 276.

In an embodiment, the expression regulatory domain may be a transcriptional inhibitor protein. Here, the transcriptional inhibitor protein may be HDAC3. The expression regulatory domain may have the amino acid sequence set forth in SEQ ID NO: 277.

### 3) Linker

The Cas12f1 fusion protein for the CRISPR regulatory system provided herein comprises a linker to link the dCas12f1 protein and the expression regulatory domain. Here, the linker is characterized by being an amino acid sequence that does not affect functions and structures of the dCas12f1 protein and the expression regulatory domain.

### 4) Additional domain

The Cas12f1 fusion protein for the CRISPR regulatory system provided herein may further comprise at least one additional domain. The additional domain may be located at the N-terminus and/or the C-terminus of the Cas12f1 fusion protein. Alternatively, the additional domain may be located between the dCas12f1 protein and the expression regulatory domain included in the Cas12f1 fusion protein.

In an embodiment, the additional domain may be a nuclear localization sequence (NLS) or a nuclear export sequence (NES). Specifically, the NLS may be, but is not limited to, any one of the examples described in the paragraph for NLS in the section of defining terms.

In an embodiment, the additional domain may be a tag. Specifically, the tag may be, but is not limited to, any one of the examples described in the paragraph for tag in the section of defining terms.

### 5) Cas12f1 fusion protein

### Cas12f1 fusion protein - Overview

The Cas12f1 fusion protein for the CRISPR regulatory system provided herein is divided into two types depending on the function. First, the Cas12f1 fusion protein may be a transcriptional activator Cas12f1 fusion protein that functions to increase or promote expression of a target gene. Specifically, the transcriptional activator Cas12f1 fusion protein comprises: a dCas12f1 protein; and a transcriptional activator protein as the expression regulatory domain. The transcriptional activator Cas12f1 fusion protein is used in a CRISPR regulatory system for increasing or enhancing expression of a target gene, that is, a CRISPR activation system. Second, the Cas12f1 fusion protein may be a transcriptional inhibitor Cas12f1 fusion protein that functions to inhibit or suppress expression of a target gene. Specifically, the transcriptional inhibitor Cas12f1 fusion protein comprises: a dCas12f1 protein; and a transcriptional inhibitor protein as the expression regulatory domain. The transcriptional inhibitor Cas12f1 fusion protein is used in a CRISPR regulatory system for inhibiting or suppressing expression of a target gene, that is, a CRISPR interference system.

### Cas12f1 fusion protein - Modularization

The Cas12f1 fusion protein for the CRISPR regulatory system provided herein may have an improved or optimized function through modularization done in varisous ways. Here, the modularization is characterized by variously adjusting the number and type of expression regulatory domains included in the Cas12f1 fusion protein and the location thereof in the Cas12f1 fusion protein. This modularization allows an optimized transcriptional activator Cas12f1 fusion protein or transcriptional inhibitor Cas12f1 fusion protein to be developed and more effectively used in a CRISPR activation system or a CRISPR interference system. In addition, for the Cas12f1 fusion protein, in addition to the expression regulatory domain, it is possible to variously adjust the number and type of additional domains and the location thereof in the Cas12f1 fusion protein.

In an embodiment, the Cas12f1 fusion protein may comprise a dCas12f1 protein and two transcriptional inhibitor proteins. Here, the two transcriptional inhibitor proteins may be the same type of transcriptional inhibitor proteins. Alternatively, the two transcriptional inhibitor proteins may be different types of transcriptional inhibitor proteins. Here, all of the two transcriptional inhibitor proteins may be located at the N-terminus of the dCas12f1 protein. Alternatively, all of the two transcriptional inhibitor proteins may be located at the C-terminus of the dCas12f1 protein. Alternatively, the two transcriptional inhibitor proteins may be located one each at the N-terminus and the C-terminus of the dCas12f1 protein. The Cas12f1 fusion protein may further comprise at least one additional domain. Here, the additional domain may be located at the N terminus and/or the C-terminus of the Cas12f1 fusion protein. Alternatively, the additional domain may be located between the dCas12f1 protein and the transcriptional inhibitor protein. Locations of the dCas12f1 protein, the transcriptional inhibitor protein and the additional domain included in the Cas12f1 fusion protein may be adjusted variously.

### Cas12f1 fusion protein - Embodiment of transcriptional activator Cas12f1 fusion protein

The Cas12f1 fusion protein provided herein may be a transcriptional activator Cas12f1 fusion protein.

In an embodiment, the transcriptional activator Cas12f1 fusion protein may comprise a dCas12f1 protein and at least one transcriptional activator protein. Here, the transcriptional activator protein may be located at the N-terminus or the C-terminus of the dCas12f1 protein. The transcriptional activator Cas12f1 fusion protein may further comprise at least one NLS as the additional domain. Here, the NLS may be located at the N-terminus and/or the C-terminus of the transcriptional activator Cas12f1 fusion protein. Alternatively, the NLS may be located between the dCas12f1 protein and the transcriptional activator protein. Here, the dCas12f1 protein, the transcriptional activator protein and the NLS included in the transcriptional activator Cas12f1 fusion protein may be linked via linkers.

In an embodiment, the transcriptional activator Cas12f1 fusion protein may comprise a dCas12f1 protein and at least two transcriptional activator proteins. Here, the at least two transcriptional activator proteins may be located at the N-terminus and/or the C-terminus of the dCas12f1 protein. The transcriptional activator Cas12f1 fusion protein may further comprise at least one NLS as the additional domain. Here, the NLS may be located at the N-terminus and/or the C-terminus of the transcriptional activator Cas12f1 fusion protein. Alternatively, the NLS may be located between the dCas12f1 protein and the transcriptional activator protein. Here, the dCas12f1 protein, the transcriptional activator protein and the NLS included in the transcriptional activator Cas12f1 fusion protein may be linked via linkers.

Hereinafter, various examples of the transcriptional activator Cas12f1 fusion protein will be described. The following examples are provided for illustrative purposes only and are not intended to limit the disclosure.

### Cas12f1 fusion protein - Example 1 of transcriptional activator Cas12f1 fusion protein

In an embodiment, the transcriptional activator Cas12f1 fusion protein may be such that from the N-terminus to the C-terminus; the dCas12f1 protein and the transcriptional activator protein are sequentially linked to each other; or the transcriptional activator protein and the dCas12f1 protein are sequentially linked to each other. Here, the dCas12f1 protein and the transcriptional activator protein may be linked via a linker.

The dCas12f1 protein may be a modified form of the wild-type Cas12f1 protein in which arginine (R) which is the 490^{th} amino acid in the amino acid sequence constituting the wild-type Cas12f1 protein is substituted with alanine (A), glutamine (Q), leucine (L), or tryptophan (W). Here, the dCas12f1 protein may be a dCas12f1 R490A protein having alanine at position 490, as compared with the amino acid sequence of the wild-type Cas12f1 protein. Alternatively, the dCas12f1 protein may be a dCas12f1 R490Q protein having glutamine at position 490, as compared with the amino acid sequence of the wild-type Cas12f1 protein. Alternatively, the dCas12f1 protein may be a dCas12f1 R490L protein having leucine at position 490, as compared with the amino acid sequence of the wild-type Cas12f1 protein. Alternatively, the dCas12f1 protein may be a dCas12f1 R490W protein having tryptophan at position 490, as compared with the amino acid sequence of the wild-type Cas12f1 protein. Here, the dCas12f1 protein may have at least one of the amino acid sequences set forth in SEQ ID NOs: 261, 262, 264, and 265.

Alternatively, the dCas12f1 protein may be a modified form of the wild-type Cas12f1 protein in which aspartic acid (D) which is the 510^{th} amino acid in the amino acid sequence constituting the wild-type Cas12f1 protein is substituted with alanine (A), leucine (L), or valine (V). Here, the dCas12f1 protein may be a dCas12f1 D510A protein having alanine at position 510, as compared with the amino acid sequence of the wild-type Cas12f1 protein. Alternatively, the dCas12f1 protein may be a dCas12f1 D510L protein having leucine at position 510, as compared with the amino acid sequence of the wild-type Cas12f1 protein. Alternatively, the dCas12f1 protein may be a dCas12f1 D510V protein having valine at position 510, as compared with the amino acid sequence of the wild-type Cas12f1 protein. Here, the dCas12f1 protein may have at least one of the amino acid sequences set forth in SEQ ID NOs: 266 to 268.

The transcriptional activator protein may be VP64. Here, the transcriptional activator protein may have the amino acid sequence set forth in SEQ ID NO: 272.

The transcriptional activator Cas12f1 fusion protein may further comprise at least one NLS as the additional domain. Here, the at least one NLS may be located at the N-terminus and/or the C-terminus of the transcriptional activator Cas12f1 fusion protein. Alternatively, the at least one NLS may be located between the dCas12f1 protein and the transcriptional activator protein. Here, the at least one NLS may be linked to the dCas12f1 protein and/or the transcriptional activator protein via a linker.

As an example, the transcriptional activator Cas12f1 fusion protein may have the following structure from the N-terminus to the C-terminus: [dCas12f1 R490A protein]-[VP64]; [dCas12f1 R490Q protein]-[VP64]; [dCas12f1 R490L protein]-[VP64]; [dCas12f1 R490W protein]-[VP64]; [VP64]-[dCas12f1 R490A protein]; [VP64]-[dCas12f1 R490Q protein]; [VP64]-[dCas12f1 R490L protein]; or [VP64]-[dCas12f1 R490W protein]. The transcriptional activator Cas12f1 fusion protein may further comprise at least one NLS. Here, the at least one NLS may be located at the N-terminus of the transcriptional activator Cas12f1 fusion protein, at the C-terminus of the transcriptional activator Cas12f1 fusion protein, and/or between the dCas12f1 protein (the dCas12f1 R490A protein, the dCas12f1 R490Q protein, the dCas12f1 R490L protein, or the dCas12f1 R490W protein) and VP64.

As an example, the transcriptional activator Cas12f1 fusion protein may have the following structure from the N-terminus to the C-terminus: [dCas12f1 D510A protein]-[VP64]; [dCas12f1 D510L protein]-[VP64]; [dCas12f1 D510V protein]-[VP64]; [VP64]-[dCas12f1 D510A protein]; [VP64]-[dCas12f1 D510L protein]; or [VP64]-[dCas12f1 D510V protein]. The transcriptional activator Cas12f1 fusion protein may further comprise at least one NLS. Here, the at least one NLS may be located at the N-terminus of the transcriptional activator Cas12f1 fusion protein, at the C-terminus of the transcriptional activator Cas12f1 fusion protein, and/or between the dCas12f1 protein (the dCas12f1 D510A protein, the dCas12f1 D510L protein, or the dCas12f1 D510V protein) and VP64.

### Cas12f1 fusion protein - Example 2 of transcriptional activator Cas12f1 fusion protein

In an embodiment, the transcriptional activator Cas12f1 fusion protein may be such that from the N-terminus to the C-terminus; the dCas12f1 protein, the transcriptional activator protein, and the transcriptional activator protein are sequentially linked to each other; or the transcriptional activator protein, the transcriptional activator protein, and the dCas12f1 protein are sequentially linked to each other. Here, the dCas12f1 protein and the transcriptional activator proteins may be linked via linkers.

The dCas12f1 protein may be a modified form of the wild-type Cas12f1 protein in which arginine (R) which is the 490^{th} amino acid in the amino acid sequence constituting the wild-type Cas12f1 protein is substituted with alanine (A), glutamine (Q), leucine (L), or tryptophan (W). Here, the dCas12f1 protein may be a dCas12f1 R490A protein having alanine at position 490, as compared with the amino acid sequence of the wild-type Cas12f1 protein. Alternatively, the dCas12f1 protein may be a dCas12f1 R490Q protein having glutamine at position 490, as compared with the amino acid sequence of the wild-type Cas12f1 protein. Alternatively, the dCas12f1 protein may be a dCas12f1 R490L protein having leucine at position 490, as compared with the amino acid sequence of the wild-type Cas12f1 protein. Alternatively, the dCas12f1 protein may be a dCas12f1 R490W protein having tryptophan at position 490, as compared with the amino acid sequence of the wild-type Cas12f1 protein. Here, the dCas12f1 protein may have at least one of the amino acid sequences set forth in SEQ ID NOs: 261, 262, 264, and 265.

Alternatively, the dCas12f1 protein may be a modified form of the wild-type Cas12f1 protein in which aspartic acid (D) which is the 510^{th} amino acid in the amino acid sequence constituting the wild-type Cas12f1 protein is substituted with alanine (A), leucine (L), or valine (V). Here, the dCas12f1 protein may be a dCas12f1 D510A protein having alanine at position 510, as compared with the amino acid sequence of the wild-type Cas12f1 protein. Alternatively, the dCas12f1 protein may be a dCas12f1 D510L protein having leucine at position 510, as compared with the amino acid sequence of the wild-type Cas12f1 protein. Alternatively, the dCas12f1 protein may be a dCas12f1 D510V protein having valine at position 510, as compared with the amino acid sequence of the wild-type Cas12f1 protein. Here, the dCas12f1 protein may have at least one of the amino acid sequences set forth in SEQ ID NOs: 266 to 268.

The transcriptional activator protein may be VP64. Here, the transcriptional activator protein may have the amino acid sequence set forth in SEQ ID NO: 272.

The transcriptional activator Cas12f1 fusion protein may further comprise at least one NLS as the additional domain. Here, the at least one NLS may be located at the N-terminus and/or the C-terminus of the transcriptional activator Cas12f1 fusion protein. Alternatively, the at least one NLS may be located between the dCas12f1 protein and the transcriptional activator protein. Alternatively, the at least one NLS may be located between the transcriptional activator proteins. Here, the at least one NLS may be linked to the dCas12f1 protein and/or the transcriptional activator proteins via linkers.

As an example, the transcriptional activator Cas12f1 fusion protein may have the following structure from the N-terminus to the C-terminus: [dCas12f1 R490A protein]-[VP64]-[VP64]; [dCas12f1 R490Q protein]-[VP64]-[VP64]; [dCas12f1 R490L protein]-[VP64]-[VP64]; [dCas12f1 R490W protein]-[VP64]-[VP64]; [VP64]-[VP64]-[dCas12f1 R490A protein]; [VP64]-[VP64]-[dCas12f1 R490Q protein]; [VP64]-[VP64]-[dCas12f1 R490L protein]; or [VP64]-[VP64]-[dCas12f1 R490W protein]. The transcriptional activator Cas12f1 fusion protein may further comprise at least one NLS. Here, the at least one NLS may be located at the N-terminus of the transcriptional activator Cas12f1 fusion protein, at the C-terminus of the transcriptional activator Cas12f1 fusion protein, between the dCas12f1 protein (the dCas12f1 R490A protein, the dCas12f1 R490Q protein, the dCas12f1 R490L protein, or the dCas12f1 R490W protein) and VP64, and/or between VP64 and VP64.

As an example, the transcriptional activator Cas12f1 fusion protein may have the following structure from the N-terminus to the C-terminus: [dCas12f1 D510A protein]-[VP64]-[VP64]; [dCas12f1 D510L protein]-[VP64]-[VP64]; [dCas12f1 D510V protein]-[VP64]-[VP64]; [VP64]-[VP64]-[dCas12f1 D510A protein]; [VP64]-[VP64]-[dCas12f1 D510L protein]; or [VP64]-[VP64]-[dCas12f1 D510V protein]. The transcriptional activator Cas12f1 fusion protein may further comprise at least one NLS. Here, the at least one NLS may be located at the N-terminus of the transcriptional activator Cas12f1 fusion protein, at the C-terminus of the transcriptional activator Cas12f1 fusion protein, between the dCas12f1 protein (the dCas12f1 D510A protein, the dCas12f1 D510L protein, or the dCas12f1 D510V protein) and VP64, and/or between VP64 and VP64.

### Cas12f1 fusion protein - Embodiments of transcriptional inhibitor Cas12f1 fusion protein

The Cas12f1 fusion protein provided herein may be a transcriptional inhibitor Cas12f1 fusion protein.

In an embodiment, the transcriptional inhibitor Cas12f1 fusion protein may comprise a dCas12f1 protein and at least one transcriptional inhibitor protein. Here, the transcriptional inhibitor protein may be located at the N-terminus or the C-terminus of the dCas12f1 protein. The transcriptional inhibitor Cas12f1 fusion protein may further comprise at least one NLS as the additional domain. Here, the NLS may be located at the N-terminus and/or the C-terminus of the transcriptional inhibitor Cas12f1 fusion protein. Alternatively, the NLS may be located between the dCas12f1 protein and the transcriptional inhibitor protein. Here, the dCas12f1 protein, the transcriptional inhibitor protein and the NLS included in the transcriptional inhibitor Cas12f1 fusion protein may be linked via linkers.

In an embodiment, the transcriptional inhibitor Cas12f1 fusion protein may comprise a dCas12f1 protein and at least two transcriptional inhibitor proteins. Here, the at least two transcriptional inhibitor proteins may be located at the N-terminus and/or the C-terminus of the dCas12f1 protein. The transcriptional inhibitor Cas12f1 fusion protein may further comprise at least one NLS as the additional domain. Here, the NLS may be located at the N-terminus and/or the C-terminus of the transcriptional inhibitor Cas12f1 fusion protein. Alternatively, the NLS may be located between the dCas12f1 protein and the transcriptional inhibitor protein. Here, the dCas12f1 protein, the transcriptional inhibitor protein and the NLS included in the transcriptional inhibitor Cas12f1 fusion protein may be linked via linkers.

Hereinafter, various examples of the transcriptional inhibitor Cas12f1 fusion protein will be described. The following examples are provided for illustrative purposes only, and are not intended to limit the disclosure.

### Cas12f1 fusion protein - Example 1 of transcriptional inhibitor Cas12f1 fusion protein

In an embodiment, the transcriptional inhibitor Cas12f1 fusion protein may be such that from the N-terminus to the C-terminus, the dCas12f1 protein and the transcriptional inhibitor protein are sequentially linked to each other; or the transcriptional inhibitor protein and the dCas12f1 protein are sequentially linked to each other. Here, the dCas12f1 protein and the transcriptional inhibitor protein may be linked via a linker.

The dCas12f1 protein may be a modified form of the wild-type Cas12f1 protein in which arginine (R) which is the 490^{th} amino acid in the amino acid sequence constituting the wild-type Cas12f1 protein is substituted with alanine (A), glutamine (Q), leucine (L), or tryptophan (W). Here, the dCas12f1 protein may be a dCas12f1 R490A protein having alanine at position 490, as compared with the amino acid sequence of the wild-type Cas12f1 protein. Alternatively, the dCas12f1 protein may be a dCas12f1 R490Q protein having glutamine at position 490, as compared with the amino acid sequence of the wild-type Cas12f1 protein. Alternatively, the dCas12f1 protein may be a dCas12f1 R490L protein having leucine at position 490, as compared with the amino acid sequence of the wild-type Cas12f1 protein. Alternatively, the dCas12f1 protein may be a dCas12f1 R490W protein having tryptophan at position 490, as compared with the amino acid sequence of the wild-type Cas12f1 protein. Here, the dCas12f1 protein may have at least one of the amino acid sequences set forth in SEQ ID NOs: 261, 262, 264, and 265.

Alternatively, the dCas12f1 protein may be a modified form of the wild-type Cas12f1 protein in which aspartic acid (D) which is the 510^{th} amino acid in the amino acid sequence constituting the wild-type Cas12f1 protein is substituted with alanine (A), leucine (L), or valine (V). Here, the dCas12f1 protein may be a dCas12f1 D510A protein having alanine at position 510, as compared with the amino acid sequence of the wild-type Cas12f1 protein. Alternatively, the dCas12f1 protein may be a dCas12f1 D510L protein having leucine at position 510, as compared with the amino acid sequence of the wild-type Cas12f1 protein. Alternatively, the dCas12f1 protein may be a dCas12f1 D510V protein having valine at position 510, as compared with the amino acid sequence of the wild-type Cas12f1 protein. Here, the dCas12f1 protein may have at least one of the amino acid sequences set forth in SEQ ID NOs: 266 to 268.

The transcriptional inhibitor protein may be KRAB, MeCP2, DNMT3, or HDAC3. Here, the transcriptional inhibitor protein may have at least one of the amino acid sequences set forth in SEQ ID NOs: 274 to 277.

The transcriptional inhibitor Cas12f1 fusion protein may further comprise at least one NLS as the additional domain. Here, the at least one NLS may be located at the N-terminus and/or the C-terminus of the transcriptional inhibitor Cas12f1 fusion protein. Alternatively, the at least one NLS may be located between the dCas12f1 protein and the transcriptional inhibitor protein. Here, the at least one NLS may be linked to the dCas12f1 protein and/or the transcriptional inhibitor protein via a linker.

As an example, the transcriptional inhibitor Cas12f1 fusion protein may have the following structure from the N-terminus to the C-terminus: [dCas12f1 R490A protein]-[KRAB]; [dCas12f1 R490Q protein]-[KRAB]; [dCas12f1 R490L protein]-[KRAB]; [dCas12f1 R490W protein]-[KRAB]; [KRAB]-[dCas12f1 R490A protein]; KRAB]-[dCas12f1 R490Q protein]; [KRAB]-[dCas12f1 R490L protein]; or [KRAB]-[dCas12f1 R490W protein]. The transcriptional inhibitor Cas12f1 fusion protein may further comprise at least one NLS. Here, the at least one NLS may be located at the N-terminus of the transcriptional inhibitor Cas12f1 fusion protein, at the C-terminus of the transcriptional inhibitor Cas12f1 fusion protein, and/or between the dCas12f1 protein (the dCas12f1 R490A protein, the dCas12f1 R490Q protein, the dCas12f1 R490L protein, or the dCas12f1 R490W protein) and KRAB.

As an example, the transcriptional inhibitor Cas12f1 fusion protein may have the following structure from the N-terminus to the C-terminus: [dCas12f1 D510A protein]-[KRAB]; [dCas12f1 D510L protein]-[KRAB]; [dCas12f1 D510V protein]-[KRAB]; [KRAB]-[dCas12f1 D510A protein]; [KRAB]-[dCas12f1 D510L protein]; or [KRAB]-[dCas12f1 D510V protein]. The transcriptional inhibitor Cas12f1 fusion protein may further comprise at least one NLS. Here, the at least one NLS may be located at the N-terminus of the transcriptional inhibitor Cas12f1 fusion protein, at the C-terminus of the transcriptional inhibitor Cas12f1 fusion protein, and/or between the dCas12f1 protein (the dCas12f1 D510A protein, the dCas12f1 D510L protein, or the dCas12f1 D510V protein) and KRAB.

As an example, the transcriptional inhibitor Cas12f1 fusion protein may have the following structure from the N-terminus to the C-terminus: [dCas12f1 R490A protein]-[DNMT3]; [dCas12f1 R490Q protein]-[DNMT3]; [dCas12f1 R490L protein]-[DNMT3]; [dCas12f1 R490W protein]-[DNMT3]; [DNMT3]-[dCas12f1 R490A protein]; [DNMT3]-[dCas12f1 R490Q protein]; [DNMT3]-[dCas12f1 R490L protein]; or [DNMT3]-[dCas12f1 R490W protein]. The transcriptional inhibitor Cas12f1 fusion protein may further comprise at least one NLS. Here, the at least one NLS may be located at the N-terminus of the transcriptional inhibitor Cas12f1 fusion protein, at the C-terminus of the transcriptional inhibitor Cas12f1 fusion protein, and/or between the dCas12f1 protein (the dCas12f1 R490A protein, the dCas12f1 R490Q protein, the dCas12f1 R490L protein, or the dCas12f1 R490W protein) and DNMT3.

As an example, the transcriptional inhibitor Cas12f1 fusion protein may have the following structure from the N-terminus to the C-terminus: [dCas12f1 D510A protein]-[DNMT3]; [dCas12f1 D510L protein]-[DNMT3]; [dCas12f1 D510V protein]-[DNMT3]; [DNMT3]-[dCas12f1 D510A protein]; [DNMT3]-[dCas12f1 D510L protein]; or [DNMT3]-[dCas12f1 D510V protein]. The transcriptional inhibitor Cas12f1 fusion protein may further comprise at least one NLS. Here, the at least one NLS may be located at the N-terminus of the transcriptional inhibitor Cas12f1 fusion protein, at the C-terminus of the transcriptional inhibitor Cas12f1 fusion protein, and/or between the dCas12f1 protein (the dCas12f1 D510A protein, the dCas12f1 D510L protein, or the dCas12f1 D510V protein) and DNMT3.

As an example, the transcriptional inhibitor Cas12f1 fusion protein may have the following structure from the N-terminus to the C-terminus: [dCas12f1 R490A protein]-[MeCP2]; [dCas12f1 R490Q protein]-[MeCP2]; [dCas12f1 R490L protein]-[MeCP2]; [dCas12f1 R490W protein]-[MeCP2]; [MeCP2]-[dCas12f1 R490A protein]; [MeCP2]-[dCas12f1 R490Q protein]; [MeCP2]-[dCas12f1 R490L protein]; or [MeCP2]-[dCas12f1 R490W protein]. The transcriptional inhibitor Cas12f1 fusion protein may further comprise at least one NLS. Here, the at least one NLS may be located at the N-terminus of the transcriptional inhibitor Cas12f1 fusion protein, at the C-terminus of the transcriptional inhibitor Cas12f1 fusion protein, and/or between the dCas12f1 protein (the dCas12f1 R490A protein, the dCas12f1 R490Q protein, the dCas12f1 R490L protein, or the dCas12f1 R490W protein) and MeCP2.

As an example, the transcriptional inhibitor Cas12f1 fusion protein may have the following structure from the N-terminus to the C-terminus: [dCas12f1 D510A protein]-[MeCP2]; [dCas12f1 D510L protein]-[MeCP2]; [dCas12f1 D510V protein]-[MeCP2]; [MeCP2]-[dCas12f1 D510A protein]; [MeCP2]-[dCas12f1 D510L protein]; or [MeCP2]-[dCas12f1 D510V protein]. The transcriptional inhibitor Cas12f1 fusion protein may further comprise at least one NLS. Here, the at least one NLS may be located at the N-terminus of the transcriptional inhibitor Cas12f1 fusion protein, at the C-terminus of the transcriptional inhibitor Cas12f1 fusion protein, and/or between the dCas12f1 protein (the dCas12f1 D510A protein, the dCas12f1 D510L protein, or the dCas12f1 D510V protein) and MeCP2.

As an example, the transcriptional inhibitor Cas12f1 fusion protein may have the following structure from the N-terminus to the C-terminus: [dCas12f1 R490A protein]-[HDAC3]; [dCas12f1 R490Q protein]-[HDAC3]; [dCas12f1 R490L protein]-[HDAC3]; [dCas12f1 R490W protein]-[HDAC3]; [HDAC3]-[dCas12f1 R490A protein]; [HDAC3]-[dCas12f1 R490Q protein]; [HDAC3]-[dCas12f1 R490L protein]; or [HDAC3]-[dCas12f1 R490W protein]. The transcriptional inhibitor Cas12f1 fusion protein may further comprise at least one NLS. Here, the at least one NLS may be located at the N-terminus of the transcriptional inhibitor Cas12f1 fusion protein, at the C-terminus of the transcriptional inhibitor Cas12f1 fusion protein, and/or between the dCas12f1 protein (the dCas12f1 R490A protein, the dCas12f1 R490Q protein, the dCas12f1 R490L protein, or the dCas12f1 R490W protein) and HDAC3.

As an example, the transcriptional inhibitor Cas12f1 fusion protein may have the following structure from the N-terminus to the C-terminus: [dCas12f1 D510A protein]-[HDAC3]; [dCas12f1 D510L protein]-[HDAC3]; [dCas12f1 D510V protein]-[HDAC3]; [HDAC3]-[dCas12f1 D510A protein]; [HDAC3]-[dCas12f1 D510L protein]; or [HDAC3]-[dCas12f1 D510V protein]. The transcriptional inhibitor Cas12f1 fusion protein may further comprise at least one NLS. Here, the at least one NLS may be located at the N-terminus of the transcriptional inhibitor Cas12f1 fusion protein, at the C-terminus of the transcriptional inhibitor Cas12f1 fusion protein, and/or between the dCas12f1 protein (the dCas12f1 D510A protein, the dCas12f1 D510L protein, or the dCas12f1 D510V protein) and HDAC3.

### Cas12f1 fusion protein - Example 2 of transcriptional inhibitor Cas12f1 fusion protein

In an embodiment, the transcriptional inhibitor Cas12f1 fusion protein may be such that from the N-terminus to the C-terminus, the dCas12f1 protein, the transcriptional inhibitor protein, and the transcriptional inhibitor protein are sequentially linked to each other; or the transcriptional inhibitor protein, the transcriptional inhibitor protein, and the dCas12f1 protein are sequentially linked to each other. Here, the dCas12f1 protein and the transcriptional inhibitor proteins may be linked via linkers.

The dCas12f1 protein may be a modified form of the wild-type Cas12f1 protein in which arginine (R) which is the 490^{th} amino acid in the amino acid sequence constituting the wild-type Cas12f1 protein is substituted with alanine (A), glutamine (Q), leucine (L), or tryptophan (W). Here, the dCas12f1 protein may be a dCas12f1 R490A protein having alanine at position 490, as compared with the amino acid sequence of the wild-type Cas12f1 protein. Alternatively, the dCas12f1 protein may be a dCas12f1 R490Q protein having glutamine at position 490, as compared with the amino acid sequence of the wild-type Cas12f1 protein. Alternatively, the dCas12f1 protein may be a dCas12f1 R490L protein having leucine at position 490, as compared with the amino acid sequence of the wild-type Cas12f1 protein. Alternatively, the dCas12f1 protein may be a dCas12f1 R490W protein having tryptophan at position 490, as compared with the amino acid sequence of the wild-type Cas12f1 protein. Here, the dCas12f1 protein may have at least one of the amino acid sequences set forth in SEQ ID NOs: 261, 262, 264, and 265.

Alternatively, the dCas12f1 protein may be a modified form of the wild-type Cas12f1 protein in which aspartic acid (D) which is the 510^{th} amino acid in the amino acid sequence constituting the wild-type Cas12f1 protein is substituted with alanine (A), leucine (L), or valine (V). Here, the dCas12f1 protein may be a dCas12f1 D510A protein having alanine at position 510, as compared with the amino acid sequence of the wild-type Cas12f1 protein. Alternatively, the dCas12f1 protein may be a dCas12f1 D510L protein having leucine at position 510, as compared with the amino acid sequence of the wild-type Cas12f1 protein. Alternatively, the dCas12f1 protein may be a dCas12f1 D510V protein having valine at position 510, as compared with the amino acid sequence of the wild-type Cas12f1 protein. Here, the dCas12f1 protein may have at least one of the amino acid sequences set forth in SEQ ID NOs: 266 to 268.

The transcriptional inhibitor protein may be KRAB, MeCP2, DNMT3, or HDAC3. Here, the transcriptional inhibitor protein may have at least one of the amino acid sequences set forth in SEQ ID NOs: 274 to 277.

The transcriptional inhibitor Cas12f1 fusion protein may further comprise at least one NLS as the additional domain. Here, the at least one NLS may be located at the N-terminus and/or the C-terminus of the transcriptional inhibitor Cas12f1 fusion protein. Alternatively, the at least one NLS may be located between the dCas12f1 protein and the transcriptional inhibitor protein. Alternatively, the at least one NLS may be located between the transcriptional inhibitor proteins. Here, the at least one NLS may be linked to the dCas12f1 protein and/or the transcriptional inhibitor proteins via linkers.

As an example, the transcriptional inhibitor Cas12f1 fusion protein may have the following structure from the N-terminus to the C-terminus: [dCas12f1 R490A protein]-[KRAB]-[KRAB]; [dCas12f1 R490Q protein]-[KRAB]-[KRAB]; [dCas12f1 R490L protein]-[KRAB]-[KRAB]; [dCas12f1 R490W protein]-[KRAB]-[KRAB]; [KRAB]-[KRAB]-[dCas12f1 R490A protein]; [KRAB]-[KRAB]-[dCas12f1 R490Q protein]; [KRAB]-[KRAB]-[dCas12f1 R490L protein]; or [KRAB]-[KRAB]-[dCas12f1 R490W protein]. The transcriptional inhibitor Cas12f1 fusion protein may further comprise at least one NLS. Here, the at least one NLS may be located at the N-terminus of the transcriptional inhibitor Cas12f1 fusion protein, at the C-terminus of the transcriptional inhibitor Cas12f1 fusion protein, between the dCas12f1 protein (the dCas12f1 R490A protein, the dCas12f1 R490Q protein, the dCas12f1 R490L protein, or the dCas12f1 R490W protein) and KRAB, and/or between KRAB and KRAB.

As an example, the transcriptional inhibitor Cas12f1 fusion protein may have the following structure from the N-terminus to the C-terminus: [dCas12f1 D510A protein]-[KRAB]-[KRAB]; [dCas12f1 D510L protein]-[KRAB]-[KRAB]; [dCas12f1 D510V protein]-[KRAB]-[KRAB]; [KRAB]-[KRAB]-[dCas12f1 D510A protein]; [KRAB]-[KRAB]-[dCas12f1 D510L protein]; or [KRAB]-[KRAB]-[dCas12f1 D510V protein]. The transcriptional inhibitor Cas12f1 fusion protein may further comprise at least one NLS. Here, the at least one NLS may be located at the N-terminus of the transcriptional inhibitor Cas12f1 fusion protein, at the C-terminus of the transcriptional inhibitor Cas12f1 fusion protein, between the dCas12f1 protein (the dCas12f1 D510A protein, the dCas12f1 D510L protein, or the dCas12f1 D510V protein) and KRAB, and/or between KRAB and KRAB.

As an example, the transcriptional inhibitor Cas12f1 fusion protein may have the following structure from the N-terminus to the C-terminus: [dCas12f1 R490A protein]-[KRAB]-[MeCP2]; [dCas12f1 R490Q protein]-[KRAB]-[MeCP2]; [dCas12f1 R490L protein]-[KRAB]-[MeCP2]; [dCas12f1 R490W protein]-[KRAB]-[MeCP2]; [dCas12f1 R490A protein]-[MeCP2]-[KRAB]; [dCas12f1 R490Q protein]-[MeCP2]-[KRAB]; [dCas12f1 R490L protein]-[MeCP2]-[KRAB]; [dCas12f1 R490W protein]-[MeCP2]-[KRAB]; [KRAB]-[MeCP2]-[dCas12f1 R490A protein]; [KRAB]-[MeCP2]-[dCas12f1 R490Q protein]; [KRAB]-[MeCP2]-[dCas12f1 R490L protein]; [KRAB]-[MeCP2]-[dCas12f1 R490W protein]; [MeCP2]-[KRAB]-[dCas12f1 R490A protein]; [MeCP2]-[KRAB]-[dCas12f1 R490Q protein]; [MeCP2]-[KRAB]-[dCas12f1 R490L protein]; or [MeCP2]-[KRAB]-[dCas12f1 R490W protein]. The transcriptional inhibitor Cas12f1 fusion protein may further comprise at least one NLS. Here, the at least one NLS may be located at the N-terminus of the transcriptional inhibitor Cas12f1 fusion protein, at the C-terminus of the transcriptional inhibitor Cas12f1 fusion protein, between the dCas12f1 protein (the dCas12f1 R490A protein, the dCas12f1 R490Q protein, the dCas12f1 R490L protein, or the dCas12f1 R490W protein) and KRAB, between the dCas12f1 protein (the dCas12f1 R490A protein, the dCas12f1 R490Q protein, the dCas12f1 R490L protein, or the dCas12f1 R490W protein) and MeCP2, and/or between KRAB and MeCP2.

As an example, the transcriptional inhibitor Cas12f1 fusion protein may have the following structure from the N-terminus to the C-terminus: [dCas12f1 D510A protein]-[KRAB]-[MeCP2]; [dCas12f1 D510L protein]-[KRAB]-[MeCP2]; [dCas12f1 D510V protein]-[KRAB]-[MeCP2]; [dCas12f1 D510A protein]-[MeCP2]-[KRAB]; [dCas12f1 D510L protein]-[MeCP2]-[KRAB]; [dCas12f1 D510V protein]-[MeCP2]-[KRAB]; [KRAB]-[MeCP2]-[dCas12f1 D510A protein]; [KRAB]-[MeCP2]-[dCas12f1 D510L protein]; [KRAB]-[MeCP2]-[dCas12f1 D510V protein]; [MeCP2]-[KRAB]-[dCas12f1 D510A protein]; [MeCP2]-[KRAB]-[dCas12f1 D510L protein]; or [MeCP2]-[KRAB]-[dCas12f1 D510V protein]. The transcriptional inhibitor Cas12f1 fusion protein may further comprise at least one NLS. Here, the at least one NLS may be located at the N-terminus of the transcriptional inhibitor Cas12f1 fusion protein, at the C-terminus of the transcriptional inhibitor Cas12f1 fusion protein, between the dCas12f1 protein (the dCas12f1 D510A protein, the dCas12f1 D510L protein, or the dCas12f1 D510V protein) and MeCP2, and/or between KRAB and MeCP2.

As an example, the transcriptional inhibitor Cas12f1 fusion protein may have the following structure from the N-terminus to the C-terminus: [dCas12f1 R490A protein]-[KRAB]-[DNMT3]; [dCas12f1 R490Q protein]-[KRAB]-[DNMT3]; [dCas12f1 R490L protein]-[KRAB]-[DNMT3]; [dCas12f1 R490W protein]-[KRAB]-[DNMT3]; [dCas12f1 R490A protein]-[DNMT3]-[KRAB]; [dCas12f1 R490Q protein]-[DNMT3]-[KRAB]; [dCas12f1 R490L protein]-[DNMT3]-[KRAB]; [dCas12f1 R490W protein]-[DNMT3]-[KRAB]; [KRAB]-[DNMT3]-[dCas12f1 R490A protein]; [KRAB]-[DNMT3]-[dCas12f1 R490Q protein]; [KRAB]-[DNMT3]-[dCas12f1 R490L protein]; [KRAB]-[DNMT3]-[dCas12f1 R490W protein]; [DNMT3]-[KRAB]-[dCas12f1 R490A protein]; [DNMT3]-[KRAB]-[dCas12f1 R490Q protein]; [DNMT3]-[KRAB]-[dCas12f1 R490L protein]; or [DNMT3]-[KRAB]-[dCas12f1 R490W protein]. The transcriptional inhibitor Cas12f1 fusion protein may further comprise at least one NLS. Here, the at least one NLS may be located at the N-terminus of the transcriptional inhibitor Cas12f1 fusion protein, at the C-terminus of the transcriptional inhibitor Cas12f1 fusion protein, between the dCas12f1 protein (the dCas12f1 R490A protein, the dCas12f1 R490Q protein, the dCas12f1 R490L protein, or the dCas12f1 R490W protein) and KRAB, between the dCas12f1 protein (the dCas12f1 R490A protein, the dCas12f1 R490Q protein, the dCas12f1 R490L protein, or the dCas12f1 R490W protein) and DNMT3, and/or between KRAB and DNMT3.

As an example, the transcriptional inhibitor Cas12f1 fusion protein may have the following structure from the N-terminus to the C-terminus: [dCas12f1 D510A protein]-[KRAB]-[DNMT3]; [dCas12f1 D510L protein]-[KRAB]-[DNMT3]; [dCas12f1 D510V protein]-[KRAB]-[DNMT3]; [dCas12f1 D510A protein]-[DNMT3]-[KRAB]; [dCas12f1 D510L protein]-[DNMT3]-[KRAB]; [dCas12f1 D510V protein]-[DNMT3]-[KRAB]; [KRAB]-[DNMT3]-[dCas12f1 D510A protein]; [KRAB]-[DNMT3]-[dCas12f1 D510L protein]; [KRAB]-[DNMT3]-[dCas12f1 D510V protein]; [DNMT3]-[KRAB]-[dCas12f1 D510A protein]; [DNMT3]-[KRAB]-[dCas12f1 D510L protein]; or [DNMT3]-[KRAB]-[dCas12f1 D510V protein]. The transcriptional inhibitor Cas12f1 fusion protein may further comprise at least one NLS. Here, the at least one NLS may be located at the N-terminus of the transcriptional inhibitor Cas12f1 fusion protein, at the C-terminus of the transcriptional inhibitor Cas12f1 fusion protein, between the dCas12f1 protein (the dCas12f1 D510A protein, the dCas12f1 D510L protein, or the dCas12f1 D510V protein) and DNMT3, and/or between KRAB and DNMT3.

As an example, the transcriptional inhibitor Cas12f1 fusion protein may have the following structure from the N-terminus to the C-terminus: [dCas12f1 R490A protein]-[KRAB]-[HDAC3]; [dCas12f1 R490Q protein]-[KRAB]-[HDAC3]; [dCas12f1 R490L protein]-[KRAB]-[HDAC3]; [dCas12f1 R490W protein]-[KRAB]-[HDAC3]; [dCas12f1 R490A protein]-[HDAC3]-[KRAB]; [dCas12f1 R490Q protein]-[HDAC3]-[KRAB]; [dCas12f1 R490L protein]-[HDAC3]-[KRAB]; [dCas12f1 R490W protein]-[HDAC3]-[KRAB]; [KRAB]-[HDAC3]-[dCas12f1 R490A protein]; [KRAB]-[HDAC3]-[dCas12f1 R490Q protein]; [KRAB]-[HDAC3]-[dCas12f1 R490L protein]; [KRAB]-[HDAC3]-[dCas12f1 R490W protein]; [HDAC3]-[KRAB]-[dCas12f1 R490A protein]; [HDAC3]-[KRAB]-[dCas12f1 R490Q protein]; [HDAC3]-[KRAB]-[dCas12f1 R490L protein]; or [HDAC3]-[KRAB]-[dCas12f1 R490W protein]. The transcriptional inhibitor Cas12f1 fusion protein may further comprise at least one NLS. Here, the at least one NLS may be located at the N-terminus of the transcriptional inhibitor Cas12f1 fusion protein, at the C-terminus of the transcriptional inhibitor Cas12f1 fusion protein, between the dCas12f1 protein (the dCas12f1 R490A protein, the dCas12f1 R490Q protein, the dCas12f1 R490L protein, or the dCas12f1 R490W protein) and KRAB, between the dCas12f1 protein (the dCas12f1 R490A protein, the dCas12f1 R490Q protein, the dCas12f1 R490L protein, or the dCas12f1 R490W protein) and HDAC3, and/or between KRAB and HDAC3.

As an example, the transcriptional inhibitor Cas12f1 fusion protein may have the following structure from the N-terminus to the C-terminus: [dCas12f1 D510A protein]-[KRAB]-[HDAC3]; [dCas12f1 D510L protein]-[KRAB]-[HDAC3]; [dCas12f1 D510V protein]-[KRAB]-[HDAC3]; [dCas12f1 D510A protein]-[HDAC3]-[KRAB]; [dCas12f1 D510L protein]-[HDAC3]-[KRAB]; [dCas12f1 D510V protein]-[HDAC3]-[KRAB]; [KRAB]-[HDAC3]-[dCas12f1 D510A protein]; [KRAB]-[HDAC3]-[dCas12f1 D510L protein]; [KRAB]-[HDAC3]-[dCas12f1 D510V protein]; [HDAC3]-[KRAB]-[dCas12f1 D510A protein]; [HDAC3]-[KRAB]-[dCas12f1 D510L protein]; or [HDAC3]-[KRAB]-[dCas12f1 D510V protein]. The transcriptional inhibitor Cas12f1 fusion protein may further comprise at least one NLS. Here, the at least one NLS may be located at the N-terminus of the transcriptional inhibitor Cas12f1 fusion protein, at the C-terminus of the transcriptional inhibitor Cas12f1 fusion protein, between the dCas12f1 protein (the dCas12f1 D510A protein, the dCas12f1 D510L protein, or the dCas12f1 D510V protein) and HDAC3, and/or between KRAB and HDAC3.

### Cas12f1 fusion protein - Example 3 of transcriptional inhibitor Cas12f1 fusion protein

In an embodiment, the transcriptional inhibitor Cas12f1 fusion protein may be such that from the N-terminus to the C-terminus, the transcriptional inhibitor protein, the dCas12f1 protein, and the transcriptional inhibitor protein are sequentially linked to each other. Here, the dCas12f1 protein and the transcriptional inhibitor proteins may be linked via linkers.

The transcriptional inhibitor protein may be KRAB, MeCP2, DNMT3, or HDAC3. Here, the transcriptional inhibitor protein may have at least one of the amino acid sequences set forth in SEQ ID NOs: 274 to 277.

The dCas12f1 protein may be a modified form of the wild-type Cas12f1 protein in which arginine (R) which is the 490^{th} amino acid in the amino acid sequence constituting the wild-type Cas12f1 protein is substituted with alanine (A), glutamine (Q), leucine (L), or tryptophan (W). Here, the dCas12f1 protein may be a dCas12f1 R490A protein having alanine at position 490, as compared with the amino acid sequence of the wild-type Cas12f1 protein. Alternatively, the dCas12f1 protein may be a dCas12f1 R490Q protein having glutamine at position 490, as compared with the amino acid sequence of the wild-type Cas12f1 protein. Alternatively, the dCas12f1 protein may be a dCas12f1 R490L protein having leucine at position 490, as compared with the amino acid sequence of the wild-type Cas12f1 protein. Alternatively, the dCas12f1 protein may be a dCas12f1 R490W protein having tryptophan at position 490, as compared with the amino acid sequence of the wild-type Cas12f1 protein. Here, the dCas12f1 protein may have at least one of the amino acid sequences set forth in SEQ ID NOs: 261, 262, 264, and 265.

Alternatively, the dCas12f1 protein may be a modified form of the wild-type Cas12f1 protein in which aspartic acid (D) which is the 510^{th} amino acid in the amino acid sequence constituting the wild-type Cas12f1 protein is substituted with alanine (A), leucine (L), or valine (V). Here, the dCas12f1 protein may be a dCas12f1 D510A protein having alanine at position 510, as compared with the amino acid sequence of the wild-type Cas12f1 protein. Alternatively, the dCas12f1 protein may be a dCas12f1 D510L protein having leucine at position 510, as compared with the amino acid sequence of the wild-type Cas12f1 protein. Alternatively, the dCas12f1 protein may be a dCas12f1 D510V protein having valine at position 510, as compared with the amino acid sequence of the wild-type Cas12f1 protein. Here, the dCas12f1 protein may have at least one of the amino acid sequences set forth in SEQ ID NOs: 266 to 268.

The transcriptional inhibitor Cas12f1 fusion protein may further comprise at least one NLS as the additional domain. Here, the at least one NLS may be located at the N-terminus and/or the C-terminus of the transcriptional inhibitor Cas12f1 fusion protein. Alternatively, the at least one NLS may be located between the dCas12f1 protein and the transcriptional inhibitor protein. Alternatively, the at least one NLS may be located between the transcriptional inhibitor proteins. Here, the at least one NLS may be linked to the dCas12f1 protein and/or the transcriptional inhibitor proteins via linkers.

As an example, the transcriptional inhibitor Cas12f1 fusion protein may have the following structure from the N-terminus to the C-terminus: [KRAB]-[dCas12f1 R490A protein]-[KRAB]; [KRAB]-[dCas12f1 R490Q protein]-[KRAB]; [KRAB]-[dCas12f1 R490L protein]-[KRAB]; [KRAB]-[dCas12f1 R490W protein]-[KRAB]; [KRAB]-[dCas12f1 R490A protein]-[KRAB]; [KRAB]-[dCas12f1 R490Q protein]-[KRAB]; [KRAB]-[dCas12f1 R490L protein]-[KRAB]; or [KRAB]-[dCas12f1 R490W protein]-[KRAB]. The transcriptional inhibitor Cas12f1 fusion protein may further comprise at least one NLS. Here, the at least one NLS may be located at the N-terminus of the transcriptional inhibitor Cas12f1 fusion protein, at the C-terminus of the transcriptional inhibitor Cas12f1 fusion protein, and/or between the dCas12f1 protein (the dCas12f1 R490A protein, the dCas12f1 R490Q protein, the dCas12f1 R490L protein, or the dCas12f1 R490W protein) and KRAB.

As an example, the transcriptional inhibitor Cas12f1 fusion protein may have the following structure from the N-terminus to the C-terminus: [KRAB]-[dCas12f1 D510A protein]-[KRAB]; [KRAB]-[dCas12f1 D510L protein]-[KRAB]; [KRAB]-[dCas12f1 D510V protein]-[KRAB]; [KRAB]-[dCas12f1 D510A protein]-[KRAB]; [KRAB]-[dCas12f1 D510L protein]-[KRAB]; or [KRAB]-[dCas12f1 D510V protein]-[KRAB]. The transcriptional inhibitor Cas12f1 fusion protein may further comprise at least one NLS. Here, the at least one NLS may be located at the N-terminus of the transcriptional inhibitor Cas12f1 fusion protein, at the C-terminus of the transcriptional inhibitor Cas12f1 fusion protein, and/or between the dCas12f1 protein (the dCas12f1 D510A protein, the dCas12f1 D510L protein, or the dCas12f1 D510V protein) and KRAB.

As an example, the transcriptional inhibitor Cas12f1 fusion protein may have the following structure from the N-terminus to the C-terminus: [KRAB]-[dCas12f1 R490A protein]-[MeCP2]; [KRAB]-[dCas12f1 R490Q protein]-[MeCP2]; [KRAB]-[dCas12f1 R490L protein]-[MeCP2]; [KRAB]-[dCas12f1 R490W protein]-[MeCP2]; [MeCP2]-[dCas12f1 R490A protein]-[KRAB]; [MeCP2]-[dCas12f1 R490Q protein]-[KRAB]; [MeCP2]-[dCas12f1 R490L protein]-[KRAB]; or [MeCP2]-[dCas12f1 R490W protein]-[KRAB]. The transcriptional inhibitor Cas12f1 fusion protein may further comprise at least one NLS. Here, the at least one NLS may be located at the N-terminus of the transcriptional inhibitor Cas12f1 fusion protein, at the C-terminus of the transcriptional inhibitor Cas12f1 fusion protein, between the dCas12f1 protein (the dCas12f1 R490A protein, the dCas12f1 R490Q protein, the dCas12f1 R490L protein, or the dCas12f1 R490W protein) and KRAB, and/or between the dCas12f1 protein (the dCas12f1 R490A protein, the dCas12f1 R490Q protein, the dCas12f1 R490L protein, or the dCas12f1 R490W protein) and MeCP2.

As an example, the transcriptional inhibitor Cas12f1 fusion protein may have the following structure from the N-terminus to the C-terminus: [KRAB]-[dCas12f1 D510A protein]-[MeCP2]; [KRAB]-[dCas12f1 D510L protein]-[MeCP2]; [KRAB]-[dCas12f1 D510V protein]-[MeCP2]; [MeCP2]-[dCas12f1 D510A protein]-[KRAB]; [MeCP2]-[dCas12f1 D510L protein]-[KRAB]; or [MeCP2]-[dCas12f1 D510V protein]-[KRAB]. The transcriptional inhibitor Cas12f1 fusion protein may further comprise at least one NLS. Here, the at least one NLS may be located at the N-terminus of the transcriptional inhibitor Cas12f1 fusion protein, at the C-terminus of the transcriptional inhibitor Cas12f1 fusion protein, between the dCas12f1 protein (the dCas12f1 D510A protein, the dCas12f1 D510L protein, or the dCas12f1 D510V protein) and KRAB, and/or between the dCas12f1 protein (the dCas12f1 D510A protein, the dCas12f1 D510L protein, or the dCas12f1 D510V protein) and MeCP2.

As an example, the transcriptional inhibitor Cas12f1 fusion protein may have the following structure from the N-terminus to the C-terminus: [KRAB]-[dCas12f1 R490A protein]-[DNMT3]; [KRAB]-[dCas12f1 R490Q protein]-[DNMT3]; [KRAB]-[dCas12f1 R490L protein]-[DNMT3]; [KRAB]-[dCas12f1 R490Wprotein]-[DNMT3]; [DNMT3]-[dCas12f1 R490A protein]-[KRAB]; [DNMT3]-[dCas12f1 R490Q protein]-[KRAB]; [DNMT3]-[dCas12f1 R490L protein]-[KRAB]; or [DNMT3]-[dCas12f1 R490W protein]-[KRAB]. The transcriptional inhibitor Cas12f1 fusion protein may further comprise at least one NLS. In this regard, the at least one NLS may be located at the N-terminus of the transcriptional inhibitor Cas12f1 fusion protein, at the C-terminus of the transcriptional inhibitor Cas12f1 fusion protein, between the dCas12f1 protein (the dCas12f1 R490A protein, the dCas12f1 R490Q protein, the dCas12f1 R490L protein, or the dCas12f1 R490W protein) and KRAB, and/or between the dCas12f1 protein (the dCas12f1 R490A protein, the dCas12f1 R490Q protein, the dCas12f1 R490L protein, or the dCas12f1 R490W protein) and DNMT3.

As an example, the transcriptional inhibitor Cas12f1 fusion protein may have the following structure from the N-terminus to the C-terminus: [KRAB]-[dCas12f1 D510A protein]-[DNMT3]; [KRAB]-[dCas12f1 D510L protein]-[DNMT3]; [KRAB]-[dCas12f1 D510V protein]-[DNMT3]; [DNMT3]-[dCas12f1 D510A protein]-[KRAB]; [DNMT3]-[dCas12f1 D510L protein]-[KRAB]; or [DNMT3]-[dCas12f1 D510V protein]-[KRAB]. The transcriptional inhibitor Cas12f1 fusion protein may further comprise at least one NLS. Here, the at least one NLS may be located at the N-terminus of the transcriptional inhibitor Cas12f1 fusion protein, at the C-terminus of the transcriptional inhibitor Cas12f1 fusion protein, between the dCas12f1 protein (the dCas12f1 D510A protein, the dCas12f1 D510L protein, or the dCas12f1 D510V protein) and KRAB, and/or between the dCas12f1 protein (the dCas12f1 D510A protein, the dCas12f1 D510L protein, or the dCas12f1 D510V protein) and DNMT3.

As an example, the transcriptional inhibitor Cas12f1 fusion protein may have the following structure from the N-terminus to the C-terminus: [KRAB]-[dCas12f1 R490A protein]-[HDAC3]; [KRAB]-[dCas12f1 R490Q protein]-[HDAC3]; [KRAB]-[dCas12f1 R490L protein]-[HDAC3]; [KRAB]-[dCas12f1 R490W protein]-[HDAC3]; [HDAC3]-[dCas12f1 R490A protein]-[KRAB]; [HDAC3]-[dCas12f1 R490Q protein]-[KRAB]; [HDAC3]-[dCas12f1 R490L protein]-[KRAB]; or [HDAC3]-[dCas12f1 R490W protein]-[KRAB]. The transcriptional inhibitor Cas12f1 fusion protein may further comprise at least one NLS. Here, the at least one NLS may be located at the N-terminus of the transcriptional inhibitor Cas12f1 fusion protein, at the C-terminus of the transcriptional inhibitor Cas12f1 fusion protein, between the dCas12f1 protein (the dCas12f1 R490A protein, the dCas12f1 R490Q protein, the dCas12f1 R490L protein, or the dCas12f1 R490W protein) and KRAB, and/or between the dCas12f1 protein (the dCas12f1 R490A protein, the dCas12f1 R490Q protein, the dCas12f1 R490L protein, or the dCas12f1 R490W protein) and HDAC3.

As an example, the transcriptional inhibitor Cas12f1 fusion protein may have the following structure from the N-terminus to the C-terminus: [KRAB]-[dCas12f1 D510A protein]-[HDAC3]; [KRAB]-[dCas12f1 D510L protein]-[HDAC3]; [KRAB]-[dCas12f1 D510V protein]-[HDAC3]; [HDAC3]-[dCas12f1 D510A protein]-[KRAB]; [HDAC3]-[dCas12f1 D510L protein]-[KRAB]; or [HDAC3]-[dCas12f1 D510V protein]-[KRAB]. The transcriptional inhibitor Cas12f1 fusion protein may further comprise at least one NLS. Here, the at least one NLS may be located at the N-terminus of the transcriptional inhibitor Cas12f1 fusion protein, at the C-terminus of the transcriptional inhibitor Cas12f1 fusion protein, between the dCas12f1 protein (the dCas12f1 D510A protein, the dCas12f1 D510L protein, or the dCas12f1 D510V protein) and KRAB, and/or between the dCas12f1 protein (the dCas12f1 D510A protein, the dCas12f1 D510L protein, or the dCas12f1 D510V protein) and HDAC3.

### Cas12f1 fusion protein - Example 4 of transcriptional inhibitor Cas12f1 fusion protein

In an embodiment, the transcriptional inhibitor Cas12f1 fusion protein may comprise a dCas12f1 protein and at least three transcriptional activator proteins.

In an embodiment, the transcriptional inhibitor Cas12f1 fusion protein may be such that from the N-terminus to the C-terminus, the dCas12f1 protein, the transcriptional inhibitor protein, the transcriptional inhibitor protein, and the transcriptional inhibitor protein are sequentially linked to each other; the transcriptional inhibitor protein, the transcriptional inhibitor protein, the transcriptional inhibitor protein, and the dCas12f1 protein are sequentially linked to each other; the transcriptional inhibitor protein, the transcriptional inhibitor protein, the dCas12f1 protein, and the transcriptional inhibitor protein are sequentially linked to each other; or the transcriptional inhibitor protein, the dCas12f1 protein, the transcriptional inhibitor protein, and the transcriptional inhibitor protein are sequentially linked to each other. Here, the dCas12f1 protein and the transcriptional inhibitor proteins may be linked via linkers.

The transcriptional inhibitor protein may be KRAB, MeCP2, DNMT3, or HDAC3. Here, the transcriptional inhibitor protein may have at least one of the amino acid sequences set forth in SEQ ID NOs: 274 to 277.

The dCas12f1 protein may be a modified form of the wild-type Cas12f1 protein in which arginine (R) which is the 490^{th} amino acid in the amino acid sequence constituting the wild-type Cas12f1 protein is substituted with alanine (A), glutamine (Q), leucine (L), or tryptophan (W). Here, the dCas12f1 protein may be a dCas12f1 R490A protein having alanine at position 490, as compared with the amino acid sequence of the wild-type Cas12f1 protein. Alternatively, the dCas12f1 protein may be a dCas12f1 R490Q protein having glutamine at position 490, as compared with the amino acid sequence of the wild-type Cas12f1 protein. Alternatively, the dCas12f1 protein may be a dCas12f1 R490L protein having leucine at position 490, as compared with the amino acid sequence of the wild-type Cas12f1 protein. Alternatively, the dCas12f1 protein may be a dCas12f1 R490W protein having tryptophan at position 490, as compared with the amino acid sequence of the wild-type Cas12f1 protein. Here, the dCas12f1 protein may have at least one of the amino acid sequences set forth in SEQ ID NOs: 261, 262, 264, and 265.

Alternatively, the dCas12f1 protein may be a modified form of the wild-type Cas12f1 protein in which aspartic acid (D) which is the 510^{th} amino acid in the amino acid sequence constituting the wild-type Cas12f1 protein is substituted with alanine (A), leucine (L), or valine (V). Here, the dCas12f1 protein may be a dCas12f1 D510A protein having alanine at position 510, as compared with the amino acid sequence of the wild-type Cas12f1 protein. Alternatively, the dCas12f1 protein may be a dCas12f1 D510L protein having leucine at position 510, as compared with the amino acid sequence of the wild-type Cas12f1 protein. Alternatively, the dCas12f1 protein may be a dCas12f1 D510V protein having valine at position 510, as compared with the amino acid sequence of the wild-type Cas12f1 protein. Here, the dCas12f1 protein may have at least one of the amino acid sequences set forth in SEQ ID NOs: 266 to 268.

The transcriptional inhibitor Cas12f1 fusion protein may further comprise at least one NLS as the additional domain. Here, the at least one NLS may be located at the N-terminus and/or the C-terminus of the transcriptional inhibitor Cas12f1 fusion protein. Alternatively, the at least one NLS may be located between the dCas12f1 protein and the transcriptional inhibitor protein. Alternatively, the at least one NLS may be located between the transcriptional inhibitor proteins. Here, the at least one NLS may be linked to the dCas12f1 protein and/or the transcriptional inhibitor proteins via linkers.

As an example, the transcriptional inhibitor Cas12f1 fusion protein may have the following structure from the N-terminus to the C-terminus: [dCas12f1 R490A protein]-[KRAB]-[KRAB]-[MeCP2]; [dCas12f1 R490Q protein]-[KRAB]-[KRAB]-[MeCP2]; [dCas12f1 R490L protein]-[KRAB]-[KRAB]-[MeCP2]; [dCas12f1 R490W protein]-[KRAB]-[KRAB]-[MeCP2]; [dCas12f1 R490A protein]-[KRAB]-[KRAB]-[DNMT3]; [dCas12f1 R490Q protein]-[KRAB]-[KRAB]-[DNMT3]; [dCas12f1 R490L protein]-[KRAB]-[KRAB]-[DNMT3]; [dCas12f1 R490W protein]-[KRAB]-[KRAB]-[DNMT3]; [dCas12f1 R490A protein]-[KRAB]-[KRAB]-[HDAC3]; dCas12f1 R490Q protein]-[KRAB]-[KRAB]-[HDAC3]; [dCas12f1 R490L protein]-[KRAB]-[KRAB]-[HDAC3]; [dCas12f1 R490W protein]-[KRAB]-[KRAB]-[HDAC3]; [dCas12f1 R490A protein]-[KRAB]-[MeCP2]-[KRAB]; [dCas12f1 R490Q protein]-[KRAB]-[MeCP2]-[KRAB]; [dCas12f1 R490L protein]-[KRAB]-[MeCP2]-[KRAB]; [dCas12f1 R490W protein]-[KRAB]-[MeCP2]-[KRAB]; [dCas12f1 R490A protein]-[KRAB]-[DNMT3]-[KRAB]; [dCas12f1 R490Q protein]-[KRAB]-[DNMT3]-[KRAB]; [dCas12f1 R490L protein]-[KRAB]-[DNMT3]-[KRAB]; [dCas12f1 R490W protein]-[KRAB]-[DNMT3]-[KRAB]; [dCas12f1 R490A protein]-[KRAB]-[HDAC3]-[KRAB]; [dCas12f1 R490Q protein]-[KRAB]-[HDAC3]-[KRAB]; [dCas12f1 R490L protein]-[KRAB]-[HDAC3]-[KRAB]; or [dCas12f1 R490W protein]-[KRAB]-[HDAC3]-[KRAB]. The transcriptional inhibitor Cas12f1 fusion protein may further comprise at least one NLS. Here, the at least one NLS may be located at the N-terminus of the transcriptional inhibitor Cas12f1 fusion protein, at the C-terminus of the transcriptional inhibitor Cas12f1 fusion protein, between the dCas12f1 protein (the dCas12f1 R490A protein, the dCas12f1 R490Q protein, the dCas12f1 R490L protein, or the dCas12f1 R490W protein) and KRAB, between KRAB and KRAB, between KRAB and MeCP2, between KRAB and DNMT3, and/or between KRAB and HDAC3.

As an example, the transcriptional inhibitor Cas12f1 fusion protein may have the following structure from the N-terminus to the C-terminus: [dCas12f1 D510A protein]-[KRAB]-[KRAB]-[MeCP2]; [dCas12f1 D510L protein]-[KRAB]-[KRAB]-[MeCP2]; [dCas12f1 D510V protein]-[KRAB]-[KRAB]-[MeCP2]; [dCas12f1 D510A protein]-[KRAB]-[KRAB]-[DNMT3]; [dCas12f1 D510L protein]-[KRAB]-[KRAB]-[DNMT3]; [dCas12f1 D510V protein]-[KRAB]-[KRAB]-[DNMT3]; [dCas12f1 D510A protein]-[KRAB]-[KRAB]-[HDAC3]; [dCas12f1 D510L protein]-[KRAB]-[KRAB]-[HDAC3]; [dCas12f1 D510V protein]-[KRAB]-[KRAB]-[HDAC3]; [dCas12f1 D510A protein]-[KRAB]-[MeCP2]-[KRAB]; [dCas12f1 D510L protein]-[KRAB]-[MeCP2]-[KRAB]; [dCas12f1 D510V protein]-[KRAB]-[MeCP2]-[KRAB]; [dCas12f1 D510A protein]-[KRAB]-[DNMT3]-[KRAB]; [dCas12f1 D510L protein]-[KRAB]-[DNMT3]-[KRAB]; [dCas12f1 D510V protein]-[KRAB]-[DNMT3]-[KRAB]; [dCas12f1 D510A protein]-[KRAB]-[HDAC3]-[KRAB]; [dCas12f1 D510L protein]-[KRAB]-[HDAC3]-[KRAB]; or [dCas12f1 D510V protein]-[KRAB]-[HDAC3]-[KRAB]. The transcriptional inhibitor Cas12f1 fusion protein may further comprise at least one NLS. Here, the at least one NLS may be located at the N-terminus of the transcriptional inhibitor Cas12f1 fusion protein, at the C-terminus of the transcriptional inhibitor Cas12f1 fusion protein, between the dCas12f1 protein (the dCas12f1 D510A protein, the dCas12f1 D510L protein, or the dCas12f1 D510V protein) and KRAB, between KRAB and KRAB, between KRAB and MeCP2, between KRAB and DNMT3, and/or between KRAB and HDAC3.

As an example, the transcriptional inhibitor Cas12f1 fusion protein may have the following structure from the N-terminus to the C-terminus: [KRAB]-[KRAB]-[dCas12f1 R490A protein]-[MeCP2]; [KRAB]-[KRAB]-[dCas12f1 R490Q protein]-[MeCP2]; [KRAB]-[KRAB]-[dCas12f1 R490L protein]-[MeCP2]; [KRAB]-[KRAB]-[dCas12f1 R490W protein]-[MeCP2]; [KRAB]-[KRAB]-[dCas12f1 R490A protein]-[DNMT3]; [KRAB]-[KRAB]-[dCas12f1 R490Q protein]-[DNMT3]; [KRAB]-[KRAB]-[dCas12f1 R490L protein]-[DNMT3]; [KRAB]-[KRAB]-[dCas12f1 R490W protein]-[DNMT3]; [KRAB]-[KRAB]-[dCas12f1 R490A protein]-[HDAC3]; [KRAB]-[KRAB]-[dCas12f1 R490Q protein]-[HDAC3]; [KRAB]-[KRAB]-[dCas12f1 R490L protein]-[HDAC3]; [KRAB]-[KRAB]-[dCas12f1 R490W protein]-[HDAC3]; [KRAB]-[MeCP2]-[dCas12f1 R490A protein]-[KRAB]; [KRAB]-[MeCP2]-[dCas12f1 R490Q protein]-[KRAB]; [KRAB]-[MeCP2]-[dCas12f1 R490L protein]-[KRAB]; [KRAB]-[MeCP2]-[dCas12f1 R490W protein]-[KRAB]; [KRAB]-[DNMT3]-[dCas12f1 R490A protein]-[KRAB]; [KRAB]-[DNMT3]-[dCas12f1 R490Q protein]-[KRAB]; [KRAB]-[DNMT3]-[dCas12f1 R490L protein]-[KRAB]; [KRAB]-[DNMT3]-[dCas12f1 R490W protein]-[KRAB]; [KRAB]-[HDAC3]-[dCas12f1 R490A protein]-[KRAB]; [KRAB]-[HDAC3]-[dCas12f1 R490Q protein]-[KRAB]; [KRAB]-[HDAC3]-[dCas12f1 R490L protein]-[KRAB]; or [KRAB]-[HDAC3]-[dCas12f1 R490W protein]-[KRAB]. The transcriptional inhibitor Cas12f1 fusion protein may further comprise at least one NLS. Here, the at least one NLS may be located at the N-terminus of the transcriptional inhibitor Cas12f1 fusion protein, at the C-terminus of the transcriptional inhibitor Cas12f1 fusion protein, between the dCas12f1 protein (the dCas12f1 R490A protein, the dCas12f1 R490Q protein, the dCas12f1 R490L protein, or the dCas12f1 R490W protein) and KRAB, between the dCas12f1 protein (the dCas12f1 R490A protein, the dCas12f1 R490Q protein, the dCas12f1 R490L protein, or the dCas12f1 R490W protein) and MeCP2, between the dCas12f1 protein (the dCas12f1 R490A protein, the dCas12f1 R490Q protein, the dCas12f1 R490L protein, or the dCas12f1 R490W protein) and DNMT3, between the dCas12f1 protein (the dCas12f1 R490A protein, the dCas12f1 R490Q protein, the dCas12f1 R490L protein, or the dCas12f1 R490W protein) and HDAC3, between KRAB and KRAB, between KRAB and MeCP2, between KRAB and DNMT3, and/or between KRAB and HDAC3.

As an example, the transcriptional inhibitor Cas12f1 fusion protein may have the following structure from the N-terminus to the C-terminus: [KRAB]-[KRAB]-[dCas12f1 D510A protein]-[MeCP2]; [KRAB]-[KRAB]-[dCas12f1 D510L protein]-[MeCP2]; [KRAB]-[KRAB]-[dCas12f1 D510V protein]-[MeCP2]; [KRAB]-[KRAB]-[dCas12f1 D510A protein]-[DNMT3]; [KRAB]-[KRAB]-[dCas12f1 D510L protein]-[DNMT3]; [KRAB]-[KRAB]-[dCas12f1 D510V protein]-[DNMT3]; [KRAB]-[KRAB]-[dCas12f1 D510A protein]-[HDAC3]; [KRAB]-[KRAB]-[dCas12f1 D510L protein]-[HDAC3]; [KRAB]-[KRAB]-[dCas12f1 D510V protein]-[HDAC3]; [KRAB]-[MeCP2]-[dCas12f1 D510A protein]-[KRAB]; [KRAB]-[MeCP2]-[dCas12f1 D510L protein]-[KRAB]; [KRAB]-[MeCP2]-[dCas12f1 D510V protein]-[KRAB]; [KRAB]-[DNMT3]-[dCas12f1 D510A protein]-[KRAB]; [KRAB]-[DNMT3]-[dCas12f1 D510L protein]-[KRAB]; [KRAB]-[DNMT3]-[dCas12f1 D510V protein]-[KRAB]; [KRAB]-[HDAC3]-[dCas12f1 D510A protein]-[KRAB]; [KRAB]-[HDAC3]-[dCas12f1 D510L protein]-[KRAB]; or [KRAB]-[HDAC3]-[dCas12f1 D510V protein]-[KRAB]. The transcriptional inhibitor Cas12f1 fusion protein may further comprise at least one NLS. Here, the at least one NLS may be located at the N-terminus of the transcriptional inhibitor Cas12f1 fusion protein, at the C-terminus of the transcriptional inhibitor Cas12f1 fusion protein, between the dCas12f1 protein (the dCas12f1 D510A protein, the dCas12f1 D510L protein, or the dCas12f1 D510V protein) and KRAB, between the dCas12f1 protein (the dCas12f1 D510A protein, the dCas12f1 D510L protein, or the dCas12f1 D510V protein) and MeCP2, between the dCas12f1 protein (the dCas12f1 D510A protein, the dCas12f1 D510L protein, or the dCas12f1 D510V protein) and DNMT3, between the dCas12f1 protein (the dCas12f1 D510A protein, the dCas12f1 D510L protein, or the dCas12f1 D510V protein) and HDAC3, between KRAB and KRAB, between KRAB and MeCP2, between KRAB and DNMT3, and/or between KRAB and HDAC3.

### 2. Engineered guide RNA

The CRISPR regulatory system provided herein comprises a guide RNA of a CRISPR/Cas12f1 system. The guide RNA may be a naturally occurring wild-type Cas12f1 guide RNA or a Cas12f1 guide RNA engineered to improve efficiency of the CRISPR/Cas12f1 system.

The Cas12f1 guide RNA can be largely divided into a spacer and a scaffold region, and the scaffold region consists of five stems (named Stem 1 to Stem 5) and one pseudoknot (PK). The Cas12f1 guide RNA includes two structures in which a part of tracrRNA (tracrRNA anti-repeat) and a part of a crRNA repeat portion are complementarily bound to form a duplex, and this is named a crRNA repeat-tracrRNA anti-repeat (R:AR) portion. The Stem 5 (R:AR2), and PK (R:AR1) form this crRNA repeat-tracrRNA anti-repeat duplex structure.

The CRISPR regulatory system provided herein may use an engineered Cas12f1 guide RNA that targets a transcriptional regulatory region of a gene whose expression is to be regulated, that is, a target gene. More specifically, the CRISPR regulatory system comprises an engineered Cas12f1 guide RNA that complementarily binds to a target sequence present in a transcriptional regulatory region of a target gene. Here, the transcriptional regulatory region of the target gene comprises all regions that regulate transcription of the target gene, such as a promoter, an enhancer, a promoter-proximal element, an operator, and a silencer. The engineered Cas12f1 guide RNA included in the CRISPR regulatory system is characterized by forming a complex with the Cas12f1 fusion protein and causing the Cas12f1 fusion protein to be located in the transcriptional regulatory region of the target gene.

In the present disclosure, there is provided an engineered Cas12f1 guide RNA for the CRISPR regulatory system. The engineered Cas12f1 guide RNA is a naturally occurring Cas12f1 guide RNA to which a new component is added and also in which a part of its structure is modified. The engineered Cas12f1 guide RNA is characterized by comprising a U-rich tail, which is a new component, at the 3' end. In addition, the engineered Cas12f1 guide RNA is characterized in that at least a portion of the scaffold region, which serves to interact with a Cas12f1 protein, is modified.

In an embodiment, the engineered Cas12f1 guide RNA may comprise an engineered scaffold region, a spacer, and a U-rich tail. Here, the engineered scaffold region is characterized by being different from a scaffold region of a naturally occurring guide RNA.

### Characteristic 1 of engineered Cas12f1 guide RNA - Inclusion of U-rich tail

The engineered Cas12f1 guide RNA provided herein is characterized in that a U-rich tail is added to a naturally occurring guide RNA. The U-rich tail is located at the 3' end portion of the engineered Cas12f1 guide RNA and is a portion rich in uridine.

In an embodiment, the engineered Cas12f1 guide RNA may comprise a U-rich tail, which is rich in uridine, at the 3' end portion. In an embodiment, a sequence of the U-rich tail may be represented by (UₐN)_{b}U_{c}. Here, N may be one of A, U, C, or G, and a, b, and c are each an integer, with a being between 1 and 5 inclusive, b being between 0 and 2 inclusive, and c being between 1 and 10 inclusive.

### Characteristic 2 of engineered Cas12f1 guide RNA - One or more parts of scaffold region being modified

The engineered Cas12f1 guide RNA provided herein is characterized in that a part of its scaffold region is modified as compared with a naturally occurring guide RNA. The scaffold region comprises a tracrRNA and a part of a crRNA and has a function of interacting with a Cas12f1 protein. The scaffold region will be described in more detail below.

In an embodiment, the engineered Cas12f1 guide RNA may comprise an engineered scaffold region. Here, the engineered scaffold region is obtained by modifying a scaffold region of a naturally occurring guide RNA. Therefore, the engineered scaffold region has a different sequence from the scaffold region of the naturally occurring guide RNA. In an embodiment, the engineered scaffold region may be obtained by removing a part of a scaffold region of a naturally occurring guide RNA. In an embodiment, the engineered scaffold region may be obtained by removing one or more nucleotides included in a scaffold region of a naturally occurring guide RNA.

### Effect of engineered Cas12f1 guide RNA

By using an engineered Cas12f1 guide RNA provided herein, the CRISPR/Cas12f1 system show dramatically improved gene editing activity in a cell as compared with when a naturally occurring guide RNA is used. In addition, the engineered Cas12f1 guide RNA has a length equal to or shorter than a naturally occurring guide RNA, and thus has a high potential for application in the field of gene editing technology. The engineered Cas12f1 guide RNA makes it possible to fully utilize the advantages of the CRISPR/Cas12f1 system (for example, the advantage of having a very small size) in gene editing and gene expression regulation techniques

### Use of engineered Cas12f1 guide RNA

The engineered Cas12f1 guide RNA provided herein may be used for regulating gene expression together with a Cas12f1 protein. In addition, the engineered Cas12f1 guide RNA may be used for preparing a gene expression regulatory composition.

Hereinafter, the configuration and various embodiments of the engineered Cas12f1 guide RNA will be described.

### 1) U-Rich tail

### Overview of U-rich tail

In the present disclosure, there is provided a U-rich tail that can be introduced into a CRISPR regulatory system to improve efficiency thereof. A sequence of the U-rich tail is characterized by being linked to the 3' end of a spacer of a crRNA in the engineered Cas12f1 guide RNA, and this sequence serves to increase editing efficiency, against a target nucleic acid, of a CRISPR/Cas12f1 system in which the engineered Cas12f1 guide RNA is used. The U-rich tail sequence is basically rich in uridine and comprises a sequence having one or more consecutive uridines. The U-rich tail sequence may further comprise an additional nucleotide in addition to uridine depending on an actual environment in which the engineered CRISPR/Cas12f1 system is used and expression occurs (for example, an environment in a eukaryotic cell or a prokaryotic cell).

The U-rich tail sequence may be a U-rich tail sequence disclosed in the international application PCT/KR2020/014961. Hereinafter, when referring to the U-rich tail sequence herein, it should be understood as including all of the contents and experimental results related to the U-rich tail sequence disclosed in the international application PCT/KR2020/014961.

### Structure 1 of U-Rich tail sequence - Uridine repeat sequence

One of the important factors in designing the U-rich tail sequence is to ensure that it abundantly contains a sequence having one or more consecutive uridines. The present inventors have found through experiments that introduction of a U-rich tail sequence, which is a sequence having one or more consecutive uridines, into a CRISPR/Cas12f1 system enables the CRISPR/Cas12f1 complex to show improved gene editing efficiency. Accordingly, the U-rich tail sequence provided herein comprises a sequence having one or more consecutive uridines.

In an embodiment, the U-rich tail sequence may comprise a sequence having 1, 2, 3, 4, 5, 6, 7, 8, 9, 10, 11, 12, 13, 14, 15, 16, 17, 18, 19, or 20 consecutive uridines.

### Structure 2 of U-rich tail sequence- Modified uridine repeat sequence

The U-rich tail sequence provided herein may comprise a modified uridine repeat sequence that contains one of ribonucleosides (A, C, and G) other than uridine for every repetition of 1 to 5 uridines. The modified uridine repeat sequence is particularly useful when designing a vector expressing an engineered crRNA.

In an embodiment, the U-rich tail sequence may comprise a sequence in which one or more UV, UUV, UUUV, UUUUV, and/or UUUUUV are repeated. Here, V is one of adenosine (A), cytidine (C), and guanosine (G).

### U-rich tail sequence - Embodiments

In an embodiment, the U-rich tail sequence may be represented by (UₐN)_{b}U_{c}. Here, N is one of adenosine (A), uridine (U), cytidine (C), and guanosine (G). Here, a, b, and c are each an integer, with a being 1 to 5, and b being 0 or greater. In an embodiment, b may be 0 to 2 inclusive. In an embodiment, c may be 0, 1, 2, 3, 4, 5, 6, 7, 8, 9, or 10. In an embodiment, c may be an integer within a range of two numbers selected from the immediately preceding sentence. For example, c may be 1 to 6 inclusive.

### Examples of U-rich tail sequence

In an embodiment, a sequence of the U-rich tail may be 5'-U-3', 5'-UU-3', 5'-UUU-3', 5'-UUUU-3', 5'-UUUUU-3', 5'-UUUUUU-3', 5'-UUURUUU-3', 5'-UUURUUURUUU-3' (SEQ ID NO: 646), 5'-UUUURU-3', 5'-UUUURUU-3', 5'-UUUURUUU-3', 5'-UUUURUUUU-3', 5'-UUUURUUUUU-3' (SEQ ID NO: 647), or 5'-UUUURUUUUUU-3' (SEQ ID NO: 648). In an embodiment, a sequence of the U-rich tail may be 5'-U-3', 5'-UU-3', 5'-UUU-3', 5'-UUUU-3', 5'-UUUUU-3', 5'-UUUUUU-3', 5'-UUUAUUU-3', 5'-UUUAUUUAUUU-3' (SEQ ID NO: 254), 5'-UUUUAU-3', 5'-UUUUAUU-3', 5'-UUUUAUUU-3', 5'-UUUUAUUUU-3', 5'-UUUUAUUUUU-3' (SEQ ID NO: 255), 5'-UUUUAUUUUUU-3' (SEQ ID NO: 256), 5'-UUUGUUU-3', 5'-UUUGUUUGUUU-3' (SEQ ID NO: 257), 5'-UUUUGU-3', 5'-UUUUGUU-3', 5'-UUUUGUUU-3', 5'-UUUUGUUUU-3', 5'-UUUUGUUUUU-3' (SEQ ID NO: 258), or 5'-UUUUGUUUUUU-3' (SEQ ID NO: 259).

In an embodiment, a sequence of the U-rich tail may be 5'-UUUUUU-3', 5'-UUUUAUUUUUU-3' (SEQ ID NO: 256), or 5'-UUUUGUUUUUU-3' (SEQ ID NO: 259). In an embodiment, a sequence of the U-rich tail may be 5'-UUUUAUUUU-3'.

### 2) Explanation of terms - Terms referring to parts of Cas12f1 guide RNA

### Parts of Cas12f1 guide RNA - Overview

It is well known to those of ordinary skill in the art that the naturally occurring Cas12f1 guide RNA is divided into a tracrRNA and a crRNA, in which the crRNA may be further divided into a crRNA repeat sequence and a spacer.

Apart from the above criteria, in the present disclosure, parts of the Cas12f1 guide RNA, which interacts with a Cas12f1 protein, are collectively referred to as a scaffold region. The scaffold region comprises a tracrRNA and a part of a crRNA and does not necessarily refer to a single molecule of RNA. The scaffold region may be further subdivided into a first region, a second region, a third region, a fourth region, a fifth region, and a sixth region. When the subdivided regions are described with respect to the tracrRNA and crRNA, the first to fourth regions are included in the tracrRNA, and the fifth to sixth regions are included in the crRNA, specifically, in the crRNA repeat sequence portion.

The "n-th region" or "naturally occurring n-th region" (n is an integer between 1 to 6 inclusive) as described below basically refers to each part of the naturally occurring Cas12f1 guide RNA. The region in an engineered Cas12f1 guide RNA, which corresponds to the above classification criteria, is generally described as "modified n-th region" or "n-th region of an engineered scaffold region."

However, an n-th region included in an engineered scaffold region may not be modified and thus may be identical to a naturally occurring n-th region, and only in that case, the term "n-th region" may be used interchangeably. Here, what is referred to by the "n-th region" (for example, whether it is a region included in an engineered Cas12f1 guide RNA or a region included in a naturally occurring guide RNA) should be appropriately interpreted depending on the context.

### tracrRNA and crRNA

As used herein, the terms "tracrRNA" and "crRNA" include all meanings that can be recognized by those of ordinary skill in the field of CRISPR/Cas technology. The terms are generally used to refer to respective molecules of a naturally occurring dual guide RNA, and may also be used to refer to respective corresponding parts of a single guide RNA in which a tracrRNA and a crRNA are linked via a linker. Unless otherwise specified, in a case of being merely written as "tracrRNA" and "crRNA", the terms refer to a tracrRNA and a crRNA constituting a CRISPR/Cas12f1 system, respectively.

In an embodiment, a sequence of the tracrRNA may be 5'-CUUCACUGAUAAAGUGGAGAACCGCUUCACCAAAAGCUGUCCCUUAGGGGAUUAGA ACUUGAGUGAAGGUGGGCUGCUUGCAUCAGCCUAAUGUCGAGAAGUGCUUUCUUC GGAAAGUAACCCUCGAAACAAAUUCAUUU-3' (SEQ ID NO: 1) or 5'-CUUCACUGAUAAAGUGGAGAACCGCUUCACCAAAAGCUGUCCCUUAGGGGAUUAGA ACUUGAGUGAAGGUGGGCUGCUUGCAUCAGCCUAAUGUCGAGAAGUGCUUUCUUC GGAAAGUAACCCUCGAAACAAAUUCAUUUUUCCUCUCCAAUUCUGCACAA-3' (SEQ ID NO: 2). In an embodiment, the tracrRNA comprises a first region, a second region, a third region, and a fourth region. In an embodiment, the tracrRNA is one in which the first region, the second region, the third region, and the fourth region are sequentially linked to each other in a 5' to 3' direction.

In an embodiment, a sequence of the crRNA comprises a crRNA repeat sequence and a spacer sequence. Here, the crRNA repeat sequence may be 5'-GAAUGAAGGAAUGCAAC-3' (SEQ ID NO: 3) or 5'-GUUGCAGAACCCGAAUAGACGAAUGAAGGAAUGCAAC-3' (SEQ ID NO: 4). The crRNA repeat sequence comprises a fifth region and a sixth region. The spacer sequence may vary depending on a target sequence, and generally comprises 10 to 50 nucleotides. In an embodiment, the crRNA is one in which the fifth region, the sixth region, and the spacer are sequentially linked to each other in a 5' to 3' direction (SEQ ID NO: 5 or 6).

### Scaffold region - Overview

As used herein, the term "scaffold region" refers collectively to the rest of a naturally occurring guide RNA excluding the spacer. Specifically, the scaffold region comprises the tracrRNA, and a part of the crRNA. Specifically, the part of the crRNA may be a crRNA repeat sequence portion. The scaffold region is generally known as a portion capable of interacting with a Cas protein. In the present disclosure, the scaffold region is divided into first to sixth regions for description, and each region will be described in more detail below.

### Scaffold region 1 - First region

As used herein, the term "first region" refers to a region comprising the 5' end of the tracrRNA. The first region may comprise nucleotides forming a stem structure in the CRISPR/Cas12f1 complex, and may comprise nucleotides adjacent thereto.

The first region comprises a Stem 1 portion (Takeda et al., Structure of the miniature type V-F CRISPR-Cas effector enzyme, Molecular Cell 81, 1-13 (2021)). The first region may comprise one or more nucleotides adjacent to the Stem 1 portion.

The first region comprises a disordered region that does not interact with a Cas12f1 protein in the CRISPR/Cas12f1 complex.

In an embodiment, the first region may refer to the 1^{st} to 11^{th} nucleotides from the 5' end of the tracrRNA represented by SEQ ID NO: 1 or 2. In an embodiment, a sequence of the first region may be 5'-CUUCACUGAUAAAGUGGAGAA-3' (SEQ ID NO: 10).

### Scaffold Region 2 - Second region

As used herein, the term "second region" refers to a region located at the 3' end of the first region in the tracrRNA. The second region may comprise nucleotides forming a stem structure in a CRISPR/Cas12f1 complex and may comprise nucleotides adjacent thereto. Here, the stem structure is different from the stem included in the first region.

The second region comprises a Stem 2 portion (Takeda et al., Structure of the miniature type V-F CRISPR-Cas effector enzyme, Molecular Cell 81, 1-13 (2021)). The second region may comprise one or more nucleotides adjacent to the Stem 2 portion.

The second region may comprise one or more nucleotides that interact with a RuvC domain of one dimer-forming Cas12f1 protein and/or a RuvC domain of the other dimer-forming Cas12f1 protein in the CRISPR/Cas12f1 complex. The second region comprises a disordered region that does not interact with a Cas12f1 protein in the CRISPR/Cas12f1 complex.

In an embodiment, the second region may refer to the 22^{nd} to 72^{nd} nucleotides from the 5' end of the tracrRNA represented by SEQ ID NO: 1 or 2. In an embodiment, a sequence of the second region may be 5'-CCGCUUCACCAAAAGCUGUCCCUUAGGGGAUUAGAACUUGAGUGAAGGUGG-3' (SEQ ID NO: 11).

### Scaffold region 3 - Third region

As used herein, the term "third region" refers to a region located at the 3' end of the second region in a tracrRNA. The third region may comprise nucleotides forming a stem structure in the CRISPR/Cas12f1 complex and nucleotides forming complementary bonds with some nucleotides included in the crRNA and may comprise nucleotides adjacent thereto.

The third region comprises nucleotides that belong to the tracrRNA in a Stem 4 portion (Takeda et al., Structure of the miniature type V-F CRISPR-Cas effector enzyme, Molecular Cell 81, 1-13 (2021)) and a Stem 3-PK (R:AR-1) portion (Takeda et al., Structure of the miniature type V-F CRISPR-Cas effector enzyme, Molecular Cell 81, 1-13 (2021)). The third region may comprise one or more nucleotides adjacent to the nucleotides which belong to the tracrRNA in the Stem 4 portion and/or the Stem 3-PK (R:AR-1) portion.

The third region comprises one or more nucleotides that interact with a WED domain and/or a RuvC domain of one dimer-forming Cas12f1 protein in the CRISPR/Cas12f1 complex. Here, the nucleotides may be nucleotides which belong to the tracrRNA in the Stem 3-PK (R:AR-1) portion.

The third region comprises one or more nucleotides that interact with a RuvC domain of one dimer-forming Cas12f1 protein and/or an REC domain of the other dimer-forming Cas12f1 protein in the CRISPR/Cas12f1 complex. Here, the nucleotides may be nucleotides included in the Stem 4 portion.

The third region may comprise one or more nucleotides complementarily binding to one or more nucleotides included in the sixth region of the crRNA.

In an embodiment, the third region may refer to the 73^{rd} to 127^{th} nucleotides from the 5' end of the tracrRNA represented by SEQ ID NO: 1 or 2. In an embodiment, a sequence of the third region may be 5'-GCUGCUUGCAUCAGCCUAAUGUCGAGAAGUGCUUUCUUCGGAAAGUAACCCUCGA-3' (SEQ ID NO: 12).

### Scaffold region 4 - Fourth region

As used herein, the term "fourth region" refers to a region located at the 3' end of the third region in the tracrRNA. The fourth region may comprise nucleotides capable of forming complementary bonds with some nucleotides included in the crRNA in the CRISPR/Cas12f1 complex and may comprise nucleotides adjacent thereto.

The fourth region comprises nucleotides which belong to the tracrRNA in Stem 5 (R:AR-2) (Takeda et al., Structure of the miniature type V-F CRISPR-Cas effector enzyme, Molecular Cell 81, 1-13 (2021)). The fourth region may comprise one or more nucleotides adjacent to the nucleotides which belong to the tracrRNA in Stem 5 (R:AR-2).

The fourth region comprises one or more nucleotides that interact with a WED domain and/or a ZF domain of one dimer-forming Cas12f1 protein in the CRISPR/Cas12f1 complex. Here, the nucleotides may be nucleotides which belong to the tracrRNA in Stem 5 (R:AR-2).

The fourth region may comprise one or more nucleotides complementarily binding to one or more nucleotides included in the fifth region of the crRNA. The fourth region comprises a disordered region that does not interact with a Cas12f1 protein in a CRISPR/Cas12f1 complex.

In an embodiment, the fourth region may refer to the 128^{th} to 140^{th} nucleotides from the 5' end of the tracrRNA represented by SEQ ID NO: 1. In an embodiment, the fourth region may refer to the 128^{th} to 162^{nd} nucleotides from the 5' end of the tracrRNA represented by SEQ ID NO: 2.

In an embodiment, a sequence of the fourth region may be 5'-AACAAAUUCAUUU-3' (SEQ ID NO: 13) or 5'-AACAAAUUCAUUUUUCCUCUCCAAUUCUGCACAA-3' (SEQ ID NO: 14).

### Scaffold region 5 - Fifth region

As used herein, the term "fifth region" refers to a region comprising the 5' end of the crRNA. The fifth region may comprise nucleotides that form complementary bonds with one or more nucleotides of the fourth region in a CRISPR/Cas12f1 complex and may comprise any nucleotide adjacent thereto.

The fifth region comprises nucleotides which belong to the crRNA in Stem 5 (R:AR-2) (Takeda et al., Structure of the miniature type V-F CRISPR-Cas effector enzyme, Molecular Cell 81, 1-13 (2021)). The fifth region may comprise any one or more nucleotides adjacent to the nucleotides which belong to the crRNA in Stem 5 (R:AR-2).

The fifth region comprises one or more nucleotides that interact with a WED domain, an REC domain, and/or a ZF domain of one dimer-forming Cas12f1 protein in a CRISPR/Cas12f1 complex. Here, the nucleotides may be nucleotides which belong to the crRNA in Stem 5 (R:AR-2).

The fifth region may comprise one or more nucleotides complementarily binding to one or more nucleotides included in the fourth region. The fifth region comprises a disordered region that does not interact with a Cas12f1 protein in the CRISPR/Cas12f1 complex.

In an embodiment, the fifth region may refer to the 1^{st} to 10^{th} nucleotides from the 5' end of the crRNA represented by SEQ ID NO: 3. In an embodiment, the fifth region may refer to the 1^{st} to 30^{th} nucleotides from the 5' end of the crRNA represented by SEQ ID NO: 4. In an embodiment, a sequence of the fifth region may be 5'-GAAUGAAGGA-3' (SEQ ID NO: 15) or 5'-GUUGCAGAACCCGAAUAGACGAAUGAAGGA-3' (SEQ ID NO: 16).

### Scaffold region 6 - Sixth region

As used herein, the term "sixth region" refers to a region located at the 3' end of the fifth region in the crRNA. The sixth region may comprise nucleotides that form complementary bonds with one or more nucleotides of the third region in a CRISPR/Cas12f1 complex, and may comprise any nucleotide adjacent thereto.

The sixth region comprises nucleotides which belong to the crRNA in Stem 3-PK (R:AR-1) (Takeda et al., Structure of the miniature type V-F CRISPR-Cas effector enzyme, Molecular Cell 81, 1-13 (2021)). The sixth region may comprise any one or more nucleotides adjacent to the nucleotides which belong to the crRNA in Stem 3-PK (R:AR-1).

The sixth region comprises one or more nucleotides that interact with a WED domain, a ZF domain, and/or a RuvC domain of one dimer-forming Cas12f1 protein in the CRISPR/Cas12f1 complex. Here, the nucleotides may be nucleotides which belong to the crRNA in Stem 3-PK (R:AR-1).

In an embodiment, the sixth region may refer to the 11^{th} to 17^{th} nucleotides from the 5' end of the crRNA represented by SEQ ID NO: 3. In an embodiment, the sixth region may refer to the 31^{st} to 37^{th} nucleotides from the 5' end of the crRNA represented by SEQ ID NO: 4. In an embodiment, a sequence of the sixth region may be 5'-AUGCAAC-3'.

### Spacer

As used herein, the term "spacer" as used herein refers to one or more nucleotides which hybridize with a target sequence in a CRISPR/Cas12f1 system. The spacer refers to 10 to 50 consecutive nucleotides near the 3' end of the crRNA of the guide RNA in the CRISPR/Cas12f1 system. The spacer is designed to match a target sequence in the target nucleic acid to be edited using the CRISPR/Cas12f1 system. In other words, the spacer may have a different sequence depending on a target sequence of the target nucleic acid.

### 3) Engineered scaffold region - Overview

### Overview of engineered scaffold region

In the present disclosure, there is provided an engineered scaffold region that can be introduced into a CRISPR regulatory system to improve its targeting efficiency in a target gene. The engineered scaffold region synergizes with the above-described U-rich tail to improve targeting efficiency of a CRISPR regulatory system, in which an engineered Cas12f1 guide RNA is used, in a target gene. The engineered scaffold region is characterized in that it is obtained by applying one or more mutations in the scaffold region of a naturally occurring Cas12f1 guide RNA (hereinafter, naturally occurring scaffold region), and thus is different therefrom in terms of sequence and/or structure.

Here, functions of the engineered scaffold region are identical or similar to those of the naturally occurring scaffold region.

In an embodiment, the engineered scaffold region comprises regions corresponding to respective portions of the naturally occurring scaffold region. Specifically, the engineered scaffold region comprises a first region, a second region, a third region, a fourth region, a fifth region, and a sixth region, which respectively correspond to the first to sixth regions included in the naturally occurring scaffold region.

In an embodiment, the engineered scaffold region may not comprise regions corresponding to the first region and/or the second region included in the naturally occurring scaffold region.

### Modifications to make single guide RNA

The engineered Cas12f1 guide RNA provided herein may be a single guide RNA of one molecule. Accordingly, the engineered scaffold region provided herein may have a modification(s) in one or more of the respective regions, and additionally, the 3' end of the fourth region of the tracrRNA and the 5' end of the fifth region of the crRNA may be linked via a linker.

In an embodiment, the engineered scaffold region may be an engineered form of a naturally occurring scaffold region in which one or more regions are modified and the 3' end of the fourth region and the 5' end of the fifth region are linked via a linker. Here, the linker may be 5'-GAAA-3'.

### 4) Engineered scaffold region 1 - Modification of first region

### Overview of modification of first region

The engineered scaffold region provided herein may be an engineered form of a naturally occurring scaffold region in which the first region is modified.

In an embodiment, the engineered scaffold region may comprise a modified first region. Here, the modified first region is obtained by removing one or more nucleotides from the first region of the naturally occurring scaffold region. Here, the removed nucleotide(s) is a nucleotide(s) selected from a region forming a stem structure in a CRISPR/Cas12f1 complex.

In an embodiment, the engineered scaffold region included in the engineered Cas12f1 guide RNA may include an engineered form of a naturally occurring scaffold region in which one or more nucleotides included in the first region are removed. In an embodiment, the removed nucleotide(s) may be a nucleotide(s) included in a portion, which forms a stem structure in the CRISPR/Cas12f1 complex, in the naturally occurring first region. In an embodiment, the removed nucleotide(s) may be a nucleotide(s), which belong to Stem 1 (Takeda et al., Structure of the miniature type V-F CRISPR-Cas effector enzyme, Molecular Cell 81, 1-13 (2021)), in the naturally occurring first region. In an embodiment, the removed nucleotide(s) may be a nucleotide(s), which does not interact with a Cas12f1 protein in the CRISPR/Cas12f1 complex, in the naturally occurring first region.

In an embodiment, the modified first region comprises the sequence 5'-A-3' at the 3' end.

In an embodiment, the engineered scaffold region may be an engineered form of a naturally occurring scaffold region from which the first region is removed. In other words, the engineered scaffold region may not comprise a region corresponding to the first region of the naturally occurring scaffold region.

### Modification detail 1 of first region - Removal of some nucleotides

The first region of the engineered scaffold region may be a modified form of a first region of the naturally occurring scaffold region from which one or more nucleotides are removed.

In an embodiment, the modified first region of the engineered scaffold region may be a modified form of a first region of the naturally occurring scaffold region from which 1 to 20 nucleotides at the 5' end are removed. In an embodiment, the modified first region may be a modified form of a first region of the naturally occurring scaffold region from which 1, 2, 3, 4, 5, 6, 7, 8, 9, 10, 11, 12, 13, 14, 15, 16, 17, 18, 19, or 20 consecutive nucleotides at the 5' end are removed. In an embodiment, the modified first region may be a modified form of a first region of the naturally occurring scaffold region from which consecutive nucleotides at the 5' end, the number of which is within a range of two numbers selected from the immediately preceding sentence, are removed. For example, the modified first region may be a modified form of a first region of the naturally occurring scaffold region from which 1 to 3 consecutive nucleotides at the 5' end are removed.

In an embodiment, the modified first region comprises at least one nucleotide, which may be 5'-A-3'.

### Modification detail 2 of first region - Removal of first region

The engineered scaffold region provided herein may be a modified form of a naturally occurring scaffold region from which the first region is removed.

In an embodiment, the engineered scaffold region may not comprise a region corresponding to the first region of the naturally occurring scaffold region.

### Examples of engineered scaffold sequences in which first region is modified

In an embodiment, a sequence of the modified first region may be selected from 5'-A-3', 5'-AA-3', 5'-GAA-3', 5'-AGAA-3', 5'-GAGAA-3', 5'-GGAGAA-3', 5'-UGGAGAA-3', 5'-GUGGAGAA-3', 5'-AGUGGAGAA-3', 5'-AAGUGGAGAA-3' (SEQ ID NO: 17), 5'-AAAGUGGAGAA-3' (SEQ ID NO: 18), 5'-UAAAGUGGAGAA-3' (SEQ ID NO: 19), 5'-AUAAAGUGGAGAA-3' (SEQ ID NO: 20), 5'-GAUAAAGUGGAGAA-3' (SEQ ID NO: 21), 5'-UGAUAAAGUGGAGAA-3' (SEQ ID NO: 22), 5'-CUGAUAAAGUGGAGAA-3' (SEQ ID NO: 23), 5'-ACUGAUAAAGUGGAGAA-3' (SEQ ID NO: 24), 5'-CACUGAUAAAGUGGAGAA-3' (SEQ ID NO: 25), 5'-UCACUGAUAAAGUGGAGAA-3' (SEQ ID NO: 26), and 5'-UUCACUGAUAAAGUGGAGAA-3' (SEQ ID NO: 27).

In an embodiment, a sequence of the engineered scaffold region in which the first region is modified may comprise:
a sequence in which the following sequences are linked to each other in a 5' to 3' direction:
a sequence selected from the group consisting of 5'-A-3', 5'-AA-3', 5'-GAA-3', 5'-AGAA-3', 5'-GAGAA-3', 5'-GGAGAA-3', 5'-UGGAGAA-3', 5'-GUGGAGAA-3', 5'-AGUGGAGAA-3', and SEQ ID NOs: 17 to 27,
SEQ ID NO: 11,
SEQ ID NO: 12, and
SEQ ID NO: 13; and
a sequence in which SEQ ID NO: 15 and 5'-AUGCAAC-3' are linked to each other in a 5' to 3' direction.

In an embodiment, a sequence of the engineered scaffold region in which the first region is modified may comprise:
a sequence selected from the group consisting of 5'-ACCGCUUCACCAAAAGCUGUCCCUUAGGGGAUUAGAACUUGAGUGAAGGUGGGCUG CUUGCAUCAGCCUAAUGUCGAGAAGUGCUUUCUUCGGAAAGUAACCCUCGAAACAA AUUCAUUU-3' (SEQ ID NO: 119), 5'-AACCGCUUCACCAAAAGCUGUCCCUUAGGGGAUUAGAACUUGAGUGAAGGUGGGCU GCUUGCAUCAGCCUAAUGUCGAGAAGUGCUUUCUUCGGAAAGUAACCCUCGAAACA AAUUCAUUU-3' (SEQ ID NO: 120), 5'-GAACCGCUUCACCAAAAGCUGUCCCUUAGGGGAUUAGAACUUGAGUGAAGGUGGGC UGCUUGCAUCAGCCUAAUGUCGAGAAGUGCUUUCUUCGGAAAGUAACCCUCGAAAC AAAUUCAUUU-3' (SEQ ID NO: 121), 5'-AGAACCGCUUCACCAAAAGCUGUCCCUUAGGGGAUUAGAACUUGAGUGAAGGUGGG CUGCUUGCAUCAGCCUAAUGUCGAGAAGUGCUUUCUUCGGAAAGUAACCCUCGAAA CAAAUUCAUUU-3' (SEQ ID NO: 122), 5'-GAGAACCGCUUCACCAAAAGCUGUCCCUUAGGGGAUUAGAACUUGAGUGAAGGUGG GCUGCUUGCAUCAGCCUAAUGUCGAGAAGUGCUUUCUUCGGAAAGUAACCCUCGAA ACAAAUUCAUUU-3' (SEQ ID NO: 123), 5'-GGAGAACCGCUUCACCAAAAGCUGUCCCUUAGGGGAUUAGAACUUGAGUGAAGGUG GGCUGCUUGCAUCAGCCUAAUGUCGAGAAGUGCUUUCUUCGGAAAGUAACCCUCGA AACAAAUUCAUUU-3' (SEQ ID NO: 124), 5'-UGGAGAACCGCUUCACCAAAAGCUGUCCCUUAGGGGAUUAGAACUUGAGUGAAGGU GGGCUGCUUGCAUCAGCCUAAUGUCGAGAAGUGCUUUCUUCGGAAAGUAACCCUCG AAACAAAUUCAUUU-3' (SEQ ID NO: 125), 5'-GUGGAGAACCGCUUCACCAAAAGCUGUCCCUUAGGGGAUUAGAACUUGAGUGAAGG UGGGCUGCUUGCAUCAGCCUAAUGUCGAGAAGUGCUUUCUUCGGAAAGUAACCCUC GAAACAAAUUCAUUU-3' (SEQ ID NO: 126), 5'-AGUGGAGAACCGCUUCACCAAAAGCUGUCCCUUAGGGGAUUAGAACUUGAGUGAAG GUGGGCUGCUUGCAUCAGCCUAAUGUCGAGAAGUGCUUUCUUCGGAAAGUAACCCU CGAAACAAAUUCAUUU-3' (SEQ ID NO: 127), 5'-AAGUGGAGAACCGCUUCACCAAAAGCUGUCCCUUAGGGGAUUAGAACUUGAGUGAA GGUGGGCUGCUUGCAUCAGCCUAAUGUCGAGAAGUGCUUUCUUCGGAAAGUAACC CUCGAAACAAAUUCAUUU-3' (SEQ ID NO: 128), 5'-AAAGUGGAGAACCGCUUCACCAAAAGCUGUCCCUUAGGGGAUUAGAACUUGAGUGA AGGUGGGCUGCUUGCAUCAGCCUAAUGUCGAGAAGUGCUUUCUUCGGAAAGUAACC CUCGAAACAAAUUCAUUU-3' (SEQ ID NO: 129), 5'-UAAAGUGGAGAACCGCUUCACCAAAAGCUGUCCCUUAGGGGAUUAGAACUUGAGUG AAGGUGGGCUGCUUGCAUCAGCCUAAUGUCGAGAAGUGCUUUCUUCGGAAAGUAAC CCUCGAAACAAAUUCAUUU-3' (SEQ ID NO: 130), 5'-AUAAAGUGGAGAACCGCUUCACCAAAAGCUGUCCCUUAGGGGAUUAGAACUUGAGU GAAGGUGGGCUGCUUGCAUCAGCCUAAUGUCGAGAAGUGCUUUCUUCGGAAAGUAA CCCUCGAAACAAAUUCAUUU-3' (SEQ ID NO: 131), 5'-GAUAAAGUGGAGAACCGCUUCACCAAAAGCUGUCCCUUAGGGGAUUAGAACUUGAG UGAAGGUGGGCUGCUUGCAUCAGCCUAAUGUCGAGAAGUGCUUUCUUCGGAAAGU AACCCUCGAAACAAAUUCAUUU-3' (SEQ ID NO: 132), 5'-UGAUAAAGUGGAGAACCGCUUCACCAAAAGCUGUCCCUUAGGGGAUUAGAACUUGA GUGAAGGUGGGCUGCUUGCAUCAGCCUAAUGUCGAGAAGUGCUUUCUUCGGAAAG UAACCCUCGAAACAAAUUCAUUU-3' (SEQ ID NO: 133), 5'-CUGAUAAAGUGGAGAACCGCUUCACCAAAAGCUGUCCCUUAGGGGAUUAGAACUUG AGUGAAGGUGGGCUGCUUGCAUCAGCCUAAUGUCGAGAAGUGCUUUCUUCGGAAA GUAACCCUCGAAACAAAUUCAUUU-3' (SEQ ID NO: 134), 5'-ACUGAUAAAGUGGAGAACCGCUUCACCAAAAGCUGUCCCUUAGGGGAUUAGAACUU GAGUGAAGGUGGGCUGCUUGCAUCAGCCUAAUGUCGAGAAGUGCUUUCUUCGGAA AGUAACCCUCGAAACAAAUUCAUUU-3' (SEQ ID NO: 135), 5'-CACUGAUAAAGUGGAGAACCGCUUCACCAAAAGCUGUCCCUUAGGGGAUUAGAACU UGAGUGAAGGUGGGCUGCUUGCAUCAGCCUAAUGUCGAGAAGUGCUUUCUUCGGA AAGUAACCCUCGAAACAAAUUCAUUU-3' (SEQ ID NO: 136), 5'-UCACUGAUAAAGUGGAGAACCGCUUCACCAAAAGCUGUCCCUUAGGGGAUUAGAAC UUGAGUGAAGGUGGGCUGCUUGCAUCAGCCUAAUGUCGAGAAGUGCUUUCUUCGG AAAGUAACCCUCGAAACAAAUUCAUUU-3' (SEQ ID NO: 137), and 5'-UUCACUGAUAAAGUGGAGAACCGCUUCACCAAAAGCUGUCCCUUAGGGGAUUAGAA CUUGAGUGAAGGUGGGCUGCUUGCAUCAGCCUAAUGUCGAGAAGUGCUUUCUUCG GAAAGUAACCCUCGAAACAAAUUCAUUU-3' (SEQ ID NO: 138); and
5'-GAAUGAAGGAAUGCAAC-3' (SEQ ID NO: 3).

In an embodiment, a sequence of the engineered scaffold region in which the first region is modified may comprise:
a sequence in which the following sequences are linked to each other in a 5' to 3' direction:
a sequence selected from the group consisting of 5'-A-3', 5'-AA-3', 5'-GAA-3', 5'-AGAA-3', 5'-GAGAA-3', 5'-GGAGAA-3', 5'-UGGAGAA-3', 5'-GUGGAGAA-3', 5'-AGUGGAGAA-3', and SEQ ID NOs: 17 to 27,
SEQ ID NO: 11,
SEQ ID NO: 12, and
SEQ ID NO: 14; and
a sequence in which SEQ ID NO: 16 and 5'-AUGCAAC-3' are linked to each other in a 5' to 3' direction.

In an embodiment, a sequence of the engineered scaffold region in which the first region is modified may be a sequence in which the following sequences are linked to each other in a 5' to 3' direction:
a sequence selected from the group consisting of 5'-A-3', 5'-AA-3', 5'-GAA-3', 5'-AGAA-3', 5'-GAGAA-3', 5'-GGAGAA-3', 5'-UGGAGAA-3', 5'-GUGGAGAA-3', 5'-AGUGGAGAA-3', and SEQ ID NOs: 17 to 27,
SEQ ID NO: 11,
SEQ ID NO: 12,
SEQ ID NO: 13,
a linker,
SEQ ID NO: 15, and
5'-AUGCAAC-3'.

Here, the linker may be 5'-GAAA-3'.

In an embodiment, a sequence of the engineered scaffold region in which a first region is modified may be a sequence selected from the group consisting of 5'-ACCGCUUCACCAAAAGCUGUCCCUUAGGGGAUUAGAACUUGAGUGAAGGUGGGCUG CUUGCAUCAGCCUAAUGUCGAGAAGUGCUUUCUUCGGAAAGUAACCCUCGAAACAA AUUCAUUUGAAAGAAUGAAGGAAUGCAAC-3' (SEQ ID NO: 168), 5'-AACCGCUUCACCAAAAGCUGUCCCUUAGGGGAUUAGAACUUGAGUGAAGGUGGGCU GCUUGCAUCAGCCUAAUGUCGAGAAGUGCUUUCUUCGGAAAGUAACCCUCGAAACA AAUUCAUUUGAAAGAAUGAAGGAAUGCAAC-3' (SEQ ID NO: 169), 5'-GAACCGCUUCACCAAAAGCUGUCCCUUAGGGGAUUAGAACUUGAGUGAAGGUGGGC UGCUUGCAUCAGCCUAAUGUCGAGAAGUGCUUUCUUCGGAAAGUAACCCUCGAAAC AAAUUCAUUUGAAAGAAUGAAGGAAUGCAAC-3' (SEQ ID NO: 170), 5'-AGAACCGCUUCACCAAAAGCUGUCCCUUAGGGGAUUAGAACUUGAGUGAAGGUGGG CUGCUUGCAUCAGCCUAAUGUCGAGAAGUGCUUUCUUCGGAAAGUAACCCUCGAAA CAAAUUCAUUUGAAAGAAUGAAGGAAUGCAAC-3' (SEQ ID NO: 171), 5'-GAGAACCGCUUCACCAAAAGCUGUCCCUUAGGGGAUUAGAACUUGAGUGAAGGUGG GCUGCUUGCAUCAGCCUAAUGUCGAGAAGUGCUUUCUUCGGAAAGUAACCCUCGAA ACAAAUUCAUUUGAAAGAAUGAAGGAAUGCAAC-3' (SEQ ID NO: 172), 5'-GGAGAACCGCUUCACCAAAAGCUGUCCCUUAGGGGAUUAGAACUUGAGUGAAGGUG GGCUGCUUGCAUCAGCCUAAUGUCGAGAAGUGCUUUCUUCGGAAAGUAACCCUCGA AACAAAUUCAUUUGAAAGAAUGAAGGAAUGCAAC-3' (SEQ ID NO: 173), 5'-UGGAGAACCGCUUCACCAAAAGCUGUCCCUUAGGGGAUUAGAACUUGAGUGAAGGU GGGCUGCUUGCAUCAGCCUAAUGUCGAGAAGUGCUUUCUUCGGAAAGUAACCCUCG AAACAAAUUCAUUUGAAAGAAUGAAGGAAUGCAAC-3' (SEQ ID NO: 174), 5'-GUGGAGAACCGCUUCACCAAAAGCUGUCCCUUAGGGGAUUAGAACUUGAGUGAAGG UGGGCUGCUUGCAUCAGCCUAAUGUCGAGAAGUGCUUUCUUCGGAAAGUAACCCUC GAAACAAAUUCAUUUGAAAGAAUGAAGGAAUGCAAC-3' (SEQ ID NO: 175), 5'-AGUGGAGAACCGCUUCACCAAAAGCUGUCCCUUAGGGGAUUAGAACUUGAGUGAAG GUGGGCUGCUUGCAUCAGCCUAAUGUCGAGAAGUGCUUUCUUCGGAAAGUAACCCU CGAAACAAAUUCAUUUGAAAGAAUGAAGGAAUGCAAC-3' (SEQ ID NO: 176), 5'-AAGUGGAGAACCGCUUCACCAAAAGCUGUCCCUUAGGGGAUUAGAACUUGAGUGAA GGUGGGCUGCUUGCAUCAGCCUAAUGUCGAGAAGUGCUUUCUUCGGAAAGUAACC CUCGAAACAAAUUCAUUUGAAAGAAUGAAGGAAUGCAAC-3' (SEQ ID NO: 177), 5'-AAAGUGGAGAACCGCUUCACCAAAAGCUGUCCCUUAGGGGAUUAGAACUUGAGUGA AGGUGGGCUGCUUGCAUCAGCCUAAUGUCGAGAAGUGCUUUCUUCGGAAAGUAACC CUCGAAACAAAUUCAUUUGAAAGAAUGAAGGAAUGCAAC-3' (SEQ ID NO: 178), 5'-UAAAGUGGAGAACCGCUUCACCAAAAGCUGUCCCUUAGGGGAUUAGAACUUGAGUG AAGGUGGGCUGCUUGCAUCAGCCUAAUGUCGAGAAGUGCUUUCUUCGGAAAGUAAC CCUCGAAACAAAUUCAUUUGAAAGAAUGAAGGAAUGCAAC-3' (SEQ ID NO: 179), 5'-AUAAAGUGGAGAACCGCUUCACCAAAAGCUGUCCCUUAGGGGAUUAGAACUUGAGU GAAGGUGGGCUGCUUGCAUCAGCCUAAUGUCGAGAAGUGCUUUCUUCGGAAAGUAA CCCUCGAAACAAAUUCAUUUGAAAGAAUGAAGGAAUGCAAC-3' (SEQ ID NO: 180), 5'-GAUAAAGUGGAGAACCGCUUCACCAAAAGCUGUCCCUUAGGGGAUUAGAACUUGAG UGAAGGUGGGCUGCUUGCAUCAGCCUAAUGUCGAGAAGUGCUUUCUUCGGAAAGU AACCCUCGAAACAAAUUCAUUUGAAAGAAUGAAGGAAUGCAAC-3' (SEQ ID NO: 181), 
5'-
UGAUAAAGUGGAGAACCGCUUCACCAAAAGCUGUCCCUUAGGGGAUUAGAACUUGA
GUGAAGGUGGGCUGCUUGCAUCAGCCUAAUGUCGAGAAGUGCUUUCUUCGGAAAG
UAACCCUCGAAACAAAUUCAUUUGAAAGAAUGAAGGAAUGCAAC-3' (SEQ ID NO: 182),
 5'-CUGAUAAAGUGGAGAACCGCUUCACCAAAAGCUGUCCCUUAGGGGAUUAGAACUUG AGUGAAGGUGGGCUGCUUGCAUCAGCCUAAUGUCGAGAAGUGCUUUCUUCGGAAA GUAACCCUCGAAACAAAUUCAUUUGAAAGAAUGAAGGAAUGCAAC-3' (SEQ ID NO: 183), 5'-ACUGAUAAAGUGGAGAACCGCUUCACCAAAAGCUGUCCCUUAGGGGAUUAGAACUU GAGUGAAGGUGGGCUGCUUGCAUCAGCCUAAUGUCGAGAAGUGCUUUCUUCGGAA AGUAACCCUCGAAACAAAUUCAUUUGAAAGAAUGAAGGAAUGCAAC-3' (SEQ ID NO: 184), 5'-CACUGAUAAAGUGGAGAACCGCUUCACCAAAAGCUGUCCCUUAGGGGAUUAGAACU UGAGUGAAGGUGGGCUGCUUGCAUCAGCCUAAUGUCGAGAAGUGCUUUCUUCGGA AAGUAACCCUCGAAACAAAUUCAUUUGAAAGAAUGAAGGAAUGCAAC-3' (SEQ ID NO: 185), 5'-UCACUGAUAAAGUGGAGAACCGCUUCACCAAAAGCUGUCCCUUAGGGGAUUAGAAC UUGAGUGAAGGUGGGCUGCUUGCAUCAGCCUAAUGUCGAGAAGUGCUUUCUUCGG AAAGUAACCCUCGAAACAAAUUCAUUUGAAAGAAUGAAGGAAUGCAAC-3' (SEQ ID NO: 186), and 5'-UUCACUGAUAAAGUGGAGAACCGCUUCACCAAAAGCUGUCCCUUAGGGGAUUAGAA CUUGAGUGAAGGUGGGCUGCUUGCAUCAGCCUAAUGUCGAGAAGUGCUUUCUUCG GAAAGUAACCCUCGAAACAAAUUCAUUUGAAAGAAUGAAGGAAUGCAAC-3' (SEQ ID NO: 187).

### Examples of engineered scaffold sequence from which first region is removed

In an embodiment, a sequence of the engineered scaffold region from which the first region is removed may comprise:
a sequence in which the following sequences are linked to each other in a 5' to 3' direction:
SEQ ID NO: 11,
SEQ ID NO: 12, and
SEQ ID NO: 13; and
a sequence in which SEQ ID NO: 15 and 5'-AUGCAAC-3' are linked to each other in a 5' to 3' direction.

In an embodiment, a sequence of the engineered scaffold region from which the first region is removed may comprise: 
5'-
CCGCUUCACCAAAAGCUGUCCCUUAGGGGAUUAGAACUUGAGUGAAGGUGGGCUG
CUUGCAUCAGCCUAAUGUCGAGAAGUGCUUUCUUCGGAAAGUAACCCUCGAAACAA
AUUCAUUU-3' (SEQ ID NO: 379);
 and
SEQ ID NO: 3.

In an embodiment, a sequence of the engineered scaffold region from which the first region is removed may comprise:
a sequence in which the following sequences are linked to each other in a 5' to 3' direction:
SEQ ID NO: 11,
SEQ ID NO: 12, and
SEQ ID NO: 14; and
a sequence in which SEQ ID NO: 16 and 5'-AUGCAAC-3' are linked to each other in a 5' to 3' direction.

In an embodiment, a sequence of the engineered scaffold region from which the first region is removed may be one in which the following sequences are linked to each other in a 5' to 3' direction:
SEQ ID NO: 11,
SEQ ID NO: 12,
SEQ ID NO: 13,
a linker,
SEQ ID NO: 15, and
5'-AUGCAAC-3'.

Here, the linker may be 5'-GAAA-3'.

In an embodiment, a sequence of the engineered scaffold region from which the first region is removed may be 5'CCGCUUCACCAAAAGCUGUCCCUUAGGGGAUUAGAACUUGAGUGAAGGUGGGCU GCUUGCAUCAGCCUAAUGUCGAGAAGUGCUUUCUUCGGAAAGUAACCCUCGAAACA AAUUCAUUUGAAAGAAUGAAGGAAUGCAAC-3' (SEQ ID NO: 487).

### 5) Engineered scaffold region 2 - Modification of second region

### Overview of modification of second region

The engineered scaffold region included in the engineered guide RNA provided herein may be an engineered form of a naturally occurring scaffold region in which the second region is modified.

In an embodiment, the engineered scaffold region may comprise a modified second region. Here, the modified second region is a modified form of a second region of the naturally occurring scaffold region from which one or more nucleotides are removed. Here, the removed nucleotide(s) is a nucleotide(s) selected from a region forming a stem structure in a CRISPR/Cas12f1 complex.

In an embodiment, the engineered scaffold region included in the engineered Cas12f1 guide RNA may include an engineered form of a naturally occurring scaffold region from which one or more nucleotides included in the second region are removed. In an embodiment, removal of the nucleotide(s) may occur in a portion forming a stem structure in the naturally occurring second region, in which the nucleotides may be removed in base pair. In an embodiment, the removed nucleotide(s) may be a nucleotide(s) included in a portion, which forms a stem structure in the CRISPR/Cas12f1 complex, in the naturally occurring second region. In an embodiment, the removed nucleotide(s) may be a nucleotide(s), which belong to Stem 2 (Takeda et al., Structure of the miniature type V-F CRISPR-Cas effector enzyme, Molecular Cell 81, 1-13 (2021)), in the naturally occurring second region. In an embodiment, the removed nucleotide(s) may be a nucleotide(s) that does not interact with a Cas12f1 protein in the CRISPR/Cas12f1 complex in the naturally occurring second region.

In an embodiment, the modified second region comprises a sequence of 5'-G-3' at the 3' end.

In an embodiment, the engineered scaffold region may be an engineered form of a naturally occurring scaffold region from which the second region is removed. In other words, the engineered scaffold region may not comprise a region corresponding to the second region of the naturally occurring scaffold region.

### Modification detail 1 of second region - Removal of some nucleotides

The second region of the engineered scaffold region may be a modified form of a second region of the naturally occurring scaffold region from which one or more nucleotides are removed.

In an embodiment, the modified second region of the engineered scaffold region may be a modified form of a second region of the naturally occurring scaffold region from which 1 to 51 nucleotides are removed. In an embodiment, the modified second region may be a modified form of a second region of the naturally occurring scaffold region from which one or more nucleotides, of the 1^{st} to 22^{nd} nucleotides and/or the 27^{th} to 51^{st} nucleotides from the 5' end based on the sequence of SEQ ID NO: 11, are removed. In an embodiment, the modified second region may be a modified form of a second region of the naturally occurring scaffold region from which one or more nucleotides, of the 1^{st} to 22^{nd} nucleotides and/or the 27^{th} to 51^{st} nucleotides from the 5' end based on the sequence of SEQ ID NO: 11, are removed, and in which the 23^{rd} to 26^{th} nucleotides from the 5' end based on the sequence of SEQ ID NO: 11 are substituted with other nucleotides. In an embodiment, the modified second region may be a modified form of a second region of the naturally occurring scaffold region from which 1, 2, 3, 4, 5, 6, 7, 8, 9, 10, 11, 12, 13, 14, 15, 16, 17, 18, 19, 20, 21, or 22 consecutive nucleotides, of the 1^{st} to 22^{nd} nucleotides from the 5' end based on the sequence of SEQ ID NO: 11, are removed. In an embodiment, the modified second region may be a modified form of a second region of the naturally occurring scaffold region from which 1, 2, 3, 4, 5, 6, 7, 8, 9, 10, 11, 12, 13, 14, 15, 16, 17, 18, 19, 20, 21, 22, 23, 24, or 25 consecutive nucleotides, of the 27^{th} to 51^{st} nucleotides from the 5' end based on the sequence of SEQ ID NO: 11, are removed.

In an embodiment, a sequence of the modified second region comprises at least 5'-G-3'.

### Modification detail 2 of second region - Removal in base pair

The modification of the second region may be removal of one or more pairs of nucleotides that are included in a portion forming a stem structure and complementarily bind to each other.

In an embodiment, the modified second region may be a modified form of a second region of the naturally occurring scaffold region from which one or more pairs of nucleotides forming base pairs in the CRISPR/Cas12f1 complex, of the 1^{st} to 22^{nd} nucleotides and the 27^{th} to 50^{th} nucleotides from the 5' end based on the sequence of SEQ ID NO: 11, are removed.

In an embodiment, the modified second region may be a modified form of a second region of the naturally occurring scaffold region from which one or more pairs of nucleotides forming base pairs and/or one or more nucleotides not forming a base pair in the CRISPR/Cas12f1 complex, of the 1^{st} to 22^{nd} nucleotides and the 27^{th} to 50^{th} nucleotides from the 5' end based on the sequence of SEQ ID NO: 11, are removed.

In an embodiment, the modified second region may be a modified form of a second region of the naturally occurring scaffold region from which one or more pairs of nucleotides forming base pairs and/or one or more mismatched pairs of nucleotides in the CRISPR/Cas12f1 complex, of the 1^{st} to 22^{nd} nucleotides and the 27^{th} to 50th nucleotides from the 5' end based on the sequence of SEQ ID NO: 11, are removed.

In an embodiment, the modified second region may have at least one of the sequences set forth in SEQ ID NOs: 139 to 149.

### Modification detail 3 of second region - Removal of second region

The engineered scaffold region provided herein may be an engineered form of a naturally occurring scaffold region from which the second region is removed.

In an embodiment, the engineered scaffold region may not comprise a region corresponding to the second region of the naturally occurring scaffold region.

### Examples of engineered scaffold sequence in which second region is modified

In an embodiment, a sequence of the modified second region may be a sequence selected from the group consisting of 5'-G-3', 5'-UUAGG-3', 5'-CUUAGGG-3', 5'-CUUAGUGG-3', 5'-CCUUAGGUGG-3' (SEQ ID NO: 342), 5'-CCGUUAGGUGG-3' (SEQ ID NO: 343), 5'-CCGCUUAGGGUGG-3' (SEQ ID NO: 344),
5'-CCGCUUUAGAGGUGG-3' (SEQ ID NO: 345),
5'-CCGCUUUUAGAAGGUGG-3' (SEQ ID NO: 346),
5'-CCGCUUCUUAGGAAGGUGG-3' (SEQ ID NO: 347),
5'-CCGCUUCAUUAGUGAAGGUGG-3' (SEQ ID NO: 348),
5'-CCGCUUCACUUAGGUGAAGGUGG-3' (SEQ ID NO: 349),
5'-CCGCUUCACUUAGAGUGAAGGUGG-3' (SEQ ID NO: 350),
5'-CCGCUUCACCUUAGGAGUGAAGGUGG-3' (SEQ ID NO: 351),
5'-CCGCUUCACCAUUAGUGAGUGAAGGUGG-3' (SEQ ID NO: 352),
5'-CCGCUUCACCAAUUAGUUGAGUGAAGGUGG-3' (SEQ ID NO: 353),
5'-CCGCUUCACCAAAUUAGCUUGAGUGAAGGUGG-3' (SEQ ID NO: 354),
5'-CCGCUUCACCAAAAUUAGACUUGAGUGAAGGUGG-3' (SEQ ID NO: 355),
5'-CCGCUUCACCAAAAGUUAGAACUUGAGUGAAGGUGG-3' (SEQ ID NO: 356),
5'-CCGCUUCACCAAAAGCUUAGGAACUUGAGUGAAGGUGG-3' (SEQ ID NO: 357),
5'-CCGCUUCACCAAAAGCUUUAGAGAACUUGAGUGAAGGUGG-3' (SEQ ID NO: 358),
5'-CCGCUUCACCAAAAGCUGUUAGUAGAACUUGAGUGAAGGUGG-3' (SEQ ID NO: 359),
5'-CCGCUUCACCAAAAGCUGUUAGUUAGAACUUGAGUGAAGGUGG-3' (SEQ ID NO: 360),
5'-CCGCUUCACCAAAAGCUGUUUAGAUUAGAACUUGAGUGAAGGUGG-3' (SEQ ID NO: 361), and
5'-CCGCUUCACCAAAAGCUGUCUUAGGAUUAGAACUUGAGUGAAGGUGG-3' (SEQ ID NO: 362).

In an embodiment, the sequence of the modified second region may be at least one of the sequences set forth in SEQ ID NOs: 363 to 378.

In an embodiment, a sequence of the engineered scaffold region in which the second region is modified may comprise:
a sequence in which the following sequences are linked to each other in a 5' to 3' direction:
SEQ ID NO: 10,
a sequence selected from the group consisting of 5'-G-3', 5'-UUAGG-3', 5'-CUUAGGG-3', 5'-CUUAGUGG-3', and SEQ ID NOs: 342 to 362,
SEQ ID NO: 12, and
SEQ ID NO: 13; and
a sequence in which SEQ ID NO: 15 and 5'-AUGCAAC-3' are linked to each other in a 5' to 3' direction.

In an embodiment, a sequence of the engineered scaffold region in which the second region is modified may comprise:
a sequence selected from the group consisting of 5'-CUUCACUGAUAAAGUGGAGAAGGCUGCUUGCAUCAGCCUAAUGUCGAGAAGUGCUU UCUUCGGAAAGUAACCCUCGAAACAAAUUCAUUU-3' (SEQ ID NO: 381), 5'-CUUCACUGAUAAAGUGGAGAAUUAGGGCUGCUUGCAUCAGCCUAAUGUCGAGAAGU GCUUUCUUCGGAAAGUAACCCUCGAAACAAAUUCAUUU-3' (SEQ ID NO: 382), 5'-CUUCACUGAUAAAGUGGAGAACUUAGGGGCUGCUUGCAUCAGCCUAAUGUCGAGAA GUGCUUUCUUCGGAAAGUAACCCUCGAAACAAAUUCAUUU-3' (SEQ ID NO: 383), 5'-CUUCACUGAUAAAGUGGAGAACUUAGUGGGCUGCUUGCAUCAGCCUAAUGUCGAGA AGUGCUUUCUUCGGAAAGUAACCCUCGAAACAAAUUCAUUU-3' (SEQ ID NO: 384), 5'-CUUCACUGAUAAAGUGGAGAACCUUAGGUGGGCUGCUUGCAUCAGCCUAAUGUCGA GAAGUGCUUUCUUCGGAAAGUAACCCUCGAAACAAAUUCAUUU-3' (SEQ ID NO: 385), 
5'-
CUUCACUGAUAAAGUGGAGAACCGUUAGGUGGGCUGCUUGCAUCAGCCUAAUGUCG
AGAAGUGCUUUCUUCGGAAAGUAACCCUCGAAACAAAUUCAUUU-3' (SEQ ID NO: 386),
 5'-CUUCACUGAUAAAGUGGAGAACCGCUUAGGGUGGGCUGCUUGCAUCAGCCUAAUGU CGAGAAGUGCUUUCUUCGGAAAGUAACCCUCGAAACAAAUUCAUUU-3' (SEQ ID NO: 387), 5'-CUUCACUGAUAAAGUGGAGAACCGCUUUAGAGGUGGGCUGCUUGCAUCAGCCUAAU GUCGAGAAGUGCUUUCUUCGGAAAGUAACCCUCGAAACAAAUUCAUUU-3' (SEQ ID NO: 388), 5'-CUUCACUGAUAAAGUGGAGAACCGCUUUUAGAAGGUGGGCUGCUUGCAUCAGCCUA AUGUCGAGAAGUGCUUUCUUCGGAAAGUAACCCUCGAAACAAAUUCAUUU-3' (SEQ ID NO: 389), 5'-CUUCACUGAUAAAGUGGAGAACCGCUUCUUAGGAAGGUGGGCUGCUUGCAUCAGCC UAAUGUCGAGAAGUGCUUUCUUCGGAAAGUAACCCUCGAAACAAAUUCAUUU-3' (SEQ ID NO: 390), 5'-CUUCACUGAUAAAGUGGAGAACCGCUUCAUUAGUGAAGGUGGGCUGCUUGCAUCAG CCUAAUGUCGAGAAGUGCUUUCUUCGGAAAGUAACCCUCGAAACAAAUUCAUUU-3' (SEQ ID NO: 391), 5'-CUUCACUGAUAAAGUGGAGAACCGCUUCACUUAGGUGAAGGUGGGCUGCUUGCAUC AGCCUAAUGUCGAGAAGUGCUUUCUUCGGAAAGUAACCCUCGAAACAAAUUCAUUU-3' (SEQ ID NO: 392), 5'-CUUCACUGAUAAAGUGGAGAACCGCUUCACUUAGAGUGAAGGUGGGCUGCUUGCAU CAGCCUAAUGUCGAGAAGUGCUUUCUUCGGAAAGUAACCCUCGAAACAAAUUCAUU U-3' (SEQ ID NO: 393), 5'-CUUCACUGAUAAAGUGGAGAACCGCUUCACCUUAGGAGUGAAGGUGGGCUGCUUG CAUCAGCCUAAUGUCGAGAAGUGCUUUCUUCGGAAAGUAACCCUCGAAACAAAUUC AUUU-3' (SEQ ID NO: 394), 5'-CUUCACUGAUAAAGUGGAGAACCGCUUCACCAUUAGUGAGUGAAGGUGGGCUGCUU GCAUCAGCCUAAUGUCGAGAAGUGCUUUCUUCGGAAAGUAACCCUCGAAACAAAUU CAUUU-3' (SEQ ID NO: 395), 5'-CUUCACUGAUAAAGUGGAGAACCGCUUCACCAAUUAGUUGAGUGAAGGUGGGCUGC UUGCAUCAGCCUAAUGUCGAGAAGUGCUUUCUUCGGAAAGUAACCCUCGAAACAAA UUCAUUU-3' (SEQ ID NO: 396), 5'-CUUCACUGAUAAAGUGGAGAACCGCUUCACCAAAUUAGCUUGAGUGAAGGUGGGCU GCUUGCAUCAGCCUAAUGUCGAGAAGUGCUUUCUUCGGAAAGUAACCCUCGAAACA AAUUCAUUU-3' (SEQ ID NO: 397), 5'-CUUCACUGAUAAAGUGGAGAACCGCUUCACCAAAAUUAGACUUGAGUGAAGGUGGG CUGCUUGCAUCAGCCUAAUGUCGAGAAGUGCUUUCUUCGGAAAGUAACCCUCGAAA CAAAUUCAUUU-3' (SEQ ID NO: 398), 5'-CUUCACUGAUAAAGUGGAGAACCGCUUCACCAAAAGUUAGAACUUGAGUGAAGGUG GGCUGCUUGCAUCAGCCUAAUGUCGAGAAGUGCUUUCUUCGGAAAGUAACCCUCGA AACAAAUUCAUUU-3' (SEQ ID NO: 399), 5'-CUUCACUGAUAAAGUGGAGAACCGCUUCACCAAAAGCUUAGGAACUUGAGUGAAGG UGGGCUGCUUGCAUCAGCCUAAUGUCGAGAAGUGCUUUCUUCGGAAAGUAACCCUC GAAACAAAUUCAUUU-3' (SEQ ID NO: 400), 5'-CUUCACUGAUAAAGUGGAGAACCGCUUCACCAAAAGCUUUAGAGAACUUGAGUGAA GGUGGGCUGCUUGCAUCAGCCUAAUGUCGAGAAGUGCUUUCUUCGGAAAGUAACC CUCGAAACAAAUUCAUUU-3' (SEQ ID NO: 401), 5'-CUUCACUGAUAAAGUGGAGAACCGCUUCACCAAAAGCUGUUAGUAGAACUUGAGUG AAGGUGGGCUGCUUGCAUCAGCCUAAUGUCGAGAAGUGCUUUCUUCGGAAAGUAAC CCUCGAAACAAAUUCAUUU-3' (SEQ ID NO: 402), 5'-CUUCACUGAUAAAGUGGAGAACCGCUUCACCAAAAGCUGUUAGUUAGAACUUGAGU GAAGGUGGGCUGCUUGCAUCAGCCUAAUGUCGAGAAGUGCUUUCUUCGGAAAGUAA CCCUCGAAACAAAUUCAUUU-3' (SEQ ID NO: 403), 5'-CUUCACUGAUAAAGUGGAGAACCGCUUCACCAAAAGCUGUUUAGAUUAGAACUUGA GUGAAGGUGGGCUGCUUGCAUCAGCCUAAUGUCGAGAAGUGCUUUCUUCGGAAAG UAACCCUCGAAACAAAUUCAUUU-3' (SEQ ID NO: 404), 5'-CUUCACUGAUAAAGUGGAGAACCGCUUCACCAAAAGCUGUCUUAGGAUUAGAACUU GAGUGAAGGUGGGCUGCUUGCAUCAGCCUAAUGUCGAGAAGUGCUUUCUUCGGAA AGUAACCCUCGAAACAAAUUCAUUU-3' (SEQ ID NO: 405), and 5'-CUUCACUGAUAAAGUGGAGAACCGCUUCACCAAAAGCUGUCCUUAGGGAUUAGAAC UUGAGUGAAGGUGGGCUGCUUGCAUCAGCCUAAUGUCGAGAAGUGCUUUCUUCGG AAAGUAACCCUCGAAACAAAUUCAUUU-3' (SEQ ID NO: 406); and
SEQ ID NO: 3.

In an embodiment, a sequence of the engineered scaffold region in which the second region is modified may comprise:
a sequence in which the following sequences are linked to each other in a 5' to 3' direction:
SEQ ID NO: 10,
a sequence selected from the group consisting of 5'-G-3', 5'-UUAGG-3', 5'-CUUAGGG-3', 5'-CUUAGUGG-3', and SEQ ID NOs: 342 to 362,
SEQ ID NO: 12, and
SEQ ID NO: 14; and
a sequence in which SEQ ID NO: 16 and 5'-AUGCAAC-3' are linked to each other in a 5' to 3' direction.

In an embodiment, a sequence of the engineered scaffold region in which the second region is modified may be a sequence in which the following sequences are linked to each other in a 5' to 3' direction: a sequence selected from the group consisting of SEQ ID NO: 10, 5'-G-3', 5'-UUAGG-3', 5'-CUUAGGG-3', 5'-CUUAGUGG-3', and SEQ ID NOs: 342 to 362; SEQ ID NO: 12; SEQ ID NO: 13; a linker; SEQ ID NO: 15; and 5'-AUGCAAC-3'.

Here, the linker may be 5'-GAAA-3'.

In an embodiment, a sequence of the engineered scaffold region in which the second region is modified may be a sequence selected from the group consisting of 5'-CUUCACUGAUAAAGUGGAGAAGGCUGCUUGCAUCAGCCUAAUGUCGAGAAGUGCUU UCUUCGGAAAGUAACCCUCGAAACAAAUUCAUUUGAAAGAAUGAAGGAAUGCAAC-3' (SEQ ID NO: 408), 5'-CUUCACUGAUAAAGUGGAGAAUUAGGGCUGCUUGCAUCAGCCUAAUGUCGAGAAGU GCUUUCUUCGGAAAGUAACCCUCGAAACAAAUUCAUUUGAAAGAAUGAAGGAAUGCA AC-3' (SEQ ID NO: 409), 5'-CUUCACUGAUAAAGUGGAGAACUUAGGGGCUGCUUGCAUCAGCCUAAUGUCGAGAA GUGCUUUCUUCGGAAAGUAACCCUCGAAACAAAUUCAUUUGAAAGAAUGAAGGAAU GCAAC-3' (SEQ ID NO: 410), 5'-CUUCACUGAUAAAGUGGAGAACUUAGUGGGCUGCUUGCAUCAGCCUAAUGUCGAGA AGUGCUUUCUUCGGAAAGUAACCCUCGAAACAAAUUCAUUUGAAAGAAUGAAGGAAU GCAAC-3' (SEQ ID NO: 411), 5'-CUUCACUGAUAAAGUGGAGAACCUUAGGUGGGCUGCUUGCAUCAGCCUAAUGUCGA GAAGUGCUUUCUUCGGAAAGUAACCCUCGAAACAAAUUCAUUUGAAAGAAUGAAGGA AUGCAAC-3' (SEQ ID NO: 412), 5'-CUUCACUGAUAAAGUGGAGAACCGUUAGGUGGGCUGCUUGCAUCAGCCUAAUGUCG AGAAGUGCUUUCUUCGGAAAGUAACCCUCGAAACAAAUUCAUUUGAAAGAAUGAAGG AAUGCAAC-3' (SEQ ID NO: 413), 5'-CUUCACUGAUAAAGUGGAGAACCGCUUAGGGUGGGCUGCUUGCAUCAGCCUAAUGU CGAGAAGUGCUUUCUUCGGAAAGUAACCCUCGAAACAAAUUCAUUUGAAAGAAUGAA GGAAUGCAAC-3' (SEQ ID NO: 414), 5'-CUUCACUGAUAAAGUGGAGAACCGCUUUAGAGGUGGGCUGCUUGCAUCAGCCUAAU GUCGAGAAGUGCUUUCUUCGGAAAGUAACCCUCGAAACAAAUUCAUUUGAAAGAAU GAAGGAAUGCAAC-3' (SEQ ID NO: 415), 5'-CUUCACUGAUAAAGUGGAGAACCGCUUUUAGAAGGUGGGCUGCUUGCAUCAGCCUA AUGUCGAGAAGUGCUUUCUUCGGAAAGUAACCCUCGAAACAAAUUCAUUUGAAAGAA UGAAGGAAUGCAAC-3' (SEQ ID NO: 416), 5'-CUUCACUGAUAAAGUGGAGAACCGCUUCUUAGGAAGGUGGGCUGCUUGCAUCAGCC UAAUGUCGAGAAGUGCUUUCUUCGGAAAGUAACCCUCGAAACAAAUUCAUUUGAAA GAAUGAAGGAAUGCAAC-3' (SEQ ID NO: 417), 5'-CUUCACUGAUAAAGUGGAGAACCGCUUCAUUAGUGAAGGUGGGCUGCUUGCAUCAG CCUAAUGUCGAGAAGUGCUUUCUUCGGAAAGUAACCCUCGAAACAAAUUCAUUUGA AAGAAUGAAGGAAUGCAAC-3' (SEQ ID NO: 418), 5'-CUUCACUGAUAAAGUGGAGAACCGCUUCACUUAGGUGAAGGUGGGCUGCUUGCAUC AGCCUAAUGUCGAGAAGUGCUUUCUUCGGAAAGUAACCCUCGAAACAAAUUCAUUU GAAAGAAUGAAGGAAUGCAAC-3' (SEQ ID NO: 419), 5'-CUUCACUGAUAAAGUGGAGAACCGCUUCACUUAGAGUGAAGGUGGGCUGCUUGCAU CAGCCUAAUGUCGAGAAGUGCUUUCUUCGGAAAGUAACCCUCGAAACAAAUUCAUU UGAAAGAAUGAAGGAAUGCAAC-3' (SEQ ID NO: 420), 5'-CUUCACUGAUAAAGUGGAGAACCGCUUCACCUUAGGAGUGAAGGUGGGCUGCUUG CAUCAGCCUAAUGUCGAGAAGUGCUUUCUUCGGAAAGUAACCCUCGAAACAAAUUC AUUUGAAAGAAUGAAGGAAUGCAAC-3' (SEQ ID NO: 421), 5'-CUUCACUGAUAAAGUGGAGAACCGCUUCACCAUUAGUGAGUGAAGGUGGGCUGCUU GCAUCAGCCUAAUGUCGAGAAGUGCUUUCUUCGGAAAGUAACCCUCGAAACAAAUU CAUUUGAAAGAAUGAAGGAAUGCAAC-3' (SEQ ID NO: 422), 5'-CUUCACUGAUAAAGUGGAGAACCGCUUCACCAAUUAGUUGAGUGAAGGUGGGCUGC UUGCAUCAGCCUAAUGUCGAGAAGUGCUUUCUUCGGAAAGUAACCCUCGAAACAAA UUCAUUUGAAAGAAUGAAGGAAUGCAAC-3' (SEQ ID NO: 423), 5'-CUUCACUGAUAAAGUGGAGAACCGCUUCACCAAAUUAGCUUGAGUGAAGGUGGGCU GCUUGCAUCAGCCUAAUGUCGAGAAGUGCUUUCUUCGGAAAGUAACCCUCGAAACA AAUUCAUUUGAAAGAAUGAAGGAAUGCAAC-3' (SEQ ID NO: 424), 5'-CUUCACUGAUAAAGUGGAGAACCGCUUCACCAAAAUUAGACUUGAGUGAAGGUGGG CUGCUUGCAUCAGCCUAAUGUCGAGAAGUGCUUUCUUCGGAAAGUAACCCUCGAAA CAAAUUCAUUUGAAAGAAUGAAGGAAUGCAAC-3' (SEQ ID NO: 425), 5'-CUUCACUGAUAAAGUGGAGAACCGCUUCACCAAAAGUUAGAACUUGAGUGAAGGUG GGCUGCUUGCAUCAGCCUAAUGUCGAGAAGUGCUUUCUUCGGAAAGUAACCCUCGA AACAAAUUCAUUUGAAAGAAUGAAGGAAUGCAAC-3' (SEQ ID NO: 426), 5'-CUUCACUGAUAAAGUGGAGAACCGCUUCACCAAAAGCUUAGGAACUUGAGUGAAGG UGGGCUGCUUGCAUCAGCCUAAUGUCGAGAAGUGCUUUCUUCGGAAAGUAACCCUC GAAACAAAUUCAUUUGAAAGAAUGAAGGAAUGCAAC-3' (SEQ ID NO: 427), 5'-CUUCACUGAUAAAGUGGAGAACCGCUUCACCAAAAGCUUUAGAGAACUUGAGUGAA GGUGGGCUGCUUGCAUCAGCCUAAUGUCGAGAAGUGCUUUCUUCGGAAAGUAACC CUCGAAACAAAUUCAUUUGAAAGAAUGAAGGAAUGCAAC-3' (SEQ ID NO: 428), 5'-CUUCACUGAUAAAGUGGAGAACCGCUUCACCAAAAGCUGUUAGUAGAACUUGAGUG AAGGUGGGCUGCUUGCAUCAGCCUAAUGUCGAGAAGUGCUUUCUUCGGAAAGUAAC CCUCGAAACAAAUUCAUUUGAAAGAAUGAAGGAAUGCAAC-3' (SEQ ID NO: 429), 5'-CUUCACUGAUAAAGUGGAGAACCGCUUCACCAAAAGCUGUUAGUUAGAACUUGAGU GAAGGUGGGCUGCUUGCAUCAGCCUAAUGUCGAGAAGUGCUUUCUUCGGAAAGUAA CCCUCGAAACAAAUUCAUUUGAAAGAAUGAAGGAAUGCAAC-3' (SEQ ID NO: 430), 5'-CUUCACUGAUAAAGUGGAGAACCGCUUCACCAAAAGCUGUUUAGAUUAGAACUUGA GUGAAGGUGGGCUGCUUGCAUCAGCCUAAUGUCGAGAAGUGCUUUCUUCGGAAAG UAACCCUCGAAACAAAUUCAUUUGAAAGAAUGAAGGAAUGCAAC-3' (SEQ ID NO: 431), 5'-CUUCACUGAUAAAGUGGAGAACCGCUUCACCAAAAGCUGUCUUAGGAUUAGAACUU GAGUGAAGGUGGGCUGCUUGCAUCAGCCUAAUGUCGAGAAGUGCUUUCUUCGGAA AGUAACCCUCGAAACAAAUUCAUUUGAAAGAAUGAAGGAAUGCAAC-3' (SEQ ID NO: 432), and 5'-CUUCACUGAUAAAGUGGAGAACCGCUUCACCAAAAGCUGUCCUUAGGGAUUAGAAC UUGAGUGAAGGUGGGCUGCUUGCAUCAGCCUAAUGUCGAGAAGUGCUUUCUUCGG AAAGUAACCCUCGAAACAAAUUCAUUUGAAAGAAUGAAGGAAUGCAAC-3' (SEQ ID NO: 433).

### Examples of engineered scaffold sequence from which second region is removed

In an embodiment, a sequence of the engineered scaffold region from which the second region is removed may comprise:
a sequence in which the following sequences are linked to each other in a 5' to 3' direction:
SEQ ID NO: 10,
SEQ ID NO: 12, and
SEQ ID NO: 13; and
a sequence in which SEQ ID NO: 15 and 5'-AUGCAAC-3' are linked to each other in a 5' to 3' direction.

In an embodiment, a sequence of the engineered scaffold region from which the second region is removed may comprise: 
5'-
CUUCACUGAUAAAGUGGAGAAGCUGCUUGCAUCAGCCUAAUGUCGAGAAGUGCUUU
CUUCGGAAAGUAACCCUCGAAACAAAUUCAUUU-3' (SEQ ID NO: 380);
 and
SEQ ID NO: 3.

In an embodiment, a sequence of the engineered scaffold region from which the second region is removed may comprise:
a sequence in which the following sequences are linked to each other in a 5' to 3' direction:
SEQ ID NO: 10,
SEQ ID NO: 12, and
SEQ ID NO: 14; and
a sequence in which SEQ ID NO: 16 and 5'-AUGCAAC-3' are linked to each other in a 5' to 3' direction.

In an embodiment, a sequence of the engineered scaffold region from which the second region is removed may be a sequence in which SEQ ID NO: 10, SEQ ID NO: 12, SEQ ID NO: 13, a linker, SEQ ID NO: 15, and 5'-AUGCAAC-3' are linked to each other in a 5' to 3' direction.

Here, the linker may be 5'-GAAA-3'.

In an embodiment, a sequence of the engineered scaffold region from which the second region is removed may be 5'-CUUCACUGAUAAAGUGGAGAAGCUGCUUGCAUCAGCCUAAUGUCGAGAAGUGCUUU CUUCGGAAAGUAACCCUCGAAACAAAUUCAUUUGAAAGAAUGAAGGAAUGCAAC-3'(SEQ ID NO: 407).

### 6) Engineered scaffold region 3 - Modification of third region

### Overview of modification of third region

The engineered scaffold region included in the engineered guide RNA provided herein may be an engineered form of a naturally occurring scaffold region in which the third region is modified.

In an embodiment, the engineered scaffold region may comprise a modified third region. Here, the modified third region is obtained by removing one or more nucleotides from the third region of the naturally occurring scaffold region. Here, the removed nucleotide(s) is a nucleotide(s) selected from a region forming a stem structure in the CRISPR/Cas12f1 complex.

In an embodiment, the engineered scaffold region included in the engineered Cas12f1 guide RNA may comprise an engineered form of a naturally occurring scaffold region from which one or more nucleotides included in the third region are removed. In an embodiment, removal of the nucleotide(s) may occur in a portion forming a stem structure in the naturally occurring third region, in which the nucleotide(s) may be removed in base pair. In an embodiment, the removed nucleotide(s) may be a nucleotide(s) included in a portion, which forms a stem structure in a CRISPR/Cas12f1 complex, in the naturally occurring third region. In an embodiment, the removed nucleotide(s) may be a nucleotide(s), which belong to Stem 4 (Takeda et al., Structure of the miniature type V-F CRISPR-Cas effector enzyme, Molecular Cell 81, 1-13 (2021)), in the naturally occurring third region.

In an embodiment, the modified third region may be characterized by having 5'GCUGCUUGCAUCAGCCUAAUGUCGAG-3', 5'-UUCG-3', and 5'-CUCGA-3'.

### Modification detail 1 of third region - Removal of some nucleotides

The third region of the engineered scaffold region may be a modified form of a third region of the naturally occurring scaffold region from which one or more nucleotides are removed.

In an embodiment, the modified third region of the engineered scaffold region may be a modified form of a third region of the naturally occurring scaffold region from which 1 to 20 nucleotides are removed. In an embodiment, the modified third region may be a modified form of a third region of the naturally occurring scaffold region from which one or more nucleotides, of the 27^{th} to 36^{th} nucleotides and/or the 41^{st} to 50^{th} nucleotides from the 5' end based on the sequence of SEQ ID NO: 12, are removed. In an embodiment, the modified third region may be a modified form of a third region of the naturally occurring scaffold region from which 1, 2, 3, 4, 5, 6, 7, 8, 9, or 10 consecutive nucleotides, of the 27^{th} to 36^{th} nucleotides from the 5'-end based on the sequence of SEQ ID NO: 12, are removed. In an embodiment, the modified third region may be a modified form of a third region of the naturally occurring scaffold region from which 1, 2, 3, 4, 5, 6, 7, 8, 9, or 10 consecutive nucleotides, of the 41^{st} to 50^{th} nucleotides from the 5' end based on the sequence of SEQ ID NO: 12, are removed.

### Modification detail 2 of third region - Removal in base pair

The modification of the third region may be removal of one or more pairs of nucleotides that are included in a portion forming a stem structure and complementarily bind to each other.

In an embodiment, the modified third region may be a modified form of a third region of the naturally occurring scaffold region from which one or more pairs of nucleotides forming base pairs in the CRISPR/Cas12f1 complex, of the 27^{th} to 36^{th} nucleotides and the 41^{st} to 50^{th} nucleotides from the 5' end based on the sequence of SEQ ID NO: 12, are removed.

In an embodiment, the modified third region may be a modified form of a third region of the naturally occurring scaffold region from which one or more pairs of nucleotides forming base pairs and/or one or more nucleotides not forming a base pair in the CRISPR/Casl2f1 complex, of the 27^{th} to 36^{th} nucleotides and/or the 41^{st} to 50^{th} nucleotides from the 5' end based on the sequence of SEQ ID NO: 12, are removed.

In an embodiment, the modified third region may be a modified form of a third region of the naturally occurring scaffold region from which one or more pairs of nucleotides forming base pairs and/or one or more mismatched pairs of nucleotides in the CRISPR/Cas12f1 complex, of the 27^{th} to 36^{th} nucleotides and the 41^{st} to 50^{th} nucleotides from the 5' end based on the sequence of SEQ ID NO: 12, are removed.

### Examples of engineered scaffold sequence in which third region is modified

In an embodiment, a sequence of the modified third region may be a sequence selected from the group consisting of 5'-GCUGCUUGCAUCAGCCUAAUGUCGAGAAGUGCUUUCUUCGGAAAGUAACCCUCGA-3' (SEQ ID NO: 434), 5'-GCUGCUUGCAUCAGCCUAAUGUCGAGAAGUGCUUUUUCGAAAGUAACCCUCGA-3' (SEQ ID NO: 435), 5'-GCUGCUUGCAUCAGCCUAAUGUCGAGAAGUGCUUUUCGAAGUAACCCUCGA-3'(SEQ ID NO: 436), 5'-GCUGCUUGCAUCAGCCUAAUGUCGAGAAGUGCUCUUCGGAGUAACCCUCGA-3' (SEQ ID NO: 437), 5'-GCUGCUUGCAUCAGCCUAAUGUCGAGAAGUGCUUUCGAGUAACCCUCGA-3' (SEQ ID NO: 438), 5'-GCUGCUUGCAUCAGCCUAAUGUCGAGAAGUGCUUCGGUAACCCUCGA-3' (SEQ ID NO: 439), 5'-GCUGCUUGCAUCAGCCUAAUGUCGAGAAGUGUUCGUAACCCUCGA-3' (SEQ ID NO: 440), 5'-GCUGCUUGCAUCAGCCUAAUGUCGAGAAGUUUCGAACCCUCGA-3' (SEQ ID NO: 441), 5'-GCUGCUUGCAUCAGCCUAAUGUCGAGAAGUUCGACCCUCGA-3' (SEQ ID NO: 442), 5'-GCUGCUUGCAUCAGCCUAAUGUCGAGAAGUUCGCCCUCGA-3' (SEQ ID NO: 443), 5'-GCUGCUUGCAUCAGCCUAAUGUCGAGAAUUCGCCCUCGA-3' (SEQ ID NO: 444), 5'-GCUGCUUGCAUCAGCCUAAUGUCGAGAAUUCGCCUCGA-3' (SEQ ID NO: 445), 5'-GCUGCUUGCAUCAGCCUAAUGUCGAGAUUCGCCUCGA-3' (SEQ ID NO: 446), and 5'-GCUGCUUGCAUCAGCCUAAUGUCGAGUUCGCUCGA-3' (SEQ ID NO: 447).

In an embodiment, a sequence of the engineered scaffold region in which the third region is modified may comprise:
a sequence in which the following sequences are linked to each other in a 5' to 3' direction:
SEQ ID NO: 10,
SEQ ID NO: 11,
a sequence selected from the group consisting of SEQ ID NOs: 434 to 447, and
SEQ ID NO: 13; and
a sequence in which SEQ ID NO: 15 and 5'-AUGCAAC-3' are linked to each other in a 5' to 3' direction.

In an embodiment, a sequence of the engineered scaffold region in which the third region is modified may comprise:
a sequence selected from the group consisting of 5'-CUUCACUGAUAAAGUGGAGAACCGCUUCACCAAAAGCUGUCCCUUAGGGGAUUAGA ACUUGAGUGAAGGUGGGCUGCUUGCAUCAGCCUAAUGUCGAGAAGUGCUUUUUCG AAAGUAACCCUCGAAACAAAUUCAUUU-3' (SEQ ID NO: 448), 5'-CUUCACUGAUAAAGUGGAGAACCGCUUCACCAAAAGCUGUCCCUUAGGGGAUUAGA ACUUGAGUGAAGGUGGGCUGCUUGCAUCAGCCUAAUGUCGAGAAGUGCUUUUCGA AGUAACCCUCGAAACAAAUUCAUUU-3' (SEQ ID NO: 449), 5'-CUUCACUGAUAAAGUGGAGAACCGCUUCACCAAAAGCUGUCCCUUAGGGGAUUAGA ACUUGAGUGAAGGUGGGCUGCUUGCAUCAGCCUAAUGUCGAGAAGUGCUCUUCGG AGUAACCCUCGAAACAAAUUCAUUU-3' (SEQ ID NO: 450), 5'-CUUCACUGAUAAAGUGGAGAACCGCUUCACCAAAAGCUGUCCCUUAGGGGAUUAGA ACUUGAGUGAAGGUGGGCUGCUUGCAUCAGCCUAAUGUCGAGAAGUGCUUUCGAG UAACCCUCGAAACAAAUUCAUUU-3' (SEQ ID NO: 451), 5'-CUUCACUGAUAAAGUGGAGAACCGCUUCACCAAAAGCUGUCCCUUAGGGGAUUAGA ACUUGAGUGAAGGUGGGCUGCUUGCAUCAGCCUAAUGUCGAGAAGUGCUUCGGUA ACCCUCGAAACAAAUUCAUUU-3' (SEQ ID NO: 452), 5'-CUUCACUGAUAAAGUGGAGAACCGCUUCACCAAAAGCUGUCCCUUAGGGGAUUAGA ACUUGAGUGAAGGUGGGCUGCUUGCAUCAGCCUAAUGUCGAGAAGUGUUCGUAAC CCUCGAAACAAAUUCAUUU-3' (SEQ ID NO: 453), 5'-CUUCACUGAUAAAGUGGAGAACCGCUUCACCAAAAGCUGUCCCUUAGGGGAUUAGA ACUUGAGUGAAGGUGGGCUGCUUGCAUCAGCCUAAUGUCGAGAAGUUUCGAACCCU CGAAACAAAUUCAUUU-3' (SEQ ID NO: 454), 5'-CUUCACUGAUAAAGUGGAGAACCGCUUCACCAAAAGCUGUCCCUUAGGGGAUUAGA ACUUGAGUGAAGGUGGGCUGCUUGCAUCAGCCUAAUGUCGAGAAGUUCGACCCUC GAAACAAAUUCAUUU-3' (SEQ ID NO: 455), 5'-CUUCACUGAUAAAGUGGAGAACCGCUUCACCAAAAGCUGUCCCUUAGGGGAUUAGA ACUUGAGUGAAGGUGGGCUGCUUGCAUCAGCCUAAUGUCGAGAAGUUCGCCCUCG AAACAAAUUCAUUU-3' (SEQ ID NO: 456), 5'-CUUCACUGAUAAAGUGGAGAACCGCUUCACCAAAAGCUGUCCCUUAGGGGAUUAGA ACUUGAGUGAAGGUGGGCUGCUUGCAUCAGCCUAAUGUCGAGAAUUCGCCCUCGAA ACAAAUUCAUUU-3' (SEQ ID NO: 457), 5'-CUUCACUGAUAAAGUGGAGAACCGCUUCACCAAAAGCUGUCCCUUAGGGGAUUAGA ACUUGAGUGAAGGUGGGCUGCUUGCAUCAGCCUAAUGUCGAGAAUUCGCCUCGAAA CAAAUUCAUUU-3' (SEQ ID NO: 458), 5'-CUUCACUGAUAAAGUGGAGAACCGCUUCACCAAAAGCUGUCCCUUAGGGGAUUAGA ACUUGAGUGAAGGUGGGCUGCUUGCAUCAGCCUAAUGUCGAGAUUCGCCUCGAAAC AAAUUCAUUU-3' (SEQ ID NO: 459), and 5'-CUUCACUGAUAAAGUGGAGAACCGCUUCACCAAAAGCUGUCCCUUAGGGGAUUAGA ACUUGAGUGAAGGUGGGCUGCUUGCAUCAGCCUAAUGUCGAGUUCGCUCGAAACAA AUUCAUUU-3' (SEQ ID NO: 460); and
SEQ ID NO: 3.

In an embodiment, a sequence of the engineered scaffold region in which the third region is modified may comprise:
a sequence in which the following sequences are linked to each other in a 5' to 3' direction:
SEQ ID NO: 10,
SEQ ID NO: 11,
a sequence selected from the group consisting of SEQ ID NOs: 434 to 447, and
SEQ ID NO: 14; and
a sequence in which SEQ ID NO: 16 and 5'-AUGCAAC-3' are linked to each other in a 5' to 3' direction.

In an embodiment, a sequence of the engineered scaffold region in which the third region is modified may be one in which SEQ ID NO: 10, SEQ ID NO: 11, SEQ ID NO: 12, a sequence selected from the group consisting of SEQ ID NOs: 434 to 447, a linker, SEQ ID NO: 15, and 5'-AUGCAAC-3' are linked to each other in a 5' to 3' direction.

Here, the linker may be 5'-GAAA-3'.

In an embodiment, a sequence of the engineered scaffold region in which the third region is modified may be a sequence selected from the group consisting of 5'-CUUCACUGAUAAAGUGGAGAACCGCUUCACCAAAAGCUGUCCCUUAGGGGAUUAGA ACUUGAGUGAAGGUGGGCUGCUUGCAUCAGCCUAAUGUCGAGAAGUGCUUUCGAAA GUAACCCUCGAAACAAAUUCAUUUGAAAGAAUGAAGGAAUGCAAC-3'(SEQ ID NO: 461). 5'-CUUCACUGAUAAAGUGGAGAACCGCUUCACCAAAAGCUGUCCCUUAGGGGAUUAGA ACUUGAGUGAAGGUGGGCUGCUUGCAUCAGCCUAAUGUCGAGAAGUGCUUUUUCG AAAGUAACCCUCGAAACAAAUUCAUUUGAAAGAAUGAAGGAAUGCAAC-3'(SEQ ID NO: 462), 5'-CUUCACUGAUAAAGUGGAGAACCGCUUCACCAAAAGCUGUCCCUUAGGGGAUUAGA ACUUGAGUGAAGGUGGGCUGCUUGCAUCAGCCUAAUGUCGAGAAGUGCUUUUCGA AGUAACCCUCGAAACAAAUUCAUUUGAAAGAAUGAAGGAAUGCAAC-3'(SEQ ID NO: 463), 5'-CUUCACUGAUAAAGUGGAGAACCGCUUCACCAAAAGCUGUCCCUUAGGGGAUUAGA ACUUGAGUGAAGGUGGGCUGCUUGCAUCAGCCUAAUGUCGAGAAGUGCUCUUCGG AGUAACCCUCGAAACAAAUUCAUUUGAAAGAAUGAAGGAAUGCAAC-3'(SEQ ID NO: 464), 5'-CUUCACUGAUAAAGUGGAGAACCGCUUCACCAAAAGCUGUCCCUUAGGGGAUUAGA ACUUGAGUGAAGGUGGGCUGCUUGCAUCAGCCUAAUGUCGAGAAGUGCUUUCGAG UAACCCUCGAAACAAAUUCAUUUGAAAGAAUGAAGGAAUGCAAC-3'(SEQ ID NO: 465), 5'-CUUCACUGAUAAAGUGGAGAACCGCUUCACCAAAAGCUGUCCCUUAGGGGAUUAGA ACUUGAGUGAAGGUGGGCUGCUUGCAUCAGCCUAAUGUCGAGAAGUGCUUCGGUA ACCCUCGAAACAAAUUCAUUUGAAAGAAUGAAGGAAUGCAAC-3'(SEQ ID NO: 466), 5'-CUUCACUGAUAAAGUGGAGAACCGCUUCACCAAAAGCUGUCCCUUAGGGGAUUAGA ACUUGAGUGAAGGUGGGCUGCUUGCAUCAGCCUAAUGUCGAGAAGUGUUCGUAAC CCUCGAAACAAAUUCAUUUGAAAGAAUGAAGGAAUGCAAC-3'(SEQ ID NO: 467), 5'-CUUCACUGAUAAAGUGGAGAACCGCUUCACCAAAAGCUGUCCCUUAGGGGAUUAGA ACUUGAGUGAAGGUGGGCUGCUUGCAUCAGCCUAAUGUCGAGAAGUUUCGAACCCU CGAAACAAAUUCAUUUGAAAGAAUGAAGGAAUGCAAC-3'(SEQ ID NO: 468), 5'-CUUCACUGAUAAAGUGGAGAACCGCUUCACCAAAAGCUGUCCCUUAGGGGAUUAGA ACUUGAGUGAAGGUGGGCUGCUUGCAUCAGCCUAAUGUCGAGAAGUUCGACCCUC GAAACAAAUUCAUUUGAAAGAAUGAAGGAAUGCAAC-3'(SEQ ID NO: 469), 5'-CUUCACUGAUAAAGUGGAGAACCGCUUCACCAAAAGCUGUCCCUUAGGGGAUUAGA ACUUGAGUGAAGGUGGGCUGCUUGCAUCAGCCUAAUGUCGAGAAGUUCGCCCUCG AAACAAAUUCAUUUGAAAGAAUGAAGGAAUGCAAC-3'(SEQ ID NO: 470), 5'-CUUCACUGAUAAAGUGGAGAACCGCUUCACCAAAAGCUGUCCCUUAGGGGAUUAGA ACUUGAGUGAAGGUGGGCUGCUUGCAUCAGCCUAAUGUCGAGAAUUCGCCCUCGAA ACAAAUUCAUUUGAAAGAAUGAAGGAAUGCAAC-3'(SEQ ID NO: 471), 5'-CUUCACUGAUAAAGUGGAGAACCGCUUCACCAAAAGCUGUCCCUUAGGGGAUUAGA ACUUGAGUGAAGGUGGGCUGCUUGCAUCAGCCUAAUGUCGAGAAUUCGCCUCGAAA CAAAUUCAUUUGAAAGAAUGAAGGAAUGCAAC-3'(SEQ ID NO: 472), 5'-CUUCACUGAUAAAGUGGAGAACCGCUUCACCAAAAGCUGUCCCUUAGGGGAUUAGA ACUUGAGUGAAGGUGGGCUGCUUGCAUCAGCCUAAUGUCGAGAUUCGCCUCGAAAC AAAUUCAUUUGAAAGAAUGAAGGAAUGCAAC-3'(SEQ ID NO: 473), and 5'-CUUCACUGAUAAAGUGGAGAACCGCUUCACCAAAAGCUGUCCCUUAGGGGAUUAGA ACUUGAGUGAAGGUGGGCUGCUUGCAUCAGCCUAAUGUCGAGUUCGCUCGAAACAA AUUCAUUUGAAAGAAUGAAGGAAUGCAAC-3'(SEQ ID NO: 474).

### 7) Engineered scaffold region 4 - Modification of fourth and fifth regions

### Overview of modification of fourth and fifth regions

The engineered scaffold region provided herein may be an engineered form of a naturally occurring scaffold region in which the fourth and fifth regions are modified. The fourth and fifth regions comprise parts that hybridize to each other to form a stem in the CRISPR/Cas12f1 complex, and thus the corresponding parts may be modified together to constitute an engineered scaffold region.

In an embodiment, the engineered scaffold region may comprise a modified fourth region and/or a modified fifth region.

The modified fourth region is characterized in that it is obtained by removing one or more nucleotides from the fourth region of a naturally occurring scaffold region. The modified fifth region is characterized in that it is obtained by removing one or more nucleotides from the fifth region of a naturally occurring scaffold region.

In an embodiment, the engineered scaffold region included in the engineered Cas12f1 guide RNA may be an engineered form of a naturally occurring scaffold region in which one or more nucleotides are removed from the fourth region and/or the fifth region.

In an embodiment, the modified fourth region has 5'-AACAAA-3' at the 5' end. In an embodiment, the modified fifth region has 5'-GGA-3' at the 3' end.

### Modification detail 1 of fourth and fifth regions - Removal of some nucleotides

The fourth region of the engineered scaffold region may be a modified form of a fourth region of the naturally occurring scaffold region from which one or more nucleotides are removed. The fifth region of the engineered scaffold region may be a modified form of a fifth region of the naturally occurring scaffold region from which one or more nucleotides are removed.

In an embodiment, the modified fourth region of the engineered scaffold region may be a modified form of a fourth region of the naturally occurring scaffold region from which 1 to 7 nucleotides are removed. In an embodiment, the modified fourth region of the engineered scaffold region may be a modified form of a fourth region of the naturally occurring scaffold region from which 1 to 28 nucleotides are removed. In an embodiment, the modified fourth region may be a modified form of a fourth region of the naturally occurring scaffold region from which one or more nucleotides, of the 7^{th} to 13^{th} nucleotides from the 5' end based on the sequence of SEQ ID NO: 13, are removed. In an embodiment, the modified fourth region may be a modified form of a fourth region of the naturally occurring scaffold region from which one or more nucleotides, of the 7th to 34^{th} nucleotides from the 5' end based on the sequence of SEQ ID NO: 14, are removed.

In an embodiment, a sequence of the modified fourth region comprises at least 5'-AACAAA-3'.

In an embodiment, the modified fifth region of the engineered scaffold region may be a modified form of a fifth region of the naturally occurring scaffold region from which 1 to 7 nucleotides are removed. In an embodiment, the modified fifth region of the engineered scaffold region may be a modified form of a fifth region of the naturally occurring scaffold region from which 1 to 27 nucleotides are removed. In an embodiment, the modified fifth region may be a modified form of a fifth region of the naturally occurring scaffold region from which one or more nucleotides, of the 1^{st} to 7^{th} nucleotides from the 5' end based on the sequence of SEQ ID NO: 15, are removed. In an embodiment, the modified fifth region may be a modified form of a fifth region of the naturally occurring scaffold region from which one or more nucleotides, of the 1^{st} to 27^{th} nucleotides from the 5' end based on the sequence of SEQ ID NO: 16, are removed.

In an embodiment, the modified fifth region comprises at least 5'-GGA-3'.

### Modification detail 2 of fourth and fifth regions - Removal in base pair

The fourth region and the fifth region are known to form a stem by complementarily binding to each other in the CRISPR/Cas12 complex. Since the above-described modifications of the fourth and fifth regions are subject to one or more nucleotides constituting the stem, the modifications of the fourth and fifth regions may be made to remove nucleotides constituting the stem in base pair.

In an embodiment, the modified fourth and fifth regions may be engineered forms of fourth and fifth regions of the naturally occurring scaffold region from which one or more pairs of nucleotides forming base pairs in the CRISPR/Cas12f1 complex, of the 7^{th} to 13^{th} nucleotides from the 5' end based on the sequence of SEQ ID NO: 13 and the 1^{st} to 7^{th} nucleotides from the 5' end based on the sequence of SEQ ID NO: 15, are removed.

In an embodiment, the modified fourth and fifth regions may be engineered forms of fourth and fifth regions of the naturally occurring scaffold region from which one or more pairs of nucleotides forming base pairs and/or one or more nucleotides not forming a base pair in the CRISPR/Cas12f1 complex, of the 7^{th} to 13^{th} nucleotides from the 5' end based on the sequence of SEQ ID NO: 13 and the 1^{st} to 7^{th} nucleotides from the 5' end based on the sequence of SEQ ID NO: 15, are removed.

In an embodiment, the modified fourth and fifth regions may be engineered forms of fourth and fifth regions of the naturally occurring scaffold region from which one or more pairs of nucleotides forming base pairs and/or one or more mismatched pairs of nucleotides in the CRISPR/Cas12f1 complex, of the 7^{th} to 13^{th} nucleotides from the 5' end based on the sequence of SEQ ID NO: 13 and the 1^{st} to 7^{th} nucleotides from the 5' end based on the sequence of SEQ ID NO: 15, are removed.

In an embodiment, the modified fourth and fifth regions may be engineered forms of fourth and fifth regions of the naturally occurring scaffold region from which one or more pairs of nucleotides forming base pairs in the CRISPR/Cas12f1 complex, of the 7^{th} to 34^{th} nucleotides from the 5' end based on the sequence of SEQ ID NO: 14 and the 1^{st} to 27^{th} nucleotides from the 5' end based on the sequence of SEQ ID NO: 16, are removed.

In an embodiment, the modified fourth and fifth regions may be engineered forms of fourth and fifth regions of the naturally occurring scaffold region from which one or more pairs of nucleotides forming base pairs and/or one or more nucleotides not forming a base pair in the CRISPR/Cas12f1 complex, of the 7^{th} to 34^{th} nucleotides from the 5' end based on the sequence of SEQ ID NO: 14 and the 1^{st} to 27^{th} nucleotides from the 5' end based on the sequence of SEQ ID NO: 16, are removed.

In an embodiment, the modified fourth and fifth regions may be engineered forms of fourth and fifth regions of the naturally occurring scaffold region from which one or more pairs of nucleotides forming base pairs and/or one or more mismatched pairs of nucleotides in the CRISPR/Cas12f1 complex, of the 7^{th} to 34^{th} nucleotides based on the sequence of SEQ ID NO: 14 and the 1^{st} to 27^{th} nucleotides based on the sequence of SEQ ID NO: 16, are removed.

### Examples of sequences of modified fourth region and fifth region

In an embodiment, a sequence of the modified fourth region may be selected from the group consisting of 5'-AACAAA-3', 5'-AACAAAU-3', 5'-AACAAAUU-3', 5'-AACAAAUUC-3', 5'-AACAAAUUCA-3' (SEQ ID NO: 67), 5'-AACAAAUUCAU-3' (SEQ ID NO: 68), and 5'-AACAAAUUCAUU-3' (SEQ ID NO: 69).

In an embodiment, the sequence of the modified fourth region may be selected from the group consisting of 5'-AACAAA-3', 5'-AACAAAU-3', 5'-AACAAAUU-3', 5'-AACAAAUUC-3', 5'-AACAAAUUCA-3' (SEQ ID NO: 67), 5'-AACAAAUUCAU-3' (SEQ ID NO: 68), 5'-AACAAAUUCAUU-3' (SEQ ID NO: 69), 5'-AACAAAUUCAUUU-3' (SEQ ID NO: 70), 5'-AACAAAUUCAUUUU-3' (SEQ ID NO: 71), 5'-AACAAAUUCAUUUUU-3' (SEQ ID NO: 72), 5'-AACAAAUUCAUUUUUC-3' (SEQ ID NO: 73), 5'-AACAAAUUCAUUUUUCC-3' (SEQ ID NO: 74), 5'-AACAAAUUCAUUUUUCCU-3' (SEQ ID NO: 75), 5'-AACAAAUUCAUUUUUCCUC-3' (SEQ ID NO: 76), 5'-AACAAAUUCAUUUUUCCUCU-3' (SEQ ID NO: 77), 5'-AACAAAUUCAUUUUUCCUCUC-3' (SEQ ID NO: 78), 5'-AACAAAUUCAUUUUUCCUCUCC-3' (SEQ ID NO: 79), 5'-AACAAAUUCAUUUUUCCUCUCCA-3' (SEQ ID NO: 80), 5'-AACAAAUUCAUUUUUCCUCUCCAA-3' (SEQ ID NO: 81), 5'-AACAAAUUCAUUUUUCCUCUCCAAU-3' (SEQ ID NO: 82), 5'-AACAAAUUCAUUUUUCCUCUCCAAUU-3' (SEQ ID NO: 83), 5'-AACAAAUUCAUUUUUCCUCUCCAAUUC-3' (SEQ ID NO: 84), 5'-AACAAAUUCAUUUUUCCUCUCCAAUUCU-3' (SEQ ID NO: 85), 5'-AACAAAUUCAUUUUUCCUCUCCAAUUCUG-3' (SEQ ID NO: 86), 5'-AACAAAUUCAUUUUUCCUCUCCAAUUCUGC-3' (SEQ ID NO: 87), 5'-AACAAAUUCAUUUUUCCUCUCCAAUUCUGCA-3' (SEQ ID NO: 88), 5'-AAACAAAUUCAUUUUUCCUCUCCAAUUCUGCAC-3' (SEQ ID NO: 89), and 5'-AACAAAUUCAUUUUUCCUCUCCAAUUCUGCACA-3' (SEQ ID NO: 90).

In an embodiment, a sequence of the modified fifth region may be selected from 5'-GGA-3', 5'-AGGA-3', 5'-AAGGA-3', 5'-GAAGGA-3', 5'-UGAAGGA-3', 5'-AUGAAGGA-3', and 5'-AAUGAAGGA-3'.

In an embodiment, the sequence of the modified fifth region may be selected from the group consisting of 5'-GGA-3', 5'-AGGA-3', 5'-AAGGA-3', 5'-GAAGGA-3', 5'-UGAAGGA-3', 5'-AUGAAGGA-3', 5'-AAUGAAGGA-3', 5'-GAAUGAAGGA-3' (SEQ ID NO: 91), 5'-CGAAUGAAGGA-3' (SEQ ID NO: 92), 5'-ACGAAUGAAGGA-3' (SEQ ID NO: 93), 5'-GACGAAUGAAGGA-3' (SEQ ID NO: 94), 5'-AGACGAAUGAAGGA-3' (SEQ ID NO: 95), 5'-UAGACGAAUGAAGGA-3' (SEQ ID NO: 96), 5'-AUAGACGAAUGAAGGA-3' (SEQ ID NO: 97), 5'-AAUAGACGAAUGAAGGA-3' (SEQ ID NO: 98), 5'-GAAUAGACGAAUGAAGGA-3' (SEQ ID NO: 99), 5'-CGAAUAGACGAAUGAAGGA-3' (SEQ ID NO: 100), 5'-CCGAAUAGACGAAUGAAGGA-3' (SEQ ID NO: 101), 5'-CCCGAAUAGACGAAUGAAGGA-3' (SEQ ID NO: 102), 5'-ACCCGAAUAGACGAAUGAAGGA-3' (SEQ ID NO: 103), 5'-AACCCGAAUAGACGAAUGAAGGA-3' (SEQ ID NO: 104), 5'-GAACCCGAAUAGACGAAUGAAGGA-3' (SEQ ID NO: 105), 5'-AGAACCCGAAUAGACGAAUGAAGGA-3' (SEQ ID NO: 106), 5'-CAGAACCCGAAUAGACGAAUGAAGGA-3' (SEQ ID NO: 107), 5'-GCAGAACCCGAAUAGACGAAUGAAGGA-3' (SEQ ID NO: 108), 5'-UGCAGAACCCGAAUAGACGAAUGAAGGA-3' (SEQ ID NO: 109), and 5'-UUGCAGAACCCGAAUAGACGAAUGAAGGA-3' (SEQ ID NO: 110).

In an embodiment, a sequence of the engineered scaffold region in which the fourth and fifth regions are modified may comprise:
a sequence in which the following sequences are linked to each other in a 5' to 3' direction:
SEQ ID NO: 10,
SEQ ID NO: 11,
SEQ ID NO: 12, and
a sequence selected from the group consisting of 5'-AACAAA-3', 5'-AACAAAU-3', 5'-AACAAAUU-3', 5'-AACAAAUUC-3', and SEQ ID NOs: 67 to 69; and
a sequence in which the following sequences are linked to each other in a 5' to 3' direction:
   a sequence selected from the group consisting of 5'-GGA-3', 5'-AGGA-3', 5'-AAGGA-3', 5'-GAAGGA-3', 5'-UGAAGGA-3', 5'-AUGAAGGA-3', and 5'-AAUGAAGGA-3'; and
   5'-AUGCAAC-3'.

In an embodiment, a sequence of the engineered scaffold region in which the fourth and fifth regions are modified may comprise:
a sequence selected from the group consisting of 5'-CUUCACUGAUAAAGUGGAGAACCGCUUCACCAAAAGCUGUCCCUUAGGGGAUUAGA ACUUGAGUGAAGGUGGGCUGCUUGCAUCAGCCUAAUGUCGAGAAGUGCUUUCUUC GGAAAGUAACCCUCGAAACAAA-3' (SEQ ID NO: 150), 5'-CUUCACUGAUAAAGUGGAGAACCGCUUCACCAAAAGCUGUCCCUUAGGGGAUUAGA ACUUGAGUGAAGGUGGGCUGCUUGCAUCAGCCUAAUGUCGAGAAGUGCUUUCUUC GGAAAGUAACCCUCGAAACAAAU-3' (SEQ ID NO: 151), 5'-CUUCACUGAUAAAGUGGAGAACCGCUUCACCAAAAGCUGUCCCUUAGGGGAUUAGA ACUUGAGUGAAGGUGGGCUGCUUGCAUCAGCCUAAUGUCGAGAAGUGCUUUCUUC GGAAAGUAACCCUCGAAACAAAUU-3' (SEQ ID NO: 152), 5'-CUUCACUGAUAAAGUGGAGAACCGCUUCACCAAAAGCUGUCCCUUAGGGGAUUAGA ACUUGAGUGAAGGUGGGCUGCUUGCAUCAGCCUAAUGUCGAGAAGUGCUUUCUUC GGAAAGUAACCCUCGAAACAAAUUC-3' (SEQ ID NO: 153), 5'-CUUCACUGAUAAAGUGGAGAACCGCUUCACCAAAAGCUGUCCCUUAGGGGAUUAGA ACUUGAGUGAAGGUGGGCUGCUUGCAUCAGCCUAAUGUCGAGAAGUGCUUUCUUC GGAAAGUAACCCUCGAAACAAAUUCA-3' (SEQ ID NO: 154), 5'-CUUCACUGAUAAAGUGGAGAACCGCUUCACCAAAAGCUGUCCCUUAGGGGAUUAGA ACUUGAGUGAAGGUGGGCUGCUUGCAUCAGCCUAAUGUCGAGAAGUGCUUUCUUC GGAAAGUAACCCUCGAAACAAAUUCAU-3' (SEQ ID NO: 155), and 5'-CUUCACUGAUAAAGUGGAGAACCGCUUCACCAAAAGCUGUCCCUUAGGGGAUUAGA ACUUGAGUGAAGGUGGGCUGCUUGCAUCAGCCUAAUGUCGAGAAGUGCUUUCUUC GGAAAGUAACCCUCGAAACAAAUUCAUU-3' (SEQ ID NO: 156); and
a sequence selected from the group consisting of 5'-GGAAUGCAAC-3' (SEQ ID NO: 161), 5'-AGGAAUGCAAC-3' (SEQ ID NO: 162), 5'-AAGGAAUGCAAC-3' (SEQ ID NO: 163), 5'-GAAGGAAUGCAAC-3' (SEQ ID NO: 164), 5'-UGAAGGAAUGCAAC-3' (SEQ ID NO: 165), 5'-AUGAAGGAAUGCAAC-3' (SEQ ID NO: 166), and 5'-AAUGAAGGAAUGCAAC-3' (SEQ ID NO: 167).

In an embodiment, a sequence of the engineered scaffold region in which the fourth and fifth regions are modified may comprise:
a sequence in which the following sequences are linked to each other in a 5' to 3' direction:
SEQ ID NO: 10,
SEQ ID NO: 11,
SEQ ID NO: 12, and
a sequence selected from the group consisting of 5'-AACAAA-3', 5'-AACAAAU-3', 5'-AACAAAUU-3', 5'-AACAAAUUC-3', and SEQ ID NOs: 67 to 90; and
a sequence in which the following sequences are linked to each other in a 5' to 3' direction:
   a sequence selected from the group consisting of 5'-GGA-3', 5'-AGGA-3', 5'-AAGGA-3', 5'-GAAGGA-3', 5'-UGAAGGA-3', 5'-AUGAAGGA-3', 5'-AAUGAAGGA-3', and SEQ ID NOs: 91 to 110; and
   5'-AUGCAAC-3'.

In an embodiment, a sequence of the engineered scaffold region in which the fourth and fifth regions are modified may be a sequence in which the following sequences are linked to each other in a 5' to 3' direction:
SEQ ID NO: 10,
SEQ ID NO: 11,
SEQ ID NO: 12,
a sequence selected from 5'-AACAAAGAAAGGA-3' (SEQ ID NO: 111), 5'-AACAAAUGAAAAGGA-3' (SEQ ID NO: 112), 5'-AACAAAUUGAAAAAGGA-3' (SEQ ID NO: 113), 5'-AACAAAUUCGAAAGAAGGA-3' (SEQ ID NO: 114), 5'-AACAAAUUCAGAAAUGAAGGA-3' (SEQ ID NO: 115), 5'-AACAAAUUCAUGAAAAUGAAGGA-3' (SEQ ID NO: 116), and 5'-AACAAAUUCAUUGAAAAAUGAAGGA-3' (SEQ ID NO: 117), and
5'-AUGCAAC-3'.

In an embodiment, a sequence of the engineered scaffold region in which the fourth and fifth regions are modified may be selected from the group consisting of 5'-CUUCACUGAUAAAGUGGAGAACCGCUUCACCAAAAGCUGUCCCUUAGGGGAUUAGA ACUUGAGUGAAGGUGGGCUGCUUGCAUCAGCCUAAUGUCGAGAAGUGCUUUCUUC GGAAAGUAACCCUCGAAACAAAGAAAGGAAUGCAAC-3' (SEQ ID NO: 200), 5'-CUUCACUGAUAAAGUGGAGAACCGCUUCACCAAAAGCUGUCCCUUAGGGGAUUAGA ACUUGAGUGAAGGUGGGCUGCUUGCAUCAGCCUAAUGUCGAGAAGUGCUUUCUUC GGAAAGUAACCCUCGAAACAAAUGAAAAGGAAUGCAAC-3' (SEQ ID NO: 201), 5'-CUUCACUGAUAAAGUGGAGAACCGCUUCACCAAAAGCUGUCCCUUAGGGGAUUAGA ACUUGAGUGAAGGUGGGCUGCUUGCAUCAGCCUAAUGUCGAGAAGUGCUUUCUUC GGAAAGUAACCCUCGAAACAAAUUGAAAAAGGAAUGCAAC-3' (SEQ ID NO: 202), 5'-CUUCACUGAUAAAGUGGAGAACCGCUUCACCAAAAGCUGUCCCUUAGGGGAUUAGA ACUUGAGUGAAGGUGGGCUGCUUGCAUCAGCCUAAUGUCGAGAAGUGCUUUCUUC GGAAAGUAACCCUCGAAACAAAUUCGAAAGAAGGAAUGCAAC-3' (SEQ ID NO: 203), 5'-CUUCACUGAUAAAGUGGAGAACCGCUUCACCAAAAGCUGUCCCUUAGGGGAUUAGA ACUUGAGUGAAGGUGGGCUGCUUGCAUCAGCCUAAUGUCGAGAAGUGCUUUCUUC GGAAAGUAACCCUCGAAACAAAUUCAGAAAUGAAGGAAUGCAAC-3' (SEQ ID NO: 204), 5'-CUUCACUGAUAAAGUGGAGAACCGCUUCACCAAAAGCUGUCCCUUAGGGGAUUAGA ACUUGAGUGAAGGUGGGCUGCUUGCAUCAGCCUAAUGUCGAGAAGUGCUUUCUUC GGAAAGUAACCCUCGAAACAAAUUCAUGAAAAUGAAGGAAUGCAAC-3' (SEQ ID NO: 205), and 5'-CUUCACUGAUAAAGUGGAGAACCGCUUCACCAAAAGCUGUCCCUUAGGGGAUUAGA ACUUGAGUGAAGGUGGGCUGCUUGCAUCAGCCUAAUGUCGAGAAGUGCUUUCUUC GGAAAGUAACCCUCGAAACAAAUUCAUUGAAAAAUGAAGGAAUGCAAC-3' (SEQ ID NO: 206).

### 8) Engineered scaffold region 5 - Combination of respective modifications

### Overview of combination of respective modifications

The engineered scaffold region included in the engineered Cas12f1 guide RNA provided herein may be a modified form of a naturally occurring scaffold region in which one or more of the above-mentioned modifications for respective regions are combined.

In an embodiment, the engineered scaffold region may comprise a modified first region and a modified second region.

In an embodiment, the engineered scaffold region may be one in which a modified second region is included and the first region is removed.

In an embodiment, the engineered scaffold region may be one in which a modified first region is included and the second region is removed.

In an embodiment, the engineered scaffold region may be one in which the first region and the second region are removed.

In an embodiment, the engineered scaffold region may comprise a modified first region and a modified third region.

In an embodiment, the engineered scaffold region may be one in which a modified third region is included and the first region is removed.

In an embodiment, the engineered scaffold region may comprise a modified first region and modified fourth and fifth regions.

In an embodiment, the engineered scaffold region may be one in which modified fourth and fifth regions are included and the first region is removed.

In an embodiment, the engineered scaffold region may comprise a modified second region and a modified third region.

In an embodiment, the engineered scaffold region may be one in which a modified third region is included and the second region is removed.

In an embodiment, the engineered scaffold region may comprise a modified second region and modified fourth and fifth regions.

In an embodiment, the engineered scaffold region may be one in which modified fourth and fifth regions are included and the second region is removed.

In an embodiment, the engineered scaffold region may comprise a modified third region, and modified fourth and fifth regions.

In an embodiment, the engineered scaffold region may comprise a modified first region, a modified second region, and a modified third region.

In an embodiment, the engineered scaffold region may be one in which a modified second region and a modified third region are included and the first region is removed.

In an embodiment, the engineered scaffold region may be one in which a modified first region and a modified third region are included and the second region is removed.

In an embodiment, the engineered scaffold region may be one in which a modified third region is included and the first region and the second region are removed.

In an embodiment, the engineered scaffold region may comprise a modified first region, a modified second region, and modified fourth and fifth regions.

In an embodiment, the engineered scaffold region may be one in which a modified second region and modified fourth and fifth regions are included and the first region is removed.

In an embodiment, the engineered scaffold region may be one in which a modified first region and modified fourth and fifth regions are included and the second region is removed.

In an embodiment, the engineered scaffold region may be one in which modified fourth and fifth regions are included, and the first region and the second region are removed.

In an embodiment, the engineered scaffold region may comprise a modified first region, a modified third region, and modified fourth and fifth regions.

In an embodiment, the engineered scaffold region may be one in which a modified third region and modified fourth and fifth regions are included and the first region is removed.

In an embodiment, the engineered scaffold region may comprise a modified second region, a modified third region, and modified fourth and fifth regions.

In an embodiment, the engineered scaffold region may be one in which a modified third region and modified fourth and fifth regions are included and the second region is removed.

In an embodiment, the engineered scaffold region may comprise a modified first region, a modified second region, a modified third region, and modified fourth and fifth regions.

In an embodiment, the engineered scaffold region may be one in which a modified second region, a modified third region, and modified fourth and fifth regions are included, and the first region is removed.

In an embodiment, the engineered scaffold region may be one in which a modified first region, a modified third region, and modified fourth and fifth regions are included and the second region is removed.

In an embodiment, the engineered scaffold region may be one in which a modified third region and modified fourth and fifth regions are included and the first region and the second region are removed.

Here, the modified regions are as described above in the section for modification of each of the regions.

### Combination 1 of respective modifications - Modification of first region and modification of second region

In an embodiment, the engineered scaffold region may comprise a modified first region and a modified second region. Here, the modified first region includes any one of the modifications described in the section "Engineered scaffold region 1 - Modification of first region." Here, the modified second region includes any one of the modifications described in the section "Engineered scaffold region 2 - Modification of second region."

In an embodiment, a sequence of the engineered scaffold region in which the first region and the second region are modified may comprise:
a sequence in which the following sequences are linked to each other in a 5' to 3' direction:
a sequence selected from the group consisting of 5'-A-3', 5'-AA-3', 5'-GAA-3', 5'-AGAA-3', 5'-GAGAA-3', 5'-GGAGAA-3', 5'-UGGAGAA-3', 5'-GUGGAGAA-3', 5'-AGUGGAGAA-3', and SEQ ID NOs: 17 to 27,
a sequence selected from the group consisting of 5'-G-3', 5'-UUAGG-3', 5'-CUUAGGG-3', 5'-CUUAGUGG-3', and SEQ ID NOs: 342 to 362,
SEQ ID NO: 12, and
SEQ ID NO: 13; and
a sequence in which SEQ ID NO: 15 and 5'-AUGCAAC-3' are linked to each other in a 5' to 3' direction.

In an embodiment, a sequence of the engineered scaffold region in which the first region and the second region are modified may comprise in a 5' to 3' direction: 
5'-
ACCGCUUCACCAUUAGUGAGUGAAGGUGGGCUGCUUGCAUCAGCCUAAUGUCGAGA
AGUGCUUUCUUCGGAAAGUAACCCUCGAAACAAAUUCAUUU-3' (SEQ ID NO: 157);
 and
5'-GAAUGAAGGAAUGCAAC-3' (SEQ ID NO: 3).

In an embodiment, a sequence of the engineered scaffold region in which the first region and the second region are modified may be a sequence in which the following sequences are linked to each other in a 5' to 3' direction:
a sequence selected from the group consisting of 5'-A-3', 5'-AA-3', 5'-GAA-3', 5'-AGAA-3', 5'-GAGAA-3', 5'-GGAGAA-3', 5'-UGGAGAA-3', 5'-GUGGAGAA-3', 5'-AGUGGAGAA-3', and SEQ ID NOs: 17 to 27,
a sequence selected from the group consisting of 5'-G-3', 5'-UUAGG-3', 5'-CUUAGGG-3', 5'-CUUAGUGG-3', and SEQ ID NOs: 342 to 362,
SEQ ID NO: 12,
SEQ ID NO: 13,
a linker,
SEQ ID NO: 15, and
5'-AUGCAAC-3'.

Here, the linker may be 5'-GAAA-3'.

In an embodiment, a sequence of the engineered scaffold region in which the first region and the second region are modified may be 5'-ACCGCUUCACCAUUAGUGAGUGAAGGUGGGCUGCUUGCAUCAGCCUAAUGUCGAGA AGUGCUUUCUUCGGAAAGUAACCCUCGAAACAAAUUCAUUUGAAAGAAUGAAGGAAU GCAAC-3' (SEQ ID NO: 207).

### Combination 2 of respective modifications - Modification of second region and removal of first region

In an embodiment, the engineered scaffold region may be one in which a modified second region is included and the first region is removed. Here, the engineered scaffold region may not comprise a region corresponding to the first region of the naturally occurring scaffold region. Here, the modified second region includes any one of the modifications described in the section "Engineered scaffold region 2 - Modification of second region."

In an embodiment, a sequence of the engineered scaffold region in which the second region is modified and the first region is removed may comprise:
a sequence in which the following sequences are linked to each other in a 5' to 3' direction:
a sequence selected from the group consisting of 5'-G-3', 5'-UUAGG-3', 5'-CUUAGGG-3', 5'-CUUAGUGG-3', and SEQ ID NOs: 342 to 362,
SEQ ID NO: 12, and
SEQ ID NO: 13; and
a sequence in which SEQ ID NO: 15 and 5'-AUGCAAC-3' are linked to each other in a 5' to 3' direction.

In an embodiment, a sequence of the engineered scaffold region in which the second region is modified and the first region is removed may be a sequence in which the following sequences are linked to each other in a 5' to 3' direction:
a sequence selected from the group consisting of 5'-G-3', 5'-UUAGG-3', 5'-CUUAGGG-3', 5'-CUUAGUGG-3', and SEQ ID NOs: 342 to 362,
SEQ ID NO: 12,
SEQ ID NO: 13,
a linker,
SEQ ID NO: 15, and
5'-AUGCAAC-3'.

Here, the linker may be 5'-GAAA-3'.

### Combination 3 of respective modifications - Modification of first region and removal of second region

In an embodiment, the engineered scaffold region may be one in which a modified first region is included and the second region is removed. Here, the modified first region includes any one of the modifications described in the section "Engineered scaffold region 1 - Modification of first region." Here, the engineered scaffold region may not comprise a region corresponding to the second region of the naturally occurring scaffold region.

In an embodiment, a sequence of the engineered scaffold region in which the first region is modified and the second region is removed may comprise:
a sequence in which the following sequences are linked to each other in a 5' to 3' direction:
a sequence selected from the group consisting of 5'-A-3', 5'-AA-3', 5'-GAA-3', 5'-AGAA-3', 5'-GAGAA-3', 5'-GGAGAA-3', 5'-UGGAGAA-3', 5'-GUGGAGAA-3', 5'-AGUGGAGAA-3', and SEQ ID NOs: 17 to 27,
SEQ ID NO: 12, and
SEQ ID NO: 13; and
a sequence in which SEQ ID NO: 15 and 5'-AUGCAAC-3' are linked to each other in a 5' to 3' direction.

In an embodiment, a sequence of the engineered scaffold region in which the first region is modified and the second region is removed may be one in which the following sequences are linked to each other in a 5' to 3' direction:
a sequence selected from the group consisting of 5'-A-3', 5'-AA-3', 5'-GAA-3', 5'-AGAA-3', 5'-GAGAA-3', 5'-GGAGAA-3', 5'-UGGAGAA-3', 5'-GUGGAGAA-3', 5'-AGUGGAGAA-3', and SEQ ID NOs: 17 to 27,
SEQ ID NO: 12,
SEQ ID NO: 13,
a linker,
SEQ ID NO: 15, and
5'-AUGCAAC-3'.

Here, the linker may be 5'-GAAA-3'.

### Combination 4 of respective modifications - Removal of first region and removal of second region

In an embodiment, the engineered scaffold region may be one in which the first region and the second region are removed. Here, the engineered scaffold region may not comprise a region corresponding to the first region of the naturally occurring scaffold region. Here, the engineered scaffold region may not comprise a region corresponding to the second region of the naturally occurring scaffold region.

In an embodiment, a sequence of the engineered scaffold region in which the first region and the second region are removed may comprise:
a sequence in which the following sequences are linked to each other in a 5' to 3' direction:
SEQ ID NO: 12, and
SEQ ID NO: 13; and
a sequence in which SEQ ID NO: 15 and 5'-AUGCAAC-3' are linked to each other in a 5' to 3' direction.

In an embodiment, a sequence of the engineered scaffold region in which the first region and the second region are removed may be one in which the following sequences are linked to each other in a 5' to 3' direction:
SEQ ID NO: 12,
SEQ ID NO: 13,
a linker,
SEQ ID NO: 15, and
5'-AUGCAAC-3'.

Here, the linker may be 5'-GAAA-3'.

### Combination 5 of respective modifications - Modification of first region and modification of third region

In an embodiment, the engineered scaffold region may comprise a modified first region and a modified third region. Here, the modified first region includes any one of the modifications described in the section "Engineered scaffold region 1 - Modification of first region." Here, the modified third region includes any one of the modifications described in the section "Engineered scaffold region 3 - Modification of third region."

In an embodiment, a sequence of the engineered scaffold region in which the first region and the third region are modified may comprise:
a sequence in which the following sequences are linked to each other in a 5' to 3' direction:
a sequence selected from the group consisting of 5'-A-3', 5'-AA-3', 5'-GAA-3', 5'-AGAA-3', 5'-GAGAA-3', 5'-GGAGAA-3', 5'-UGGAGAA-3', 5'-GUGGAGAA-3', 5'-AGUGGAGAA-3', and SEQ ID NOs: 17 to 27,
SEQ ID NO: 11,
a sequence selected from SEQ ID NOs: 434 to 447, and
SEQ ID NO: 13; and
a sequence in which SEQ ID NO: 15 and 5'-AUGCAAC-3' are linked to each other in a 5' to 3' direction.

In an embodiment, a sequence of the engineered scaffold region in which the first region and the third region are modified may be a sequence in which the following sequences are linked to each other in a 5' to 3' direction:
a sequence selected from the group consisting of 5'-A-3', 5'-AA-3', 5'-GAA-3', 5'-AGAA-3', 5'-GAGAA-3', 5'-GGAGAA-3', 5'-UGGAGAA-3', 5'-GUGGAGAA-3', 5'-AGUGGAGAA-3', and SEQ ID NOs: 17 to 27,
SEQ ID NO: 11,
SEQ ID NO: 12,
a sequence selected from the group consisting of SEQ ID NOs: 434 to 447,
a linker,
SEQ ID NO: 15, and
5'-AUGCAAC-3'.

Here, the linker may be 5'-GAAA-3'.

### Combination 6 of respective modifications - Modification of third region and removal of first region

In an embodiment, the engineered scaffold region may be one in which a modified third region is included and the first region is removed. Here, the modified third region includes any one of the modifications described in the section "Engineered scaffold region 3 - Modification of third region." Here, the engineered scaffold region may not comprise a region corresponding to the first region of the naturally occurring scaffold region.

In an embodiment, a sequence of the engineered scaffold region in which the third region is modified and the first region is removed may comprise:
a sequence in which the following sequences are linked to each other in a 5' to 3' direction:
SEQ ID NO: 11,
a sequence selected from the group consisting of SEQ ID NOs: 434 to 447, and
SEQ ID NO: 13; and
a sequence in which SEQ ID NO: 15 and 5'-AUGCAAC-3' are linked to each other in a 5' to 3' direction.

In an embodiment, a sequence of the engineered scaffold region in which the third region is modified and the first region is removed may be a sequence in which the following sequences are linked to each other in a 5' to 3' direction:
SEQ ID NO: 11,
a sequence selected from the group consisting of SEQ ID NOs: 434 to 447,
a linker,
SEQ ID NO: 15, and
5'-AUGCAAC-3'.

Here, the linker may be 5'-GAAA-3'.

### Combination 7 of respective modifications - Modification of first region, and modification of fourth and fifth regions

In an embodiment, the engineered scaffold region may comprise a modified first region and modified fourth and fifth regions. Here, the modified first region includes any one of the modifications described in the section "Engineered scaffold region 1 - Modification of first region."Here, the modified fourth and fifth regions include any one of the modifications described in the section "Engineered scaffold region 4 - Modification of fourth and fifth regions."

In an embodiment, a sequence of the engineered scaffold region in which the first region and the fourth and fifth regions are modified may comprise:
a sequence in which the following sequences are linked to each other in a 5' to 3' direction:
a sequence selected from the group consisting of 5'-A-3', 5'-AA-3', 5'-GAA-3', 5'-AGAA-3', 5'-GAGAA-3', 5'-GGAGAA-3', 5'-UGGAGAA-3', 5'-GUGGAGAA-3', 5'-AGUGGAGAA-3', and SEQ ID NOs: 17 to 27,
SEQ ID NO: 11,
SEQ ID NO: 12, and
a sequence selected from the group consisting of 5'-AACAAA-3', 5'-AACAAAU-3', 5'-AACAAAUU-3', 5'-AACAAAUUC-3', and SEQ ID NOs: 67 to 69; and
a sequence in which the following sequences are linked to each other in a 5' to 3' direction:
   a sequence selected from the group consisting of 5'-GGA-3', 5'-AGGA-3', 5'-AAGGA-3', 5'-GAAGGA-3', 5'-UGAAGGA-3', 5'-AUGAAGGA-3', and 5'-AAUGAAGGA-3', and
   5'-AUGCAAC-3'.

In an embodiment, a sequence of the engineered scaffold region in which the first region, and the fourth and fifth regions are modified may comprise in a 5' to 3' direction:
a sequence of 5'-ACCGCUUCACCAAAAGCUGUCCCUUAGGGGAUUAGAACUUGAGUGAAGGUGGGCUG CUUGCAUCAGCCUAAUGUCGAGAAGUGCUUUCUUCGGAAAGUAACCCUCGAAACAA A-3' (SEQ ID NO: 158); and
5'-GGAAUGCAAC-3' (SEQ ID NO: 161).

In an embodiment, a sequence of the engineered scaffold region in which the first region, and the fourth and fifth regions are modified may be a sequence in which the following sequences are linked to each other in a 5' to 3' direction:
a sequence selected from the group consisting of 5'-A-3', 5'-AA-3', 5'-GAA-3', 5'-AGAA-3', 5'-GAGAA-3', 5'-GGAGAA-3', 5'-UGGAGAA-3', 5'-GUGGAGAA-3', 5'-AGUGGAGAA-3', and SEQ ID NOs: 17 to 27,
SEQ ID NO: 11,
SEQ ID NO: 12,
a sequence selected from SEQ ID NOs: 111 to 117, and
5'-AUGCAAC-3'.

In an embodiment, a sequence of the engineered scaffold region in which the first region and the fourth and fifth regions are modified may be 5'-ACCGCUUCACCAAAAGCUGUCCCUUAGGGGAUUAGAACUUGAGUGAAGGUGGGCUG CUUGCAUCAGCCUAAUGUCGAGAAGUGCUUUCUUCGGAAAGUAACCCUCGAAACAA AGAAAGGAAUGCAAC-3' (SEQ ID NO: 208).

### Combination 8 of respective modifications - Modification of fourth and fifth regions and removal of first region

In an embodiment, the engineered scaffold region may be one in which modified fourth and fifth regions are included and the first region is removed. Here, the modified fourth and fifth regions include any one of the modifications described in the section "Engineered scaffold region 4 - Modification of fourth and fifth regions." Here, the engineered scaffold region may not comprise a region corresponding to the first region of the naturally occurring scaffold region.

In an embodiment, a sequence of the engineered scaffold region in which the fourth and fifth regions are modified and the first region is removed may comprise:
a sequence in which the following sequences are linked to each other in a 5' to 3' direction:
SEQ ID NO: 11,
SEQ ID NO: 12, and
a sequence selected from the group consisting of 5'-AACAAA-3', 5'-AACAAAU-3', 5'-AACAAAUU-3', 5'-AACAAAUUC-3', and SEQ ID NOs: 67 to 69; and
a sequence in which the following sequences are linked to each other in a 5' to 3' direction:
   a sequence selected from the group consisting of 5'-GGA-3', 5'-AGGA-3', 5'-AAGGA-3', 5'-GAAGGA-3', 5'-UGAAGGA-3', 5'-AUGAAGGA-3', and 5'-AAUGAAGGA-3', and
   5'-AUGCAAC-3'.

In an embodiment, a sequence of the engineered scaffold region in which the fourth and fifth regions are modified and the first region is removed may be a sequence in which the following sequences are linked to each other in a 5' to 3' direction:
SEQ ID NO: 11,
SEQ ID NO: 12,
a sequence selected from SEQ ID NOs: 111 to 117, and
5'-AUGCAAC-3'.

### Combination 9 of respective modifications - Modification of second region and modification of third region

In an embodiment, the engineered scaffold region may comprise a modified second region and a modified third region. Here, the modified second region includes any one of the modifications described in the section "Engineered scaffold region 2 - Modification of second region." Here, the modified third region includes any one of the modifications described in the section "Engineered scaffold region 3 - Modification of third region."

In an embodiment, a sequence of the engineered scaffold region in which the second region, and the third region are modified may comprise:
a sequence in which the following sequences are linked to each other in a 5' to 3' direction:
SEQ ID NO: 10,
a sequence selected from the group consisting of 5'-G-3', 5'-UUAGG-3', 5'-CUUAGGG-3', 5'-CUUAGUGG-3', and SEQ ID NOs: 342 to 362,
a sequence selected from the group consisting of SEQ ID NOs: 434 to 447, and
SEQ ID NO: 13; and
a sequence in which SEQ ID NO: 15 and 5'-AUGCAAC-3' are linked to each other in a 5' to 3' direction.

In an embodiment, a sequence of the engineered scaffold region in which the second region and the third region are modified may be a sequence in which the following sequences are linked to each other in a 5' to 3' direction:
SEQ ID NO: 10,
a sequence selected from the group consisting of 5'-G-3', 5'-UUAGG-3', 5'-CUUAGGG-3', 5'-CUUAGUGG-3', and SEQ ID NOs: 342 to 362,
a sequence selected from the group consisting of SEQ ID NOs: 434 to 447;
a linker,
SEQ ID NO: 15, and
5'-AUGCAAC-3'.

Here, the linker may be 5'-GAAA-3'.

### Combination 10 of respective modifications - Modification of third region and removal of second region

In an embodiment, the engineered scaffold region may be one in which a modified third region is included and the second region is removed. Here, the modified third region includes any one of the modifications described in the section "Engineered scaffold region 3 - Modification of third region." Here, the engineered scaffold region may not comprise a region corresponding to the second region of the naturally occurring scaffold region.

In an embodiment, a sequence of the engineered scaffold region in which the third region is modified and the second region is removed may comprise:
a sequence in which the following sequences are linked to each other in a 5' to 3' direction:
SEQ ID NO: 10,
a sequence selected from the group consisting of SEQ ID NOs: 434 to 447, and
SEQ ID NO: 13; and
a sequence in which SEQ ID NO: 15 and 5'-AUGCAAC-3' are linked to each other in a 5' to 3' direction.

In an embodiment, a sequence of the engineered scaffold region in which the third region is modified and the second region is removed may be a sequence in which the following sequences are linked to each other in a 5' to 3' direction:
SEQ ID NO: 10,
a sequence selected from the group consisting of SEQ ID NOs: 434 to 447;
a linker,
SEQ ID NO: 15, and
5'-AUGCAAC-3'.

Here, the linker may be 5'-GAAA-3'.

### Combination 11 of respective modifications - Modification of second region, and modification of fourth and fifth regions

In an embodiment, the engineered scaffold region may comprise a modified second region and modified fourth and fifth regions. Here, the modified second region includes any one of the modifications described in the section "Engineered scaffold region 2 - Modification of second region." Here, the modified fourth and fifth regions include any one of the modifications described in the section "Engineered scaffold region 4 - Modification of fourth and fifth regions."

In an embodiment, a sequence of the engineered scaffold region in which the second region and the fourth and fifth regions are modified may comprise:
a sequence in which the following sequences are linked to each other in a 5' to 3' direction:
SEQ ID NO: 10,
a sequence selected from the group consisting of 5'-G-3', 5'-UUAGG-3', 5'-CUUAGGG-3', 5'-CUUAGUGG-3', and SEQ ID NOs: 342 to 362,
SEQ ID NO: 12, and
a sequence selected from the group consisting of 5'-AACAAA-3', 5'-AACAAAU-3', 5'-AACAAAUU-3', 5'-AACAAAUUC-3', and SEQ ID NOs: 67 to 69; and
a sequence in which the following sequences are linked to each other in a 5' to 3' direction:
   a sequence selected from the group consisting of 5'-GGA-3', 5'-AGGA-3', 5'-AAGGA-3', 5'-GAAGGA-3', 5'-UGAAGGA-3', 5'-AUGAAGGA-3', and 5'-AAUGAAGGA-3', and
   5'-AUGCAAC-3'.

In an embodiment, the sequence of the engineered scaffold region in which the second region and the fourth and fifth regions are modified may comprise in a 5' to 3' direction: 
5'-
CUUCACUGAUAAAGUGGAGAACCGCUUCACCAUUAGUGAGUGAAGGUGGGCUGCUU
GCAUCAGCCUAAUGUCGAGAAGUGCUUUCUUCGGAAAGUAACCCUCGAAACAAA-3'
(SEQ ID NO: 159),
 and
5'-GGAAUGCAAC-3' (SEQ ID NO: 161).

In an embodiment, a sequence of the engineered scaffold region in which the second region, and the fourth and fifth regions are modified may be a sequence in which the following sequences are linked to each other in a 5' to 3' direction:
SEQ ID NO: 10,
a sequence selected from the group consisting of 5'-G-3', 5'-UUAGG-3', 5'-CUUAGGG-3', 5'-CUUAGUGG-3', and SEQ ID NOs: 342 to 362,
SEQ ID NO: 12,
a sequence selected from SEQ ID NOs: 111 to 117, and
5'-AUGCAAC-3'.

In an embodiment, the sequence of the engineered scaffold region in which the second region, and the fourth and fifth regions are modified may be 5'-CUUCACUGAUAAAGUGGAGAACCGCUUCACUUAGAGUGAAGGUGGGGCUGCUUGCA UCAGCCUAAUGUCGAGAAGUGCUUUCUUCGGAAAGUAACCCUCGAAACAAAGAAAG GAAUGCAAC-3' (SEQ ID NO: 209).

### Combination 12 of respective modifications - Modification of fourth and fifth regions and removal of second region

In an embodiment, the engineered scaffold region may be one in which modified fourth and fifth regions are included, and the second region is removed. Here, the modified fourth and fifth regions include any one of the modifications described in the section "Engineered scaffold region 4 - Modification of fourth and fifth regions." Here, the engineered scaffold region may not comprise a region corresponding to the second region of the naturally occurring scaffold region.

In an embodiment, a sequence of the engineered scaffold region in which the fourth and fifth regions are modified and the second region is removed may comprise:
a sequence in which the following sequences are linked to each other in a 5' to 3' direction:
SEQ ID NO: 10,
SEQ ID NO: 12, and
a sequence selected from the group consisting of 5'-AACAAA-3', 5'-AACAAAU-3', 5'-AACAAAUU-3', 5'-AACAAAUUC-3', and SEQ ID NOs: 67 to 69; and
a sequence in which the following sequences are linked to each other in a 5' to 3' direction:
   a sequence selected from the group consisting of 5'-GGA-3', 5'-AGGA-3', 5'-AAGGA-3', 5'-GAAGGA-3', 5'-UGAAGGA-3', 5'-AUGAAGGA-3', and 5'-AAUGAAGGA-3', and
   5'-AUGCAAC-3'.

In an embodiment, a sequence of the engineered scaffold region in which the fourth and fifth regions are modified and the second region is removed may be one in which the following sequences are linked to each other in a 5' to 3' direction:
SEQ ID NO: 10,
SEQ ID NO: 12,
a sequence selected from SEQ ID NOs: 111 to 117, and
5'-AUGCAAC-3'.

### Combination 13 of respective modifications - Modification of third region, and modification of fourth and fifth regions

In an embodiment, the engineered scaffold region may comprise a modified third region and modified fourth and fifth regions. Here, the modified third region includes any one of the modifications described in the section "Engineered scaffold region 3 - Modification of third region." Here, the modified fourth and fifth regions include any one of the modifications described in the section "Engineered scaffold region 4 - Modification of fourth and fifth regions."

In an embodiment, a sequence of the engineered scaffold region in which the third region and the fourth and fifth regions are modified may comprise:
a sequence in which the following sequences are linked to each other in a 5' to 3' direction:
SEQ ID NO: 10,
SEQ ID NO: 11,
a sequence selected from SEQ ID NOs: 434 to 447, and
a sequence selected from the group consisting of 5'-AACAAA-3', 5'-AACAAAU-3', 5'-AACAAAUU-3', 5'-AACAAAUUC-3', and SEQ ID NOs: 67 to 69; and
a sequence in which the following sequences are linked to each other in a 5' to 3' direction:
   a sequence selected from the group consisting of 5'-GGA-3', 5'-AGGA-3', 5'-AAGGA-3', 5'-GAAGGA-3', 5'-UGAAGGA-3', 5'-AUGAAGGA-3', and 5'-AAUGAAGGA-3', and
   5'-AUGCAAC-3'.

In an embodiment, a sequence of the engineered scaffold region in which the third region, and the fourth and fifth regions are modified may be a sequence in which the following sequences are linked to each other in a 5' to 3' direction:
SEQ ID NO: 10,
SEQ ID NO: 11,
a sequence selected from the group consisting of SEQ ID NOs: 434 to 447;
a sequence selected from SEQ ID NOs: 111 to 117, and
5'-AUGCAAC-3'.

### Combination 14 of respective modifications - Modification of first region, modification of second region, and modification of third region

In an embodiment, the engineered scaffold region may comprise a modified first region, a modified second region, and a modified third region. Here, the modified first region includes any one of the modifications described in the section "Engineered scaffold region 1 - Modification of first region." Here, the modified second region includes any one of the modifications described in the section "Engineered scaffold region 2 - Modification of second region." Here, the modified third region includes any one of the modifications described in the section "Engineered scaffold region 3 - Modification of third region."

In an embodiment, a sequence of the engineered scaffold region in which the first region, the second region, and the third region are modified may comprise:
a sequence in which the following sequences are linked to each other in a 5' to 3' direction:
a sequence selected from the group consisting of 5'-A-3', 5'-AA-3', 5'-GAA-3', 5'-AGAA-3', 5'-GAGAA-3', 5'-GGAGAA-3', 5'-UGGAGAA-3', 5'-GUGGAGAA-3', 5'-AGUGGAGAA-3', and SEQ ID NOs: 17 to 27,
a sequence selected from the group consisting of 5'-G-3', 5'-UUAGG-3', 5'-CUUAGGG-3', 5'-CUUAGUGG-3', and SEQ ID NOs: 342 to 362,
a sequence selected from the group consisting of SEQ ID NOs: 434 to 447, and
SEQ ID NO: 13; and
a sequence in which SEQ ID NO: 15 and 5'-AUGCAAC-3' are linked to each other in a 5' to 3' direction.

In an embodiment, a sequence of the engineered scaffold region in which the first region, the second region, and the third region are modified may be a sequence in which the following sequences are linked to each other in a 5' to 3' direction:
a sequence selected from the group consisting of 5'-A-3', 5'-AA-3', 5'-GAA-3', 5'-AGAA-3', 5'-GAGAA-3', 5'-GGAGAA-3', 5'-UGGAGAA-3', 5'-GUGGAGAA-3', 5'-AGUGGAGAA-3', and SEQ ID NOs: 17 to 27,
a sequence selected from the group consisting of 5'-G-3', 5'-UUAGG-3', 5'-CUUAGGG-3', 5'-CUUAGUGG-3', and SEQ ID NOs: 342 to 362,
a sequence selected from the group consisting of SEQ ID NOs: 434 to 447,
SEQ ID NO: 13,
a linker,
SEQ ID NO: 15, and
5'-AUGCAAC-3'.

Here, the linker may be 5'-GAAA-3'.

### Combination 15 of respective modifications - Modification of second region, modification of third region, and removal of first region

In an embodiment, the engineered scaffold region may be one in which a modified second region and a modified third region are included and the first region is removed. Here, the modified second region includes any one of the modifications described in the section "Engineered scaffold region 2 - Modification of second region." Here, the modified third region includes any one of the modifications described in the section "Engineered scaffold region 3 - Modification of third region." Here, the engineered scaffold region may not comprise a region corresponding to the first region of the naturally occurring scaffold region.

In an embodiment, a sequence of the engineered scaffold region in which the second region and the third region are modified and the first region is removed may comprise:
a sequence in which the following sequences are linked to each other in a 5' to 3' direction:
a sequence selected from the group consisting of 5'-G-3', 5'-UUAGG-3', 5'-CUUAGGG-3', 5'-CUUAGUGG-3', and SEQ ID NOs: 342 to 362,
a sequence selected from the group consisting of SEQ ID NOs: 434 to 447, and
SEQ ID NO: 13; and
a sequence in which SEQ ID NO: 15 and 5'-AUGCAAC-3' are linked to each other in a 5' to 3' direction.

In an embodiment, a sequence of the engineered scaffold region in which the second region and the third region are modified and the first region is removed may be a sequence in which the following sequences are linked to each other in a 5' to 3' direction:
a sequence selected from the group consisting of 5'-G-3', 5'-UUAGG-3', 5'-CUUAGGG-3', 5'-CUUAGUGG-3', and SEQ ID NOs: 342 to 362,
a sequence selected from the consisting of SEQ ID NOs: 434 to 447,
SEQ ID NO: 13,
a linker,
SEQ ID NO: 15, and
5'-AUGCAAC-3'.

Here, the linker may be 5'-GAAA-3'.

### Combination 16 of respective modifications - Modification of first region, modification of third region, and removal of second region

In an embodiment, the engineered scaffold region may be one in which a modified first region and a modified third region are included and the second region is removed. Here, the modified first region includes any one of the modifications described in the section "Engineered scaffold region 1 - Modification of first region." Here, the modified third region includes any one of the modifications described in the section "Engineered scaffold region 3 - Modification of third region." Here, the engineered scaffold region may not comprise a region corresponding to the second region of the naturally occurring scaffold region.

In an embodiment, a sequence of the engineered scaffold region in which the first region and the third region are modified and the second region is removed may comprise:
a sequence in which the following sequences are linked to each other in a 5' to 3' direction:
a sequence selected from the group consisting of 5'-A-3', 5'-AA-3', 5'-GAA-3', 5'-AGAA-3', 5'-GAGAA-3', 5'-GGAGAA-3', 5'-UGGAGAA-3', 5'-GUGGAGAA-3', 5'-AGUGGAGAA-3', and SEQ ID NOs: 17 to 27,
a sequence selected from the group consisting of SEQ ID NOs: 434 to 447, and
SEQ ID NO: 13; and
a sequence in which SEQ ID NO: 15 and 5'-AUGCAAC-3' are linked to each other in a 5' to 3' direction.

In an embodiment, a sequence of the engineered scaffold region in which the first region and the third region are modified, and the second region is removed may be a sequence in which the following sequences are linked to each other in a 5' to 3' direction:
a sequence selected from the group consisting of 5'-A-3', 5'-AA-3', 5'-GAA-3', 5'-AGAA-3', 5'-GAGAA-3', 5'-GGAGAA-3', 5'-UGGAGAA-3', 5'-GUGGAGAA-3', 5'-AGUGGAGAA-3', and SEQ ID NOs: 17 to 27,
a sequence selected from the group consisting of SEQ ID NOs: 434 to 447,
SEQ ID NO: 13,
a linker,
SEQ ID NO: 15, and
5'-AUGCAAC-3'.

Here, the linker may be 5'-GAAA-3'.

### Combination 17 of respective modifications - Modification of third region, removal of first region, and removal of second region

In an embodiment, the engineered scaffold region may be one in which a modified third region is included and the first region and the second region are removed. Here, the modified third region includes any one of the modifications described in the section "Engineered scaffold region 3 - Modification of third region." Here, the engineered scaffold region may not comprise a region corresponding to the first region of the naturally occurring scaffold region. Here, the engineered scaffold region may not comprise a region corresponding to the second region of the naturally occurring scaffold region.

In an embodiment, a sequence of the engineered scaffold region in which the third region is modified, and the first region and the second region are removed may comprise:
a sequence in which the following sequences are linked to each other in a 5' to 3' direction:
a sequence selected the group consisting of SEQ ID NOs: 434 to 447, and
SEQ ID NO: 13; and
a sequence in which SEQ ID NO: 15 and 5'-AUGCAAC-3' are linked to each other in a 5' to 3' direction.

In an embodiment, a sequence of the engineered scaffold region in which the third region is modified and the first region and the second region are removed may comprise:
a sequence in which the following sequences are linked to each other in a 5' to 3' direction:
a sequence selected from the group consisting of SEQ ID NOs: 434 to 447,
SEQ ID NO: 13,
a linker,
SEQ ID NO: 15, and
5'-AUGCAAC-3'.

Here, the linker may be 5'-GAAA-3'.

### Combination 18 of respective modifications - Modification of first region, modification of second region, and modification of fourth and fifth regions

In an embodiment, the engineered scaffold region may comprise a modified first region, a modified second region, and modified fourth and fifth regions. Here, the modified first region includes any one of the modifications described in the section "Engineered scaffold region 1 - Modification of first region." Here, the modified second region includes any one of the modifications described in the section "Engineered scaffold region 2 - Modification of second region." Here, the modified fourth and fifth regions include any one of the modifications described in the section "Engineered scaffold region 4 - Modification of fourth and fifth regions."

In an embodiment, a sequence of the engineered scaffold region in which the first region, the second region, and the fourth and fifth regions are modified may comprise:
a sequence in which the following sequences are linked to each other in a 5' to 3' direction:
a sequence selected from the group consisting of 5'-A-3', 5'-AA-3', 5'-GAA-3', 5'-AGAA-3', 5'-GAGAA-3', 5'-GGAGAA-3', 5'-UGGAGAA-3', 5'-GUGGAGAA-3', 5'-AGUGGAGAA-3', and SEQ ID NOs: 17 to 27,
a sequence selected from the group consisting of 5'-G-3', 5'-UUAGG-3', 5'-CUUAGGG-3', 5'-CUUAGUGG-3', and SEQ ID NOs: 342 to 362,
SEQ ID NO: 12, and
a sequence selected from the group consisting of 5'-AACAAA-3', 5'-AACAAAU-3', 5'-AACAAAUU-3', 5'-AACAAAUUC-3', and SEQ ID NOs: 67 to 69; and
a sequence in which the following sequences are linked to each other in a 5' to 3' direction:
   a sequence selected from the group consisting of 5'-GGA-3', 5'-AGGA-3', 5'-AAGGA-3', 5'-GAAGGA-3', 5'-UGAAGGA-3', 5'-AUGAAGGA-3', and 5'-AAUGAAGGA-3', and
   5'-AUGCAAC-3'.

In an embodiment, a sequence of the engineered scaffold region in which the first region, the second region, and the fourth and fifth regions are modified may comprise in a 5' to 3' direction: 
5'-
ACCGCUUCACCAUUAGUGAGUGAAGGUGGGCUGCUUGCAUCAGCCUAAUGUCGAGA
AGUGCUUUCUUCGGAAAGUAACCCUCGAAACAAA-3' (SEQ ID NO: 160),
 and
5'-GGAAUGCAAC-3' (SEQ ID NO: 161).

In an embodiment, a sequence of the engineered scaffold region in which the first region, the second region, and fourth and fifth regions are modified may be a sequence in which the following sequences are linked to each other in a 5' to 3' direction:
a sequence selected from the group consisting of 5'-A-3', 5'-AA-3', 5'-GAA-3', 5'-AGAA-3', 5'-GAGAA-3', 5'-GGAGAA-3', 5'-UGGAGAA-3', 5'-GUGGAGAA-3', 5'-AGUGGAGAA-3', and SEQ ID NOs: 17 to 27,
a sequence selected from the group consisting of 5'-G-3', 5'-UUAGG-3', 5'-CUUAGGG-3', 5'-CUUAGUGG-3', and SEQ ID NOs: 342 to 362,
SEQ ID NO: 12,
a sequence selected from SEQ ID NOs: 111 to 117, and
5'-AUGCAAC-3'.

In an embodiment, a sequence of the engineered scaffold region in which the first region, the second region, and the fourth and fifth regions are modified may be 5'-ACCGCUUCACUUAGAGUGAAGGUGGGGCUGCUUGCAUCAGCCUAAUGUCGAGAAG UGCUUUCUUCGGAAAGUAACCCUCGAAACAAAGAAAGGAAUGCAAC-3' (SEQ ID NO: 210).

### Combination 19 of respective modifications - Modification of second region, modification of fourth and fifth regions, and removal of first region

In an embodiment, the engineered scaffold region may be one in which a modified second region and modified fourth and fifth regions are included and the first region is removed. Here, the modified second region includes any one of the modifications described in the section "Engineered scaffold region 2 - Modification of second region." Here, the modified fourth and fifth regions include any one of the modifications described in the section "Engineered scaffold region 4 - Modification of fourth and fifth regions." Here, the engineered scaffold region may not comprise a region corresponding to the first region of the naturally occurring scaffold region.

In an embodiment, a sequence of the engineered scaffold region in which the second region and the fourth and fifth regions are modified and the first region is removed may comprise:
a sequence in which the following sequences are linked to each other in a 5' to 3' direction:
a sequence selected from the group consisting of 5'-G-3', 5'-UUAGG-3', 5'-CUUAGGG-3', 5'-CUUAGUGG-3', and SEQ ID NOs: 342 to 362,
SEQ ID NO: 12,
a sequence selected from the group consisting of 5'-AACAAA-3', 5'-AACAAAU-3', 5'-AACAAAUU-3', 5'-AACAAAUUC-3', and SEQ ID NOs: 67 to 69; and
a sequence in which the following sequences are linked to each other in a 5' to 3' direction:
   a sequence selected from the group consisting of 5'-GGA-3', 5'-AGGA-3', 5'-AAGGA-3', 5'-GAAGGA-3', 5'-UGAAGGA-3', 5'-AUGAAGGA-3', and 5'-AAUGAAGGA-3', and
   5'-AUGCAAC-3'.

In an embodiment, a sequence of the engineered scaffold region in which the second region and the fourth and fifth regions are modified and the first region is removed may be a sequence in which the following sequences are linked to each other in a 5' to 3' direction:
a sequence selected from the group consisting of 5'-G-3', 5'-UUAGG-3', 5'-CUUAGGG-3', 5'-CUUAGUGG-3', and SEQ ID NOs: 342 to 362,
SEQ ID NO: 12,
a sequence selected from SEQ ID NOs: 111 to 117, and
5'-AUGCAAC-3'.

### Combination 20 of respective modifications - Modification of first region, modification of fourth and fifth regions, and removal of second region

In an embodiment, the engineered scaffold region may be one in which a modified first region, and modified fourth and fifth regions are included, and the second region is removed. Here, the modified first region includes any one of the modifications described in the section "Engineered scaffold region 1 - Modification of first region." Here, the modified fourth and fifth regions include any one of the modifications described in the section "Engineered scaffold region 4 - Modification of fourth and fifth regions." Here, the engineered scaffold region may not comprise a region corresponding to the second region of the naturally occurring scaffold region.

In an embodiment, a sequence of the engineered scaffold region in which the first region and the fourth and fifth regions are modified and the second region is removed may comprise:
a sequence in which the following sequences are linked to each other in a 5' to 3' direction:
a sequence selected from the group consisting of 5'-A-3', 5'-AA-3', 5'-GAA-3', 5'-AGAA-3', 5'-GAGAA-3', 5'-GGAGAA-3', 5'-UGGAGAA-3', 5'-GUGGAGAA-3', 5'-AGUGGAGAA-3', and SEQ ID NOs: 17 to 27,
SEQ ID NO: 12, and
a sequence selected from the group consisting of 5'-AACAAA-3', 5'-AACAAAU-3', 5'-AACAAAUU-3', 5'-AACAAAUUC-3', and SEQ ID NOs: 67 to 69; and
a sequence in which the following sequences are linked to each other in a 5' to 3' direction:
   a sequence selected from the group consisting of 5'-GGA-3', 5'-AGGA-3', 5'-AAGGA-3', 5'-GAAGGA-3', 5'-UGAAGGA-3', 5'-AUGAAGGA-3', and 5'-AAUGAAGGA-3', and
   5'-AUGCAAC-3'.

In an embodiment, a sequence of the engineered scaffold region in which the first region and the fourth and fifth regions are modified and the second region is removed may be a sequence in which the following sequences are linked to each other in a 5' to 3' direction:
a sequence selected from the group consisting of 5'-A-3', 5'-AA-3', 5'-GAA-3', 5'-AGAA-3', 5'-GAGAA-3', 5'-GGAGAA-3', 5'-UGGAGAA-3', 5'-GUGGAGAA-3', 5'-AGUGGAGAA-3', and SEQ ID NOs: 17 to 27,
SEQ ID NO: 12,
a sequence selected from SEQ ID NOs: 111 to 117, and
5'-AUGCAAC-3'.

### Combination 21 of respective modifications - Modification of fourth and fifth regions, removal of first region, and removal of second region

In an embodiment, the engineered scaffold region may be one in which the modified fourth and fifth regions are included and the first region and the second region are removed. Here, the modified fourth and fifth regions include any one of the modifications described in the section "Engineered scaffold region 4 - Modification of fourth and fifth regions." Here, the engineered scaffold region may not comprise a region corresponding to the first region of the naturally occurring scaffold region. Here, the engineered scaffold region may not comprise a region corresponding to the second region of the naturally occurring scaffold region.

In an embodiment, a sequence of the engineered scaffold region in which the fourth and fifth regions are modified and the first region and the second region are removed may comprise:
a sequence in which the following sequences are linked to each other in a 5' to 3' direction:
SEQ ID NO: 12, and
a sequence selected from the group consisting of 5'-AACAAA-3', 5'-AACAAAU-3', 5'-AACAAAUU-3', 5'-AACAAAUUC-3', and SEQ ID NOs: 67 to 69; and
a sequence in which the following sequences are linked to each other in a 5' to 3' direction:
   a sequence selected from the group consisting of 5'-GGA-3', 5'-AGGA-3', 5'-AAGGA-3', 5'-GAAGGA-3', 5'-UGAAGGA-3', 5'-AUGAAGGA-3', and 5'-AAUGAAGGA-3', and
   5'-AUGCAAC-3'.

In an embodiment, a sequence of the engineered scaffold region in which the fourth and fifth regions are modified and the first region and the second region are removed may be a sequence in which the following sequences are linked to each other in a 5' to 3' direction:
SEQ ID NO: 12,
a sequence selected from SEQ ID NOs: 111 to 117, and
5'-AUGCAAC-3'.

### Combination 22 of respective modifications - Modification of first region, modification of third region, and modification of fourth and fifth regions

In an embodiment, the engineered scaffold region may comprise a modified first region, a modified third region, and modified fourth and fifth regions. Here, the modified first region includes any one of the modifications described in the section "Engineered scaffold region 1 - Modification of first region." Here, the modified third region includes any one of the modifications described in the section "Engineered scaffold region 3 - Modification of third region." Here, the modified fourth and fifth regions include any one of the modifications described in the section "Engineered scaffold region 4 - Modification of fourth and fifth regions."

In an embodiment, the engineered scaffold sequence in which the first region, the third region, and the fourth and fifth regions are modified may comprise:
a sequence in which the following sequences are linked to each other in a 5' to 3' direction:
a sequence selected from the group consisting of 5'-A-3', 5'-AA-3', 5'-GAA-3', 5'-AGAA-3', 5'-GAGAA-3', 5'-GGAGAA-3', 5'-UGGAGAA-3', 5'-GUGGAGAA-3', 5'-AGUGGAGAA-3', and SEQ ID NOs: 17 to 27,
SEQ ID NO: 11,
a sequence selected from the group consisting of SEQ ID NOs: 434 to 447, and
a sequence selected from the group consisting of 5'-AACAAA-3', 5'-AACAAAU-3', 5'-AACAAAUU-3', 5'-AACAAAUUC-3', and SEQ ID NOs: 67 to 69; and
a sequence in which the following sequences are linked to each other in a 5' to 3' direction:
   a sequence selected from the group consisting of 5'-GGA-3', 5'-AGGA-3', 5'-AAGGA-3', 5'-GAAGGA-3', 5'-UGAAGGA-3', 5'-AUGAAGGA-3', and 5'-AAUGAAGGA-3', and
   5'-AUGCAAC-3'.

In an embodiment, a sequence of the engineered scaffold region in which the first region, the third region, and the fourth and fifth regions are modified may be a sequence in which the following sequences are linked to each other in a 5' to 3' direction:
a sequence selected from the group consisting of 5'-A-3', 5'-AA-3', 5'-GAA-3', 5'-AGAA-3', 5'-GAGAA-3', 5'-GGAGAA-3', 5'-UGGAGAA-3', 5'-GUGGAGAA-3', 5'-AGUGGAGAA-3', and SEQ ID NOs: 17 to 27,
SEQ ID NO: 11,
a sequence selected from the group consisting of SEQ ID NOs: 434 to 447,
a sequence selected from SEQ ID NOs: 111 to 117, and
5'-AUGCAAC-3'.

### Combination 23 of respective modifications - Modification of third region, modification of fourth and fifth regions, and removal of first region

In an embodiment, the engineered scaffold region may be one in which a modified third region, and modified fourth and fifth regions are included, and the first region is removed. Here, the modified third region includes any one of the modifications described in the section "Engineered scaffold region 3 - Modification of third region." Here, the modified fourth and fifth regions include any one of the modifications described in the section "Engineered scaffold region 4 - Modification of fourth and fifth regions." Here, the engineered scaffold region may not comprise a region corresponding to the first region of the naturally occurring scaffold region.

In an embodiment, a sequence of the engineered scaffold region in which the third region, and the fourth and fifth regions are modified, and the first region is removed may comprise:
a sequence in which the following sequences are linked to each other in a 5' to 3' direction:
SEQ ID NO: 11,
a sequence selected from the group consisting fo SEQ ID NOs: 434 to 447, and
a sequence selected from the group consisting of 5'-AACAAA-3', 5'-AACAAAU-3', 5'-AACAAAUU-3', 5'-AACAAAUUC-3', and SEQ ID NOs: 67 to 69; and
a sequence in which the following sequences are linked to each other in a 5' to 3' direction:
   a sequence selected from the group consisting of 5'-GGA-3', 5'-AGGA-3', 5'-AAGGA-3', 5'-GAAGGA-3', 5'-UGAAGGA-3', 5'-AUGAAGGA-3', and 5'-AAUGAAGGA-3', and
   5'-AUGCAAC-3'.

In an embodiment, a sequence of the engineered scaffold region in which the third region, and the fourth and fifth regions are modified and the first region is removed may be a sequence in which the following sequences are linked to each other in a 5' to 3' direction:
SEQ ID NO: 11,
a sequence selected from the group consisting of SEQ ID NOs: 434 to 447;
a sequence selected from SEQ ID NOs: 111 to 117, and
5'-AUGCAAC-3'.

### Combination 24 of respective modifications - Modification of second region, modification of third region, and modification of fourth and fifth regions

In an embodiment, the engineered scaffold region may comprise a modified second region, a modified third region, and modified fourth and fifth regions. Here, the modified first region includes any one of the modifications described in the section "Engineered scaffold region 2 - Modification of second region." Here, the modified third region includes any one of the modifications described in the section "Engineered scaffold region 3 - Modification of third region." Here, the modified fourth and fifth regions include any one of the modifications described in the section "Engineered scaffold region 4 - Modification of fourth and fifth regions."

In an embodiment, the engineered scaffold sequence in which the second region, the third region, and the fourth and fifth regions are modified may comprise:
a sequence in which the following sequences are linked to each other in a 5' to 3' direction:
SEQ ID NO: 10,
a sequence selected from the group consisting of 5'-G-3', 5'-UUAGG-3', 5'-CUUAGGG-3', 5'-CUUAGUGG-3', and SEQ ID NOs: 342 to 362,
a sequence selected from the group consisting of SEQ ID NOs: 434 to 447, and
a sequence selected from the group consisting of 5'-AACAAA-3', 5'-AACAAAU-3', 5'-AACAAAUU-3', 5'-AACAAAUUC-3', and SEQ ID NOs: 67 to 69; and
a sequence in which the following sequences are linked to each other in a 5' to 3' direction:
   a sequence selected from the group consisting of 5'-GGA-3', 5'-AGGA-3', 5'-AAGGA-3', 5'-GAAGGA-3', 5'-UGAAGGA-3', 5'-AUGAAGGA-3', and 5'-AAUGAAGGA-3', and
   5'-AUGCAAC-3'.

In an embodiment, a sequence of the engineered scaffold region in which the second region, the third region, and the fourth and fifth regions are modified may be a sequence in which the following sequences are linked to each other in a 5' to 3' direction:
SEQ ID NO: 10,
a sequence selected from the group consisting of 5'-G-3', 5'-UUAGG-3', 5'-CUUAGGG-3', 5'-CUUAGUGG-3', and SEQ ID NOs: 342 to 362,
a sequence selected from the group consisting of SEQ ID NOs: 434 to 447,
a sequence selected from SEQ ID NOs: 111 to 117, and
5'-AUGCAAC-3'.

### Combination 25 of respective modifications - Modification of third region, modification of fourth and fifth regions, and removal of second region

In an embodiment, the engineered scaffold region may be one in which a modified third region, and modified fourth and fifth regions are included and the second region is removed. Here, the modified third region includes any one of the modifications described in the section "Engineered scaffold region 3 - Modification of third region." Here, the modified fourth and fifth regions include any one of the modifications described in the section "Engineered scaffold region 4 - Modification of fourth and fifth regions." Here, the engineered scaffold region may not comprise a region corresponding to the second region of the naturally occurring scaffold region.

In an embodiment, a sequence of the engineered scaffold region in which the third region, and the fourth and fifth regions are modified and the second region is removed may comprise:
a sequence in which the following sequences are linked to each other in a 5' to 3' direction:
SEQ ID NO: 10,
a sequence selected from the group consisting of SEQ ID NOs: 434 to 447, and
a sequence selected from the group consisting of 5'-AACAAA-3', 5'-AACAAAU-3', 5'-AACAAAUU-3', 5'-AACAAAUUC-3', and SEQ ID NOs: 67 to 69; and
a sequence in which the following sequences are linked to each other in a 5' to 3' direction:
   a sequence selected from the group consisting of 5'-GGA-3', 5'-AGGA-3', 5'-AAGGA-3', 5'-GAAGGA-3', 5'-UGAAGGA-3', 5'-AUGAAGGA-3', and 5'-AAUGAAGGA-3', and
   5'-AUGCAAC-3'.

In an embodiment, a sequence of the engineered scaffold region in which the third region, and the fourth and fifth regions are modified and the second region is removed may be a sequence in which the following sequences are linked to each other in a 5' to 3' direction:
SEQ ID NO: 10,
a sequence selected from the group consisting of SEQ ID NOs: 434 to 447,
a sequence selected from SEQ ID NOs: 111 to 117, and
5'-AUGCAAC-3'.

### Combination 26 of respective modifications - Modification of first region, modification of second region, modification of third region, and modification of fourth and fifth regions

In an embodiment, the engineered scaffold region may comprise a modified first region, a modified second region, a modified third region, and modified fourth and fifth regions. Here, the modified first region includes any one of the modifications described in the section "Engineered scaffold region 1 - Modification of first region". Here, the modified second region includes any one of the modifications described in the section "Engineered scaffold region 2 - Modification of second region." Here, the modified third region includes any one of the modifications described in the section "Engineered scaffold region 3 - Modification of third region." Here, the modified fourth and fifth regions include any one of the modifications described in the section "Engineered scaffold region 4 - Modification of fourth and fifth regions."

In an embodiment, the engineered scaffold sequence in which the first region, the second region, the third region, and the fourth and fifth regions are modified may comprise:
a sequence in which the following sequences are linked to each other in a 5' to 3' direction:
a sequence selected from the group consisting of 5'-A-3', 5'-AA-3', 5'-GAA-3', 5'-AGAA-3', 5'-GAGAA-3', 5'-GGAGAA-3', 5'-UGGAGAA-3', 5'-GUGGAGAA-3', 5'-AGUGGAGAA-3', and SEQ ID NOs: 17 to 27,
a sequence selected from the group consisting of 5'-G-3', 5'-UUAGG-3', 5'-CUUAGGG-3', 5'-CUUAGUGG-3', and SEQ ID NOs: 342 to 362,
a sequence selected from the group consisting of SEQ ID NOs: 434 to 447, and
a sequence selected from the group consisting of 5'-AACAAA-3', 5'-AACAAAU-3', 5'-AACAAAUU-3', 5'-AACAAAUUC-3', and SEQ ID NOs: 67 to 69; and
a sequence in which the following sequences are linked to each other in a 5' to 3' direction:
   a sequence selected from the group consisting of 5'-GGA-3', 5'-AGGA-3', 5'-AAGGA-3', 5'-GAAGGA-3', 5'-UGAAGGA-3', 5'-AUGAAGGA-3', and 5'-AAUGAAGGA-3', and
   5'-AUGCAAC-3'.

In an embodiment, a sequence of the engineered scaffold region in which the first region, the second region, the third region, and the fourth and fifth regions are modified may be a sequence in which the following sequences are linked to each other in a 5' to 3' direction:
a sequence selected from the group consisting of 5'-A-3', 5'-AA-3', 5'-GAA-3', 5'-AGAA-3', 5'-GAGAA-3', 5'-GGAGAA-3', 5'-UGGAGAA-3', 5'-GUGGAGAA-3', 5'-AGUGGAGAA-3', and SEQ ID NOs: 17 to 27,
a sequence selected from the group consisting of 5'-G-3', 5'-UUAGG-3', 5'-CUUAGGG-3', 5'-CUUAGUGG-3', and SEQ ID NOs: 342 to 362,
a sequence selected from the group consisting of SEQ ID NOs: 434 to 447;
a sequence selected from SEQ ID NOs: 111 to 117, and
5'-AUGCAAC-3'.

### Combination 27 of respective modifications - Modification of second region, modification of third region, modification of fourth and fifth regions, and removal of first region

In an embodiment, the engineered scaffold region may be one in which a modified second region, a modified third region, and modified fourth and fifth regions are included and the first region is removed. Here, the modified second region includes any one of the modifications described in the section "Engineered scaffold region 2 - Modification of second region." Here, the modified third region includes any one of the modifications described in the section "Engineered scaffold region 3 - Modification of third region." Here, the modified fourth and fifth regions include any one of the modifications described in the section "Engineered scaffold region 4 - Modification of fourth and fifth regions." Here, the engineered scaffold region may not comprise a region corresponding to the first region of the naturally occurring scaffold region.

In an embodiment, a sequence of the engineered scaffold region in which the second region, the third region, and the fourth and fifth regions are modified and the first region is removed may comprise:
a sequence in which the following sequences are linked to each other in a 5' to 3' direction:
a sequence selected from the group consisting of 5'-G-3', 5'-UUAGG-3', 5'-CUUAGGG-3', 5'-CUUAGUGG-3', and SEQ ID NOs: 342 to 362,
a sequence selected from the group consisting of SEQ ID NOs: 434 to 447, and
a sequence selected from the group consisting of 5'-AACAAA-3', 5'-AACAAAU-3', 5'-AACAAAUU-3', 5'-AACAAAUUC-3', and SEQ ID NOs: 67 to 69; and
a sequence in which the following sequences are linked to each other in a 5' to 3' direction:
   a sequence selected from the group consisting of 5'-GGA-3', 5'-AGGA-3', 5'-AAGGA-3', 5'-GAAGGA-3', 5'-UGAAGGA-3', 5'-AUGAAGGA-3', and 5'-AAUGAAGGA-3', and
   5'-AUGCAAC-3'.

In an embodiment, a sequence of the engineered scaffold region in which the second region, the third region, and the fourth and fifth regions are modified and the first region is removed may be a sequence in which the following sequences are linked to each other in a 5' to 3' direction:
a sequence selected from the group consisting of 5'-G-3', 5'-UUAGG-3', 5'-CUUAGGG-3', 5'-CUUAGUGG-3', and SEQ ID NOs: 342 to 362,
a sequence selected from the group consisting of SEQ ID NOs: 434 to 447,
a sequence selected from SEQ ID NOs: 111 to 117, and
5'-AUGCAAC-3'.

### Combination 28 of respective modifications - modifications of first region, third region, and fourth and fifth regions, and removal of second region

In an embodiment, the engineered scaffold region may be one in which a modified first region, a modified third region, and modified fourth and fifth regions are included and the second region is removed. Here, the modified first region includes any one of the modifications described in the section "Engineered scaffold region 1 - Modification of first region." Here, the modified third region includes any one of the modifications described in the section "Engineered scaffold region 3 - Modification of third region." Here, the modified fourth and fifth regions include any one of the modifications described in the section "Engineered scaffold region 4 - Modification of fourth and fifth regions." Here, the engineered scaffold region may not comprise a region corresponding to the second region of the naturally occurring scaffold region.

In an embodiment, a sequence of the engineered scaffold region in which the first region, the third region, and the fourth and fifth regions are modified and the second region is removed may comprise:
a sequence in which the following sequences are linked to each other in a 5' to 3' direction:
a sequence selected from the group consisting of 5'-A-3', 5'-AA-3', 5'-GAA-3', 5'-AGAA-3', 5'-GAGAA-3', 5'-GGAGAA-3', 5'-UGGAGAA-3', 5'-GUGGAGAA-3', 5'-AGUGGAGAA-3', and SEQ ID NOs: 17 to 27,
a sequence selected from the group consisting of SEQ ID NOs: 434 to 447, and
a sequence selected from the group consisting of 5'-AACAAA-3', 5'-AACAAAU-3', 5'-AACAAAUU-3', 5'-AACAAAUUC-3', and SEQ ID NOs: 67 to 69; and
a sequence in which the following sequences are linked to each other in a 5' to 3' direction:
   a sequence selected from the group consisting of 5'-GGA-3', 5'-AGGA-3', 5'-AAGGA-3', 5'-GAAGGA-3', 5'-UGAAGGA-3', 5'-AUGAAGGA-3', and 5'-AAUGAAGGA-3', and
   5'-AUGCAAC-3'.

In an embodiment, a sequence of the engineered scaffold region in which the first region, the third region, and the fourth and fifth regions are modified and the second region is removed may be a sequence in which the following sequences are linked to each other in a 5' to 3' direction:
a sequence selected from the group consisting of 5'-A-3', 5'-AA-3', 5'-GAA-3', 5'-AGAA-3', 5'-GAGAA-3', 5'-GGAGAA-3', 5'-UGGAGAA-3', 5'-GUGGAGAA-3', 5'-AGUGGAGAA-3', and SEQ ID NOs: 17 to 27,
a sequence selected from the group consisting of SEQ ID NOs: 434 to 447,
a sequence selected from SEQ ID NOs: 111 to 117, and
5'-AUGCAAC-3'.

### Combination 29 of respective modifications - Modification of third region, modification of fourth and fifth regions, removal of first region, and removal of second region

In an embodiment, the engineered scaffold region may be one in which a modified third region and modified fourth and fifth regions are included and the first region and the second region are removed. Here, the modified third region includes any one of the modifications described in the section "Engineered scaffold region 3 - Modification of third region." Here, the modified fourth and fifth regions include any one of the modifications described in the section "Engineered scaffold region 4 - Modification of fourth and fifth regions." Here, the engineered scaffold region may not comprise a region corresponding to the first region of the naturally occurring scaffold region. Here, the engineered scaffold region may not comprise a region corresponding to the second region of the naturally occurring scaffold region.

In an embodiment, a sequence of the engineered scaffold region in which the third region, and the fourth and fifth regions are modified and the first region and the second region are removed may comprise:
a sequence in which the following sequences are linked to each other in a 5' to 3' direction:
a sequence selected from the group consisting of SEQ ID NOs: 434 to 447, and
a sequence selected from the group consisting of 5'-AACAAA-3', 5'-AACAAAU-3', 5'-AACAAAUU-3', 5'-AACAAAUUC-3', and SEQ ID NOs: 67 to 69; and
a sequence in which the following sequences are linked to each other in a 5' to 3' direction:
   a sequence selected from the group consisting of 5'-GGA-3', 5'-AGGA-3', 5'-AAGGA-3', 5'-GAAGGA-3', 5'-UGAAGGA-3', 5'-AUGAAGGA-3', and 5'-AAUGAAGGA-3', and
   5'-AUGCAAC-3'.

In an embodiment, the engineered scaffold sequence, in which the third region, and the fourth and fifth regions are modified, and the first region and the second region are removed, may be a sequence in which the following sequences are linked to each other in a 5' to 3' direction:
a sequence selected from the group consisting of SEQ ID NOs: 434 to 447;
a sequence selected from SEQ ID NOs: 111 to 117, and
5'-AUGCAAC-3'.

### Combination 30 of respective modifications - Additional modification of sixth region

As described above, since the sixth region may also be modified within a range in which its function is not impaired, the engineered scaffold region provided herein may be one in which the sixth region is additionally modified in addition to the modification(s) of the first region, the second region, the third region, the fourth region, and/or the fifth region, including the removal of the first region and/or the second region

### 9) Engineered scaffold region 6 - Inclusion of sequence having homology

The engineered scaffold region provided herein comprises a sequence having homology to the sequences of the engineered scaffold region (hereinafter, referred to as the above-described engineered scaffold region) described in the sections of "Engineered scaffold region 1 - Modification of first region," "Engineered scaffold region 2 - Modification of second region," "Engineered scaffold region 3 - Modification of third region," "Engineered scaffold region 4 - Modification of fourth and fifth regions," and "Engineered scaffold region 5 - Combination of respective modifications."

In an embodiment, a sequence of the engineered scaffold region may be a sequence having identity or homology of 100%, 99%, 98%, 97%, 96%, 95%, 94%, 93%, 92%, 91%, 90%, 89%, 88%, 87%, 86%, 85%, 84%, 83%, 82%, 81%, 80%, 79%, 78%, 77%, 76%, 75%, 74%, 73%, 72%, 71%, 70%, 69%, 68%, 67%, 66%, 65%, 64%, 63%, 62%, 61%, 60%, 59%, 58%, 57%, 56%, 55%, 54%, 53%, 52%, 51%, or 50% to any one of the sequences of the above-described engineered scaffold region. In an embodiment, the scaffold sequence may be a sequence that is identical to any one of the sequences of the above-described engineered scaffold region within a range of two numbers selected from the immediately preceding sentence. For example, the scaffold sequence may be a sequence that is 90% to 100% identical to any one of the sequences of the above-described engineered scaffold region.

### 10) Engineered Cas12f1 guide RNA

### Overview of engineered Cas12f1 guide RNA

In the present disclosure, there is provided an engineered Cas12f1 guide RNA for increasing targeting efficiency, for a target gene in a cell, of the CRISPR regulatory system. The engineered Cas12f1 guide RNA comprises an engineered scaffold, a spacer, and a U-rich tail. Here, the engineered scaffold may be any one of those described in the above-described "engineered scaffold region." Here, the U-rich tail may be any one of those described in the section "U-rich tail."

### Single guide RNA or dual guide RNA

The engineered Cas12f1 guide RNA may be a single guide RNA or a dual guide RNA. The dual guide RNA refers to a guide RNA which consists of two RNA molecules of a tracrRNA and a crRNA. The single guide RNA refers to a molecule formed by linking the 3' end of a (engineered) tracrRNA and the 5' end of a (engineered) crRNA via a linker. In other words, the single guide RNA means a molecule obtained by linking the 3 end of a fourth region and the 5' end of a fifth region via a linker, wherein the fourth and fifth regions are included in the engineered scaffold of the dual guide RNA. Here, the respective regions of the engineered scaffold may include any one of the modifications, and specific sequences thereof, as described in the sections of "Engineered scaffold region."

### Example 1 of engineered single guide RNA

In an embodiment, the engineered Cas12f1 guide RNA may be one in which an engineered scaffold region, a spacer, and a U-rich tail are sequentially linked to each other in a 5' to 3' direction.

The spacer has a length of 10 to 50 nucleotides and has a sequence complementary to a target sequence.

A sequence of the U-rich tail may be a uridine repeat sequence or a modified uridine repeat sequence. As an example, a sequence of the U-rich tail may comprise a sequence in which 1, 2, 3, 4, 5, 6, 7, 8, 9, 10, 11, 12, 13, 14, 15, 16, 17, 18, 19, or 20 consecutive uridines are contained. As another example, a sequence of the U-rich tail may comprise a sequence in which one or more UV, UUV, UUUV, UUUUV, and/or UUUUUV are repeated. Here, V is one of adenosine (A), cytidine (C), and guanosine (G).

The engineered scaffold region is one in which a first region, a second region, a third region, a fourth region, a linker, a fifth region, and a sixth region, which correspond to those of the naturally occurring scaffold region, are sequentially linked to each other in a 5' to 3' direction, and one or more regions selected from the first region, the second region, the fourth region, and the fifth region are modified as compared with the naturally occurring scaffold region.

As an example, when a first region of the engineered scaffold region is modified, the modified first region may be a modified form of a first region of the naturally occurring scaffold region from which one or more nucleotides are removed. Here, the removed nucleotide(s) may be a nucleotide(s) belonging to Stem 1 (Takeda et al., Structure of the miniature type V-F CRISPR-Cas effector enzyme, Molecular Cell 81, 1-13 (2021)) in the first region. Here, a sequence of the modified first region is characterized by comprising 5'-A-3'.

As another example, when a second region of the engineered scaffold region is modified, the modified second region may be a modified form of a second region of the naturally occurring scaffold region from which one or more nucleotides are removed. Here, removal of the nucleotide(s) may occur in a portion that forms a Stem 2 structure (Takeda et al., Structure of the miniature type V-F CRISPR-Cas effector enzyme, Molecular Cell 81, 1-13 (2021)) in the second region, and such removal may be done in pairs of nucleotides that are complementary to each other. Here, a sequence of the modified second region is characterized by comprising at least 5'-CCGCUUCACCA-3' (SEQ ID NO: 51) and 5'-UGAGUGAAGGUG-3' (SEQ ID NO: 52). More specifically, a sequence of the modified second region may be one in which 5'-CCGCUUCACCA-3' (SEQ ID NO: 51) and 5'-UGAGUGAAGGUG-3' (SEQ ID NO: 52) are sequentially linked to each other in a 5' to 3' direction, wherein the sequences may be linked by an appropriate intermediate sequence. As an example, the intermediate sequence may be selected from the group consisting of 5'-UUAG-3', 5'-AUUAGU-3', 5'-AAUUAGCU-3', 5'-AAAUUAGACU-3' (SEQ ID NO: 58), 5'-AAAGUUAGAACU-3' (SEQ ID NO: 59), 5'-AAAGCUUAGGAACU-3' (SEQ ID NO: 60), 5'-AAAGCUUUAGAGAACU-3' (SEQ ID NO: 61), 5'-AAAGCUGUUAGUUAGAACU-3' (SEQ ID NO: 62), 5'-AAAGCUGUUAGUAGAACU-3' (SEQ ID NO: 63), 5'-AAAGCUGUUUAGAUUAGAACU-3' (SEQ ID NO: 64), 5'-AAAGCUGUCUUAGGAUUAGAACU-3' (SEQ ID NO: 65), and 5'-AAAGCUGUCCUUAGGGAUUAGAACU-3' (SEQ ID NO: 66).

As yet another example, when the fourth and fifth regions of the engineered scaffold region are modified, the modified fourth and fifth regions may be a modified form of a fourth region and/or a fifth region of the naturally occurring scaffold region from which one or more nucleotides are removed. Here, removal of the nucleotide may occur in a portion that forms a Stem 5 (R:AR-2) structure (Takeda et al., Structure of the miniature type V-F CRISPR-Cas effector enzyme, Molecular Cell 81, 1-13 (2021)) in the fourth and fifth regions, and such removal may be done in pairs of nucleotides that form base pairs. Here, a sequence of the modified fourth region is characterized by comprising at least 5'-AACAAA-3'. Here, a sequence of the modified fifth region is characterized by comprising at least 5'-GGA-3'.

### Example 2 of engineered single guide RNA

In an embodiment, the engineered Cas12f1 guide RNA may be one in which an engineered scaffold region, a spacer, and a U-rich tail are sequentially linked to each other in a 5' to 3' direction.

The spacer has a length of 10 to 50 nucleotides and has a sequence complementary to a target sequence.

A sequence of the U-rich tail may be represented by (UₐN)_{b}U_{c}. Here, N is one of adenosine (A), uridine (U), cytidine (C), and guanosine (G), and a, b, and c are each an integer, with a being 1 to 5, and b being 0 or greater. As an example, a sequence of the U-rich tail may be 5'-UUUUAUUUU-3'. As an example, a sequence of the U-rich tail may be 5'-UUUUGUUUU-3'.

A sequence of the engineered scaffold region is such that the following sequences are sequentially linked to each other in a 5' to 3' direction:
a sequence selected from the group consisting of 5'-A-3', 5'-AA-3', 5'-GAA-3', 5'-AGAA-3', 5'-GAGAA-3', 5'-GGAGAA-3', 5'-UGGAGAA-3', 5'-GUGGAGAA-3', 5'-AGUGGAGAA-3', 5'-AAGUGGAGAA-3' (SEQ ID NO: 17), 5'-AAAGUGGAGAA-3' (SEQ ID NO: 18), 5'-UAAAGUGGAGAA-3' (SEQ ID NO: 19), 5'-AUAAAGUGGAGAA-3' (SEQ ID NO: 20), 5'-GAUAAAGUGGAGAA-3' (SEQ ID NO: 21), 5'-UGAUAAAGUGGAGAA-3' (SEQ ID NO: 22), 5'-CUGAUAAAGUGGAGAA-3' (SEQ ID NO: 23), 5'-ACUGAUAAAGUGGAGAA-3' (SEQ ID NO: 24), 5'-CACUGAUAAAGUGGAGAA-3' (SEQ ID NO: 25), 5'-UCACUGAUAAAGUGGAGAA-3' (SEQ ID NO: 26), 5'-UUCACUGAUAAAGUGGAGAA-3' (SEQ ID NO: 27), and 5'-CUUCACUGAUAAAGUGGAGAA-3' (SEQ ID NO: 10);
a sequence selected from the group consisting of 5'-CCGCUUCACUUAGAGUGAAGGUGG-3' (SEQ ID NO: 350), 5'-CCGCUUCACCUUAGGAGUGAAGGUGG-3' (SEQ ID NO: 351), 5'-CCGCUUCACCAUUAGUGAGUGAAGGUGG-3' (SEQ ID NO: 39), 5'-CCGCUUCACCAAUUAGUUGAGUGAAGGUGG-3' (SEQ ID NO: 40), 5'-CCGCUUCACCAAAUUAGCUUGAGUGAAGGUGG-3' (SEQ ID NO: 41), 5'-CCGCUUCACCAAAAUUAGACUUGAGUGAAGGUGG-3' (SEQ ID NO: 42), 5'-CCGCUUCACCAAAAGUUAGAACUUGAGUGAAGGUGG-3' (SEQ ID NO: 43), 5'-CCGCUUCACCAAAAGCUUAGGAACUUGAGUGAAGGUGG-3' (SEQ ID NO: 44), 5'-CCGCUUCACCAAAAGCUUUAGAGAACUUGAGUGAAGGUGG-3' (SEQ ID NO: 45), 5'-CCGCUUCACCAAAAGCUGUUAGUUAGAACUUGAGUGAAGGUGG-3' (SEQ ID NO: 46), 5'-CCGCUUCACCAAAAGCUGUUAGUAGAACUUGAGUGAAGGUGG-3' (SEQ ID NO: 47), 5'-CCGCUUCACCAAAAGCUGUUUAGAUUAGAACUUGAGUGAAGGUGG-3' (SEQ ID NO: 48), 5'-CCGCUUCACCAAAAGCUGUCUUAGGAUUAGAACUUGAGUGAAGGUGG-3' (SEQ ID NO: 49), 5'-CCGCUUCACCAAAAGCUGUCCUUAGGGAUUAGAACUUGAGUGAAGGUGG-3' (SEQ ID NO: 50), and 5'-CCGCUUCACCAAAAGCUGUCCCUUAGGGGAUUAGAACUUGAGUGAAGGUGG-3' (SEQ ID NO: 11);
5'-GCUGCUUGCAUCAGCCUAAUGUCGAGAAGUGCUUUCUUCGGAAAGUAACCCUCGA-3' (SEQ ID NO: 12);
a sequence selected from 5'-AACAAAGAAAGGA-3' (SEQ ID NO: 111), 5'-AACAAAUGAAAAGGA-3' (SEQ ID NO: 112), 5'-AACAAAUUGAAAAAGGA-3' (SEQ ID NO: 113), 5'-AACAAAUUCGAAAGAAGGA-3' (SEQ ID NO: 114), 5'-AACAAAUUCAGAAAUGAAGGA-3' (SEQ ID NO: 115), 5'-AACAAAUUCAUGAAAAUGAAGGA-3' (SEQ ID NO: 116), 5'-AACAAAUUCAUUGAAAAAUGAAGGA-3' (SEQ ID NO: 117), and 5'-AACAAAUUCAUUUGAAAGAAUGAAGGA-3' (SEQ ID NO: 118); and
5'-AUGCAAC-3',
wherein the sequence of the engineered scaffold region is different from 5'-CUUCACUGAUAAAGUGGAGAACCGCUUCACCAAAAGCUGUCCCUUAGGGGAUUAGA ACUUGAGUGAAGGUGGGCUGCUUGCAUCAGCCUAAUGUCGAGAAGUGCUUUCUUC GGAAAGUAACCCUCGAAACAAAUUCAUUUGAAAGAAUGAAGGAAUGCAAC-3' (SEQ ID NO: 7).

### Example 3 of engineered single guide RNA

In an embodiment, the engineered Cas12f1 guide RNA may be one in which an engineered scaffold region, a spacer, and a U-rich tail are sequentially linked to each other in a 5' to 3' direction.

The spacer has a length of 10 to 50 nucleotides and has a sequence complementary to a target sequence.

A sequence of the U-rich tail may be represented by (UₐN)_{b}U_{c}. Here, N is one of adenosine (A), uridine (U), cytidine (C), and guanosine (G). Here, a, b, and c are each an integer, with a being 1 to 5, and b being 0 or greater. As an example, a sequence of the U-rich tail may be 5'-UUUUAUUUU-3'. As an example, a sequence of the U-rich tail may be 5'-UUUUGUUUU-3'.

A sequence of the engineered scaffold region is such that the following sequences are sequentially linked to each other in a 5' to 3' direction:
a first sequence represented by 5'-A-3';
a second sequence represented by 5'-CCGCUUCACCA-3' (SEQ ID NO: 51);
a third sequence represented by 5'-UUAG-3';
a fourth sequence represented by 5'-UGAGUGAAGGUG-3' (SEQ ID NO: 52);
a fifth sequence represented by 5'-GGCUGCUUGCAUCAGCCUAAUGUCGAGAAGUGCUUUCUUCGGAAAGUAACCCUCGA -3' (SEQ ID NO: 12);
a sixth sequence represented by 5'-AACAAA-3';
a linker;
a seventh sequence represented by 5'-GGA-3'; and
an eighth sequence represented by 5'-AUGCAAC-3'.

As an example, the linker may be 5'-GAAA-3'.

As another example, the linker may be selected from the group consisting of 5'-GAAA-3', 5'-UGAAAA-3', 5'-UUGAAAAA-3', 5'-UUCGAAAGAA-3' (SEQ ID NO: 642), 5'-UUCAGAAAUGAA-3' (SEQ ID NO: 643), 5'-UUCAUGAAAAUGAA-3' (SEQ ID NO: 644), and 5'-UUCAUUGAAAAAUGAA-3' (SEQ ID NO: 645).

As a specific example of the embodiment, a sequence of the engineered scaffold region may further comprise a ninth sequence selected from the group consisting of 5'-A-3', 5'-GA-3', 5'-AGA-3', 5'-GAGA-3', 5'-GGAGA-3', 5'-UGGAGA-3', 5'-GUGGAGA-3', 5'-AGUGGAGA-3', 5'-AAGUGGAGA-3', 5'-AAAGUGGAGA-3' (SEQ ID NO: 28), 5'-UAAAGUGGAGA-3' (SEQ ID NO: 29), 5'-AUAAAGUGGAGA-3' (SEQ ID NO: 30), 5'-GAUAAAGUGGAGA-3' (SEQ ID NO: 31), 5'-UGAUAAAGUGGAGA-3' (SEQ ID NO: 32), 5'-CUGAUAAAGUGGAGA-3' (SEQ ID NO: 33), 5'-ACUGAUAAAGUGGAGA-3' (SEQ ID NO: 34), 5'-CACUGAUAAAGUGGAGA-3' (SEQ ID NO: 35), 5'-UCACUGAUAAAGUGGAGA-3' (SEQ ID NO: 36), 5'-UUCACUGAUAAAGUGGAGA-3' (SEQ ID NO: 37), and 5'-CUUCACUGAUAAAGUGGAGA-3' (SEQ ID NO: 38). Here, the 3' end of the ninth sequence may be linked to the 5' end of the first sequence.

As another specific example of the embodiment, a sequence of the engineered scaffold region may further comprise a tenth sequence selected from the group consisting of 5'-A-3', 5'-AA-3', 5'-AAA-3', 5'-AAAG-3', 5'-AAAGC-3', 5'-AAAGCU-3', 5'-AAAGCUG-3', 5'-AAAGCUGU-3', 5'-AAAGCUGUC-3', 5'-AAAGCUGUCC-3' (SEQ ID NO: 53), and 5'-AAAGCUGUCCC-3' (SEQ ID NO: 54). Here, the 3' end of the second sequence and the 5' end of the third sequence may be linked to each other via the tenth sequence.

As yet another specific example of the embodiment, a sequence of the engineered scaffold region may further comprise an eleventh sequence selected from the group consisting of 5'-U-3', 5'-CU-3', 5'-ACU-3', 5'-AACU-3', 5'-GAACU-3', 5'-AGAACU-3', 5'-UAGAACU-3', 5'-UUAGAACU-3', 5'-AUUAGAACU-3', 5'-GAUUAGAACU-3' (SEQ ID NO: 55), 5'-GGAUUAGAACU-3' (SEQ ID NO: 56), and 5'-GGGAUUAGAACU-3' (SEQ ID NO: 57). Here, the 3' end of the third sequence and the 5' end of the fourth sequence may be linked to each other via the eleventh sequence.

As still yet another specific example of the embodiment, a sequence of the engineered scaffold region may further comprise a tenth sequence selected from the group consisting of 5'-A-3', 5'-AA-3', 5'-AAA-3', 5'-AAAG-3', 5'-AAAGC-3', 5'-AAAGCU-3', 5'-AAAGCUG-3', 5'-AAAGCUGU-3', 5'-AAAGCUGUC-3', 5'-AAAGCUGUCC-3' (SEQ ID NO: 53), and 5'-AAAGCUGUCCC-3' (SEQ ID NO: 54), and an eleventh sequence selected from the group consisting of 5'-U-3', 5'-CU-3', 5'-ACU-3', 5'-AACU-3', 5'-GAACU-3', 5'-AGAACU-3', 5'-UAGAACU-3', 5'-UUAGAACU-3', 5'-AUUAGAACU-3', 5'-GAUUAGAACU-3' (SEQ ID NO: 55), 5'-GGAUUAGAACU-3' (SEQ ID NO: 56), and 5'-GGGAUUAGAACU-3' (SEQ ID NO: 57). Here, the 3' end of the second sequence and the 5' end of the third sequence may be linked via the tenth sequence, and the 3' end of the third sequence and the 5' end of the fourth sequence may be linked via the eleventh sequence.

As an example, when the tenth sequence is 5'-A-3', the eleventh sequence may be 5'-U-3'. As another example, when the tenth sequence is 5'-AA-3', the eleventh sequence may be 5'-CU-3'. As yet another example, when the tenth sequence is 5'-AAA-3', the eleventh sequence may be 5'-ACU-3'. As still yet another example, when the tenth sequence is 5'-AAAG-3', the eleventh sequence may be 5'-AACU-3'. As still yet another example, when the tenth sequence is 5'-AAAGC-3', the eleventh sequence may be 5'-GAACU-3'. As still yet another example, when the tenth sequence is 5'-AAAGCU-3', the eleventh sequence may be 5'-AGAACU-3'. As still yet another example, when the tenth sequence is 5'-AAAGCUG-3', the eleventh sequence may be 5'-UAGAACU-3' or 5'-UUAGAACU-3'. As still yet another example, when the tenth sequence is 5'-AAAGCUGU-3', the eleventh sequence may be 5'-AUUAGAACU-3'. As still yet another example, when the tenth sequence is 5'-AAAGCUGUC-3', the eleventh sequence may be 5'-GAUUAGAACU-3' (SEQ ID NO: 55). As still yet another example, when the tenth sequence is 5'-AAAGCUGUCC-3' (SEQ ID NO: 53), the eleventh sequence may be 5'-GGAUUAGAACU-3' (SEQ ID NO: 56). As still yet another example, when the tenth sequence is 5'-AAAGCUGUCCC-3' (SEQ ID NO: 54), the eleventh sequence may be 5'-GGGAUUAGAACU-3' (SEQ ID NO: 57).

As still yet another specific example of the embodiment, a sequence of the engineered scaffold region may further comprise a twelfth sequence selected from the group consisting of 5'-U-3', 5'-UU-3', 5'-UUC-3', 5'-UUCA-3', 5'-UUCAU-3', 5'-UUCAUU-3', and 5'-UUCAUUU-3'. Here, the 3' end of the sixth sequence and the 5' end of the linker may be linked via the twelfth sequence.

As still yet another specific example of the embodiment, a sequence of the engineered scaffold region may further comprise a thirteenth sequence selected from the group consisting of 5'-A-3', 5'-AA-3', 5'-GAA-3', 5'-UGAA-3', 5'-AUGAA-3', 5'-AAUGAA-3', and 5'-GAAUGAA-3'. Here, the 3' end of the linker and the 5' end of the seventh sequence may be linked via the thirteenth sequence.

As still yet another specific example of the embodiment, a sequence of the engineered scaffold region may further comprise a twelfth sequence selected from the group consisting of 5'-U-3', 5'-UU-3', 5'-UUC-3', 5'-UUCA-3', 5'-UUCAU-3', 5'-UUCAUU-3', and 5'-UUCAUUU-3', and a thirteenth sequence selected from the group consisting of 5'-A-3', 5'-AA-3', 5'-GAA-3', 5'-UGAA-3', 5'-AUGAA-3', 5'-AAUGAA-3', and 5'-GAAUGAA-3'. Here, the 3' end of the sixth sequence and the 5' end of the linker may be linked via the twelfth sequence, and the 3' end of the linker and the 5' end of the seventh sequence may be linked via the thirteenth sequence.

As an example, when the twelfth sequence is 5'-U-3', the thirteenth sequence may be 5'-A-3'. As another example, when the twelfth sequence is 5'-UU-3', the thirteenth sequence may be 5'-AA-3'. As yet another example, when the twelfth sequence is 5'-UUC-3', the thirteenth sequence may be 5'-GAA-3'. As still yet another example, when the twelfth sequence is 5'-UUCA-3', the thirteenth sequence may be 5'-UGAA-3'. As still yet another example, when the twelfth sequence is 5'-UUCAU-3', the thirteenth sequence may be 5'-AUGAA-3'. As still yet another example, when the twelfth sequence is 5'-UUCAUU-3', the thirteenth sequence may be 5'-AAUGAA-3'. As still yet another example, when the twelfth sequence is 5'-UUCAUUU-3', the thirteenth sequence may be 5'-GAAUGAA-3'.

### Example 4 of engineered single guide RNA

In an embodiment, the engineered Cas12f1 guide RNA may be one in which an engineered scaffold region, a spacer, and a U-rich tail are sequentially linked in a 5' to 3' direction.

The spacer has a length of 10 to 50 nucleotides and has a sequence complementary to a target sequence.

A sequence of the U-rich tail may be a uridine repeat sequence, or a modified uridine repeat sequence. As an example, a sequence of the U-rich tail may comprise a sequence in which 1, 2, 3, 4, 5, 6, 7, 8, 9, 10, 11, 12, 13, 14, 15, 16, 17, 18, 19, or 20 consecutive uridines are contained. As another example, a sequence of the U-rich tail may comprise a sequence in which one or more UV, UUV, UUUV, UUUUV, and/or UUUUUV are repeated. Here, V is one of adenosine (A), cytidine (C), and guanosine (G).

The engineered scaffold region is one in which a first region, a second region, a third region, a fourth region, a linker, a fifth region, and a sixth region, which correspond to those of the naturally occurring scaffold region, are sequentially linked to each other in a 5' to 3' direction, and one or more regions selected from the first region, the second region, the third region, the fourth region, and the fifth region are modified as compared with the naturally occurring scaffold region. Furthermore, the engineered scaffold region may be one from which a first region and/or a second region corresponding to those of the naturally occurring scaffold region are removed.

As an example, when a first region of the engineered scaffold region is modified, the modified first region may be a modified form of a first region of the naturally occurring scaffold region from which one or more nucleotides are removed. Here, the removed nucleotide may be a nucleotide belonging to Stem 1 (Takeda et al., Structure of the miniature type V-F CRISPR-Cas effector enzyme, Molecular Cell 81, 1-13 (2021)) in the first region. Here, a sequence of the modified first region is characterized by comprising 5'-A-3'.

As another example, when a second region of the engineered scaffold region is modified, the modified second region may be a modified form of a second region of the naturally occurring scaffold region from which one or more nucleotides are removed. Here, removal of the nucleotide may occur in a portion that forms a Stem 2 structure (Takeda et al., Structure of the miniature type V-F CRISPR-Cas effector enzyme, Molecular Cell 81, 1-13 (2021)) of the second region, and such removal may be done in pairs of nucleotides that form base pairs. Here, the modified second region is characterized by comprising at least 5'-G-3'.

As yet another example, when a third region of the engineered scaffold region is modified, the modified third region may be a modified form of a third region of the naturally occurring scaffold region from which one or more nucleotides are removed. Here, removal of the nucleotide may occur in a portion that forms a Stem 4 structure (Takeda et al., Structure of the miniature type V-F CRISPR-Cas effector enzyme, Molecular Cell 81, 1-13 (2021)) in the third region, and such removal may be done in pairs of nucleotides that form base pairs. Here, the sequence of the modified third region is characterized by comprising 5'-GCUGCUUGCAUCAGCCUAAUGUCGAG-3' (SEQ ID NO: 475) and 5'-CUCGA-3'. More specifically, a sequence of the modified third region may be one in which 5'-GCUGCUUGCAUCAGCCUAAUGUCGAG-3' (SEQ ID NO: 475) and 5'-CUCGA-3' are sequentially linked to each other in a 5' to 3' direction, and the sequences may be linked via an appropriate intermediate sequence. As an example, the intermediate sequence may be selected from the group consisting of 5'-UUCG-3', 5'-AUUCGC-3', 5'-AAUUCGC-3', 5'-AAUUCGCC-3', 5'-AAGUUCGCC-3', 5'-AAGUUCGACC-3' (SEQ ID NO: 476), 5'-AAGUUUCGAACC-3' (SEQ ID NO: 477), 5'-AAGUGUUCGUAACC-3' (SEQ ID NO: 478), 5'-AAGUGCUUCGGUAACC-3' (SEQ ID NO: 479), 5'-AAGUGCUUUCGAGUAACC-3' (SEQ ID NO: 480), 5'-AAGUGCUCUUCGGAGUAACC-3' (SEQ ID NO: 481), 5'-AAGUGCUUUUCGAAGUAACC-3' (SEQ ID NO: 482), 5'-AAGUGCUUUUUCGAAAGUAACC-3' (SEQ ID NO: 483), and 5'-AAGUGCUUUCUUCGGAAAGUAACC-3' (SEQ ID NO: 484).

As still yet another example, when the fourth and fifth regions of the engineered scaffold region are modified, the modified fourth and fifth regions may be modified forms of a fourth region and/or a fifth region of the naturally occurring scaffold region from which one or more nucleotides are removed. Here, removal of the nucleotide may occur in a portion that forms a Stem 5 (R:AR-2) structure (Takeda et al., Structure of the miniature type V-F CRISPR-Cas effector enzyme, Molecular Cell 81, 1-13 (2021)) in the fourth and fifth regions, and such removal may be done in pairs of nucleotides that form base pairs. Here, a sequence of the modified fourth region is characterized by comprising at least 5'-AACAAA-3'. Here, a sequence of the modified fifth region is characterized by comprising at least 5'-GGA-3'.

### Example 5 of engineered single guide RNA

In an embodiment, the engineered Cas12f1 guide RNA may be one in which an engineered scaffold region, a spacer, and a U-rich tail are sequentially linked to each other in a 5' to 3' direction.

The spacer has a length of 10 to 50 nucleotides and has a sequence complementary to a target sequence.

A sequence of the U-rich tail may be represented by (UₐN)_{b}U_{c}. Here, N is one of adenosine (A), uridine (U), cytidine (C), and guanosine (G), and a, b, and c are each an integer, with a being 1 to 5, and b being 0 or greater. As an example, a sequence of the U-rich tail may be 5'-UUUUAUUUU-3'. As an example, a sequence of the U-rich tail may be 5'-UUUUGUUUU-3'.

A sequence of the engineered scaffold region is such that the following sequences are sequentially linked to each other in a 5' to 3' direction:
a sequence selected from the group consisting of 5'-A-3', 5'-AA-3', 5'-GAA-3', 5'-AGAA-3', 5'-GAGAA-3', 5'-GGAGAA-3', 5'-UGGAGAA-3', 5'-GUGGAGAA-3', 5'-AGUGGAGAA-3', 5'-AAGUGGAGAA-3' (SEQ ID NO: 17), 5'-AAAGUGGAGAA-3' (SEQ ID NO: 18), 5'-UAAAGUGGAGAA-3' (SEQ ID NO: 19), 5'-AUAAAGUGGAGAA-3' (SEQ ID NO: 20), 5'-GAUAAAGUGGAGAA-3' (SEQ ID NO: 21), 5'-UGAUAAAGUGGAGAA-3' (SEQ ID NO: 22), 5'-CUGAUAAAGUGGAGAA-3' (SEQ ID NO: 23), 5'-ACUGAUAAAGUGGAGAA-3' (SEQ ID NO: 24), 5'-CACUGAUAAAGUGGAGAA-3' (SEQ ID NO: 25), 5'-UCACUGAUAAAGUGGAGAA-3' (SEQ ID NO: 26), 5'-UUCACUGAUAAAGUGGAGAA-3' (SEQ ID NO: 27), and 5'-CUUCACUGAUAAAGUGGAGAA-3' (SEQ ID NO: 10);
a sequence selected from the group consisting of 5'-G-3', 5'-UUAGG-3', 5'-CUUAGGG-3', 5'-CUUAGUGG-3', 5'-CCUUAGGUGG-3' (SEQ ID NO: 342), 5'-CCGUUAGGUGG-3' (SEQ ID NO: 343), 5'-CCGCUUAGGGUGG-3' (SEQ ID NO: 344),
5'-CCGCUUUAGAGGUGG-3' (SEQ ID NO: 345),
5'-CCGCUUUUAGAAGGUGG-3' (SEQ ID NO: 346),
5'-CCGCUUCUUAGGAAGGUGG-3' (SEQ ID NO: 347),
5'-CCGCUUCAUUAGUGAAGGUGG-3' (SEQ ID NO: 348),
5'-CCGCUUCACUUAGGUGAAGGUGG-3' (SEQ ID NO: 349),
5'-CCGCUUCACUUAGAGUGAAGGUGG-3' (SEQ ID NO: 350),
5'-CCGCUUCACCUUAGGAGUGAAGGUGG-3' (SEQ ID NO: 351),
5'-CCGCUUCACCAUUAGUGAGUGAAGGUGG-3' (SEQ ID NO: 352),
5'-CCGCUUCACCAAUUAGUUGAGUGAAGGUGG-3' (SEQ ID NO: 353),
5'-CCGCUUCACCAAAUUAGCUUGAGUGAAGGUGG-3' (SEQ ID NO: 354),
5'-CCGCUUCACCAAAAUUAGACUUGAGUGAAGGUGG-3' (SEQ ID NO: 355),
5'-CCGCUUCACCAAAAGUUAGAACUUGAGUGAAGGUGG-3' (SEQ ID NO: 356),
5'-CCGCUUCACCAAAAGCUUAGGAACUUGAGUGAAGGUGG-3' (SEQ ID NO: 357),
5'-CCGCUUCACCAAAAGCUUUAGAGAACUUGAGUGAAGGUGG-3' (SEQ ID NO: 358),
5'-CCGCUUCACCAAAAGCUGUUAGUAGAACUUGAGUGAAGGUGG-3' (SEQ ID NO: 359),
5'-CCGCUUCACCAAAAGCUGUUAGUUAGAACUUGAGUGAAGGUGG-3' (SEQ ID NO: 360),
5'-CCGCUUCACCAAAAGCUGUUUAGAUUAGAACUUGAGUGAAGGUGG-3' (SEQ ID NO: 361),
5'-CCGCUUCACCAAAAGCUGUCUUAGGAUUAGAACUUGAGUGAAGGUGG-3' (SEQ ID NO: 362), and 
5'-
CCGCUUCACCAAAAGCUGUCCCUUAGGGGAUUAGAACUUGAGUGAAGGUGG-3'
(SEQ ID NO: 11);
a sequence selected from the group consisting of 5'-GCUGCUUGCAUCAGCCUAAUGUCGAGAAGUGCUUUCUUCGGAAAGUAACCCUCGA-3' (SEQ ID NO: 434), 
5'-
GCUGCUUGCAUCAGCCUAAUGUCGAGAAGUGCUUUUUCGAAAGUAACCCUCGA-3'
(SEQ ID NO: 435),
 
5'-
GCUGCUUGCAUCAGCCUAAUGUCGAGAAGUGCUUUUCGAAGUAACCCUCGA-3' (SEQ
ID NO: 436),
 
5'-
GCUGCUUGCAUCAGCCUAAUGUCGAGAAGUGCUCUUCGGAGUAACCCUCGA-3'
(SEQ ID NO: 437),
 
5'-GCUGCUUGCAUCAGCCUAAUGUCGAGAAGUGCUUUCGAGUAACCCUCGA-
3' (SEQ ID NO: 438),
5'-GCUGCUUGCAUCAGCCUAAUGUCGAGAAGUGCUUCGGUAACCCUCGA-3' (SEQ ID NO: 439),
5'-GCUGCUUGCAUCAGCCUAAUGUCGAGAAGUGUUCGUAACCCUCGA-3' (SEQ ID NO: 440),
5'-GCUGCUUGCAUCAGCCUAAUGUCGAGAAGUUUCGAACCCUCGA-3' (SEQ ID NO: 441),
5'-GCUGCUUGCAUCAGCCUAAUGUCGAGAAGUUCGACCCUCGA-3' (SEQ ID NO: 442),
5'-GCUGCUUGCAUCAGCCUAAUGUCGAGAAGUUCGCCCUCGA-3' (SEQ ID NO: 443),
5'-GCUGCUUGCAUCAGCCUAAUGUCGAGAAUUCGCCCUCGA-3' (SEQ ID NO: 444),
5'-GCUGCUUGCAUCAGCCUAAUGUCGAGAAUUCGCCUCGA-3' (SEQ ID NO: 445),
5'-GCUGCUUGCAUCAGCCUAAUGUCGAGAUUCGCCUCGA-3' (SEQ ID NO: 446),
5'-GCUGCUUGCAUCAGCCUAAUGUCGAGUUCGCUCGA-3' (SEQ ID NO: 447), and 
5'-
GCUGCUUGCAUCAGCCUAAUGUCGAGAAGUGCUUUCUUCGGAAAGUAACCCUCGA-
3' (SEQ ID NO: 12);
a sequence selected from 5'-AACAAAGAAAGGA-3' (SEQ ID NO: 111),
5'-AACAAAUGAAAAGGA-3' (SEQ ID NO: 112),
5'-AACAAAUUGAAAAAGGA-3' (SEQ ID NO: 113),
5'-AACAAAUUCGAAAGAAGGA-3' (SEQ ID NO: 114),
5'-AACAAAUUCAGAAAUGAAGGA-3' (SEQ ID NO: 115),
5'-AACAAAUUCAUGAAAAUGAAGGA-3' (SEQ ID NO: 116),
5'-AACAAAUUCAUUGAAAAAUGAAGGA-3' (SEQ ID NO: 117), and
5'-AACAAAUUCAUUUGAAAGAAUGAAGGA-3' (SEQ ID NO: 118); and
5'-AUGCAAC-3',
wherein the sequence of the engineered scaffold region is different from 5'-CUUCACUGAUAAAGUGGAGAACCGCUUCACCAAAAGCUGUCCCUUAGGGGAUUAGA ACUUGAGUGAAGGUGGGCUGCUUGCAUCAGCCUAAUGUCGAGAAGUGCUUUCUUC GGAAAGUAACCCUCGAAACAAAUUCAUUUGAAAGAAUGAAGGAAUGCAAC-3' (SEQ ID NO: 7).

### Example 6 of engineered single guide RNA

In an embodiment, the engineered Cas12f1 guide RNA may be one in which an engineered scaffold region, a spacer, and a U-rich tail are sequentially linked to each other in a 5' to 3' direction.

The spacer has a length of 10 to 50 nucleotides and has a sequence complementary to a target sequence.

The sequence of the U-rich tail may be represented by (UₐN)_{b}U_{c}. Here, N is one of adenosine (A), uridine (U), cytidine (C), and guanosine (G). Here, a, b, and c are each an integer, with a being 1 to 5, and b being 0 or greater. As an example, a sequence of the U-rich tail may be 5'-UUUUAUUUU-3'. As an example, a sequence of the U-rich tail may be 5'-UUUUGUUUU-3'.

A sequence of the engineered scaffold region may be such that the following sequences are sequentially linked to each other in a 5' to 3' direction:
a first sequence represented by 5'-GCUGCUUGCAUCAGCCUAAUGUCGAG-3' (SEQ ID NO: 475);
a second sequence represented by 5'-UUCG-3';
a third sequence represented by 5'-CUCGA-3';
a fourth sequence represented by 5'-AACAAA-3';
a linker;
a fifth sequence represented by 5'-GGA-3'; and
a sixth sequence represented by 5'-AUGCAAC-3'.

As an example, the linker may be 5'-GAAA-3'.

As another example, the linker may be selected from the group consisting of 5'-GAAA-3', 5'-UGAAAA-3', 5'-UUGAAAAA-3', 5'-UUCGAAAGAA-3' (SEQ ID NO: 642), 5'-UUCAGAAAUGAA-3' (SEQ ID NO: 643), 5'-UUCAUGAAAAUGAA-3' (SEQ ID NO: 644), and 5'-UUCAUUGAAAAAUGAA-3' (SEQ ID NO: 645).

As a specific example of the embodiment, a sequence of the engineered scaffold region may further comprise a seventh sequence selected from the group consisting of 5'-A-3', 5'-AA-3', 5'-GAA-3', 5'-AGAA-3', 5'-GAGAA-3', 5'-GGAGAA-3', 5'-UGGAGAA-3', 5'-GUGGAGAA-3', 5'-AGUGGAGAA-3', 5'-AAGUGGAGAA-3' (SEQ ID NO: 17), 5'-AAAGUGGAGAA-3' (SEQ ID NO: 18), 5'-UAAAGUGGAGAA-3' (SEQ ID NO: 19), 5'-AUAAAGUGGAGAA-3' (SEQ ID NO: 20), 5'-GAUAAAGUGGAGAA-3' (SEQ ID NO: 21), 5'-UGAUAAAGUGGAGAA-3' (SEQ ID NO: 22), 5'-CUGAUAAAGUGGAGAA-3' (SEQ ID NO: 23), 5'-ACUGAUAAAGUGGAGAA-3' (SEQ ID NO: 24), 5'-CACUGAUAAAGUGGAGAA-3' (SEQ ID NO: 25), 5'-UCACUGAUAAAGUGGAGAA-3' (SEQ ID NO: 26), 5'-UUCACUGAUAAAGUGGAGAA-3' (SEQ ID NO: 27), and 5'-CUUCACUGAUAAAGUGGAGAA-3' (SEQ ID NO: 10). Here, the 3' end of the seventh sequence may be linked to the 5' end of the first sequence.

As a specific example of the embodiment, a sequence of the engineered scaffold region may further comprise an eighth sequence selected from the group consisting of 5'-G-3', 5'-UUAGG-3', 5'-CUUAGGG-3', 5'-CUUAGUGG-3', 5'-CCUUAGGUGG-3' (SEQ ID NO: 342), 5'-CCGUUAGGUGG-3' (SEQ ID NO: 343), 5'-CCGCUUAGGGUGG-3' (SEQ ID NO: 344),
5'-CCGCUUUAGAGGUGG-3' (SEQ ID NO: 345),
5'-CCGCUUUUAGAAGGUGG-3' (SEQ ID NO: 346),
5'-CCGCUUCUUAGGAAGGUGG-3' (SEQ ID NO: 347),
5'-CCGCUUCAUUAGUGAAGGUGG-3' (SEQ ID NO: 348),
5'-CCGCUUCACUUAGGUGAAGGUGG-3' (SEQ ID NO: 349),
5'-CCGCUUCACUUAGAGUGAAGGUGG-3' (SEQ ID NO: 350),
5'-CCGCUUCACCUUAGGAGUGAAGGUGG-3' (SEQ ID NO: 351),
5'-CCGCUUCACCAUUAGUGAGUGAAGGUGG-3' (SEQ ID NO: 352),
5'-CCGCUUCACCAAUUAGUUGAGUGAAGGUGG-3' (SEQ ID NO: 353),
5'-CCGCUUCACCAAAUUAGCUUGAGUGAAGGUGG-3' (SEQ ID NO: 354),
5'-CCGCUUCACCAAAAUUAGACUUGAGUGAAGGUGG-3' (SEQ ID NO: 355),
5'-CCGCUUCACCAAAAGUUAGAACUUGAGUGAAGGUGG-3' (SEQ ID NO: 356),
5'-CCGCUUCACCAAAAGCUUAGGAACUUGAGUGAAGGUGG-3' (SEQ ID NO: 357),
5'-CCGCUUCACCAAAAGCUUUAGAGAACUUGAGUGAAGGUGG-3' (SEQ ID NO: 358),
5'-CCGCUUCACCAAAAGCUGUUAGUAGAACUUGAGUGAAGGUGG-3' (SEQ ID NO: 359),
5'-CCGCUUCACCAAAAGCUGUUAGUUAGAACUUGAGUGAAGGUGG-3' (SEQ ID NO: 360),
5'-CCGCUUCACCAAAAGCUGUUUAGAUUAGAACUUGAGUGAAGGUGG-3' (SEQ ID NO: 361),
5'-CCGCUUCACCAAAAGCUGUCUUAGGAUUAGAACUUGAGUGAAGGUGG-3' (SEQ ID NO: 362), and
5'-CCGCUUCACCAAAAGCUGUCCCUUAGGGGAUUAGAACUUGAGUGAAGGUGG-3' (SEQ ID NO: 11). Here, the 3' end of the eighth sequence may be linked to the 5' end of the first sequence.

As a specific example of the embodiment, a sequence of the engineered scaffold region may further comprise a seventh sequence selected from the group consisting of 5'-A-3', 5'-AA-3', 5'-GAA-3', 5'-AGAA-3', 5'-GAGAA-3', 5'-GGAGAA-3', 5'-UGGAGAA-3', 5'-GUGGAGAA-3', 5'-AGUGGAGAA-3', 5'-AAGUGGAGAA-3' (SEQ ID NO: 17), 5'-AAAGUGGAGAA-3' (SEQ ID NO: 18), 5'-UAAAGUGGAGAA-3' (SEQ ID NO: 19), 5'-AUAAAGUGGAGAA-3' (SEQ ID NO: 20), 5'-GAUAAAGUGGAGAA-3' (SEQ ID NO: 21), 5'-UGAUAAAGUGGAGAA-3' (SEQ ID NO: 22), 5'-CUGAUAAAGUGGAGAA-3' (SEQ ID NO: 23), 5'-ACUGAUAAAGUGGAGAA-3' (SEQ ID NO: 24), 5'-CACUGAUAAAGUGGAGAA-3' (SEQ ID NO: 25), 5'-UCACUGAUAAAGUGGAGAA-3' (SEQ ID NO: 26), 5'-UUCACUGAUAAAGUGGAGAA-3' (SEQ ID NO: 27), and 5'-CUUCACUGAUAAAGUGGAGAA-3' (SEQ ID NO: 10), and an eighth sequence selected from the group consisting of 5'-G-3', 5'-UUAGG-3', 5'-CUUAGGG-3', 5'-CUUAGUGG-3', 5'-CCUUAGGUGG-3' (SEQ ID NO: 342), 5'-CCGUUAGGUGG-3' (SEQ ID NO: 343), 5'-CCGCUUAGGGUGG-3' (SEQ ID NO: 344),
5'-CCGCUUUAGAGGUGG-3' (SEQ ID NO: 345),
5'-CCGCUUUUAGAAGGUGG-3' (SEQ ID NO: 346),
5'-CCGCUUCUUAGGAAGGUGG-3' (SEQ ID NO: 347),
5'-CCGCUUCAUUAGUGAAGGUGG-3' (SEQ ID NO: 348),
5'-CCGCUUCACUUAGGUGAAGGUGG-3' (SEQ ID NO: 349),
5'-CCGCUUCACUUAGAGUGAAGGUGG-3' (SEQ ID NO: 350),
5'-CCGCUUCACCUUAGGAGUGAAGGUGG-3' (SEQ ID NO: 351),
5'-CCGCUUCACCAUUAGUGAGUGAAGGUGG-3' (SEQ ID NO: 352),
5'-CCGCUUCACCAAUUAGUUGAGUGAAGGUGG-3' (SEQ ID NO: 353),
5'-CCGCUUCACCAAAUUAGCUUGAGUGAAGGUGG-3' (SEQ ID NO: 354),
5'-CCGCUUCACCAAAAUUAGACUUGAGUGAAGGUGG-3' (SEQ ID NO: 355),
5'-CCGCUUCACCAAAAGUUAGAACUUGAGUGAAGGUGG-3' (SEQ ID NO: 356),
5'-CCGCUUCACCAAAAGCUUAGGAACUUGAGUGAAGGUGG-3' (SEQ ID NO: 357),
5'-CCGCUUCACCAAAAGCUUUAGAGAACUUGAGUGAAGGUGG-3' (SEQ ID NO: 358),
5'-CCGCUUCACCAAAAGCUGUUAGUAGAACUUGAGUGAAGGUGG-3' (SEQ ID NO: 359),
5'-CCGCUUCACCAAAAGCUGUUAGUUAGAACUUGAGUGAAGGUGG-3' (SEQ ID NO: 360),
5'-CCGCUUCACCAAAAGCUGUUUAGAUUAGAACUUGAGUGAAGGUGG-3' (SEQ ID NO: 361),
5'-CCGCUUCACCAAAAGCUGUCUUAGGAUUAGAACUUGAGUGAAGGUGG-3' (SEQ ID NO: 362), and
5'-CCGCUUCACCAAAAGCUGUCCCUUAGGGGAUUAGAACUUGAGUGAAGGUGG-3' (SEQ ID NO: 11). Here, the 3' end of the eighth sequence may be linked to the 5' end of the first sequence, and the 3' end of the seventh sequence may be linked to the 5' end of the eighth sequence.

As a specific example of the embodiment, a sequence of the engineered scaffold region may further comprise a ninth sequence selected from the group consisting of 5'-A-3', 5'-AA-3', 5'-AAG-3', 5'-AAGU-3', 5'-AAGUG-3', 5'-AAGUGC-3', 5'-AAGUGCU-3', 5'-AAGUGCUU-3', 5'-AAGUGCUUU-3', and 5'-AAGUGCUUUC-3' (SEQ ID NO: 485). Here, the 3' end of the first sequence and the 5' end of the second sequence may be linked via the ninth sequence.

As an example of the embodiment, a sequence of the engineered scaffold region may further comprise a tenth sequence selected from the group consisting of 5'-C-3', 5'-CC-3', 5'-ACC-3', 5'-AACC-3', 5'-UAACC-3', 5'-GUAACC-3', 5'-AGUAACC-3', 5'-AAGUAACC-3', 5'-AAAGUAACC-3', and 5'-GAAAGUAACC-3' (SEQ ID NO: 486). Here, the 3' end of the second sequence and the 5' end of the third sequence may be linked via the tenth sequence.

As a specific example of the embodiment, a sequence of the engineered scaffold region may further comprise a ninth sequence selected from the group consisting of 5'-A-3', 5'-AA-3', 5'-AAG-3', 5'-AAGU-3', 5'-AAGUG-3', 5'-AAGUGC-3', 5'-AAGUGCU-3', 5'-AAGUGCUU-3', 5'-AAGUGCUUU-3', and 5'-AAGUGCUUUC-3' (SEQ ID NO: 485), and may further comprise a tenth sequence selected from the group consisting of 5'-C-3', 5'-CC-3', 5'-ACC-3', 5'-AACC-3', 5'-UAACC-3', 5'-GUAACC-3', 5'-AGUAACC-3', 5'-AAGUAACC-3', 5'-AAAGUAACC-3', and 5'-GAAAGUAACC-3' (SEQ ID NO: 486). Here, the 3' end of the first sequence and the 5' end of the second sequence may be linked via the ninth sequence, and the 3' end of the second sequence and the 5' end of the third sequence may be linked via the tenth sequence.

As an example, when the ninth sequence is 5'-A-3', the tenth sequence may be 5'-C-3'. As another example, when the ninth sequence is 5'-AA-3', the tenth sequence may be 5'-C-3' or 5'-CC-3'. As yet another example, when the ninth sequence is 5'-AAG-3', the tenth sequence may be 5'-CC-3' or 5'-ACC-3'. As still yet another example, when the ninth sequence is 5'-AAGU-3', the tenth sequence may be 5'-AACC-3'. As still yet another example, when the ninth sequence is 5'-AAGUG-3', the tenth sequence may be 5'-UAACC-3'. As still yet another example, when the ninth sequence is 5'-AAGUGC-3', the tenth sequence may be 5'-GUAACC-3'. As still yet another example, when the ninth sequence is 5'-AAGUGCU-3', the tenth sequence may be 5'-AGUAACC-3'. As still yet another example, when the ninth sequence is 5'-AAGUGCUC-3', the tenth sequence may be 5'-GAGUAACC-3'. As still yet another example, when the ninth sequence is 5'-AAGUGCUU-3', the tenth sequence may be 5'-AAGUAACC-3'. As still yet another example, when the ninth sequence is 5'-AAGUGCUUU-3', the tenth sequence may be 5'-AAAGUAACC-3'. As still yet another example, when the ninth sequence is 5'-AAGUGCUUUC-3' (SEQ ID NO: 485), the tenth sequence may be 5'-GAAAGUAACC-3' (SEQ ID NO: 486).

As another specific example of the embodiment, a sequence of the engineered scaffold region may further comprise an eleventh sequence selected from the group consisting of 5'-U-3', 5'-UU-3', 5'-UUC-3', 5'-UUCA-3', 5'-UUCAU-3', 5'-UUCAUU-3', and 5'-UUCAUUU-3'. Here, the 3' end of the fourth sequence and the 5' end of the linker may be linked via the eleventh sequence.

As yet another specific example of the embodiment, a sequence of the engineered scaffold region may furthercomprise a twelfth sequence selected from the group consisting of 5'-A-3', 5'-AA-3', 5'-GAA-3', 5'-UGAA-3', 5'-AUGAA-3', 5'-AAUGAA-3', and 5'-GAAUGAA-3'. Here, the 3' end of the linker and the 5' end of the fifth sequence may be linked via the twelfth sequence.

As still yet another specific example of the embodiment, a sequence of the engineered scaffold region may further comprise an eleventh sequence selected from the group consisting of 5'-U-3', 5'-UU-3', 5'-UUC-3', 5'-UUCA-3', 5'-UUCAU-3', 5'-UUCAUU-3', and 5'-UUCAUUU-3', and a twelfth sequence selected from the group consisting of 5'-A-3', 5'-AA-3', 5'-GAA-3', 5'-UGAA-3', 5'-AUGAA-3', 5'-AAUGAA-3', and 5'-GAAUGAA-3'. Here, the 3' end of the fourth sequence and the 5' end of the linker may be linked via the eleventh sequence, and the 3' end of the linker and the 5' end of the fifth sequence may be linked via the twelfth sequence.

As an example, when the eleventh sequence is 5'-U-3', the twelfth sequence may be 5'-A-3'. As another example, when the eleventh sequence is 5'-UU-3', the twelfth sequence may be 5'-AA-3'. As yet another example, when the eleventh sequence is 5'-UUC-3', the twelfth sequence may be 5'-GAA-3'. As still yet another example, when the eleventh sequence is 5'-UUCA-3', the twelfth sequence may be 5'-UGAA-3'. As still yet another example, when the eleventh sequence is 5'-UUCAU-3', the twelfth sequence may be 5'-AUGAA-3'. As still yet another example, when the eleventh sequence is 5'-UUCAUU-3', the twelfth sequence may be 5'-AAUGAA-3'. As still yet another example, when the eleventh sequence is 5'-UUCAUUU-3', the twelfth sequence may be 5'-GAAUGAA-3'.

### Examples of engineered single guide RNA sequence

In an embodiment, the engineered single guide RNA may have a sequence selected from SEQ ID NOs: 211 to 253, SEQ ID NOs: 296 to 308, SEQ ID NOs: 311 to 323, SEQ ID NOs: 326 to 338, SEQ ID NOs: 488 to 541, and SEQ ID NOs: 545 to 551.

### Examples of engineered dual guide RNA

In an embodiment, the engineered Cas12f1 guide RNA may comprise an engineered scaffold region, a spacer, and a U-rich tail.

The spacer has a length of 10 to 50 nucleotides and has a sequence complementary to a target sequence.

A sequence of the U-rich tail may be represented by (UₐN)_{b}U_{c}. Here, N is one of adenosine (A), uridine (U), cytidine (C), and guanosine (G). Here, a, b, and c are each an integer, with a being 1 to 5, and b being 0 or greater.

As an example, a sequence of the U-rich tail may be 5'-UUUUAUUUU-3'.

A sequence of the engineered scaffold region comprises in a 5' to 3' direction:
an engineered tracrRNA in which the following sequences are linked to each other:
a first sequence selected from the group consisting of 5'-A-3', 5'-AA-3', 5'-GAA-3', 5'-AGAA-3', 5'-GAGAA-3', 5'-GGAGAA-3', 5'-UGGAGAA-3', 5'-GUGGAGAA-3', 5'-AGUGGAGAA-3', 5'-AAGUGGAGAA-3' (SEQ ID NO: 17), 5'-AAAGUGGAGAA-3' (SEQ ID NO: 18), 5'-UAAAGUGGAGAA-3' (SEQ ID NO: 19), 5'-AUAAAGUGGAGAA-3' (SEQ ID NO: 20), 5'-GAUAAAGUGGAGAA-3' (SEQ ID NO: 21), 5'-UGAUAAAGUGGAGAA-3' (SEQ ID NO: 22), 5'-CUGAUAAAGUGGAGAA-3' (SEQ ID NO: 23), 5'-ACUGAUAAAGUGGAGAA-3' (SEQ ID NO: 24), 5'-CACUGAUAAAGUGGAGAA-3' (SEQ ID NO: 25), 5'-UCACUGAUAAAGUGGAGAA-3' (SEQ ID NO: 26), 5'-UUCACUGAUAAAGUGGAGAA-3' (SEQ ID NO: 27), and 5'-CUUCACUGAUAAAGUGGAGAA-3' (SEQ ID NO: 10),
a second sequence selected from the group consisting of 5'-G-3', 5'-UUAGG-3', 5'-CUUAGGG-3', 5'-CUUAGUGG-3', 5'-CCUUAGGUGG-3' (SEQ ID NO: 342), 5'-CCGUUAGGUGG-3' (SEQ ID NO: 343), 5'-CCGCUUAGGGUGG-3' (SEQ ID NO: 344),
5'-CCGCUUUAGAGGUGG-3' (SEQ ID NO: 345),
5'-CCGCUUUUAGAAGGUGG-3' (SEQ ID NO: 346),
5'-CCGCUUCUUAGGAAGGUGG-3' (SEQ ID NO: 347),
5'-CCGCUUCAUUAGUGAAGGUGG-3' (SEQ ID NO: 348),
5'-CCGCUUCACUUAGGUGAAGGUGG-3' (SEQ ID NO: 349),
5'-CCGCUUCACUUAGAGUGAAGGUGG-3' (SEQ ID NO: 350),
5'-CCGCUUCACCUUAGGAGUGAAGGUGG-3' (SEQ ID NO: 351),
5'-CCGCUUCACCAUUAGUGAGUGAAGGUGG-3' (SEQ ID NO: 352),
5'-CCGCUUCACCAAUUAGUUGAGUGAAGGUGG-3' (SEQ ID NO: 353),
5'-CCGCUUCACCAAAUUAGCUUGAGUGAAGGUGG-3' (SEQ ID NO: 354),
5'-CCGCUUCACCAAAAUUAGACUUGAGUGAAGGUGG-3' (SEQ ID NO: 355),
5'-CCGCUUCACCAAAAGUUAGAACUUGAGUGAAGGUGG-3' (SEQ ID NO: 356),
5'-CCGCUUCACCAAAAGCUUAGGAACUUGAGUGAAGGUGG-3' (SEQ ID NO: 357),
5'-CCGCUUCACCAAAAGCUUUAGAGAACUUGAGUGAAGGUGG-3' (SEQ ID NO: 358),
5'-CCGCUUCACCAAAAGCUGUUAGUAGAACUUGAGUGAAGGUGG-3' (SEQ ID NO: 359),
5'-CCGCUUCACCAAAAGCUGUUAGUUAGAACUUGAGUGAAGGUGG-3' (SEQ ID NO: 360),
5'-CCGCUUCACCAAAAGCUGUUUAGAUUAGAACUUGAGUGAAGGUGG-3' (SEQ ID NO: 361),
5'-CCGCUUCACCAAAAGCUGUCUUAGGAUUAGAACUUGAGUGAAGGUGG-3' (SEQ ID NO: 362), and 
5'-
CCGCUUCACCAAAAGCUGUCCCUUAGGGGAUUAGAACUUGAGUGAAGGUGG-3'
(SEQ ID NO: 11),
a third sequence selected from the group consisting of 5'-GCUGCUUGCAUCAGCCUAAUGUCGAGAAGUGCUUUCUUCGGAAAGUAACCCUCGA-3' (SEQ ID NO: 434), 
5'-
GCUGCUUGCAUCAGCCUAAUGUCGAGAAGUGCUUUUUCGAAAGUAACCCUCGA-3'
(SEQ ID NO: 435),
 
5'-
GCUGCUUGCAUCAGCCUAAUGUCGAGAAGUGCUUUUCGAAGUAACCCUCGA-3' (SEQ
ID NO: 436),
 
5'-
GCUGCUUGCAUCAGCCUAAUGUCGAGAAGUGCUCUUCGGAGUAACCCUCGA-3'
(SEQ ID NO: 437),
 
5'-GCUGCUUGCAUCAGCCUAAUGUCGAGAAGUGCUUUCGAGUAACCCUCGA-
3' (SEQ ID NO: 438),
5'-GCUGCUUGCAUCAGCCUAAUGUCGAGAAGUGCUUCGGUAACCCUCGA-3' (SEQ ID NO: 439),
5'-GCUGCUUGCAUCAGCCUAAUGUCGAGAAGUGUUCGUAACCCUCGA-3' (SEQ ID NO: 440),
5'-GCUGCUUGCAUCAGCCUAAUGUCGAGAAGUUUCGAACCCUCGA-3' (SEQ ID NO: 441),
5'-GCUGCUUGCAUCAGCCUAAUGUCGAGAAGUUCGACCCUCGA-3' (SEQ ID NO: 442),
5'-GCUGCUUGCAUCAGCCUAAUGUCGAGAAGUUCGCCCUCGA-3' (SEQ ID NO: 443),
5'-GCUGCUUGCAUCAGCCUAAUGUCGAGAAUUCGCCCUCGA-3' (SEQ ID NO: 444),
5'-GCUGCUUGCAUCAGCCUAAUGUCGAGAAUUCGCCUCGA-3' (SEQ ID NO: 445),
5'-GCUGCUUGCAUCAGCCUAAUGUCGAGAUUCGCCUCGA-3' (SEQ ID NO: 446),
5'-GCUGCUUGCAUCAGCCUAAUGUCGAGUUCGCUCGA-3' (SEQ ID NO: 447), and 
5'-
GCUGCUUGCAUCAGCCUAAUGUCGAGAAGUGCUUUCUUCGGAAAGUAACCCUCGA-
3' (SEQ ID NO: 12),
 and
a fourth sequence selected from the group consisting of 5'-AACAAA-3', 5'-AACAAAU-3', 5'-AACAAAUU-3', 5'-AACAAAUUC-3', 5'-AACAAAUUCA-3' (SEQ ID NO: 67), 5'-AACAAAUUCAU-3' (SEQ ID NO: 68), 5'-AACAAAUUCAUU-3' (SEQ ID NO: 69), and 5'-AACAAAUUCAUUU-3' (SEQ ID NO: 13); and
an engineered crRNA repeat sequence portion in which the following sequences are linked to each other:
   a fifth sequence selected from the group consisting of 5'-GGA-3', 5'-AGGA-3', 5'-AAGGA-3', 5'-GAAGGA-3', 5'-UGAAGGA-3', 5'-AUGAAGGA-3', 5'-AAUGAAGGA-3', and 5'-GAAUGAAGGA-3' (SEQ ID NO: 15), and
   a sixth sequence represented by 5'-AUGCAAC-3',
   wherein the 3' end of the engineered crRNA repeat sequence portion is linked to the 5' end of the spacer.

Here, a sequence of the engineered tracrRNA may be different from 5'-CUUCACUGAUAAAGUGGAGAACCGCUUCACCAAAAGCUGUCCCUUAGGGGAUUAGA ACUUGAGUGAAGGUGGGCUGCUUGCAUCAGCCUAAUGUCGAGAAGUGCUUUCUUC GGAAAGUAACCCUCGAAACAAAUUCAUUU-3' (SEQ ID NO: 1), and/or the engineered crRNA repeat sequence portion may be different from 5'-GAAUGAAGGAAUGCAAC-3' (SEQ ID NO: 3).

As an example, a sequence of the engineered tracrRNA may be the same as SEQ ID NO: 1, and the engineered crRNA repeat sequence portion may be different from SEQ ID NO: 3. As another example, a sequence of the engineered tracrRNA may be different from SEQ ID NO: 1, and the engineered crRNA repeat sequence portion may be the same as the sequence of SEQ ID NO: 3. As yet another example, a sequence of the engineered tracrRNA may be different from SEQ ID NO: 1, and the engineered crRNA repeat sequence portion may be different from the sequence of SEQ ID NO: 3.

As an example, a sequence of the engineered tracrRNA may not comprise the first sequence and/or the second sequence.

Specifically, a sequence of the engineered tracrRNA may be selected from the group consisting of:
a sequence in which the second sequence, the third sequence, and the fourth sequence are linked to each other in a 5' to 3' direction,
a sequence in which the first sequence, the third sequence, and the fourth sequence are linked to each other in a 5' to 3' direction, and
a sequence in which the third sequence, and the fourth sequence are linked to each other in a 5' to 3' direction.

As an example, when the engineered tracrRNA comprises 5'-AACAAA-3', the engineered crRNA may comprise 5'-GGA-3'. As another example, when the engineered tracrRNA comprises 5'-AACAAAU-3', the engineered crRNA may comprise 5'-AGGA-3'. As yet another example, when the engineered tracrRNA comprises 5'-AACAAAUU-3', the engineered crRNA may comprise 5'-AAGGA-3'. As still yet another example, when the engineered tracrRNA comprises 5'-AACAAAUUC-3', the engineered crRNA may comprise 5'-GAAGGA-3'. As still yet another example, when the engineered tracrRNA comprises 5'-AACAAAUUCA-3' (SEQ ID NO: 67), the engineered crRNA may comprise 5'-UGAAGGA-3'. As still yet another example, when the engineered tracrRNA comprises 5'-AACAAAUUCAU-3' (SEQ ID NO: 68), the engineered crRNA may comprise 5'-AUGAAGGA-3'. As still yet another example, when the engineered tracrRNA comprises 5'-AACAAAUUCAUU-3' (SEQ ID NO: 69), the engineered crRNA may comprise 5'-AAUGAAGGA-3'. As still yet another example, when the engineered tracrRNA comprises 5'-AACAAAUUCAUUUU-3' (SEQ ID NO: 70), the engineered crRNA may comprise 5'-GAAUGAAGGA-3' (SEQ ID NO: 91).

### 3. CRISPR activation complex and CRISPR interference complex

### CRISPR activation complex and CRISPR interference complex - Overview

In the present disclosure, there are provided a CRISPR activation complex and a CRISPR interference complex. The CRISPR activation complex comprises a transcriptional activator Cas12f1 fusion protein and an engineered Cas12f1 guide RNA. The CRISPR interference complex comprises a transcriptional inhibitor Cas12f1 fusion protein and an engineered Cas12f1 guide RNA. Here, the transcriptional activator Cas12f1 fusion protein and the transcriptional inhibitor Cas12f1 fusion protein are as described in the section "5) Cas12f1 fusion protein. Here, the engineered Cas12f1 guide RNA is as described in the section "10) Engineered Cas12f1 guide RNA."

In an embodiment of the present disclosure, there is provided a CRISPR activation complex capable of increasing or promoting expression of a target gene, comprising a transcriptional activator Cas12f1 fusion protein and an engineered Cas12f1 guide RNA. Here, the transcriptional activator Cas12f1 fusion protein may be any one of those described in the section "5) Cas12f1 fusion protein." Here, the engineered Cas12f1 guide RNA may be any one of those described in the section "10) Engineered Cas12f1 guide RNA." The CRISPR activation complex may bind to a regulatory DNA site located near an enhancer or promoter of a target gene and facilitate, through protein-protein interaction, binding of general transcription machinery (RNA polymerase, common transcription factors, and the like) to the promoter, thereby promoting transcription of the gene. Alternatively, the CRISPR activation complex may bind to a regulatory DNA site located near an enhancer or promoter of a target gene so that it causes RNA polymerase to move from the promoter and proceed with synthesis along DNA, thereby promoting transcription of the gene.

In an embodiment of the present disclosure, there is provided a CRISPR interference complex capable of inhibiting or suppressing expression of a target gene, comprising a transcriptional inhibitor Cas12f1 fusion protein and an engineered Cas12f1 guide RNA. Here, the transcriptional inhibitor Cas12f1 fusion protein may be any one of those described in the section "5) Cas12f1 fusion protein." Here, the engineered Cas12f1 guide RNA may be any one of those described in the section "10) Engineered Cas12f1 guide RNA." The CRISPR interference complex may bind to an operator or silencer of a target gene to block attachment of RNA polymerase to the promoter, thereby inhibiting or suppressing transcription of the gene.

### Transcriptional activator Cas12f1 fusion protein

The transcriptional activator Cas12f1 fusion protein, which constitutes the CRISPR activation complex provided herein, has the same characteristics and structure as described in the section "5) Cas12f1 fusion protein."

### Transcriptional inhibitor Cas12f1 fusion protein

The transcriptional inhibitor Cas12f1 fusion protein, which constitutes the CRISPR interference complex provided herein, has the same characteristics and structure as described in the section "5) Cas12f1 fusion protein."

### Engineered Cas12f1 guide RNA

The engineered Cas12f1 guide RNA constituting the CRISPR activation complex or the CRISPR interference complex provided herein has the same characteristics and structure as described in the section "10) engineered Cas12f1 guide RNA."

### Examples of CRISPR activation complex

In an embodiment, the transcriptional activator Cas12f1 fusion protein may be one in which the dCas12f1 R490A protein and VP64 are sequentially linked to each other from the N-terminus to the C-terminus, the engineered Cas12f1 guide RNA may have an amino acid sequence selected from the group consisting of SEQ ID NOs: 211 to 253, SEQ ID NOs: 296 to 308, SEQ ID NOs: 311 to 323, SEQ ID NOs: 326 to 338, SEQ ID NOs: 488 to 541, and SEQ ID NOs: 545 to 551, and the transcriptional activator Cas12f1 fusion protein and the engineered Cas12f1 guide RNA may bind to each other to form a CRISPR activation complex.

### Examples of CRISPR interference complex

In an embodiment, the transcriptional inhibitor Cas12f1 fusion protein may be one in which KRAB and the dCas12f1 D510A protein are sequentially linked to each other from the N-terminus to the C-terminus, the engineered Cas12f1 guide RNA may have an amino acid sequence selected from the group consisting of SEQ ID NOs: 211 to 253, SEQ ID NOs: 296 to 308, SEQ ID NOs: 311 to 323, SEQ ID NOs: 326 to 338, SEQ ID NOs: 488 to 541, and SEQ ID NOs: 545 to 551, and the transcriptional inhibitor Cas12f1 fusion protein and the engineered Cas12f1 guide RNA may bind to each other to form a CRISPR interference complex.

In an embodiment, the transcriptional inhibitor Cas12f1 fusion protein may be one in which DNMT3 and the dCas12f1 D510A protein are sequentially linked to each other from the N-terminus to the C-terminus, the engineered Cas12f1 guide RNA may have an amino acid sequence selected from the group consisting of SEQ ID NOs: 211 to 253, SEQ ID NOs: 296 to 308, SEQ ID NOs: 311 to 323, SEQ ID NOs: 326 to 338, SEQ ID NOs: 488 to 541, and SEQ ID NOs: 545 to 551, and the transcriptional inhibitor Cas12f1 fusion protein and the engineered Cas12f1 guide RNA may bind to each other to form a CRISPR interference complex.

In an embodiment, the transcriptional inhibitor Cas12f1 fusion protein may be one in which DNMT3 and the dCas12f1 R490A protein are sequentially linked to each other from the N-terminus to the C-terminus, the engineered Cas12f1 guide RNA may have an amino acid sequence selected from the group consisting of SEQ ID NOs: 211 to 253, SEQ ID NOs: 296 to 308, SEQ ID NOs: 311 to 323, SEQ ID NOs: 326 to 338, SEQ ID NOs: 488 to 541, and SEQ ID NOs: 545 to 551, and the transcriptional inhibitor Cas12f1 fusion protein and the engineered Cas12f1 guide RNA may bind to each other to form a CRISPR interference complex.

In an embodiment, the transcriptional inhibitor Cas12f1 fusion protein may be one in which KRAB, MeCP2, and the dCas12f1 D510A protein are sequentially linked to each other from the N-terminus to the C-terminus, the engineered Cas12f1 guide RNA may have an amino acid sequence selected from the group consisting of SEQ ID NOs: 211 to 253, SEQ ID NOs: 296 to 308, SEQ ID NOs: 311 to 323, SEQ ID NOs: 326 to 338, SEQ ID NOs: 488 to 541, and SEQ ID NOs: 545 to 551, and the transcriptional inhibitor Cas12f1 fusion protein and the engineered Cas12f1 guide RNA may bind to each other to form a CRISPR interference complex.

In an embodiment, the transcriptional inhibitor Cas12f1 fusion protein may be one in which KRAB, MeCP2, and the dCas12f1 R490A protein are sequentially linked to each other from the N-terminus to the C-terminus, the engineered Cas12f1 guide RNA may have an amino acid sequence selected from the group consisting of SEQ ID NOs: 211 to 253, SEQ ID NOs: 296 to 308, SEQ ID NOs: 311 to 323, SEQ ID NOs: 326 to 338, SEQ ID NOs: 488 to 541, and SEQ ID NOs: 545 to 551, and the transcriptional inhibitor Cas12f1 fusion protein and the engineered Cas12f1 guide RNA may bind to each other to form a CRISPR interference complex.

In an embodiment, the transcriptional inhibitor Cas12f1 fusion protein may be one in which the dCas12f1 R490A protein and KRAB are sequentially linked to each other from the N-terminus to the C-terminus, the engineered Cas12f1 guide RNA may have an amino acid sequence selected from the group consisting of SEQ ID NOs: 211 to 253, SEQ ID NOs: 296 to 308, SEQ ID NOs: 311 to 323, SEQ ID NOs: 326 to 338, SEQ ID NOs: 488 to 541, and SEQ ID NOs: 545 to 551, and the transcriptional inhibitor Cas12f1 fusion protein and the engineered Cas12f1 guide RNA may bind to each other to form a CRISPR interference complex.

In an embodiment, the transcriptional inhibitor Cas12f1 fusion protein may be one in which the dCas12f1 D510A protein and KRAB are sequentially linked to each other from the N-terminus to the C-terminus, the engineered Cas12f1 guide RNA may have an amino acid sequence selected from the group consisting of SEQ ID NOs: 211 to 253, SEQ ID NOs: 296 to 308, SEQ ID NOs: 311 to 323, SEQ ID NOs: 326 to 338, SEQ ID NOs: 488 to 541, and SEQ ID NOs: 545 to 551, and the transcriptional inhibitor Cas12f1 fusion protein and the engineered Cas12f1 guide RNA may bind to each other to form a CRISPR interference complex.

In an embodiment, the transcriptional inhibitor Cas12f1 fusion protein may be one in which the dCas12f1 D510A protein, KRAB, and MeCP2 are sequentially linked to each other from the N-terminus to the C-terminus, the engineered Cas12f1 guide RNA may have an amino acid sequence selected from the group consisting of SEQ ID NOs: 211 to 253, SEQ ID NOs: 296 to 308, SEQ ID NOs: 311 to 323, SEQ ID NOs: 326 to 338, SEQ ID NOs: 488 to 541, and SEQ ID NOs: 545 to 551, and the transcriptional inhibitor Cas12f1 fusion protein and the engineered Cas12f1 guide RNA may bind to each other to form a CRISPR interference complex.

In an embodiment, the transcriptional inhibitor Cas12f1 fusion protein may be one in which the dCas12f1 R490A protein, KRAB, and MeCP2 are sequentially linked to each other from the N-terminus to the C-terminus, the engineered Cas12f1 guide RNA may have an amino acid sequence selected from the group consisting of SEQ ID NOs: 211 to 253, SEQ ID NOs: 296 to 308, SEQ ID NOs: 311 to 323, SEQ ID NOs: 326 to 338, SEQ ID NOs: 488 to 541, and SEQ ID NOs: 545 to 551, and the transcriptional inhibitor Cas12f1 fusion protein and the engineered Cas12f1 guide RNA may bind to each other to form a CRISPR interference complex.

In an embodiment, the transcriptional inhibitor Cas12f1 fusion protein may be one in which the dCas12f1 R490A protein, KRAB, and KRAB are sequentially linked to each other from the N-terminus to the C-terminus, the engineered Cas12f1 guide RNA may have an amino acid sequence selected from the group consisting of SEQ ID NOs: 211 to 253, SEQ ID NOs: 296 to 308, SEQ ID NOs: 311 to 323, SEQ ID NOs: 326 to 338, SEQ ID NOs: 488 to 541, and SEQ ID NOs: 545 to 551, and the transcriptional inhibitor Cas12f1 fusion protein and the engineered Cas12f1 guide RNA may bind to each other to form a CRISPR interference complex.

In an embodiment, the transcriptional inhibitor Cas12f1 fusion protein may be one in which the dCas12f1 D510A protein, KRAB, and KRAB are sequentially linked to each other from the N-terminus to the C-terminus, the engineered Cas12f1 guide RNA may have an amino acid sequence selected from the group consisting of SEQ ID NOs: 211 to 253, SEQ ID NOs: 296 to 308, SEQ ID NOs: 311 to 323, SEQ ID NOs: 326 to 338, SEQ ID NOs: 488 to 541, and SEQ ID NOs: 545 to 551, and the transcriptional inhibitor Cas12f1 fusion protein and the engineered Cas12f1 guide RNA may bind to each other to form a CRISPR interference complex.

In an embodiment, the transcriptional inhibitor Cas12f1 fusion protein may be one in which the dCas12f1 D510A protein, KRAB, KRAB, and MeCP2 are sequentially linked to each other from the N-terminus to the C-terminus, the engineered Cas12f1 guide RNA may have an amino acid sequence selected from the group consisting of SEQ ID NOs: 211 to 253, SEQ ID NOs: 296 to 308, SEQ ID NOs: 311 to 323, SEQ ID NOs: 326 to 338, SEQ ID NOs: 488 to 541, and SEQ ID NOs: 545 to 551, and the transcriptional inhibitor Cas12f1 fusion protein and the engineered Cas12f1 guide RNA may bind to each other to form a CRISPR interference complex.

In an embodiment, the transcriptional inhibitor Cas12f1 fusion protein may be one in which the dCas12f1 R490A protein and HDAC3 are sequentially linked to each other from the N-terminus to the C-terminus, the engineered Cas12f1 guide RNA may have an amino acid sequence selected from the group consisting of SEQ ID NOs: 211 to 253, SEQ ID NOs: 296 to 308, SEQ ID NOs: 311 to 323, SEQ ID NOs: 326 to 338, SEQ ID NOs: 488 to 541, and SEQ ID NOs: 545 to 551, and the transcriptional inhibitor Cas12f1 fusion protein and the engineered Cas12f1 guide RNA may bind to each other to form a CRISPR interference complex.

In an embodiment, the transcriptional inhibitor Cas12f1 fusion protein may be one in which KRAB, the dCas12f1 D510A protein, and MeCP2 are sequentially linked to each other from the N-terminus to the C-terminus, the engineered Cas12f1 guide RNA may have an amino acid sequence selected from the group consisting of SEQ ID NOs: 211 to 253, SEQ ID NOs: 296 to 308, SEQ ID NOs: 311 to 323, SEQ ID NOs: 326 to 338, SEQ ID NOs: 488 to 541, and SEQ ID NOs: 545 to 551, and the transcriptional inhibitor Cas12f1 fusion protein and the engineered Cas12f1 guide RNA may bind to each other to form a CRISPR interference complex.

In an embodiment, the transcriptional inhibitor Cas12f1 fusion protein may be one in which KRAB, MeCP2, the dCas12f1 R490A protein, and KRAB are sequentially linked to each other from the N-terminus to the C-terminus, the engineered Cas12f1 guide RNA may have an amino acid sequence selected from the group consisting of SEQ ID NOs: 211 to 253, SEQ ID NOs: 296 to 308, SEQ ID NOs: 311 to 323, SEQ ID NOs: 326 to 338, SEQ ID NOs: 488 to 541, and SEQ ID NOs: 545 to 551, and the transcriptional inhibitor Cas12f1 fusion protein and the engineered Cas12f1 guide RNA may bind to each other to form a CRISPR interference complex.

In an embodiment, the transcriptional inhibitor Cas12f1 fusion protein may be one in which MeCP2, the dCas12f1 R490A protein, and KRAB are sequentially linked to each other from the N-terminus to the C-terminus, the engineered Cas12f1 guide RNA may have an amino acid sequence selected from the group consisting of SEQ ID NOs: 211 to 253, SEQ ID NOs: 296 to 308, SEQ ID NOs: 311 to 323, SEQ ID NOs: 326 to 338, SEQ ID NOs: 488 to 541, and SEQ ID NOs: 545 to 551, and the transcriptional inhibitor Cas12f1 fusion protein and the engineered Cas12f1 guide RNA may bind to each other to form a CRISPR interference complex.

In an embodiment, the transcriptional inhibitor Cas12f1 fusion protein may be one in which MeCP2, the dCas12f1 D510A protein, and KRAB are sequentially linked to each other from the N-terminus to the C-terminus, the engineered Cas12f1 guide RNA may have an amino acid sequence selected from the group consisting of SEQ ID NOs: 211 to 253, SEQ ID NOs: 296 to 308, SEQ ID NOs: 311 to 323, SEQ ID NOs: 326 to 338, SEQ ID NOs: 488 to 541, and SEQ ID NOs: 545 to 551, and the transcriptional inhibitor Cas12f1 fusion protein and the engineered Cas12f1 guide RNA may bind to each other to form a CRISPR interference complex.

### 4. Vector for expressing respective components of CRISPR expression regulatory system

### Overview of vector

In the present disclosure, there is provided a vector for expression components of a CRISPR expression regulatory system. The vector is constructed to express a Cas12f1 fusion protein, and/or an engineered Cas12f1 guide RNA. A sequence of the vector may comprise a nucleic acid sequence encoding one of the components of the CRISPR expression regulatory system or may comprise a nucleic acid sequence encoding two or more of the components thereof. A sequence of the vector comprises a nucleic acid sequence encoding the Cas12f1 fusion protein and/or a nucleic acid sequence encoding the engineered Cas12f1 guide RNA. A sequence of the vector comprises one or more promoter sequences. The promoter is operatively linked with a nucleic acid sequence encoding the Cas12f1 fusion protein and/or a nucleic acid sequence encoding the engineered Cas12f1 guide RNA, so that transcription of the nucleic acid sequence(s) in a cell can be promoted. The Cas12f1 fusion protein has the same characteristics and structure as the Cas12f1 fusion protein as described in the section "2. Expression regulatory protein - Cas12f1 fusion protein." The engineered Cas12f1 guide RNA has the same characteristics and structure as the engineered Cas12f1 guide RNA as described in the section "3. Engineered Cas12f1 guide RNA."

A sequence of the vector may comprise a nucleic acid sequence encoding the Cas12f1 protein and/or a nucleic acid sequence encoding the engineered Cas12f1 guide RNA. In an embodiment, a sequence of the vector may comprise a first sequence comprising a nucleic acid sequence encoding the Cas12f1 protein and a second sequence comprising a nucleic acid sequence encoding the engineered Cas12f1 guide RNA. The sequence of the vector comprises a promoter sequence for expressing a nucleic acid sequence encoding the Cas12f1 fusion protein in a cell, and a promoter sequence for expressing a nucleic acid sequence encoding the engineered Cas12f1 guide RNA in a cell, wherein each of the promoters is operably linked to each target to be expressed. In an embodiment, a sequence of the vector may comprise a first promoter sequence operably linked to the first sequence, and a second promoter sequence operably linked to the second sequence.

A sequence of the vector may comprise a nucleic acid sequence encoding the Cas12f1 protein and a nucleic acid sequence encoding two or more engineered Cas12f1 guide RNAs that are different from each other. In an embodiment, a sequence of the vector may comprise a first sequence comprising a nucleic acid sequence encoding the Cas12f1 fusion protein, a second sequence comprising a nucleic acid sequence encoding a first engineered Cas12f1 guide RNA, and a third sequence comprising a nucleic acid sequence encoding a second engineered Cas12f1 guide RNA. Furthermore, the sequence of the vector may comprise a first promoter sequence operably linked to the first sequence, a second promoter sequence operably linked to the second sequence, and a third promoter sequence operably linked to the third sequence.

### Target to be expressed - Cas12f1 fusion protein

The vector may be constructed to express a Cas12f1 fusion protein. Here, the Cas12f1 fusion protein has the same structure and characteristics as described in the section "2. Expression regulatory protein - Cas12f1 fusion protein."

In an embodiment, the vector may be constructed to express a Cas12f1 fusion protein. Here, the Cas12f1 fusion protein may be a transcriptional activator Cas12f1 fusion protein. Alternatively, the Cas12f1 fusion protein may be a transcriptional inhibitor Cas12f1 fusion protein. In an embodiment, the vector may be constructed to express a transcriptional activator Cas12f1 fusion protein for promoting expression of a target gene. In an embodiment, the vector may be constructed to express a transcriptional inhibitor Cas12f1 fusion protein for inhibiting or suppressing expression of a target gene.

### Target to be expressed - Engineered Cas12f1 guide RNA

The vector may be constructed to express an engineered Cas12f1 guide RNA. The engineered Cas12f1 guide RNA has the same characteristics and structure as the engineered Cas12f1 guide RNA as described in the section "3. Engineered Cas12f1 guide RNA." The vector may be constructed to express two or more engineered Cas12f1 guide RNAs that are different from each other.

### Target to be expressed - Additional component

The vector may be constructed to express an additional component such as an NLS and a tag protein in addition to the above-described targets to be expressed. In an embodiment, the additional component may be expressed independently of the Cas12f1 fusion protein and/or the engineered Cas12f1 guide RNA. In another embodiment, the additional component may be expressed in conjunction with the Cas12f1 fusion protein and/or the engineered Cas12f1 guide RNA. Here, the additional component may be a component that is generally expressed when it is intended to express a CRISPR expression regulatory system. In this regard, reference may be made to the prior art. For example, the additional component may be, but is not limited to, one of the tags described in the paragraph for tag in the section of defining terms. For example, the additional component may be, but is not limited to, a herbicide resistance gene such as glyphosate, glufosinate ammonium or phosphinothricin, or an antibiotic resistance gene such as ampicillin, kanamycin, G418, bleomycin, hygromycin, or chloramphenicol.

### Construction of vector - Sequence for expressing Cas12f1 fusion protein

A sequence of the vector may comprise a nucleic acid sequence encoding Cas12f1 fusion protein. Here, the Cas12f1 fusion protein has the same structure and characteristics as described in the section "2. Expression regulatory protein - Cas12f1 fusion protein."

In an embodiment, a sequence of the vector may comprise a sequence encoding a Cas12f1 fusion protein. Here, the Cas12f1 fusion protein may be a transcriptional activator Cas12f1 fusion protein. Alternatively, the Cas12f1 fusion protein may be a transcriptional inhibitor Cas12f1 fusion protein. In an embodiment, a sequence of the vector may comprise a human codon-optimized nucleic acid sequence encoding a Cas12f1 fusion protein. In an embodiment, a sequence of the vector may comprise a sequence encoding a transcriptional activator Cas12f1 fusion protein for promoting expression of a target gene. In an embodiment, a sequence of the vector may comprise a sequence encoding a transcriptional inhibitor Cas12f1 fusion protein for inhibiting or suppressing expression of a target gene.

### Construction of vector - Sequence for expressing engineered Cas12f1 guide RNA

In an embodiment, a sequence of the vector may comprise a sequence encoding an engineered Cas12f1 guide RNA. For example, a sequence of the vector may comprise a sequence selected from the group consisting of SEQ ID NOs: 211 to 253, SEQ ID NOs: 296 to 308, SEQ ID NOs: 311 to 323, SEQ ID NOs: 326 to 338, SEQ ID NOs: 488 to 541, and SEQ ID NOs: 545 to 551.

In an embodiment, a sequence of the vector may comprise a sequence encoding two or more engineered Cas12f1 guide RNAs that are different from each other. For example, a sequence of the vector may comprise a sequence encoding a first engineered Cas12f1 guide RNA and a sequence encoding a second engineered Cas12f1 guide RNA, each of which is selected from the group consisting of SEQ ID NOs: 211 to 253, SEQ ID NOs: 296 to 308, SEQ ID NOs: 311 to 323, SEQ ID NOs: 326 to 338, SEQ ID NOs: 488 to 541, and SEQ ID NOs: 545 to 551.

### Construction of vector - Promoter sequence

A sequence of the vector may comprise a promoter sequence operably linked to a sequence encoding each component. To express a target to be expressed by the vector in a cell, a promoter sequence must be operatively linked to a sequence encoding each component so that an RNA transcription factor can be activated in the cell. The promoter sequence may be designed differently depending on the corresponding RNA transcription factor or expression environment and is not limited to any particular embodiments as long as it may properly express the components of the CRISPR/Cas system in a cell. The promoter sequence may be a promoter that promotes transcription of an RNA polymerase (for example, RNA Pol I, Pol II, or Pol III). For example, the promoter may be, but is not limited to, one selected from: an SV40 early promoter, a mouse mammary tumor virus long terminal repeat (LTR) promoter, an adenovirus major late promoter (Ad MLP), a herpes simplex virus (HSV) promoter, a cytomegalovirus (CMV) promoter such as a CMV immediate early promoter region (CMVIE), a rous sarcoma virus (RSV) promoter, a human U6 small nuclear promoter (U6) (Miyagishi et al., Nature Biotechnology 20, 497 - 500 (2002)), an enhanced U6 promoter (e.g., Xia et al., Nucleic Acids Res. 2003 Sep 1:31(17)), a human H1 promoter (H1), and a 7SK promoter.

In an embodiment, a sequence of the vector may comprise a sequence encoding a Cas12f1 fusion protein and a promoter sequence. Here, the promoter sequence is operably linked to the sequence encoding a Cas12f1 fusion protein. In an embodiment, a sequence of the vector may comprise a sequence encoding an engineered Cas12f1 guide RNA and a promoter sequence. Here, the promoter sequence may be operably linked to the sequence encoding an engineered Cas12f1 guide RNA. In an embodiment, a sequence of the vector may comprise a sequence encoding a Cas12f1 fusion protein, a sequence encoding an engineered Cas12f1 guide RNA, and a promoter sequence. Here, the promoter sequence is operatively linked to the sequence encoding a Cas12f1 fusion protein and the sequence encoding an engineered Cas12f1 guide RNA, wherein a transcription factor activated by the promoter sequence causes expression of the Cas12f1 fusion protein and the engineered Cas12f1 guide RNA.

### Construction of vector - Possible to comprise two or more promoter sequences

In an embodiment, a sequence of the vector may comprise a first promoter sequence, a first sequence encoding a Cas12f1 fusion protein, a second promoter sequence, and a second sequence encoding an engineered Cas12f1 guide RNA. Here, the first promoter sequence is operably linked to the first sequence and the second promoter sequence is operatively linked to the second sequence, wherein transcription of the first sequence is induced by the first promoter sequence and transcription of the second sequence is induced by the second promoter sequence. Here, the first promoter and the second promoter may be the same type of promoters. Here, the first promoter and the second promoter may be different types of promoters.

In an embodiment, a sequence of the vector may comprise a first promoter sequence, a first sequence encoding a Cas12f1 fusion protein, a second promoter sequence, a second sequence encoding a first engineered Cas12f1 guide RNA, a third promoter sequence, and a third sequence encoding a second engineered Cas12f1 guide RNA. Here, the first promoter sequence is operatively linked to the first sequence, the second promoter sequence is operably linked to the second sequence, and the third promoter sequence is operably linked to the third sequence, wherein transcription of the first sequence is induced by the first promoter sequence, transcription of the second sequence is induced by the second promoter sequence, and transcription of the third sequence is induced by the third promoter sequence. Here, the second promoter and the third promoter may be the same type of promoters. Specifically, the second promoter sequence and the third promoter sequence may be, but are not limited to, a U6 promoter sequence. Here, the second promoter and the third promoter may be different types of promoters. Specifically, the second promoter may be a U6 promoter sequence, and the third promoter may be a H1 promoter sequence, but these promoters are not limited thereto.

### Construction of vector - Termination signal

The vector may comprise a termination signal operably linked to the promoter sequence. In a case where a sequence of the vector comprises the promoter sequence, transcription of a sequence operably linked to the promoter is induced by an RNA transcription factor, wherein a sequence, which induces termination of transcription of the RNA transcription factor is referred to as a termination signal. The termination signal may vary depending on the type of promoter sequence. For example, when the promoter is a U6 or H1 promoter, the promoter recognizes a thymidine repeat sequence (e.g., a TTTTTT (T6) sequence) as a termination signal.

In an embodiment, when a sequence of the vector sequence comprises a U6 promoter sequence, a thymidine repeat sequence operably linked to the U6 promoter sequence may serve as a termination signal. In an embodiment, the thymidine repeat sequence may be a sequence in which five or more thymidines are consecutively linked. In an embodiment, when a sequence of the vector comprises a H1 promoter sequence, a thymidine repeat sequence operably linked to the H1 promoter sequence may serve as a termination signal. In an embodiment, the thymidine repeat sequence may be a sequence in which five or more thymidines are consecutively linked.

### Construction of vector - Other components

A sequence of the vector may comprise a component necessary depending on the purpose in addition to the above components.

In an embodiment, a sequence of the vector may comprise a sequence of a regulatory/control element, and/or a sequence of an additional component. In an embodiment, the additional component may be added for the purpose of distinguishing transfected cells from non-transfected cells. Here, a sequence of the regulatory/control element and the additional component may include, but are not limited to, a promoter, an enhancer, an intron, a polyadenylation signal, a Kozak consensus sequence, an internal ribosome entry site (IRES), a splice acceptor, a 2A sequence, and/or a replication origin. Here, the replication origin may be, but is not limited to, an f1 origin of replication, an SV40 origin of replication, a pMB1 origin of replication, an adeno origin of replication, an AAV origin of replication, and/or a BBV origin of replication.

### Type of vector - Viral vector

The vector may be a viral vector.

In an embodiment, the viral vector may be at least one selected from the group consisting of a retrovirus, a lentivirus, an adenovirus, an adeno-associated virus, a vaccinia virus, a poxvirus, and a herpes simplex virus. In an embodiment, the viral vector may be an adeno-associated virus.

### Vector type - Non-viral vector

The vector may be a non-viral vector. In an embodiment, the non-viral vector may be at least one selected from the group consisting of a plasmid, a phage, naked DNA, a DNA complex, and mRNA. In an embodiment, the plasmid may be selected from the group consisting of pcDNA series, pS456, pG1806, pACYC177, ColE1, pKT230, pME290, pBR322, pUC8/9, pUC6, pBD9, pHC79, pIJ61, pLAFR1, pHV14, pGEX series, pET series, and pUC19. In an embodiment, the phage may be selected from the group consisting of λgt4λB, λ-Charon, λΔz1, and M13. In an embodiment, the vector may be a PCR amplicon.

### Form of vector - Circular or linear vector

The vector may have a circular or linear form. When the vector is a linear vector, RNA transcription is terminated at the 3' end thereof even if a sequence of the linear vector does not separately comprise a termination signal. In comparison, when the vector is a circular vector, RNA transcription is not terminated unless a sequence of the circular vector separately comprises a termination signal. Therefore, when the vector is used in a form of a circular vector, a termination signal corresponding to a transcription factor related to each promoter sequence has to be included in order for the vector to express an intended target.

In an embodiment, the vector may be a linear vector. In an embodiment, the vector may be a linear amplicon. In an embodiment, the vector may be a linear amplicon comprising: a sequence selected from the group consisting of SEQ ID NOs: 211 to 253, SEQ ID NOs: 296 to 308, SEQ ID NOs: 311 to 323, SEQ ID NOs: 326 to 338, SEQ ID NOs: 488 to 541, and SEQ ID NOs: 545 to 551; and a sequence encoding a transcriptional activator Cas12f1 fusion protein. In an embodiment, the vector may be a linear amplicon comprising: a sequence selected from the group consisting of SEQ ID NOs: 211 to 253, SEQ ID NOs: 296 to 308, SEQ ID NOs: 311 to 323, SEQ ID NOs: 326 to 338, SEQ ID NOs: 488 to 541, and SEQ ID NOs: 545 to 551; and a sequence encoding a transcriptional inhibitor Cas12f1 fusion protein.

In an embodiment, the vector may be a circular vector. In an embodiment, the vector may be a circular vector comprising: a sequence selected from the group consisting of SEQ ID NOs: 211 to 253, SEQ ID NOs: 296 to 308, SEQ ID NOs: 311 to 323, SEQ ID NOs: 326 to 338, SEQ ID NOs: 488 to 541, and SEQ ID NOs: 545 to 551; and a sequence encoding a transcriptional activator Cas12f1 fusion protein. In an embodiment, the vector may be a circular vector comprising: a sequence selected from the group consisting of SEQ ID NOs: 211 to 253, SEQ ID NOs: 296 to 308, SEQ ID NOs: 311 to 323, SEQ ID NOs: 326 to 338, SEQ ID NOs: 488 to 541, and SEQ ID NOs: 545 to 551; and a sequence encoding a transcriptional inhibitor Cas12f1 fusion protein.

### Vector - Example of sequence

In an embodiment, a sequence of the vector may comprise: a sequence selected from the group consisting of SEQ ID NOs: 211 to 253, SEQ ID NOs: 296 to 308, SEQ ID NOs: 311 to 323, SEQ ID NOs: 326 to 338, SEQ ID NOs: 488 to 541, and SEQ ID NOs: 545 to 551; and a sequence encoding a transcriptional activator Cas12f1 fusion protein.

In an embodiment, a sequence of the vector may comprise: a sequence selected from the group consisting of SEQ ID NOs: 211 to 253, SEQ ID NOs: 296 to 308, SEQ ID NOs: 311 to 323, SEQ ID NOs: 326 to 338, SEQ ID NOs: 488 to 541, and SEQ ID NOs: 545 to 551; and a sequence encoding a transcriptional inhibitor Cas12f1 fusion protein.

### 5. Composition for regulating gene expression comprising respective components of CRISPR expression regulatory system

In the present disclosure, there is provided a gene expression regulatory composition, comprising respective components of a CRISPR expression regulatory system. In an embodiment of the present disclosure, there is provided a gene expression regulatory composition, comprising: a Cas12f1 fusion protein or a nucleic acid encoding the Cas12f1 fusion protein; and an engineered Cas12f1 guide RNA or a nucleic acid encoding the engineered Cas12f1 guide RNA. Here, the Cas12f1 fusion protein may be as described in the section "2. Expression regulatory protein - Cas12f1 fusion protein." Here, the engineered Cas12f1 guide RNA may be as described in the section "3. Engineered Cas12f1 guide RNA."

The composition for regulating gene expression may further comprise an appropriate material necessary for regulating gene expression, in addition to the respective components of the CRISPR expression regulatory system.

### 6. Chemical modification of nucleic acid

In the present disclosure, there is provided a component that comprises or consists of a nucleic acid such as an engineered crRNA or a nucleic acid encoding the engineered crRNA, an engineered Cas12f1 guide RNA or a nucleic acid encoding the engineered Cas12f1 guide RNA, and/or a vector for expressing components of a CRISPR expression regulatory system. Here, the "nucleic acid" in the component may be naturally occurring DNA or RNA, or a modified nucleic acid in which a part of or all of a constituent nucleic acid is chemically modified. In an embodiment, the constituent nucleic acid may be naturally occurring DNA and/or RNA. In an embodiment, the constituent nucleic acid may be one in which one or more nucleotides are chemically modified. Here, the chemical modification includes any of modifications of a nucleic acid known to those of ordinary skill in the art. Specifically, the chemical modification may include any of modifications of a nucleic acid as described in WO 2019/089820 A1, but is not limited thereto.

### 7. Method of regulating gene expression using CRISPR expression regulatory system

### Overview of method of regulating gene expression

In the present disclosure, there is provided a method of regulating expression of a target gene in a target cell by using a CRISPR expression regulatory system. The target gene contains a target sequence. The method of regulating gene expression comprises delivering an engineered Cas12f1 guide RNA and a Cas12f1 fusion protein, or nucleic acids, each of which encodes each of them, into a target cell including a target gene. As a result, a CRISPR activation complex or a CRISPR interference complex is introduced into the target cell, or formation of a CRISPR activation complex or a CRISPR interference complex is induced, so that expression of the target gene is regulated by the CRISPR activation complex or the CRISPR interference complex. The engineered Cas12f1 guide RNA has the same characteristics and structure as described in the section "3. Engineered Cas12f1 guide RNA." The Cas12f1 fusion protein has the same characteristics and structure as described in the section "2. Expression regulatory protein - Cas12f1 fusion protein." The CRISPR activation complex and the CRISPR activation complex have the same characteristics and structures as described in the section "4. CRISPR activation complex and CRISPR interference complex.

In an embodiment, to promote expression of a target gene, the method of regulating gene expression may comprise delivering, into a target cell, a transcriptional activator Cas12f1 fusion protein or a nucleic acid encoding the transcriptional activator Cas12f1 fusion protein, and an engineered Cas12f1 guide RNA or a nucleic acid encoding the engineered Cas12f1 guide RNA.

Here, the transcriptional activator Cas12f1 fusion protein comprises a dCas12f1 protein and a transcriptional activator protein.

Here, the dCas12f1 protein has the same characteristics and structure as any one of those described in the section "1) Modified Cas12f1 protein." As an example, the dCas12f1 protein may be represented by a sequence selected from the group consisting of SEQ ID NOs: 261, 262, 264, 265, 266, 267, 268, 269, and 271. The transcriptional activator protein has the same characteristics and structure as any one of those described in the section "2) Expression regulatory domain. As an example, the transcriptional activator protein may be VP64.

Here, the engineered Cas12f1 guide RNA may comprise an engineered scaffold region, a spacer, and a U-rich tail.

Here, the engineered scaffold region has the same characteristics and structure as any one of those described in the section "Engineered scaffold region". As an example, the engineered scaffold region may be represented by a sequence selected from the group consisting of SEQ ID NOs: 168 to 187. As another example, the engineered scaffold region may be represented by a sequence selected from the group consisting of SEQ ID NOs: 188 to 199. As yet another example, the engineered scaffold region may be represented by a sequence selected from the group consisting of SEQ ID NOs: 200 to 206. As still yet another example, the engineered scaffold region may be represented by a sequence selected from the group consisting of SEQ ID NOs: 207 to 210.

Here, the spacer sequence may complementarily bind to a target sequence present in a target gene included in the target cell.

Here, a sequence of the U-rich tail is represented by (UₐN)_{b}U_{c}, wherein N is one of adenosine (A), uridine (U), cytidine (C), and guanosine (G), and a, b, c are each an integer, with a being between 1 to 5 inclusive, and b being 0 or greater. As an example, a sequence of the U-rich tail may be 5'-UUUUAUUUU-3'. As an example, a sequence of the U-rich tail may be 5'-UUUUGUUUU-3'.

In an embodiment, to inhibit or suppress expression of a target gene, the method of regulating gene expression may comprise delivering, into a target cell, a transcriptional inhibitor Cas12f1 fusion protein or a nucleic acid encoding the transcriptional inhibitor Cas12f1 fusion protein, and an engineered Cas12f1 guide RNA or a nucleic acid encoding the engineered Cas12f1 guide RNA.

Here, the transcriptional inhibitor Cas12f1 fusion protein comprises a dCas12f1 protein and a transcriptional inhibitor protein.

Here, the dCas12f1 protein has the same characteristics and structure as any one of those described in the section "1) Modified Cas12f1 protein." As an example, the dCas12f1 protein may be represented by a sequence selected from the group consisting of SEQ ID NOs: 261, 262, 264, 265, 266, 267, 268, 269, and 271. The transcriptional inhibitor protein has the same characteristics and structure as any one of those described in the section "2) Expression regulatory domain. As an example, the transcriptional inhibitor protein may be KRAB, MeCP2, DNMT3, and/or HDAC3.

Here, the engineered Cas12f1 guide RNA may comprise an engineered scaffold region, a spacer, and a U-rich tail.

Here, the engineered scaffold region has the same characteristics and structure as any one of those described in the section "Engineered scaffold region." As an example, the engineered scaffold region may be represented by a sequence selected from the group consisting of SEQ ID NOs: 168 to 187. As another example, the engineered scaffold region may be represented by a sequence selected from the group consisting of SEQ ID NOs: 188 to 199. As yet another example, the engineered scaffold region may be represented by a sequence selected from the group consisting of SEQ ID NOs: 200 to 206. As still yet another example, the engineered scaffold region may be represented by a sequence selected from the group consisting of SEQ ID NOs: 207 to 210.

Here, the spacer sequence may complementarily bind to a target sequence present in a target gene included in the target cell.

Here, a sequence of the U-rich tail is represented by (UₐN)_{b}U_{c}, wherein N is one of adenosine (A), uridine (U), cytidine (C), and guanosine (G), and a, b, c are each an integer, with a being between 1 to 5 inclusive, and b being 0 or greater. As an example, a sequence of the U-rich tail may be 5'-UUUUAUUUU-3'. As an example, a sequence of the U-rich tail may be 5'-UUUUGUUUU-3'.

### Target cell

In an embodiment, the target cell may be a prokaryotic cell. In an embodiment, the target cell may be a eukaryotic cell. Specifically, the eukaryotic cell may be, but is not limited to, a plant cell, an animal cell, and/or a human cell.

### Determination of target sequence

A target gene whose expression is to be regulated by a CRISPR activation complex or a CRISPR interference complex may be determined in consideration of the purpose of regulating gene expression, environment of a target cell, a PAM sequence recognized by a Cas12f1 fusion protein, and/or other variables. Here, a method of determining the target sequence is not particularly limited as long as it is capable of determining a target sequence of an appropriate length present in a target gene, and a technique known in the art may be used therefor.

### Determination of spacer sequence depending on target sequence

Once the target sequence is determined, a spacer sequence corresponding thereto is designed. The spacer sequence is designed as a sequence capable of complementarily binding to the target sequence. In an embodiment, the spacer sequence may be designed as a sequence capable of complementarily binding to the target gene. In an embodiment, the spacer sequence may be designed to be capable of complementarily binding to the target nucleic acid. In an embodiment, the spacer sequence may be designed as a sequence complementary to a target sequence included in a target strand sequence of the target nucleic acid. In an embodiment, the spacer sequence is designed as an RNA sequence corresponding to a DNA sequence of a protospacer included in a non-target strand sequence of the target nucleic acid. Specifically, the spacer sequence is designed to have the same nucleotide sequence as the protospacer sequence, except that every thymidine included in the nucleotide sequence is substituted with a uridine.

### Complementarity between target sequence and spacer sequence

In an embodiment, the spacer sequence may be complementary to the target sequence by 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or 100%. In an embodiment, the spacer sequence may be a sequence complementary to the target sequence within a numerical range selected from the immediately preceding sentence. As an example, the spacer sequence may be a sequence that is 60% to 90% complementary to the target sequence. As another example, the spacer sequence may be a sequence that is 90% to 100% complementary to the target sequence.

### Number of mismatches between target sequence and spacer sequence

In an embodiment, the spacer sequence may be a sequence that is complementary to the target sequence and has 0, 1, 2, 3, 4, 5, 6, 7, 8, 9, or 10 mismatches therewith. In an embodiment, the spacer sequence may have mismatches within a numerical range selected from the immediately preceding sentence. As an example, the spacer sequence may have 1 to 5 mismatches with the target sequence. As another example, the spacer sequence may have 6 to 10 mismatches with the target sequence.

### Use of CRISPR activation complex or CRISPR interference complex

The method of regulating gene expression provided herein utilizes the fact that a CRISPR activation complex or a CRISPR interference complex has activity of regulating transcription of a gene in a target-specific manner. The CRISPR activation complex and the CRISPR activation complex have the same characteristics and structures as the CRISPR activation complex and the CRISPR interference complex as described in the section "4. CRISPR activation complex and CRISPR interference complex."

### Delivery of respective components of CRISPR activation complex or CRISPR interference complex into cell

The method of regulating gene expression provided herein comprises bringing a CRISPR activation complex or a CRISPR interference complex in contact with a target gene in a target cell. Thus, to induce the CRISPR activation complex or the CRISPR interference complex to come in contact with the target gene, the method of regulating gene expression comprises delivering respective components of the CRISPR activation complex or the CRISPR interference complex into a target cell. In an embodiment, the method of regulating gene expression may comprise delivering, into a target cell, an engineered Cas12f1 guide RNA or a nucleic acid encoding the engineered Cas12f1 guide RNA and a Cas12f1 fusion protein (a transcriptional activator Cas12f1 fusion protein or a transcriptional inhibitor Cas12f1 fusion protein) or a nucleic acid encoding the Cas12f1 fusion protein. In an embodiment, the method of regulating gene expression may comprise delivering, into a target cell, an engineered Cas12f1 guide RNA and a Cas12f1 fusion protein (a transcriptional activator Cas12f1 fusion protein or a transcriptional inhibitor Cas12f1 fusion protein). In an embodiment, the method of regulating gene expression may comprise delivering, into a target cell, a nucleic acid encoding an engineered Cas12f1 guide RNA and a Cas12f1 fusion protein (a transcriptional activator Cas12f1 fusion protein or a transcriptional inhibitor Cas12f1 fusion protein). In an embodiment, the method of regulating gene expression may comprise delivering, into a target cell, an engineered Cas12f1 guide RNA and a nucleic acid encoding a Cas12f1 fusion protein (a transcriptional activator Cas12f1 fusion protein or a transcriptional inhibitor Cas12f1 fusion protein). In an embodiment, the method of regulating gene expression may comprise delivering, into a target cell, a nucleic acid encoding an engineered Cas12f1 guide RNA and a nucleic acid encoding a Cas12f1 fusion protein (a transcriptional activator Cas12f1 fusion protein or a transcriptional inhibitor Cas12f1 fusion protein). An engineered Cas12f1 guide RNA or a nucleic acid encoding the engineered Cas12f1 guide RNA, and a Cas12f1 fusion protein (a transcriptional activator Cas12f1 fusion protein or a transcriptional inhibitor Cas12f1 fusion protein) or a nucleic acid encoding the Cas12f1 fusion protein may be delivered into a target cell in various forms of delivery using various delivery methods.

### Form of delivery - RNP

As the form of delivery, a ribonucleoprotein (RNP), in which an engineered Cas12f1 guide RNA and a Cas12f1 fusion protein (a transcriptional activator Cas12f1 fusion protein or a transcriptional inhibitor Cas12f1 fusion protein) are bound to each other, may be used. In an embodiment, the method of regulating gene expression may comprise introducing, into a target cell, a CRISPR activation complex or CRISPR interference complex in which the engineered Cas12f1 guide RNA and the Cas12f1 fusion protein (a transcriptional activator Cas12f1 fusion protein or a transcriptional inhibitor Cas12f1 fusion protein) are bound to each other.

### Form of delivery - Non-viral vector

As another form of delivery, a non-viral vector comprising a nucleic acid sequence encoding an engineered Cas12f1 guide RNA and a nucleic acid sequence encoding a Cas12f1 fusion protein (a transcriptional activator Cas12f1 fusion protein or a transcriptional inhibitor Cas12f1 fusion protein) may be used. In an embodiment, the method of regulating gene expression may comprise introducing, into a target cell, a non-viral vector comprising a nucleic acid sequence encoding an engineered Cas12f1 guide RNA and a nucleic acid sequence encoding a Cas12f1 fusion protein (a transcriptional activator Cas12f1 fusion protein or a transcriptional inhibitor Cas12f1 fusion protein). Specifically, the non-viral vector may be, but is not limited to, a plasmid, naked DNA, a DNA complex, or mRNA. In another embodiment, the method of regulating gene expression may comprise introducing, into a target cell, a first non-viral vector comprising a nucleic acid sequence encoding an engineered Cas12f1 guide RNA and a second non-viral vector comprising a nucleic acid sequence encoding a Cas12f1 fusion protein (a transcriptional activator Cas12f1 fusion protein or a transcriptional inhibitor Cas12f1 fusion protein). Specifically, each of the first non-viral vector and the second non-viral vector may be one selected from a plasmid, naked DNA, a DNA complex, and mRNA, but is not limited thereto.

### Form of delivery - Viral vector

As another form of delivery, a viral vector comprising a nucleic acid sequence encoding an engineered Cas12f1 guide RNA and a nucleic acid sequence encoding a Cas12f1 fusion protein (a transcriptional activator Cas12f1 fusion protein or a transcriptional inhibitor Cas12f1 fusion protein) may be used. In an embodiment, the method of regulating gene expression may comprise introducing, into a target cell, a viral vector comprising a nucleic acid sequence encoding an engineered Cas12f1 guide RNA and a nucleic acid sequence encoding a Cas12f1 fusion protein (a transcriptional activator Cas12f1 fusion protein or a transcriptional inhibitor Cas12f1 fusion protein). Specifically, the viral vector may be one selected from a retrovirus, a lentivirus, an adenovirus, an adeno-associated virus, a vaccinia virus, a poxvirus, and a herpes simplex virus, but is not limited thereto. In an embodiment, the viral vector may be an adeno-associated virus.

In another embodiment, the method of regulating gene expression may comprise introducing, into a target cell, a first viral vector comprising a nucleic acid sequence encoding an engineered Cas12f1 guide RNA, and a second viral vector comprising a nucleic acid sequence encoding a Cas12f1 fusion protein (a transcriptional activator Cas12f1 fusion protein or a transcriptional inhibitor Cas12f1 fusion protein). Specifically, each of the first viral vector and the second viral vector may be one selected from a retrovirus, a lentivirus, an adenovirus, an adeno-associated virus, a vaccinia virus, a poxvirus, and a herpes simplex virus, but is not limited thereto.

### Delivery method - Common means of delivery

The delivery method is not particularly limited as long as it is capable of delivering, into a cell, an engineered Cas12f1 guide RNA or a nucleic acid encoding the engineered Cas12f1 guide RNA, and a Cas12f1 fusion protein (a transcriptional activator Cas12f1 fusion protein or a transcriptional inhibitor Cas12f1 fusion protein) or a nucleic acid encoding the Cas12f1 fusion protein in an appropriate form of delivery. In an embodiment, the delivery method may electroporation, gene gun, sonoporation, magnetofection, and/or transient cell compression or squeezing.

### Delivery method - Nanoparticles

The delivery method may be delivering at least one component, which is included in the CRISPR expression regulatory system, using nanoparticles. Here, the delivery method may be a method known in the art which can be appropriately selected by those of ordinary skill in the art. For example, the nanoparticle delivery method may be a method disclosed in WO 2019/089820 A1, but is not limited thereto.

In an embodiment, the delivery method may be delivering, using nanoparticles, a Cas12f1 fusion protein (a transcriptional activator Cas12f1 fusion protein or a transcriptional inhibitor Cas12f1 fusion protein) or a nucleic acid encoding the Cas12f1 fusion protein and/or an engineered Cas12f1 guide RNA or a nucleic acid encoding the engineered Cas12f1 guide RNA. In an embodiment, the delivery method may be delivering, using nanoparticles, a Cas12f1 fusion protein (a transcriptional activator Cas12f1 fusion protein or a transcriptional inhibitor Cas12f1 fusion protein) or a nucleic acid encoding the Cas12f1 fusion protein, a first engineered Cas12f1 guide RNA or a nucleic acid encoding the first engineered Cas12f1 guide RNA, and/or a second engineered Cas12f1 guide RNA or a nucleic acid encoding the second engineered Cas12f1 guide RNA. Here, the delivery method may be, but is not limited to, a cationic liposome method, a lithium acetate-DMSO method, lipid mediated transfection, calcium phosphate precipitation, lipofection, polyethyleneimine (PEI)-mediated transfection, DEAE-dextran-mediated transfection, and/or nanoparticle-mediated nucleic acid delivery (see Panyam et., al Adv Drug Deliv Rev. 2012 Sep 13. pii: S0169-409X(12)00283-9. doi: 10.1016/j.addr.2012.09.023). Here, the component of the CRISPR/Cas12f1 system may be in the form of an RNP, a non-viral vector, and/or a viral vector. For example, each of the components of the CRISPR expression regulatory system may be in a form of mRNA encoding the same, but is not limited thereto.

### Form and method of delivery - Combination being possible

The method of regulating gene expression comprises delivering, into a cell, an engineered Cas12f1 guide RNA or a nucleic acid encoding the engineered Cas12f1 guide RNA, and a Cas12f1 fusion protein (a transcriptional activator Cas12f1 fusion protein or a transcriptional inhibitor Cas12f1 fusion protein) or a nucleic acid encoding the Cas12f1 fusion protein, wherein delivery forms and/or delivery methods of respective components may be the same as or different from each other. In an embodiment, the method of regulating gene expression may comprise delivering an engineered Cas12f1 guide RNA or a nucleic acid encoding the engineered Cas12f1 guide RNA in a first form of delivery, and delivering a Cas12f1 fusion protein (a transcriptional activator Cas12f1 fusion protein or a transcriptional inhibitor Cas12f1 fusion protein) or a nucleic acid encoding the Cas12f1 fusion protein in a second form of delivery. Here, each of the first form of delivery and the second form of delivery may be any one of the above-described forms of delivery. In an embodiment, the method of regulating gene expression may comprise delivering an engineered Cas12f1 guide RNA or a nucleic acid encoding the engineered Cas12f1 guide RNA using a first delivery method, and a Cas12f1 fusion protein (a transcriptional activator Cas12f1 fusion protein or a transcriptional inhibitor Cas12f1 fusion protein) or a nucleic acid encoding the Cas12f1 fusion protein using a second delivery method. Here, each of the first delivery method and the second delivery method may be any one of the above-described delivery methods.

### Order of Delivery

The method of regulating gene expression comprises delivering, into a cell, an engineered Cas12f1 guide RNA or a nucleic acid encoding the engineered Cas12f1 guide RNA, and a Cas12f1 fusion protein (a transcriptional activator Cas12f1 fusion protein or a transcriptional inhibitor Cas12f1 fusion protein) or a nucleic acid encoding Cas12f1 fusion protein, wherein the components may be delivered into a cell simultaneously or sequentially with a time interval.

In an embodiment, the method of regulating gene expression may comprise delivering, into a cell, an engineered Cas12f1 guide RNA or a nucleic acid encoding the engineered Cas12f1 guide RNA, and a Cas12f1 fusion protein (a transcriptional activator Cas12f1 fusion protein or a transcriptional inhibitor Cas12f1 fusion protein) or a nucleic acid encoding the Cas12f1 fusion protein simultaneously. In an embodiment, the method of regulating gene expression may comprise delivering an engineered Cas12f1 guide RNA or a nucleic acid encoding the engineered Cas12f1 guide RNA into a cell, and then delivering a Cas12f1 fusion protein (a transcriptional activator Cas12f1 fusion protein or a transcriptional inhibitor Cas12f1 fusion protein) or a nucleic acid encoding the Cas12f1 fusion protein into the cell at a time interval. In an embodiment, the method of regulating gene expression may comprise delivering a Cas12f1 fusion protein (a transcriptional activator Cas12f1 fusion protein or a transcriptional inhibitor Cas12f1 fusion protein) or a nucleic acid encoding the Cas12f1 fusion protein into a cell, and then delivering an engineered Cas12f1 guide RNA into the cell at a time interval. In an embodiment, the method of regulating gene expression may comprise delivering a nucleic acid encoding a Cas12f1 fusion protein (a transcriptional activator Cas12f1 fusion protein or a transcriptional inhibitor Cas12f1 fusion protein) into a cell, and then delivering an engineered Cas12f1 guide RNA into the cell at a time interval.

### Bringing CRISPR activation complex or CRISPR interference complex in contact with target nucleic acid

In the method of regulating gene expression provided herein, expression of a target gene is regulated as a CRISPR activation complex or a CRISPR interference complex comes in contact with the target gene in a target cell. Accordingly, the method of regulating gene expression may comprise bringing a CRISPR activation complex or a CRISPR interference complex into contact with a gene in a target cell, or inducing a CRISPR activation complex or a CRISPR interference complex to come in contact therewith. In an embodiment, the method of regulating gene expression may comprise bringing a CRISPR activation complex or a CRISPR interference complex into contact with a target gene in a target cell. In an embodiment, the method of regulating gene expression may comprise inducing a CRISPR activation complex or a CRISPR interference complex to come in contact with a target gene in a target cell. Here, the induction is not particularly limited as long as it allows the CRISPR activation complex or the CRISPR interference complex to come in contact with a target gene in a cell. In an embodiment, the induction may be achieved by delivering, into a cell, an engineered Cas12f1 guide RNA or a nucleic acid encoding the engineered Cas12f1 guide RNA, and a Cas12f1 fusion protein (a transcriptional activator Cas12f1 fusion protein or a transcriptional inhibitor Cas12f1 fusion protein) or a nucleic acid encoding the Cas12f1 fusion protein.

### Results obtained by regulating gene expression

As a result of performing the method of regulating gene expression provided herein, expression of a target gene may be promoted (or increased) or suppressed (or inhibited). Here, the expression may refer to transcription of a target gene into mRNA. Generally, when expression of a target gene is promoted (or increased), an expression level of mRNA of the corresponding gene increases and production of a protein encoded by the corresponding gene increases. In addition, when expression of a target gene is suppressed (or inhibited), an expression level of mRNA of the corresponding gene decreases and production of a protein encoded by the corresponding gene decreases. In an embodiment, as a result of performing the method of regulating gene expression, production of a protein encoded by a target gene may increase or decrease.

### Examples of method of regulating gene expression

In an embodiment, the method of regulating gene expression may comprise delivering, into a eukaryotic cell, a CRISPR activation complex or a CRISPR interference complex in a form of a ribonucleoprotein in which an engineered Cas12f1 guide RNA and a Cas12f1 fusion protein (a transcriptional activator Cas12f1 fusion protein or a transcriptional inhibitor Cas12f1 fusion protein) are bound to each other. Here, the delivery may be achieved by electroporation or lipofection.

In an embodiment, the method of regulating gene expression may comprise delivering, into a eukaryotic cell, a nucleic acid encoding an engineered Cas12f1 guide RNA and a nucleic acid encoding a Cas12f1 fusion protein (a transcriptional activator Cas12f1 fusion protein or a transcriptional inhibitor Cas12f1 fusion protein). Here, the delivery may be achieved by electroporation or lipofection.

In an embodiment, the method of regulating gene expression may comprise delivering, into a eukaryotic cell, an adeno-associated virus (AAV) vector comprising a nucleic acid sequence encoding an engineered Cas12f1 guide RNA and a nucleic acid sequence encoding a Cas12f1 fusion protein (a transcriptional activator Cas12f1 fusion protein or a transcriptional inhibitor Cas12f1 fusion protein).

### Experimental Examples

Hereinafter, the present disclosure will be described in more detail through experimental examples and examples. These examples are only for illustrating the present disclosure, and it would be obvious to those of ordinary skill in the art that a scope of the disclosure is not to be construed as being limited by these examples.

### 1. Effect of engineered Cas12f1 guide RNA

### Experimental Example 1. Preparation of materials for experiment

### Experimental Example 1.1. Design and construction of plasmid vector

A Cas12f1 gene was codon-optimized (SEQ ID NO: 270) for expression in human cells, and the optimized sequence was synthesized for vector construction. Finally, to the Cas12f1 protein-encoding sequence were added a chicken β-actin promoter, a nuclear localization signal sequence at the 5'-end and the 3'-end, and a sequence encoding an eGFP linked by a self-cleaving T2A peptide. An amino acid sequence of the Cas12f1 protein and a DNA sequence encoding the same are shown in Table 01.

**[Table 01]**

| **Label** | **Sequence (N-terminal to C-terminal/5' to 3')** | **SEQ ID NO** |
|---|---|---|
| Cas12f1 + NLS(N/C-terminal) sequence (peptide) | MPKKKRKVAKNTITKTLKLRIVRPYNSAEVEKIVADEKNNREKIALEKNKDKVKEACSKHLKVAAYCTTQVERNACLFCKARKLDD KFYQKLRGQFPDAVFWQEISEIFRQLQKQAAEIYNQSLIELYYEIFIKGKGIANASSVEHYLSDVCYTRAAELFKNAAIASGLRSKIKS NFRLKELKNMKSGLPTTKSDNFPIPLVKQKGGQYTGFEISNHNSDFIIKIPFGRWQVKKEIDKYRPWEKFDFEQVQKSPKPISLLLST QRRKRNKGWSKDEGTEAEIKKVMNGDYQTSYIEVKRGSKIGEKSAWMLNLSIDVPKIDKGVDPSIIGGIDVGVKSPLVCAINNAF SRYSISDNDLFHFNKKMFARRRILLKKNRHKRAGHGAKNKLKPITILTEKSERFRKKLIERWACEIADFFIKNKVGTVQMENLESMK RKEDSYFNIRLRGFWPYAEMQNKIEFKLKQYGIEIRKVAPNNTSKTCSKCGHLNNYFNFEYRKKNKFPHFKCEKCNFKENADYNA ALNISNPKLKSTKEEPPKKKRKV | 263 |
| Cas12f1 + NLS(NjC-terminal) sequence (DNA) | ATGccaaagaagaagcggaaagtcGCCAAGAACACAATTACAAAGACACTGAAGCTGAGGATCGTGAGACCATACAACAGCGCT GAGGTCGAGAAGATTGTGGCTGATGAAAAGAACAACAGGGAAAAGATCGCCCTCGAGAAGAACAAGGATAAGGTGAAG GAGGCCTGCTCTAAGCACCTGAAAGTGGCCGCCTACTGCACCACACAGGTGGAGAGGAACGCCTGTCTGTTTTGTAAAGCTC GGAAGCTGGATGATAAGTTTTACCAGAAGCTGCGGGGCCAGTTCCCCGATGCCGTCTTTTGGCAGGAGATTAGCGAGATCTT CAGACAGCTGCAGAAGCAGGCCGCCGAGATCTACAACCAGAGCCTGATCGAGCTCTACTACGAGATCTTCATCAAGGGCAA GGGCATTGCCAACGCCTCCTCCGTGGAGCACTACCTGAGCGACGTGTGCTACACAAGAGCCGCCGAGCTCTTTAAGAACGCC GCTATCGCTTCCGGGCTGAGGAGCAAGATTAAGAGTAACTTCCGGCTCAAGGAGCTGAAGAACATGAAGAGCGGCCTGCC CACTACAAAGAGCGACAACTTCCCAATTCCACTGGTGAAGCAGAAGGGGGGCCAGTACACAGGGTTCGAGATTTCCAACCA CAACAGCGACTTTATTATTAAGATCCCCTTTGGCAGGTGGCAGGTCAAGAAGGAGATTGACAAGTACAGGCCCTGGGAGAA GTTTGATTTCGAGCAGGTGCAGAAGAGCCCCAAGCCTATTTCCCTGCTGCTGTCCACACAGCGGCGGAAGAGGAACAAGGG GTGGTCTAAGGATGAGGGGACCGAGGCCGAGATTAAGAAAGTGATGAACGGCGACTACCAGACAAGCTACATCGAGGTC AAGCGGGGCAGTAAGATTGGCGAGAAGAGCGCCTGGATGCTGAACCTGAGCATTGACGTGCCAAAGATTGATAAGGGCG TGGATCCCAGCATCATCGGAGGGATCGATGTGGGGGTCAAGAGCCCCCTCGTGTGCGCCATCAACAACGCCTTCAGCAGGT ACAGCATCTCCGATAACGACCTGTTCCACTTTAACAAGAAGATGTTCGCCCGGCGGAGGATTTTGCTCAAGAAGAACCGGCA CAAGCGGGCCGGACACGGGGCCAAGAACAAGCTCAAGCCCATCACTATCCTGACCGAGAAGAGCGAGAGGTTCAGGAAG AAGCTCATCGAGAGATGGGCCTGCGAGATCGCCGATTTCTTTATTAAGAACAAGGTCGGAACAGTGCAGATGGAGAACCTC GAGAGCATGAAGAGGAAGGAGGATTCCTACTTCAACATTCGGCTGAGGGGGTTCTGGCCCTACGCTGAGATGCAGAACAA GATTGAGTTTAAGCTGAAGCAGTACGGGATTGAGATCCGGAAGGTGGCCCCCAACAACACCAGCAAGACCTGCAGCAAGT GCGGGCACCTCAACAACTACTTCAACTTCGAGTACCGGAAGAAGAACAAGTTCCCACACTTCAAGTGCGAGAAGTGCAACT TTAAGGAGAACGCCGATTACAACGCCGCCCTGAACATCAGCAACCCTAAGCTGAAGAGCACTAAGGAGGAGCCccaaagaag aagcggaaagtc | 273 |

A template DNA encoding a (engineered) Cas12f1 guide RNA was synthesized and cloned into a pTwist Amp plasmid vector (Twist Bioscience). When necessary, the vector was used as a template for amplifying a sequence encoding the guide RNA using a U6-complementary forward primer and a protospacer-complementary reverse primer. Using a Gibson assembly, an oligonucleotide encoding the engineered Cas12f1 guide RNA was cloned into the vector comprising the codon-optimized Cas12f1 gene, so that a vector for an engineered CRISPR/Cas12f1 system was constructed.

### Experimental Example 1.2. Engineering of Cas12f1 guide RNA

Linking a U-rich tail to the 3' end of the engineered Cas12f1 guide RNA was performed using Pfu PCR Master Mix5 (Biofact) in the presence of a sequence-modified primer and the Cas12f1 guide RNA plasmid vector. The PCR amplicon was purified by using a HiGene^{™} Gel&PCR Purification System (Biofact). Modification of the second region, and the fourth and fifth regions of the engineered scaffold region of the engineered Cas12f1 guide RNA was performed by cloning synthetic oligonucleotides, each of which delivers a modified sequence (Macrogen) into a linearized guide RNA-encoding vector, using ApoI and BamHI restriction enzymes. Modification of the first region of the engineered scaffold region of the engineered Cas12f1 guide RNA was performed by PCR amplification of a canonical or engineered template plasmid vector using a forward primer targeting the 5' end of the tracrRNA and a reverse primer targeting the U6 promoter region. The PCR amplification was performed using a Q5 Hot Start high-fidelity DNA polymerase (NEB), and ligation of the PCR products was performed using a KLD Enzyme Mix (NEB). The ligated PCR product was transformed into DH5α E. coli cells. Mutagenesis was identified by a Sanger sequencing analysis. The modified plasmid vector was purified using a NucleoBond ^{®} Xtra Midi EF kit (MN). 1 microgram of the purified plasmid was used as a template for mRNA synthesis using T7 RNA polymerase (NEB) and NTPs (Jena Bioscience). The engineered Cas12f1 guide RNA prepared above was purified using a Monarch^{®} RNA cleanup kit (NEB), aliquoted into cryogenic vials and stored in liquid nitrogen.

### Experimental Example 1.3. Cell culture and transfection

HEK293 T cells (LentX-293T, Takara) were cultured under a condition of 5% of CO₂ in Dulbecco's modified eagle medium (DMEM) supplemented with 10% heat-inactivated fetal bovine serum (FBS) (Corning) and penicillin/streptomycin. Cell transfection was performed by electroporation or lipofection. For the electroporation, each 2 µg to 5 µg of the plasmid vector encoding the Cas12f1 protein and DNA encoding the guide RNA (and the engineered guide RNA) produced in Experimental Example 1.2 were transfected into 4 x 10⁵ HEK-293 T cells using a Neon transfection system (Invitrogen). The electroporation was performed under conditions of 1300 V, 10 mA, and 3 pulses. For the lipofection, 6 µL to 15 µL of FuGene reagent (Promega) was mixed for 15 minutes with 2 µg to 5 µg of the plasmid vector encoding a Cas12f1 protein and 1.5 µg to 5 µg of the PCR amplicon. The mixture (300 µL) was added to 1.5 ml DMEM medium plated with 1 x 10⁶ cells 1 day before transfection. The cells were cultured in the presence of the mixture for 1 day to 10 days. After culturing, the cells were collected, and genomic DNA of the cells was manually isolated using a PureHelix^{™} genomic DNA preparation kit (NanoHelix) or a Maxwell RSC Cultured cells DNA Kit (Promega).

### Experimental Example 1.4. Measurement of intercellular indel efficiency

PCR was performed using target-specific primers in the presence of KAPA HiFi HotStart DNA polymerase (Roche) on a region comprising a protospacer in the genomic DNA isolated from HEK-293 T cells. The amplification was performed following the manufacturer's instructions. The PCR amplicon, which is a resulting product of the amplication and contains Illumina TruSeq HT dual indexes, was subjected to 150-bp paired end sequencing using Illumina iSeq 100. Indel frequencies were calculated by using MAUND. The MAUND is provided at https://github.com/ibs-cge/maund.

### Experimental Example 1.5. Quantitave real-time PCR

A guide RNA (or an engineered guide RNA) or a genomic DNA was each extracted from HEK293 T cells using an RNeasy Miniprep kit (Qiagen), a Maxwell RSC miRNA Tissue Kit (Promega), or a DNeasy Blood & Tissue Kit (Qiagen). To quantify the guide RNA, ligation of an RNA-specific primer was performed and cDNA was synthesized using a crRNA-specific primer. The cDNA was used as a template for quantitative real-time PCR. The real-time PCR was analyzed using a KAFA SYBR FAST qPCR Master Mix (2X) Kit (KAPAbiosystems).

### Experimental Example 1.6. Statistical analysis

For each experimental example, the experiment was performed three times, and an average of the respective values was used for analysis.

### Experimental Example 2. Indel efficiency comparison 1 between engineered CRISPR/Cas12f1 systems

To measure indel efficiency of an engineered CRISPR/Cas12f1 system using an engineered Cas12f1 guide RNA, each of the examples was prepared by Experimental Examples 1.1 to 1.2. The target sequences used for the experiments are shown in Table 02 below.

**[Table 02]**

| **Target Label** | **protospacer sequence (5' to 3')** | **SEQ ID NO** |
|---|---|---|
| DY2 | CACACACACAGTGGGCTACC | 339 |
| DY10 | CATCCCCAGGACACACACAC | 340 |
| Intergenic-22 | AGAACACATACCCCTGGGCC | 341 |

Sequences of the engineered Cas12f1 guide RNAs used in the respective examples are shown in Table 03 to Table 08.

**[Table 03]**

| **Label** | **Target** | **PAM (5' to 3')** | **Engineered Cas12f1 guide RNA sequence (5' to 3')** | **SEQ ID NOs** |
|---|---|---|---|---|
| Comparative Example 1.1.1 | DY2 | TTTG | CUUCACUGAUAAAGUGGAGAACCGCUUCACCAAAAGCUGUCCCUUAGGGGAUUAGAACUUGAGUGAAGGUGGGCUGCUUGCAUC AGCCUAAUGUCGAGAAGUGCUUUCUUCGGAAAGUAACCCUCGAAACAAAUUCAUUUGAAAGAAUGAAGGAAUGCAACCACACACA CAGUGGGCUACC | 294 |
| Comparative Example 1.1.2 | DY2 | TTTG | CUUCACUGAUAAAGUGGAGAACCGCUUCACCAAAAGCUGUCCCUUAGGGGAUUAGAACUUGAGUGAAGGUGGGCUGCUUGCAUC AGCCUAAUGUCGAGAAGUGCUUUCUUCGGAAAGUAACCCUCGAAACAAAUUCAUUUGAAAGAAUGAAGGAAUGCAACCACACACA CAGUGGGCUACCUUUUAUUUU | 295 |
| Example 1.1.1 | DY2 | TTTG | GAUAAAGUGGAGAACCGCUUCACCAAAAGCUGUCCCUUAGGGGAUUAGAACUUGAGUGAAGGUGGGCUGCUUGCAUCAGCCUAA UGUCGAGAAGUGCUUUCUUCGGAAAGUAACCCUCGAAACAAAUUCAUUUGAAAGAAUGAAGGAAUGCAACCACACACACAGUGGG CUACCUUUUAUUUU | 296 |
| Example 1.1.2 | DY2 | TTTG | UGGAGAACCGCUUCACCAAAAGCUGUCCCUUAGGGGAUUAGAACUUGAGUGAAGGUGGGCUGCUUGCAUCAGCCUAAUGUCGAG AAGUGCUUUCUUCGGAAAGUAACCCUCGAAACAAAUUCAUUUGAAAGAAUGAAGGAAUGCAACCACACACACAGUGGGCUACCUU UUAUUUU | 297 |
| Example 1.1.3 | DY2 | TTTG | ACCGCUUCACCAAAAGCUGUCCCUUAGGGGAUUAGAACUUGAGUGAAGGUGGGCUGCUUGCAUCAGCCUAAUGUCGAGAAGUGC UUUCUUCGGAAAGUAACCCUCGAAACAAAUUCAUUUGAAAGAAUGAAGGAAUGCAACCACACACACAGUGGGCUACCUUUUAUUU U | 298 |
| Example 1.1.4 | DY2 | TTTG | CUUCACUGAUAAAGUGGAGAACCGCUUCACCAAAAGCUGUUUAGAUUAGAACUUGAGUGAAGGUGGGCUGCUUGCAUCAGCCUA AUGUCGAGAAGUGCUUUCUUCGGAAAGUAACCCUCGAAACAAAUUCAUUUGAAAGAAUGAAGGAAUGCAACCACACACACAGUGG GCUACCUUUUAUUUU | 299 |
| Example 1.1.5 | DY2 | TTTG | CUUCACUGAUAAAGUGGAGAACCGCUUCACCAAAAGCUUUAGAGAACUUGAGUGAAGGUGGGCUGCUUGCAUCAGCCUAAUGUC GAGAAGUGCUUUCUUCGGAAAGUAACCCUCGAAACAAAUUCAUUUGAAAGAAUGAAGGAAUGCAACCACACACACAGUGGGCUAC CUUUUAUUUU | 300 |
| Example 1.1.6 | DY2 | TTTG | CUUCACUGAUAAAGUGGAGAACCGCUUCACCAAUUAGUUGAGUGAAGGUGGGCUGCUUGCAUCAGCCUAAUGUCGAGAAGUGCU UUCUUCGGAAAGUAACCCUCGAAACAAAUUCAUUUGAAAGAAUGAAGGAAUGCAACCACACACACAGUGGGCUACCUUUUAUUU | 301 |
| Example 117 | DY2 | TTTG | CUUCACUGAUAAAGUGGAGAACCGCUUCACCAAAAGCUGUCCCUUAGGGGAUUAGAACUUGAGUGAAGGUGGGCUGCUUGCAUC AGCCUAAUGUCGAGAAGUGCUUUCUUCGGAAAGUAACCCUCGAAACAAAUUCAUGAAAAUGAAGGAAUGCAACCACACACACAGU GGGCUACCUUUUAUUUU | 302 |
| Example 1.1.8 | DY2 | TTTG | CUUCACUGAUAAAGUGGAGAACCGCUUCACCAAAAGCUGUCCCUUAGGGGAUUAGAACUUGAGUGAAGGUGGGCUGCUUGCAUC AGCCUAAUGUCGAGAAGUGCUUUCUUCGGAAAGUAACCCUCGAAACAAAUUCGAAAGAAGGAAUGCAACCACACACACAGUGGGC UACCUUUUAUUUU | 303 |
| Example 1.1.9 | DY2 | TTTG | CUUCACUGAUAAAGUGGAGAACCGCUUCACCAAAAGCUGUCCCUUAGGGGAUUAGAACUUGAGUGAAGGUGGGCUGCUUGCAUC AGCCUAAUGUCGAGAAGUGCUUUCUUCGGAAAGUAACCCUCGAAACAAAGAAAGGAAUGCAACCACACACACAGUGGGCUACCUU UUAUUUU | 304 |

**[Table 04]**

| **Label** | **Target** | **PAM (5' to 3')** | **Engineered Cas12f1 guide RNA sequence (5' to 3')** | **SEQ ID NOs** |
|---|---|---|---|---|
| Comparative Example 1.2.1 | DY10 | TTTG | CUUCACUGAUAAAGUGGAGAACCGCUUCACCAAAAGCUGUCCCUUAGGGGAUUAGAACUUGAGUGAAGGUGGGCUGCUUGCAUC AGCCUAAUGUCGAGAAGUGCUUUCUUCGGAAAGUAACCCUCGAAACAAAUUCAUUUGAAAGAAUGAAGGAAUGCAACCAUCCCCA GGACACACACAC | 309 |
| Comparative Example 1.2.2 | DY10 | TTTG | CUUCACUGAUAAAGUGGAGAACCGCUUCACCAAAAGCUGUCCCUUAGGGGAUUAGAACUUGAGUGAAGGUGGGCUGCUUGCAUC AGCCUAAUGUCGAGAAGUGCUUUCUUCGGAAAGUAACCCUCGAAACAAAUUCAUUUGAAAGAAUGAAGGAAUGCAACCAUCCCCA GGACACACACACUUUUAUUUU | 310 |
| Example 1.2.1 | DY10 | TTTG | GAUAAAGUGGAGAACCGCUUCACCAAAAGCUGUCCCUUAGGGGAUUAGAACUUGAGUGAAGGUGGGCUGCUUGCAUCAGCCUAA UGUCGAGAAGUGCUUUCUUCGGAAAGUAACCCUCGAAACAAAUUCAUUUGAAAGAAUGAAGGAAUGCAACCAUCCCCAGGACACA CACACUUUUAUUUU | 311 |
| Example 1.2.2 | DY10 | TTTG | UGGAGAACCGCUUCACCAAAAGCUGUCCCUUAGGGGAUUAGAACUUGAGUGAAGGUGGGCUGCUUGCAUCAGCCUAAUGUCGAG AAGUGCUUUCUUCGGAAAGUAACCCUCGAAACAAAUUCAUUUGAAAGAAUGAAGGAAUGCAACCAUCCCCAGGACACACACACUUU UAUUUU | 312 |
| Example 1.2.3 | DY10 | TTTG | ACCGCUUCACCAAAAGCUGUCCCUUAGGGGAUUAGAACUUGAGUGAAGGUGGGCUGCUUGCAUCAGCCUAAUGUCGAGAAGUGC UUUCUUCGGAAAGUAACCCUCGAAACAAAUUCAUUUGAAAGAAUGAAGGAAUGCAACCAUCCCCAGGACACACACACUUUUAUUU U | 313 |
| Example 1.2.4 | DY10 | TTTG | CUUCACUGAUAAAGUGGAGAACCGCUUCACCAAAAGCUGUUUAGAUUAGAACUUGAGUGAAGGUGGGCUGCUUGCAUCAGCCUA AUGUCGAGAAGUGCUUUCUUCGGAAAGUAACCCUCGAAACAAAUUCAUUUGAAAGAAUGAAGGAAUGCAACCAUCCCCAGGACAC ACACACUUUUAUUUU | 314 |
| Example 1.2.5 | DY10 | TTTG | CUUCACUGAUAAAGUGGAGAACCGCUUCACCAAAAGCUUUAGAGAACUUGAGUGAAGGUGGGCUGCUUGCAUCAGCCUAAUGUC GAGAAGUGCUUUCUUCGGAAAGUAACCCUCGAAACAAAUUCAUUUGAAAGAAUGAAGGAAUGCAACCAUCCCCAGGACACACACAC UUUUAUUUU | 315 |
| Example 1.2.6 | DY10 | TTTG | CUUCACUGAUAAAGUGGAGAACCGCUUCACCAAUUAGUUGAGUGAAGGUGGGCUGCUUGCAUCAGCCUAAUGUCGAGAAGUGCU UUCUUCGGAAAGUAACCCUCGAAACAAAUUCAUUUGAAAGAAUGAAGGAAUGCAACCAUCCCCAGGACACACACACUUUUAUUUU | 316 |
| Example 1.2.7 | DY10 | TTTG | CUUCACUGAUAAAGUGGAGAACCGCUUCACCAAAAGCUGUCCCUUAGGGGAUUAGAACUUGAGUGAAGGUGGGCUGCUUGCAUC AGCCUAAUGUCGAGAAGUGCUUUCUUCGGAAAGUAACCCUCGAAACAAAUUCAUGAAAAUGAAGGAAUGCAACCAUCCCCAGGAC ACACACACUUUUAUUUU | 317 |
| Example 1.2.8 | DY10 | TTTG | CUUCACUGAUAAAGUGGAGAACCGCUUCACCAAAAGCUGUCCCUUAGGGGAUUAGAACUUGAGUGAAGGUGGGCUGCUUGCAUC AGCCUAAUGUCGAGAAGUGCUUUCUUCGGAAAGUAACCCUCGAAACAAAUUCGAAAGAAGGAAUGCAACCAUCCCCAGGACACACA CACUUUUAUUUU | 318 |
| Example 1.2.9 | DY10 | TTTG | CUUCACUGAUAAAGUGGAGAACCGCUUCACCAAAAGCUGUCCCUUAGGGGAUUAGAACUUGAGUGAAGGUGGGCUGCUUGCAUC AGCCUAAUGUCGAGAAGUGCUUUCUUCGGAAAGUAACCCUCGAAACAAAGAAAGGAAUGCAACCAUCCCCAGGACACACACACUUU UAUUUU | 319 |

**[Table 05]**

| **Label** | **Target** | **PAM (5' to 3')** | **Engineered Cas12f1 guide RNA sequence (5' to 3')** | **SEQ ID NOs** |
|---|---|---|---|---|
| Comparative Example 1.2.1 | DY10 | TTTG | CUUCACUGAUAAAGUGGAGAACCGCUUCACCAAAAGCUGUCCCUUAGGGGAUUAGAACUUGAGUGAAGGUGGGCUGCUUGCAUC AGCCUAAUGUCGAGAAGUGCUUUCUUCGGAAAGUAACCCUCGAAACAAAUUCAUUUGAAAGAAUGAAGGAAUGCAACCAUCCCCA GGACACACACAC | 309 |
| Comparative Example 1.2.2 | DY10 | TTTG | CUUCACUGAUAAAGUGGAGAACCGCUUCACCAAAAGCUGUCCCUUAGGGGAUUAGAACUUGAGUGAAGGUGGGCUGCUUGCAUC AGCCUAAUGUCGAGAAGUGCUUUCUUCGGAAAGUAACCCUCGAAACAAAUUCAUUUGAAAGAAUGAAGGAAUGCAACCAUCCCCA GGACACACACACUUUUAUUUU | 310 |
| Example 1.2.1 | DY10 | TTTG | GAUAAAGUGGAGAACCGCUUCACCAAAAGCUGUCCCUUAGGGGAUUAGAACUUGAGUGAAGGUGGGCUGCUUGCAUCAGCCUAA UGUCGAGAAGUGCUUUCUUCGGAAAGUAACCCUCGAAACAAAUUCAUUUGAAAGAAUGAAGGAAUGCAACCAUCCCCAGGACACA CACACUUUUAUUUU | 311 |
| Example 122 | DY10 | TTTG | UGGAGAACCGCUUCACCAAAAGCUGUCCCUUAGGGGAUUAGAACUUGAGUGAAGGUGGGCUGCUUGCAUCAGCCUAAUGUCGAG AAGUGCUUUCUUCGGAAAGUAACCCUCGAAACAAAUUCAUUUGAAAGAAUGAAGGAAUGCAACCAUCCCCAGGACACACACACUUU UAUUUU | 312 |
| Example 1.2.3 | DY10 | TTTG | ACCGCUUCACCAAAAGCUGUCCCUUAGGGGAUUAGAACUUGAGUGAAGGUGGGCUGCUUGCAUCAGCCUAAUGUCGAGAAGUGC UUUCUUCGGAAAGUAACCCUCGAAACAAAUUCAUUUGAAAGAAUGAAGGAAUGCAACCAUCCCCAGGACACACACACUUUUAUUU U | 313 |
| Example 1.2.4 | DY10 | TTTG | CUUCACUGAUAAAGUGGAGAACCGCUUCACCAAAAGCUGUUUAGAUUAGAACUUGAGUGAAGGUGGGCUGCUUGCAUCAGCCUA AUGUCGAGAAGUGCUUUCUUCGGAAAGUAACCCUCGAAACAAAUUCAUUUGAAAGAAUGAAGGAAUGCAACCAUCCCCAGGACAC ACACACUUUUAUUUU | 314 |
| Example 1.2.5 | DY10 | TTTG | CUUCACUGAUAAAGUGGAGAACCGCUUCACCAAAAGCUUUAGAGAACUUGAGUGAAGGUGGGCUGCUUGCAUCAGCCUAAUGUC GAGAAGUGCUUUCUUCGGAAAGUAACCCUCGAAACAAAUUCAUUUGAAAGAAUGAAGGAAUGCAACCAUCCCCAGGACACACACAC UUUUAUUUU | 315 |
| Example 1.2.6 | DY10 | TTTG | CUUCACUGAUAAAGUGGAGAACCGCUUCACCAAUUAGUUGAGUGAAGGUGGGCUGCUUGCAUCAGCCUAAUGUCGAGAAGUGCU UUCUUCGGAAAGUAACCCUCGAAACAAAUUCAUUUGAAAGAAUGAAGGAAUGCAACCAUCCCCAGGACACACACACUUUUAUUUU | 316 |
| Example 1.2.7 | DY10 | TTTG | CUUCACUGAUAAAGUGGAGAACCGCUUCACCAAAAGCUGUCCCUUAGGGGAUUAGAACUUGAGUGAAGGUGGGCUGCUUGCAUC AGCCUAAUGUCGAGAAGUGCUUUCUUCGGAAAGUAACCCUCGAAACAAAUUCAUGAAAAUGAAGGAAUGCAACCAUCCCCAGGAC ACACACACUUUUAUUUU | 317 |
| Example 1.2.8 | DY10 | TTTG | CUUCACUGAUAAAGUGGAGAACCGCUUCACCAAAAGCUGUCCCUUAGGGGAUUAGAACUUGAGUGAAGGUGGGCUGCUUGCAUC AGCCUAAUGUCGAGAAGUGCUUUCUUCGGAAAGUAACCCUCGAAACAAAUUCGAAAGAAGGAAUGCAACCAUCCCCAGGACACACA CACUUUUAUUUU | 318 |
| Example 1.2.9 | DY10 | TTTG | CUUCACUGAUAAAGUGGAGAACCGCUUCACCAAAAGCUGUCCCUUAGGGGAUUAGAACUUGAGUGAAGGUGGGCUGCUUGCAUC AGCCUAAUGUCGAGAAGUGCUUUCUUCGGAAAGUAACCCUCGAAACAAAGAAAGGAAUGCAACCAUCCCCAGGACACACACACUUU UAUUUU | 319 |

**[Table 06]**

| **Label** | **Target** | **PAM (5' to 3')** | **Engineered Cas12f1 guide RNA sequence (5' to 3')** | **SEQ ID NOs** |
|---|---|---|---|---|
| Example 1.2.10 | DY10 | TTTG | ACCGCUUCACCAUUAGUGAGUGAAGGUGGGCUGCUUGCAUCAGCCUAAUGUCGAGAAGUGCUUUCUUCGGAAAGUAACCCUCGA AACAAAUUCAUUUGAAAGAAUGAAGGAAUGCAACCAUCCCCAGGACACACACACUUUUAUUUU | 320 |
| Example 1.2.11 | DY10 | TTTG | ACCGCUUCACCAAAAGCUGUCCCUUAGGGGAUUAGAACUUGAGUGAAGGUGGGCUGCUUGCAUCAGCCUAAUGUCGAGAAGUGC UUUCUUCGGAAAGUAACCCUCGAAACAAAGAAAGGAAUGCAACCAUCCCCAGGACACACACACUUUUAUUUU | 321 |
| Example 1.2.12 | DY10 | TTTG | CUUCACUGAUAAAGUGGAGAACCGCUUCACCAUUAGUGAGUGAAGGUGGGCUGCUUGCAUCAGCCUAAUGUCGAGAAGUGCUUU CUUCGGAAAGUAACCCUCGAAACAAAGAAAGGAAUGCAACCAUCCCCAGGACACACACACUUUUAUUUU | 322 |
| Example 1.2.13 | DY10 | TTTG | ACCGCUUCACCAUUAGUGAGUGAAGGUGGGCUGCUUGCAUCAGCCUAAUGUCGAGAAGUGCUUUCUUCGGAAAGUAACCCUCGA AACAAAGAAAGGAAUGCAACCAUCCCCAGGACACACACACUUUUAUUUU | 323 |

**[Table 07]**

| **Label** | **Target** | **PAM (5' to 3')** | **Engineered Cas12f1 guide RNA sequence (5' to 3')** | **SEQ ID No.** |
|---|---|---|---|---|
| Comparative Example 1.3.1 | Intergenic-22 | TTTA | CUUCACUGAUAAAGUGGAGAACCGCUUCACCAAAAGCUGUCCCUUAGGGGAUUAGAACUUGAGU GAAGGUGGGCUGCUUGCAUC AGCCUAAUGUCGAGAAGUGCUUUCUUCGGAAAGUAACCCUCGAAACAAAUUCAUUUGAAAGAAU GAAGGAAUGCAACAGAACACA UACCCCUGGGCC | 324 |
| Comparative Example 1.3.2 | Intergenic-22 | TTTA | CUUCACUGAUAAAGUGGAGAACCGCUUCACCAAAAGCUGUCCCUUAGGGGAUUAGAACUUGAGUGAAGGUGGGCUGCUUGCAUC AGCCUAAUGUCGAGAAGUGCUUUCUUCGGAAAGUAACCCUCGAAACAAAUUCAUUUGAAAGAAUGAAGGAAUGCAACAGAACACA UACCCCUGGGCCUUUUAUUUU | 325 |
| Example 1.3.1 | lntergenic-22 | TTTA | GAUAAAGUGGAGAACCGCUUCACCAAAAGCUGUCCCUUAGGGGAUUAGAACUUGAGUGAAGGUGGGCUGCUUGCAUCAGCCUAA UGUCGAGAAGUGCUUUCUUCGGAAAGUAACCCUCGAAACAAAUUCAUUUGAAAGAAUGAAGGAAUGCAACAGAACACAUACCCCU GGGCCUUUUAUUUU | 326 |
| Example 1.3.2 | Intergeni-222 | TTTA | UGGAGAACCGCUUCACCAAAAGCUGUCCCUUAGGGGAUUAGAACUUGAGUGAAGGUGGGCUGCUUGCAUCAGCCUAAUGUCGAG AAGUGCUUUCUUCGGAAAGUAACCCUCGAAACAAAUUCAUUUGAAAGAAUGAAGGAAUGCAACAGAACACAUACCCCUGGGCCUU UUAUUUU | 327 |
| Example 1.3.3 | Intergenic-22 | TTTA | ACCGCUUCACCAAAAGCUGUCCCUUAGGGGAUUAGAACUUGAGUGAAGGUGGGCUGCUUGCAUCAGCCUAAUGUCGAGAAGUGC UUUCUUCGGAAAGUAACCCUCGAAACAAAUUCAUUUGAAAGAAUGAAGGAAUGCAACAGAACACAUACCCCUGGGCCUUUUAUUU U CUUCACUGAUAAAGUGGAGAACCGCUUCACCAAAAGCUGUUUAGAUUAGAACUUGAGUGAAGGUGGGCUGCUUGCAUCAGCCUA | 328 |
| Example 1.3.4 | Intergenic-22 | TTTA | AUGUCGAGAAGUGCUUUCUUCGGAAAGUAACCCUCGAAACAAAUUCAUUUGAAAGAAUGAAGGAAUGCAACAGAACACAUACCCC UGGGCCUUUUAUUUU | 329 |
| Example 1.3.5 | Intergenic-22 | TTTA | CUUCACUGAUAAAGUGGAGAACCGCUUCACCAAAAGCUUUAGAGAACUUGAGUGAAGGUGGGCUGCUUGCAUCAGCCUAAUGUC GAGAAGUGCUUUCUUCGGAAAGUAACCCUCGAAACAAAUUCAUUUGAAAGAAUGAAGGAAUGCAACAGAACACAUACCCCUGGGC CUUUUAUUUU | 330 |
| Example 1.3.6 | Intergenic-22 | TTTA | CUUCACUGAUAAAGUGGAGAACCGCUUCACCAAUUAGUUGAGUGAAGGUGGGCUGCUUGCAUCAGCCUAAUGUCGAGAAGUGCU UUCUUCGGAAAGUAACCCUCGAAACAAAUUCAUUUGAAAGAAUGAAGGAAUGCAACAGAACACAUACCCCUGGGCCUUUUAUUUU | 331 |
| Example 1.3.7 | Intergeniε-22 | TTTA | CUUCACUGAUAAAGUGGAGAACCGCUUCACCAAAAGCUGUCCCUUAGGGGAUUAGAACUUGAGUGAAGGUGGGCUGCUUGCAUC AGCCUAAUGUCGAGAAGUGCUUUCUUCGGAAAGUAACCCUCGAAACAAAUUCAUGAAAAUGAAGGAAUGCAACAGAACACAUACC CCUGGGCCUUUUAUUUU | 332 |
| Example 1.3.8 | Intergenic-22 | TTTA | CUUCACUGAUAAAGUGGAGAACCGCUUCACCAAAAGCUGUCCCUUAGGGGAUUAGAACUUGAGUGAAGGUGGGCUGCUUGCAUC AGCCUAAUGUCGAGAAGUGCUUUCUUCGGAAAGUAACCCUCGAAACAAAUUCGAAAGAAGGAAUGCAACAGAACACAUACCCCUG GGCCUUUUAUUUU | 333 |
| Example 1.3.9 | Intergenic-22 | TTTA | CUUCACUGAUAAAGUGGAGAACCGCUUCACCAAAAGCUGUCCCUUAGGGGAUUAGAACUUGAGUGAAGGUGGGCUGCUUGCAUC AGCCUAAUGUCGAGAAGUGCUUUCUUCGGAAAGUAACCCUCGAAACAAAGAAAGGAAUGCAACAGAACACAUACCCCUGGGCCUU UUAUUUU | 334 |

**[Table 08]**

| **Label** | **Target** | **PAM (5' to 3')** | **Engineered Cas12f1 guide RNA sequence (5' to 3')** | **SEQ ID NOs** |
|---|---|---|---|---|
| Example 1.3.10 | Intergenic-22 | TTTA | ACCGCUUCACCAUUAGUGAGUGAAGGUGGGCUGCUUGCAUCAGCCUAAUGUCGAGAAGUGCUUUCUUCGGAAAGUAACCCUCGA AACAAAUUCAUUUGAAAGAAUGAAGGAAUGCAACAGAACACAUACCCCUGGGCCUUUUAUUUU | 335 |
| Example 1.3.11 | Intergenic-22 | TTTA | ACCGCUUCACCAAAAGCUGUCCCUUAGGGGAUUAGAACUUGAGUGAAGGUGGGCUGCUUGCAUCAGCCUAAUGUCGAGAAGUGC UUUCUUCGGAAAGUAACCCUCGAAACAAAGAAAGGAAUGCAACAGAACACAUACCCCUGGGCCUUUUAUUUU | 336 |
| Example 1.3.12 | Intergenic-22 | TTTA | CUUCACUGAUAAAGUGGAGAACCGCUUCACCAUUAGUGAGUGAAGGUGGGCUGCUUGCAUCAGCCUAAUGUCGAGAAGUGCUUU CUUCGGAAAGUAACCCUCGAAACAAAGAAAGGAAUGCAACAGAACACAUACCCCUGGGCCUUUUAUUUU | 337 |
| Example 1.3.13 | Intergenic-22 | TTTA | ACCGCUUCACCAUUAGUGAGUGAAGGUGGGCUGCUUGCAUCAGCCUAAUGUCGAGAAGUGCUUUCUUCGGAAAGUAACCCUCGA AACAAAGAAAGGAAUGCAACAGAACACAUACCCCUGGGCCUUUUAUUUU | 338 |

Here, for each target sequence,
1) Comparative Example 1.n.1 is a single guide RNA (SEQ ID NO: 8) in which a naturally occurring Cas12f1 tracrRNA and a naturally occurring Cas12f1 crRNA are linked via 5'-GAAA-3',
2) Comparative Example 1.n.2 is a single guide RNA (SEQ ID NO: 9) having a naturally occurring scaffold region, a spacer, and a U-rich tail represented by 5'-UUUUAUUUU-3',
3) Examples 1.n.1 to 1.n.3 are each an engineered Cas12f1 guide RNA with an engineered scaffold region having a modified first region, a spacer, and a U-rich tail represented by 5'-UUUUAUUUU-3',
4) Examples 1.n.4 to 1.n.6 are each an engineered Cas12f1 guide RNA with an engineered scaffold region having a modified second region, a spacer, and a U-rich tail represented by 5'-UUUUAUUUU-3',
5) Examples 1.n.7 to 1.n.9 are each an engineered Cas12f1 guide RNA with an engineered scaffold region having modified fourth and fifth regions, a spacer, and a U-rich tail represented by 5'-UUUUAUUUU-3',
6) Example 1.n.10 is an engineered Cas12f1 guide RNA with an engineered scaffold region having a modified first region and second region, a spacer, and a U-rich tail represented by 5'-UUUUAUUUU-3',
7) Example 1.n.11 is an engineered Cas12f1 guide RNA with an engineered scaffold region having a modified first region and fourth and fifth regions, a spacer, and a U-rich tail represented by 5'-UUUUAUUUU-3',
8) Example 1.n.12 is an engineered Cas12f1 guide RNA with an engineered scaffold region having a modified second region and fourth and fifth regions, a spacer, and a U-rich tail represented by 5'-UUUUAUUUU-3', and
9) Example 1.n.13 is an engineered Cas12f1 guide RNA with an engineered scaffold region having a modified first region, a modified second region, and modified fourth and fifth regions, a spacer, and a U rich tail represented by 5'-UUUUAUUUU-3'.

Here, n is 1, 2, or 3 depending on the target sequence, wherein a case where n is 1 represents Target 1 (DY2), a case where n is 2 represents Target 2 (DY10), and a case where n is 3 represents Target 3 (Intergenic-22).

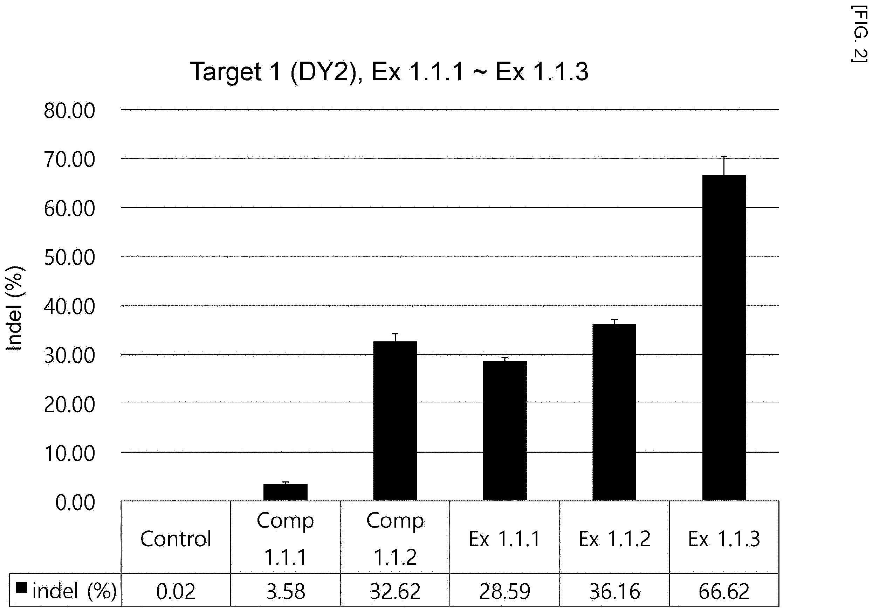

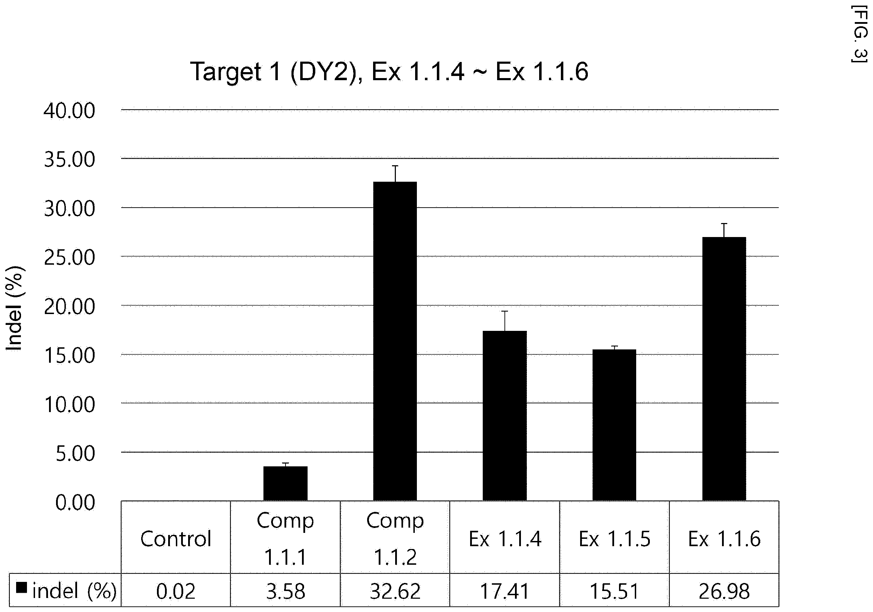

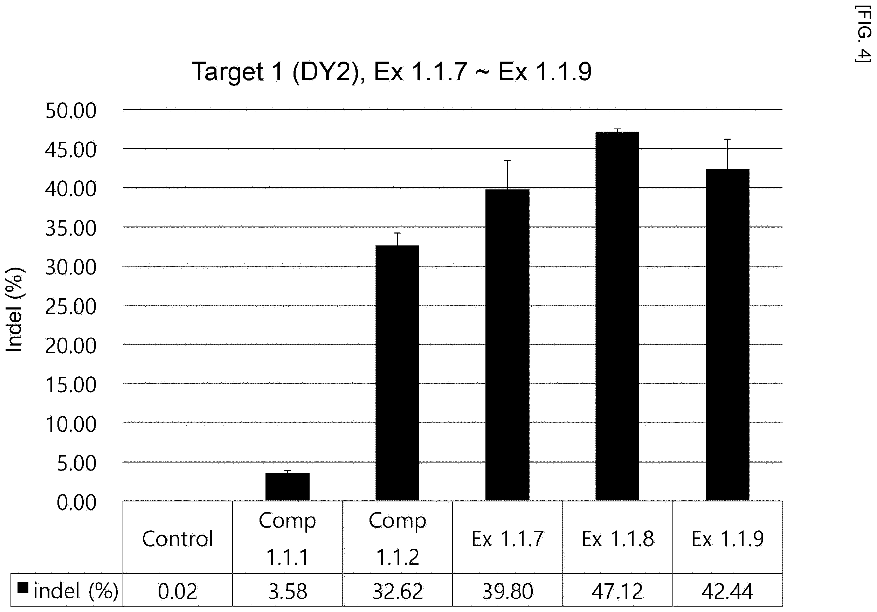

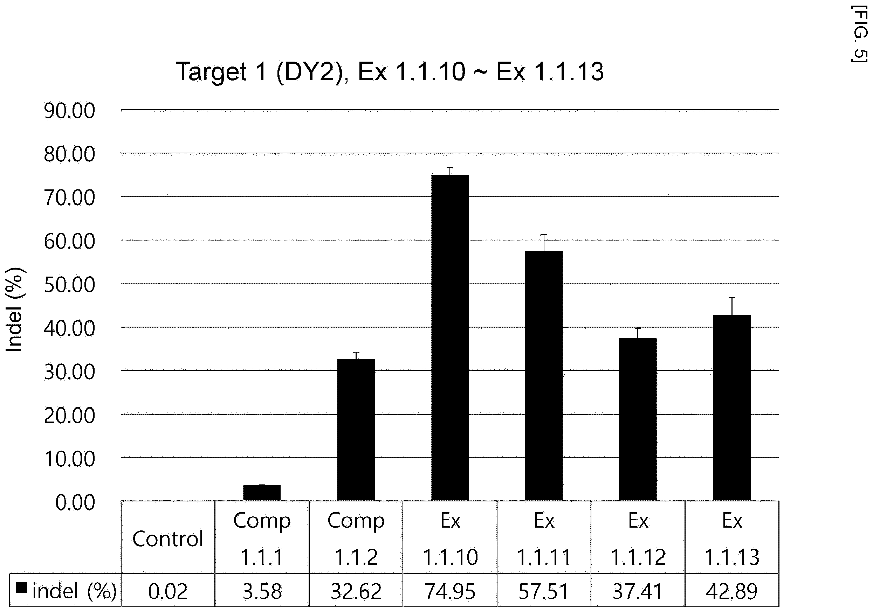

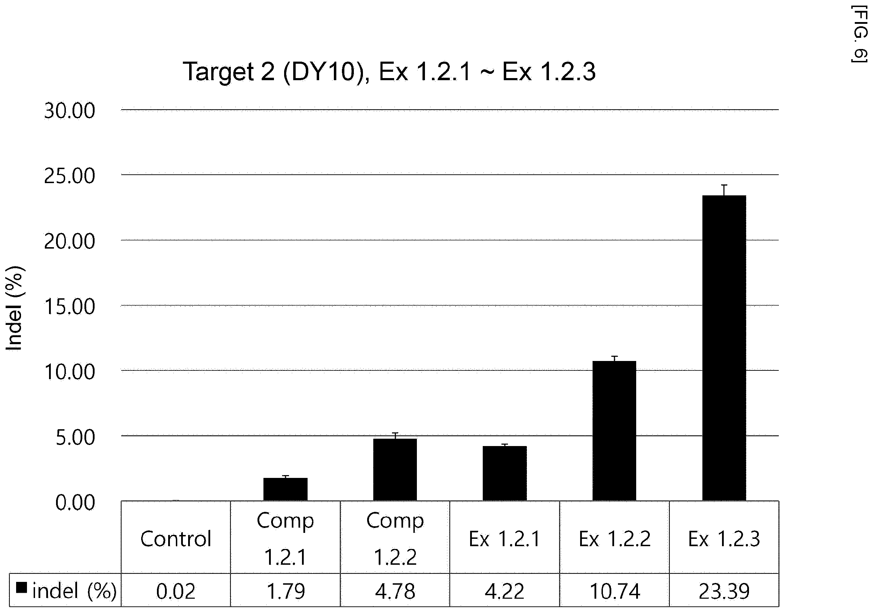

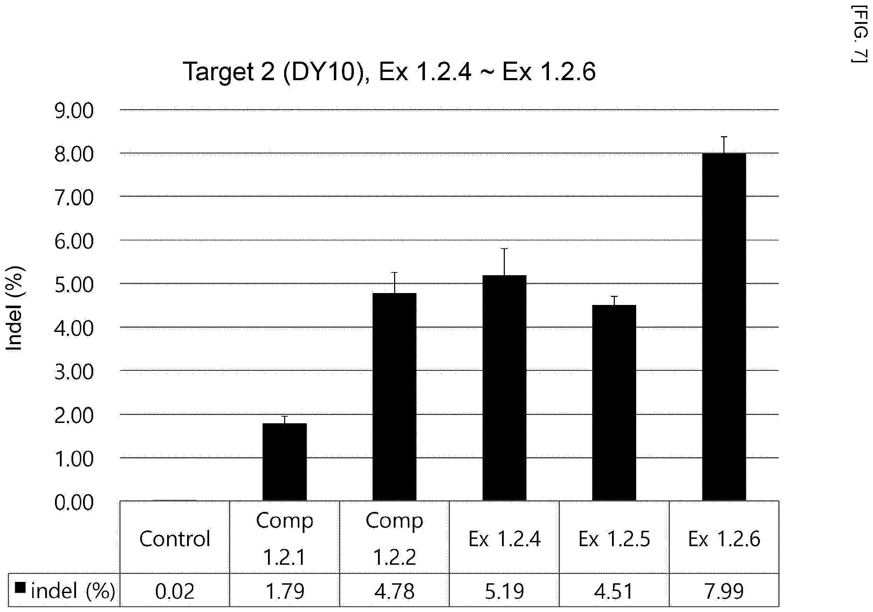

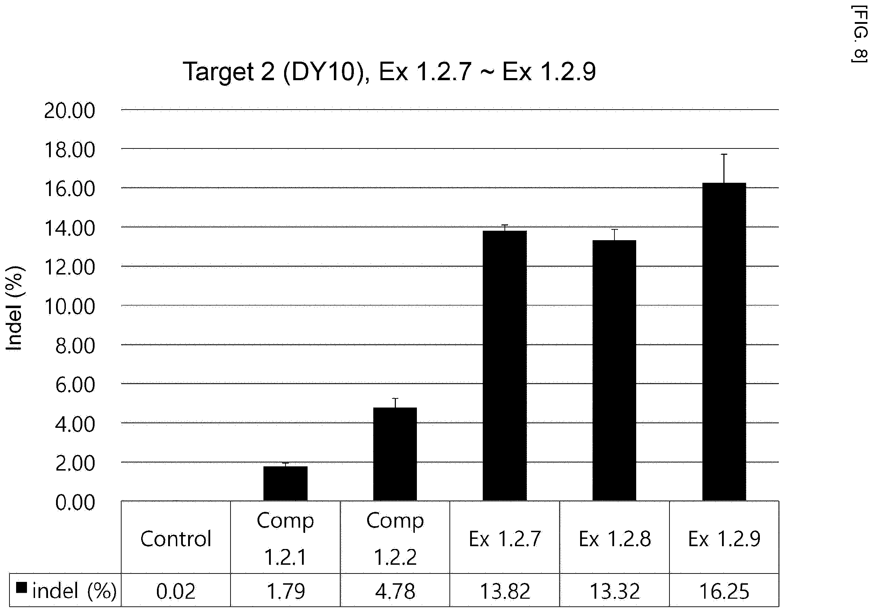

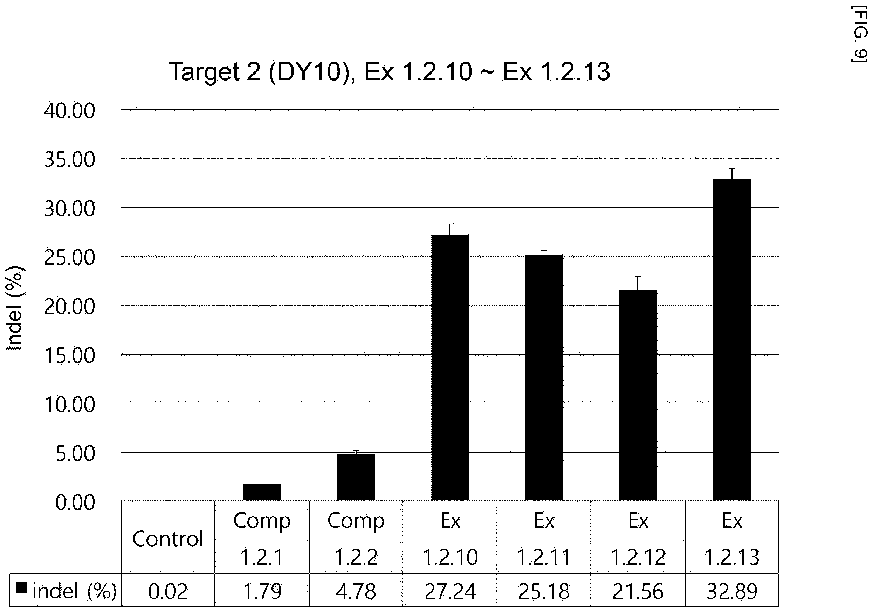

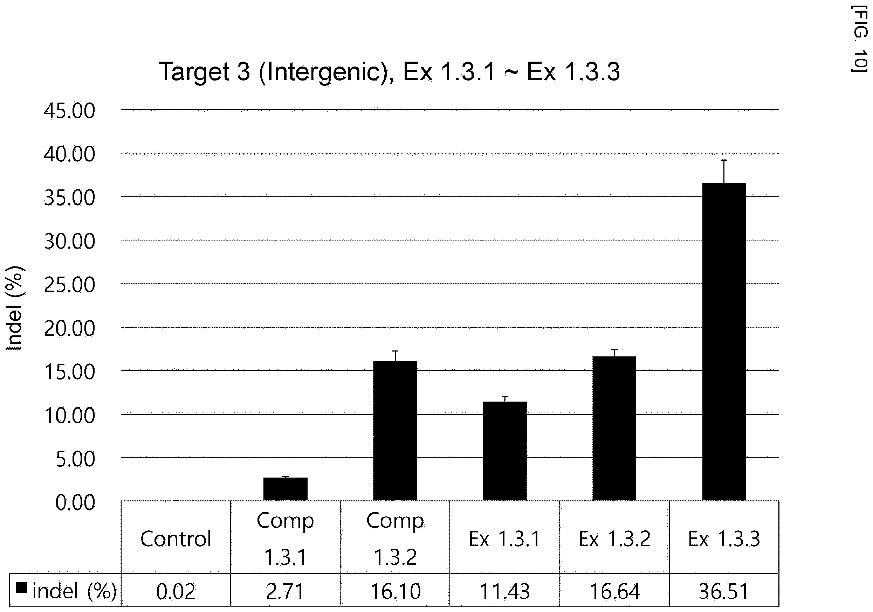

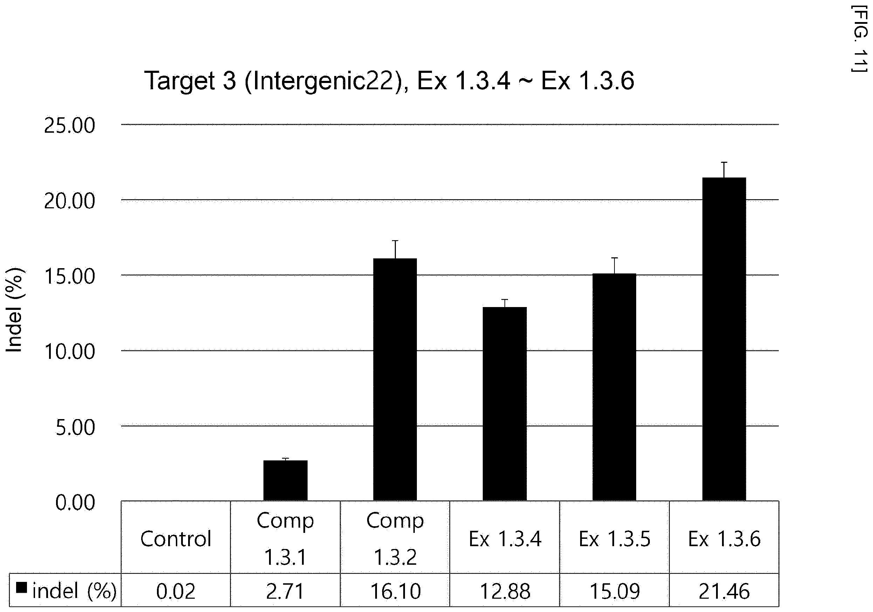

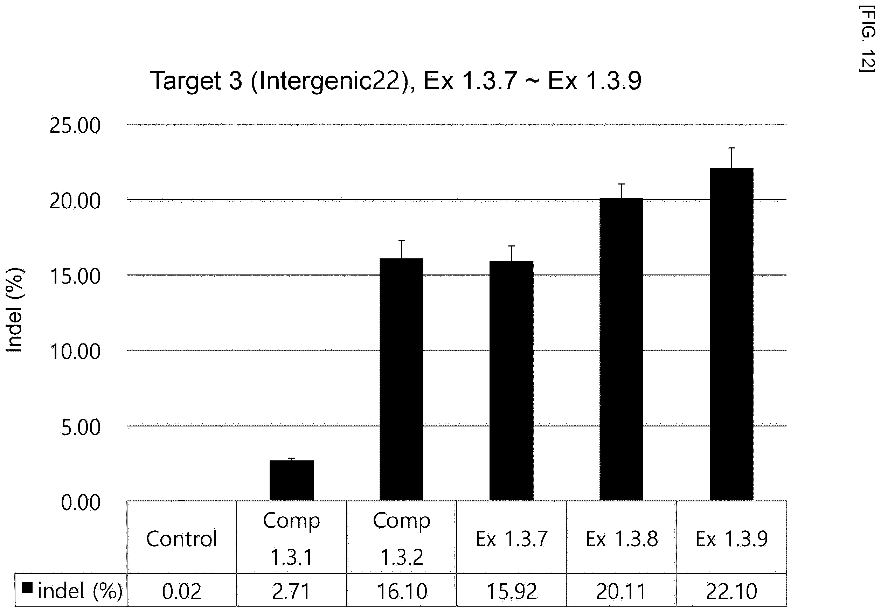

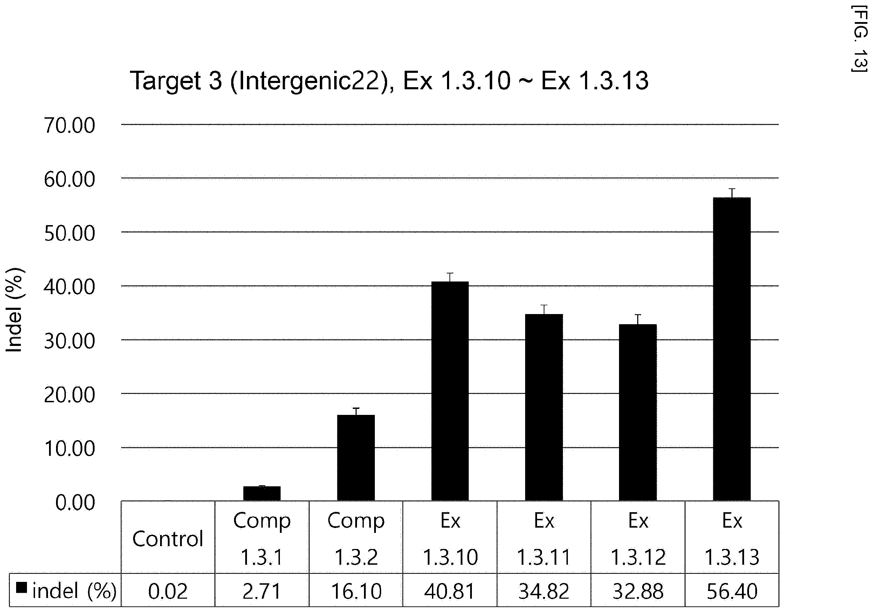

The vector constructed in each Example was transfected into HEK293 T cells according to Experimental Example 1.3, and indel generation efficiency was measured by Experimental Examples 1.4 to 1.5. The results were analyzed by Experimental Example 1.6 and are shown in FIGS. 2 to 13.

### Experimental Example 3. Indel efficiency comparison 2 between engineered CRISPR/Cas12f1 systems

### Experimental Example 3.1. Indel efficiency comparison 2-1 between engineered CRISPR/Cas12f1 systems

To measure indel efficiency of an engineered CRISPR/Cas12f1 system using an engineered Cas12f1 guide RNA, each of the examples was prepared by Experimental Examples 1.1 to 1.2. The target sequences used for the experiments are shown in Table 09 below.

**[Table 09]**

| **Target Label** | **protospacer sequence (S' to 3')** | **SEQ ID NO** |
|---|---|---|
| DY2 | CACACACACAGTGGGCTACC | 339 |
| DY10 | CATCCCCAGGACACACACAC | 340 |

The sequences of the engineered Cas12f1 guide RNAs for the respective examples are shown in Tables 10 to 13 below.

**[Table 10]**

| **Label** | **Target** | **PAM (5' to 3')** | **Engineered Cas12f1 guide RNA sequence (5' to 3')** | **SEQ ID NOs** |
|---|---|---|---|---|
| Ex 2.1.1 | DY2 | TTTG | ACCGCUUCAUUAGGAAGGUGGGCUGCUUGCAUCAGCCUAAUGUCGAGAAGUGCUUUCUUCGGAAAGUAAC CCUCGAAACAAAGAAAGGAAUGCAACCACACACACAGUGGGCUACCUUUUAUUUU | 488 |
| Ex 2.1.2 | DY2 | TTTG | ACCGCUUCAUUAGUGAAGGUGGGCUGCUUGCAUCAGCCUAAUGUCGAGAAGUGCUUUCUUCGGAAAGUAA CCCUCGAAACAAAGAAAGGAAUGCAACCACACACACAGUGGGCUACCUUUUAUUUU | 489 |
| Ex 2.1.3 | DY2 | TTTG | ACCGCUUCACUUAGGUGAAGGUGGGCUGCUUGCAUCAGCCUAAUGUCGAGAAGUGCUUUCUUCGGAAAGU AACCCUCGAAACAAAGAAAGGAAUGCAACCACACACACAGUGGGCUACCUUUUAUUUU | 490 |
| Ex 21.4 | DY2 | TTTG | ACCGCUUCACUUAGAGUGAAGGUGGGCUGCUUGCAUCAGCCUAAUGUCGAGAAGUGCUUUCUUCGGAAAG UAACCCUCGAAACAAAGAAAGGAAUGCAACCACACACACAGUGGGCUACCUUUUAUUUU | 491 |
| Ex 21.5 | DY2 | TTTG | ACCGCUUCACCUUAGGAGUGAAGGUGGGCUGCUUGCAUCAGCCUAAUGUCGAGAAGUGCUUUCUUCGGAA AGUAACCCUCGAAACAAAGAAAGGAAUGCAACCACACACACAGUGGGCUACCUUUUAUUUU | 492 |
| Ex 2.1.6 | DY2 | TTTG | ACCGCUUCACCAAUUAGUUGAGUGAAGGUGGGCUGCUUGCAUCAGCCUAAUGUCGAGAAGUGCUUUCUUC GGAAAGUAACCCUCGAAACAAAGAAAGGAAUGCAACCACACACACAGUGGGCUACCUUUUAUUUU | 493 |
| Ex 2.1.7 | DY2 | TTTG | ACCGCUUCACCAAAUUAGCUUGAGUGAAGGUGGGCUGCUUGCAUCAGCCUAAUGUCGAGAAGUGCUUUCU UCGGAAAGUAACCCUCGAAACAAAGAAAGGAAUGCAACCACACACACAGUGGGCUACCUUUUAUUUU | 494 |

**[Table 11]**

| **Label** | **Target** | **PAM (5' to 3')** | **Engineered Cas12f1 guide RNA sequence (5' to 3')** | **SEQ ID NOs** |
|---|---|---|---|---|
| Ex 2.1.8 | DY2 | TTTG | ACCGCUUCACCAAAAUUAGACUUGAGUGAAGGUGGGCUGCUUGCAUCAGCCUAAUGUCGAGAAGUGCUUU CUUCGGAAAGUAACCCUCGAAACAAAGAAAGGAAUGCAACCACACACACAGUGGGCUACCUUUUAUUUU | 495 |
| Ex 2.1.9 | DY2 | TTTG | ACCGCUUCACCAAAAGUUAGAACUUGAGUGAAGGUGGGCUGCUUGCAUCAGCCUAAUGUCGAGAAGUGCU UUCUUCGGAAAGUAACCCUCGAAACAAAGAAAGGAAUGCAACCACACACACAGUGGGCUACCUUUUAUUUU | 496 |
| Ex 2.1.10 | DY2 | TTTG | ACCGCUUCACCAAAAGCUUAGGAACUUGAGUGAAGGUGGGCUGCUUGCAUCAGCCUAAUGUCGAGAAGUG CUUUCUUCGGAAAGUAACCCUCGAAACAAAGAAAGGAAUGCAACCACACACACAGUGGGCUACCUUUUAUU UU | 497 |
| Ex 2.1.11 | DY2 | TTTG | ACCGCUUCACCAAAAGCUUUAGAGAACUUGAGUGAAGGUGGGCUGCUUGCAUCAGCCUAAUGUCGAGAAG UGCUUUCUUCGGAAAGUAACCCUCGAAACAAAGAAAGGAAUGCAACCACACACACAGUGGGCUACCUUUUA UUUU | 498 |
| Ex 2.1.12 | DY2 | TTTG | ACCGCUUCACCAAAAGCUUUAGUUAGAACUUGAGUGAAGGUGGGCUGCUUGCAUCAGCCUAAUGUCGAGA AGUGCUUUCUUCGGAAAGUAACCCUCGAAACAAAGAAAGGAAUGCAACCACACACACAGUGGGCUACCUUU UAUUUU | 499 |
| Ex 2.1.13 | DY2 | TTTG | ACCGCUUCACCAAAAGCUGUUUAGAUUAGAACUUGAGUGAAGGUGGGCUGCUUGCAUCAGCCUAAUGUCG AGAAGUGCUUUCUUCGGAAAGUAACCCUCGAAACAAAGAAAGGAAUGCAACCACACACACAGUGGGCUACC UUUUAUUUU | 500 |
| Ex 2.1.14 | DY2 | TTTG | ACCGCUUCACCAAAAGCUGUCUUAGGAUUAGAACUUGAGUGAAGGUGGGCUGCUUGCAUCAGCCUAAUGU CGAGAAGUGCUUUCUUCGGAAAGUAACCCUCGAAACAAAGAAAGGAAUGCAACCACACACACAGUGGGCUA CCUUUUAUUUU | 501 |
| Ex 2.1.15 | DY2 | TTTG | ACCGCUUCACCAAAAGCUGUCCCUUAGGGGAUUAGAACUUGAGUGAAGGUGGGCUGCUUGCAUCAGCCUA AUGUCGAGAAGUGCUUUCUUCGGAAAGUAACCCUCGAAACAAAGAAAGGAAUGCAACCACACACACAGUGG GCUACCUUUUAUUUU | 502 |

**[Table 12]**

| **Label** | **Target** | **PAM (5' to 3')** | **Engineered Cas12f1 guide RNA sequence (5' to 3')** | **SEQ ID NOs** |
|---|---|---|---|---|
| Ex 2.2.1 | DY10 | TTTG | ACCGCUUCAUUAGGAAGGUGGGCUGCUUGCAUCAGCCUAAUGUCGAGAAGUGCUUUCUUCGGAAAGUAAC CCUCGAAACAAAGAAAGGAAUGCAACCAUCCCCAGGACACACACACUUUUAUUUU | 503 |
| Ex 2.2.2 | DY10 | TTTG | ACCGCUUCAUUAGUGAAGGUGGGCUGCUUGCAUCAGCCUAAUGUCGAGAAGUGCUUUCUUCGGAAAGUAA CCCUCGAAACAAAGAAAGGAAUGCAACCAUCCCCAGGACACACACACUUUUAUUUU | 504 |
| Ex 2.2.3 | DY10 | TTTG | ACCGCUUCACUUAGGUGAAGGUGGGCUGCUUGCAUCAGCCUAAUGUCGAGAAGUGCUUUCUUCGGAAAGU AACCCUCGAAACAAAGAAAGGAAUGCAACCAUCCCCAGGACACACACACUUUUAUUUU | 505 |
| Ex 22.4 | DY10 | TTTG | ACCGCUUCACUUAGAGUGAAGGUGGGCUGCUUGCAUCAGCCUAAUGUCGAGAAGUGCUUUCUUCGGAAAG UAACCCUCGAAACAAAGAAAGGAAUGCAACCAUCCCCAGGACACACACACUUUUAUUUU | 506 |
| Ex 2.2.5 | DY10 | TTTG | ACCGCUUCACCUUAGGAGUGAAGGUGGGCUGCUUGCAUCAGCCUAAUGUCGAGAAGUGCUUUCUUCGGAA AGUAACCCUCGAAACAAAGAAAGGAAUGCAACCAUCCCCAGGACACACACACUUUUAUUUU | 507 |
| Ex 2.2.6 | DY10 | TTTG | ACCGCUUCACCAAUUAGUUGAGUGAAGGUGGGCUGCUUGCAUCAGCCUAAUGUCGAGAAGUGCUUUCUUC GGAAAGUAACCCUCGAAACAAAGAAAGGAAUGCAACCAUCCCCAGGACACACACACUUUUAUUUU | 508 |
| Ex 2.2.7 | DY10 | TTTG | ACCGCUUCACCAAAUUAGCUUGAGUGAAGGUGGGCUGCUUGCAUCAGCCUAAUGUCGAGAAGUGCUUUCU UCGGAAAGUAACCCUCGAAACAAAGAAAGGAAUGCAACCAUCCCCAGGACACACACACUUUUAUUUU | 509 |

**[Table 13]**

| **Label** | **Target** | **PAM (5' to 3')** | **Engineered Cas12f1 guide RNA sequence (5' to 3')** | **SEQ ID NOs** |
|---|---|---|---|---|
| Ex 2.2.8 | DY10 | TTTG | ACCGCUUCACCAAAAUUAGACUUGAGUGAAGGUGGGCUGCUUGCAUCAGCCUAAUGUCGAGAAGUGCUUU CUUCGGAAAGUAACCCUCGAAACAAAGAAAGGAAUGCAACCAUCCCCAGGACACACACACUUUUAUUUU | 510 |
| Ex 2.2.9 | DY10 | TTTG | ACCGCUUCACCAAAAGUUAGAACUUGAGUGAAGGUGGGCUGCUUGCAUCAGCCUAAUGUCGAGAAGUGCU UUCUUCGGAAAGUAACCCUCGAAACAAAGAAAGGAAUGCAACCAUCCCCAGGACACACACACUUUUAUUU | 511 |
| Ex 2.2.10 | DY10 | TTTG | ACCGCUUCACCAAAAGCUUAGGAACUUGAGUGAAGGUGGGCUGCUUGCAUCAGCCUAAUGUCGAGAAGUG CUUUCUUCGGAAAGUAACCCUCGAAACAAAGAAAGGAAUGCAACCAUCCCCAGGACACACACACUUUUAUUU U | 512 |
| Ex 2.2.1 1 | DY10 | TTTG | ACCGCUUCACCAAAAGCUUUAGAGAACUUGAGUGAAGGUGGGCUGCUUGCAUCAGCCUAAUGUCGAGAAG UGCUUUCUUCGGAAAGUAACCCUCGAAACAAAGAAAGGAAUGCAACCAUCCCCAGGACACACACACUUUUAU UUU | 513 |
| Ex 2.2.12 | DY10 | TTTG | ACCGCUUCACCAAAAGCUUUAGUUAGAACUUGAGUGAAGGUGGGCUGCUUGCAUCAGCCUAAUGUCGAGA AGUGCUUUCUUCGGAAAGUAACCCUCGAAACAAAGAAAGGAAUGCAACCAUCCCCAGGACACACACACUUUU AUUUU | 514 |
| Ex 2.2.13 | DY10 | TTTG | ACCGCUUCACCAAAAGCUGUUUAGAUUAGAACUUGAGUGAAGGUGGGCUGCUUGCAUCAGCCUAAUGUCG AGAAGUGCUUUCUUCGGAAAGUAACCCUCGAAACAAAGAAAGGAAUGCAACCAUCCCCAGGACACACACACU UUUAUUUU | 515 |
| Ex 2.2.14 | DY10 | TTTG | ACCGCUUCACCAAAAGCUGUCUUAGGAUUAGAACUUGAGUGAAGGUGGGCUGCUUGCAUCAGCCUAAUGU CGAGAAGUGCUUUCUUCGGAAAGUAACCCUCGAAACAAAGAAAGGAAUGCAACCAUCCCCAGGACACACACA CUUUUAUUUU | 516 |
| Ex 2.2.15 | DY10 | TTTG | ACCGCUUCACCAAAAGCUGUCCCUUAGGGGAUUAGAACUUGAGUGAAGGUGGGCUGCUUGCAUCAGCCUA AUGUCGAGAAGUGCUUUCUUCGGAAAGUAACCCUCGAAACAAAGAAAGGAAUGCAACCAUCCCCAGGACAC ACACACUUUUAUUUU | 517 |

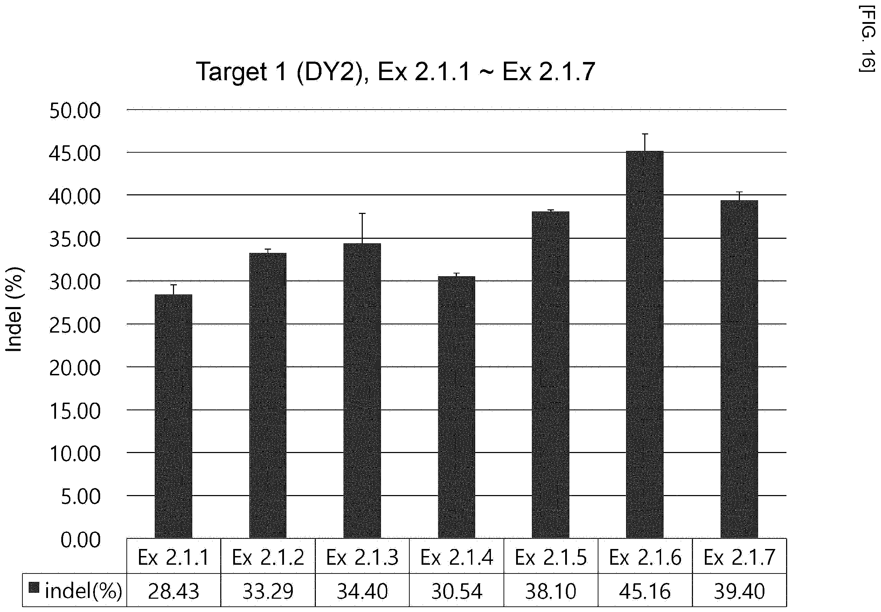

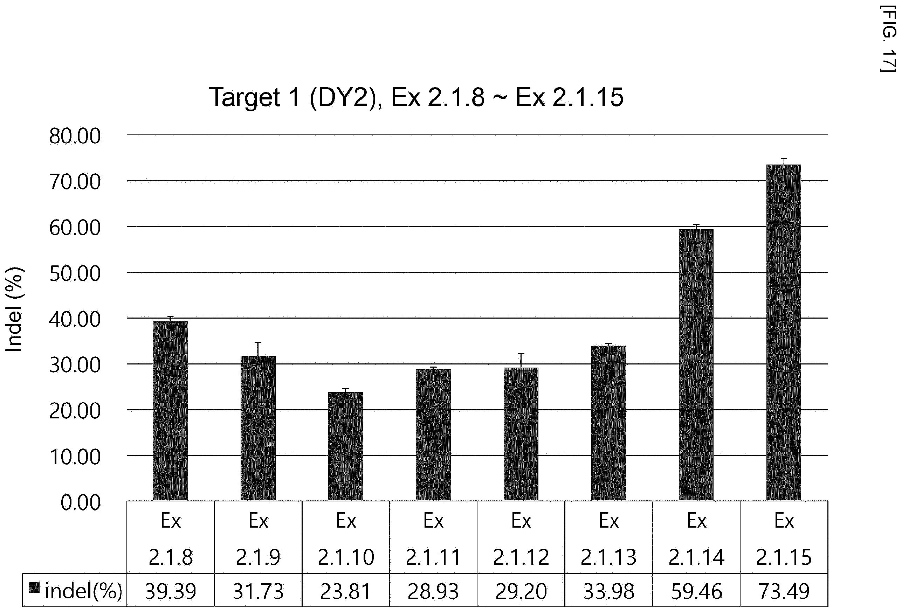

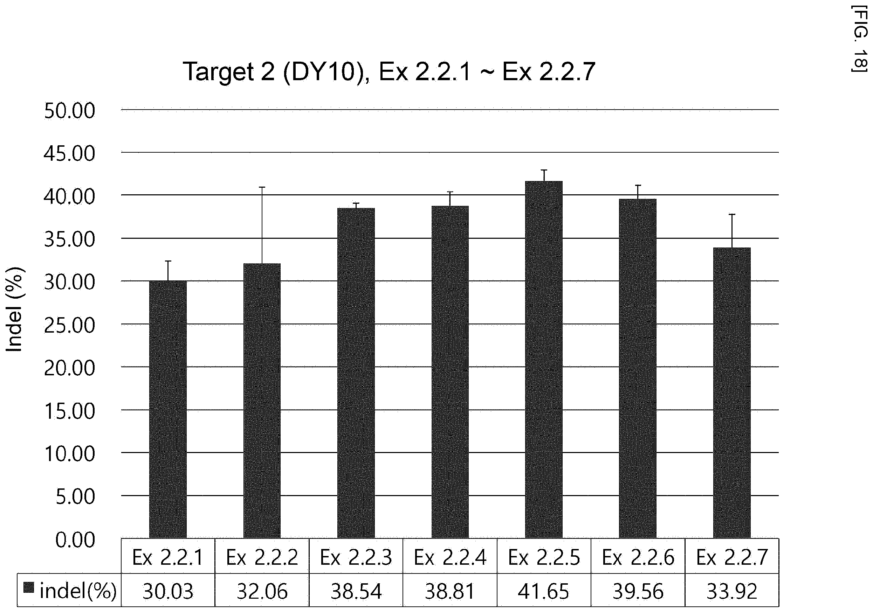

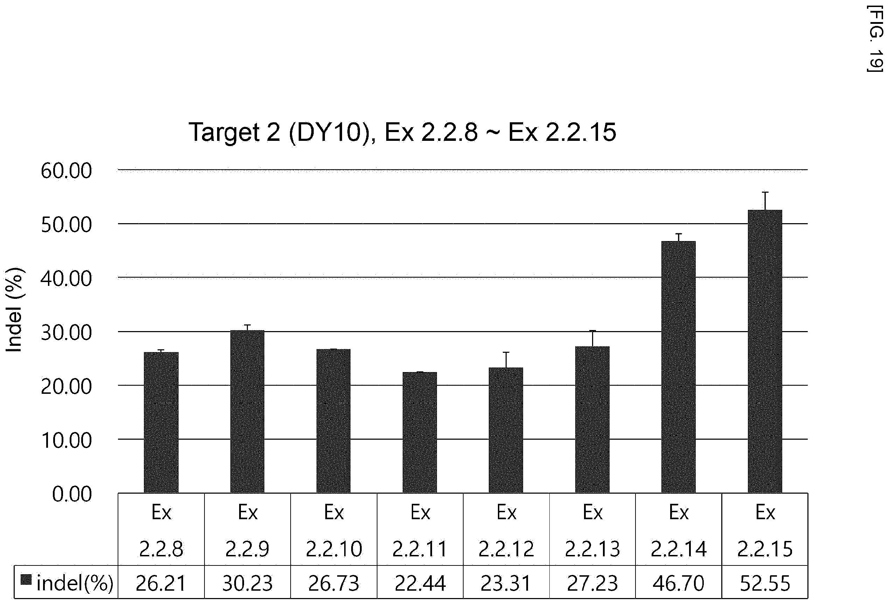

The vector constructed in each Example was transfected into HEK293 T cells according to Experimental Example 1.3, and indel generation efficiency was measured by Experimental Examples 1.4 to 1.5. The results were analyzed by Experimental Example 1.6 and are shown in FIGS. 16 to 19.

### Experimental Example 3.2. Indel efficiency comparison 2-2 between engineered CRISPR/Cas12f1 systems

To measure indel efficiency of an engineered CRISPR/Cas12f1 system using an engineered Cas12f1 guide RNA, each of the examples was prepared by Experimental Examples 1.1 to 1.2. The target sequences used for the experiments are as shown in Table 09.

The sequences of the engineered Cas12f1 guide RNAs used for the respective examples are shown in Tables 14 to 17 below.

**[Table 14]**

| **Label** | **Target** | **PAM (5' to 3')** | **Engineered Cas12f1 guide RNA sequence (5' to 3')** | **SEQ ID NOs** |
|---|---|---|---|---|
| Ex 3.1.1 | DY2 | TTTG | ACCGCUUUUAGAAGGUGGGCUGCUUGCAUCAGCCUAAUGUCGAGAAGUGCUUUCUUCGGAAAGUAACCCU CGAAACAAAGAAAGGAAUGCAACCACACACACAGUGGGCUACCUUUUAUUUU | 518 |
| Ex 3.1.2 | DY2 | TTTG | ACCGCUUUAGAGGUGGGCUGCUUGCAUCAGCCUAAUGUCGAGAAGUGCUUUCUUCGGAAAGUAACCCUCG AAACAAAGAAAGGAAUGCAACCACACACACAGUGGGCUACCUUUUAUUUU | 519 |
| Ex 3.1.3 | DY2 | TTTG | ACCGCUUAGUGGUGGGCUGCUUGCAUCAGCCUAAUGUCGAGAAGUGCUUUCUUCGGAAAGUAACCCUCGA AACAAAGAAAGGAAUGCAACCACACACACAGUGGGCUACCUUUUAUUUU | 520 |
| Ex 3.1.4 | DY2 | TTTG | ACCGUUAGGUGGGCUGCUUGCAUCAGCCUAAUGUCGAGAAGUGCUUUCUUCGGAAAGUAACCCUCGAAAC AAAGAAAGGAAUGCAACCACACACACAGUGGGCUACCUUUUAUUUU | 521 |
| Ex 3.1.5 | DY2 | TTTG | ACCUUAGGUGGGCUGCUUGCAUCAGCCUAAUGUCGAGAAGUGCUUUCUUCGGAAAGUAACCCUCGAAACA AAGAAAGGAAUGCAACCACACACACAGUGGGCUACCUUUUAUUUU | 522 |
| Ex 3.1.6 | DY2 | TTTG | ACUUAGUGGGCUGCUUGCAUCAGCCUAAUGUCGAGAAGUGCUUUCUUCGGAAAGUAACCCUCGAAACAAA GAAAGGAAUGCAACCACACACACAGUGGGCUACCUUUUAUUUU | 523 |

**[Table 15]**

| **Label** | **Target** | **PAM (5' to 3')** | **Engineered Cas12f1 guide RNA sequence (5' to 3')** | **SEQ ID NOs** |
|---|---|---|---|---|
| Ex 3.1.7 | DY2 | TTTG | ACUUAGGGGCUGCUUGCAUCAGCCUAAUGUCGAGAAGUGCUUUCUUCGGAAAGUAACCCUCGAAACAAAG AAAGGAAUGCAACCACACACACAGUGGGCUACCUUUUAUUUU | 524 |
| Ex 3.1.8 | DY2 | TTTG | AUUAGGGCUGCUUGCAUCAGCCUAAUGUCGAGAAGUGCUUUCUUCGGAAAGUAACCCUCGAAACAAAGAA AGGAAUGCAACCACACACACAGUGGGCUACCUUUUAUUUU | 525 |
| Ex 3.1.9 | DY2 | TTTG | AGGCUGCUUGCAUCAGCCUAAUGUCGAGAAGUGCUUUCUUCGGAAAGUAACCCUCGAAACAAAGAAAGGAA UGCAACCACACACACAGUGGGCUACCUUUUAUUUU | 526 |
| Ex 3.1.10 | DY2 | TTTG | GCUGCUUGCAUCAGCCUAAUGUCGAGAAGUGCUUUCUUCGGAAAGUAACCCUCGAAACAAAGAAAGGAAU GCAACCACACACACAGUGGGCUACCUUUUAUUUU | 527 |
| Ex 3.1.11 | DY2 | TTTG | ACCGCUUCACUUAGAGUGAAGGUGGGCUGCUUGCAUCAGCCUAAUGUCGAGAAGUGCUUUCUUCGGAAAG UAACCCUCGAAACAAAGAAAGGAAUGCAACCACACACACAGUGGGCUACCUUUUAUUUU | 528 |
| Ex 3.1.12 | DY2 | TTTG | ACCGCUUCACCAAAAGCUGUCCCUUAGGGGAUUAGAACUUGAGUGAAGGUGGGCUGCUUGCAUCAGCCUA AUGUCGAGAAGUGCUUUCUUCGGAAAGUAACCCUCGAAACAAAGAAAGGAAUGCAACCACACACACAGUGG GCUACCUUUUAUUUU | 529 |

**[Table 16]**

| **Label** | **Target** | **PAM (5' to 3')** | **Engineered Cas12f1 guide RNA sequence (5' to 3')** | **SEQ ID NOs** |
|---|---|---|---|---|
| Ex 3.2.1 | DY10 | TTTG | ACCGCUUUUAGAAGGUGGGCUGCUUGCAUCAGCCUAAUGUCGAGAAGUGCUUUCUUCGGAAAGUAACCCU CGAAACAAAGAAAGGAAUGCAACCAUCCCCAGGACACACACACUUUUAUUUU | 530 |
| Ex 3.2.2 | DY10 | TTTG | ACCGCUUUAGAGGUGGGCUGCUUGCAUCAGCCUAAUGUCGAGAAGUGCUUUCUUCGGAAAGUAACCCUCG AAACAAAGAAAGGAAUGCAACCAUCCCCAGGACACACACACUUUUAUUUU | 531 |
| Ex 3.2.3 | DY10 | TTTG | ACCGCUUAGUGGUGGGCUGCUUGCAUCAGCCUAAUGUCGAGAAGUGCUUUCUUCGGAAAGUAACCCUCGA AACAAAGAAAGGAAUGCAACCAUCCCCAGGACACACACACUUUUAUUUU | 532 |
| Ex 3.2.4 | DY10 | TTTG | ACCGUUAGGUGGGCUGCUUGCAUCAGCCUAAUGUCGAGAAGUGCUUUCUUCGGAAAGUAACCCUCGAAAC AAAGAAAGGAAUGCAACCAUCCCCAGGACACACACACUUUUAUUUU | 533 |
| Ex 3.2.5 | DY10 | TTTG | ACCUUAGGUGGGCUGCUUGCAUCAGCCUAAUGUCGAGAAGUGCUUUCUUCGGAAAGUAACCCUCGAAACA AAGAAAGGAAUGCAACCAUCCCCAGGACACACACACUUUUAUUUU | 534 |
| Ex 3.2.6 | DY10 | TTTG | ACUUAGUGGGCUGCUUGCAUCAGCCUAAUGUCGAGAAGUGCUUUCUUCGGAAAGUAACCCUCGAAACAAA GAAAGGAAUGCAACCAUCCCCAGGACACACACACUUUUAUUUU | 535 |

**[Table 17]**

| **Label** | **Target** | **PAM (5' to 3')** | **Engineered Cas12f1 guide RNA sequence (5' to 3')** | **SEQ ID NOs** |
|---|---|---|---|---|
| Ex 3.2.7 | DY10 | TTTG | ACUUAGGGGCUGCUUGCAUCAGCCUAAUGUCGAGAAGUGCUUUCUUCGGAAAGUAACCCUCGAAACAAAG AAAGGAAUGCAACCAUCCCCAGGACACACACACUUUUAUUUU | 536 |
| Ex 3.2.8 | DY10 | TTTG | AUUAGGGCUGCUUGCAUCAGCCUAAUGUCGAGAAGUGCUUUCUUCGGAAAGUAACCCUCGAAACAAAGAA AGGAAUGCAACCAUCCCCAGGACACACACACUUUUAUUUU | 537 |
| Ex 3.2.9 | DY10 | TTTG | AGGCUGCUUGCAUCAGCCUAAUGUCGAGAAGUGCUUUCUUCGGAAAGUAACCCUCGAAACAAAGAAAGGAA UGCAACCAUCCCCAGGACACACACACUUUUAUUUU | 538 |
| Ex 3.2.10 | DY10 | TTTG | GCUGCUUGCAUCAGCCUAAUGUCGAGAAGUGCUUUCUUCGGAAAGUAACCCUCGAAACAAAGAAAGGAAU GCAACCAUCCCCAGGACACACACACUUUUAUUUU | 539 |
| Ex 3.2.11 | DY10 | TTTG | ACCGCUUCACUUAGAGUGAAGGUGGGCUGCUUGCAUCAGCCUAAUGUCGAGAAGUGCUUUCUUCGGAAAG UAACCCUCGAAACAAAGAAAGGAAUGCAACCAUCCCCAGGACACACACACUUUUAUUUU | 540 |
| Ex 3.2.12 | DY10 | TTTG | ACCGCUUCACCAAAAGCUGUCCCUUAGGGGAUUAGAACUUGAGUGAAGGUGGGCUGCUUGCAUCAGCCUA AUGUCGAGAAGUGCUUUCUUCGGAAAGUAACCCUCGAAACAAAGAAAGGAAUGCAACCAUCCCCAGGACAC ACACACUUUUAUUUU | 541 |

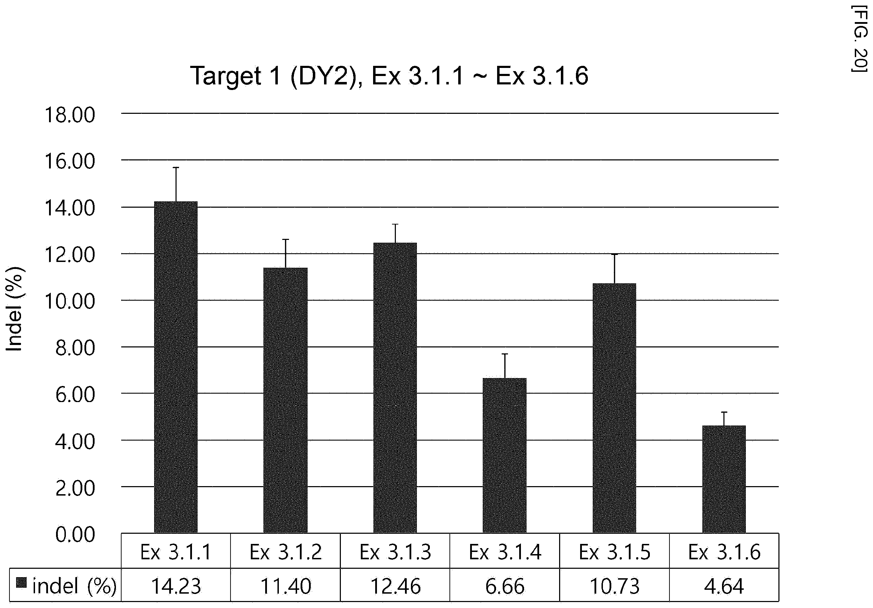

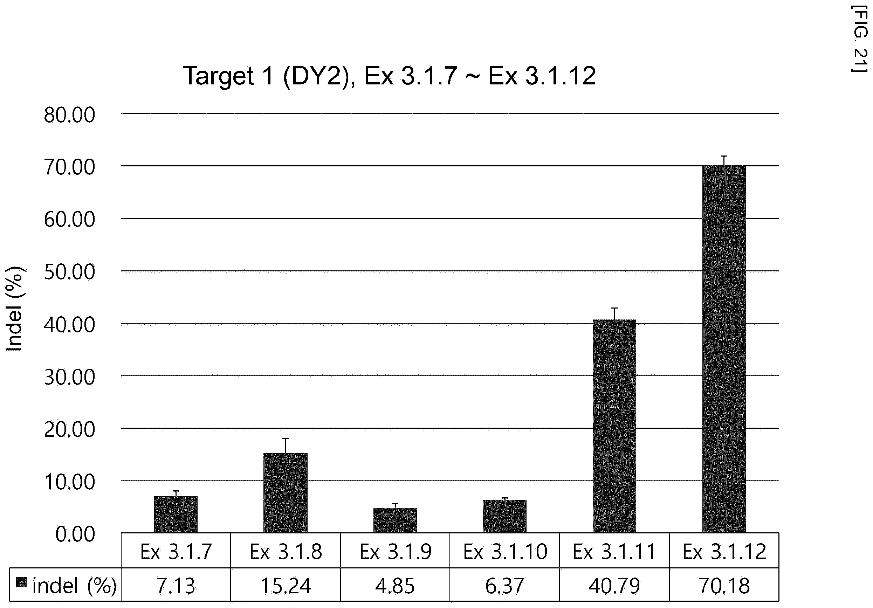

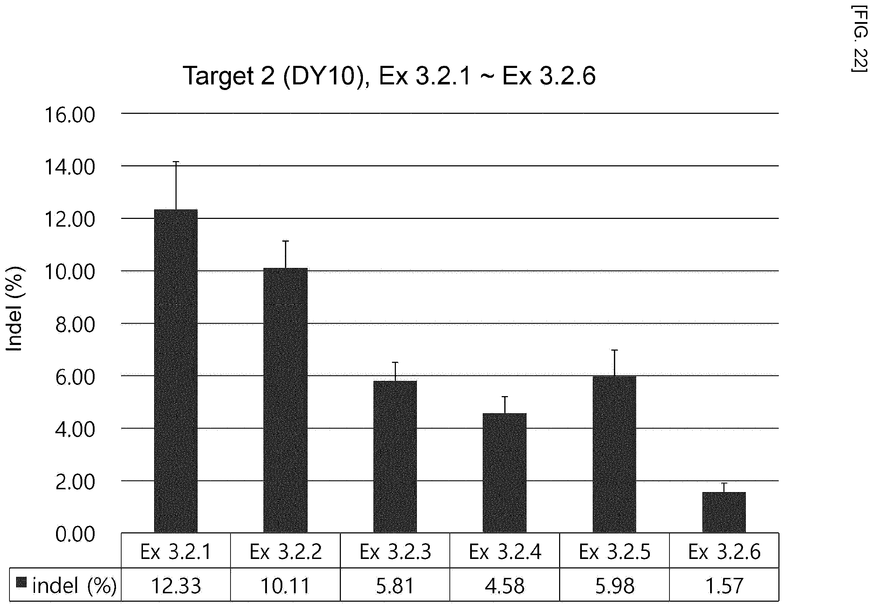

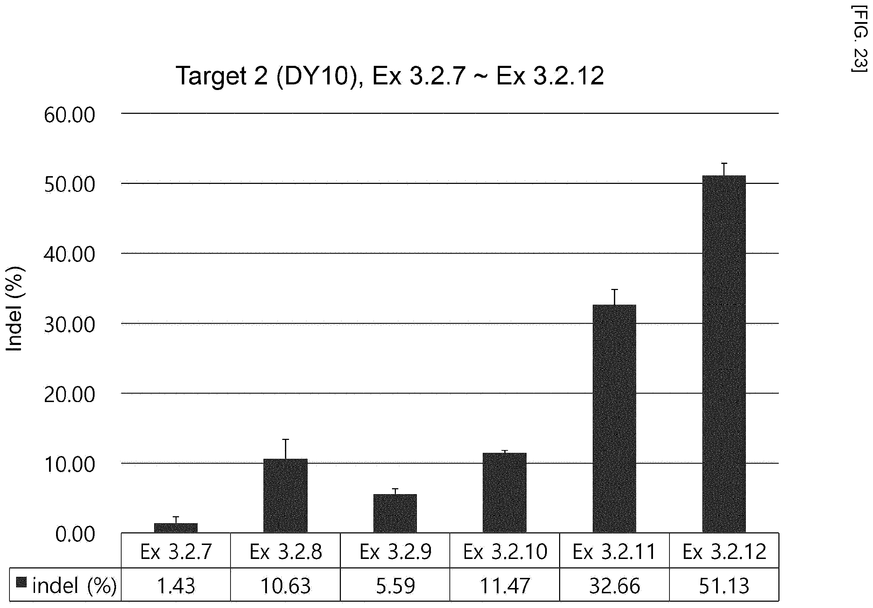

The vector constructed in each Example was transfected into HEK293 T cells according to Experimental Example 1.3, and indel generation efficiency was measured by Experimental Examples 1.4 to 1.5. The results were analyzed by Experimental Example 1.6 and are shown in FIGS. 20 to 23.

### Experimental Example 3.3. Experimental results

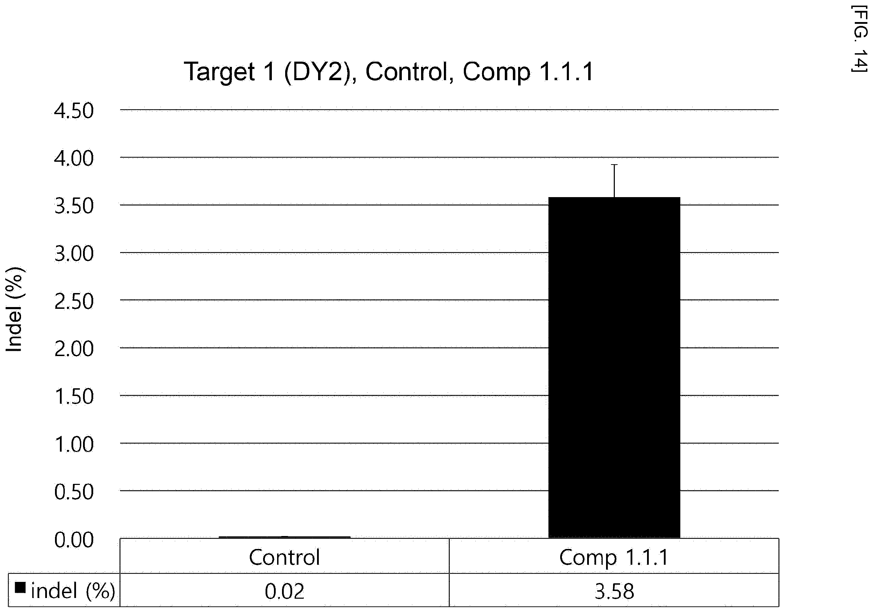

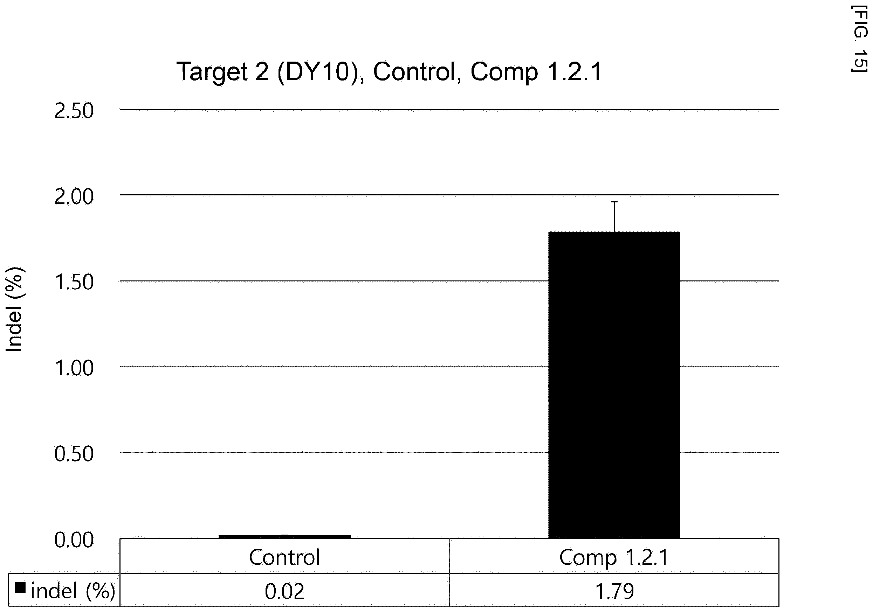

The above experimental results were compared with previous experimental data for the same targets (see FIGS. 14 and 15). As a result, it can be seen that the engineered CRISPR/Cas12f1 system exhibits high gene editing efficiency in a case of including the engineered Cas12f1 guide RNAs of Experimental Example 3.1 and Experimental Example 3.2, as compared with a case of including a Cas12f1 single guide RNA in which a naturally occurring tracrRNA and a naturally occurring crRNA are linked via a linker. From these results, it is possible to infer that the modifications of the guide RNA as shown in the examples of Experimental Examples 3.2 and 3.3 result in improved gene editing efficiency. In addition, referring to the experimental results obtained in Experimental Examples 2 and 3, it is possible to infer that the engineered CRISPR/Cas12f1 system comprising an engineered Cas12f1 guide RNA comprising a modified first region and/or a modified second region has higher gene editing efficiency than a naturally occurring CRISPR/Cas12f1 system, and a CRISPR/Cas12f1 system comprising a Cas12f1 single guide RNA in which a naturally occurring tracrRNA and a naturally occurring crRNA are linked via a linker.

### Experimental Example 4. Indel efficiency comparison 4 between engineered CRISPR/Cas12f1 systems

### Experimental Example 4.1. Indel efficiency comparison 4-1 between engineered CRISPR/Cas12f1 systems

To measure indel efficiency of an engineered CRISPR/Cas12f1 system using an engineered Cas12f1 guide RNA, each of the examples was prepared by Experimental Examples 1.1 and 1.2. The target sequences used for the experiments are shown in Table 18 below.

**[Table 18]**

| **Target Label** | **protospacer sequence (5' to 3')** | **SEQ ID NO** |
|---|---|---|
| FUS | GTGGGTAGGTCCAGTTTGGG | 542 |
| GAK | CAGAGTCCCGGGAACAAGCC | 543 |
| MLH | AGGGAATGAAAGTGAAGATG | 544 |

The sequences of the engineered Cas12f1 guide RNAs for the respective examples are shown in Table 19 below.

**[Table 19]**

| **Label** | **Target** | **PAM (5' to 3')** | **Engineered Cas12f1 guide RNA sequence (5' to 3')** | **SEQ ID NOs** |
|---|---|---|---|---|
| Ex 4.4.1 | FUS | TTTA | ACCGCUUCACCAUUAGUGAGUGAAGGUGGGCUGCUUGCAUCAGCCUAAUGUCGAGAAGUGCUCUUCG GAGUAACCCUCGAAACAAAGAAAGGAAUGCAACGUGGGUAGGUCCAGUUUGGGUUUUAUUUU | 545 |
| Ex 4.4.2 | FUS | TTTA | ACCGCUUCACCAUUAGUGAGUGAAGGUGGGCUGCUUGCAUCAGCCUAAUGUCGAGAAGUGUUCGUAA CCCUCGAAACAAAGAAAGGAAUGCAACGUGGGUAGGUCCAGUUUGGGUUUUAUUUU | 546 |
| Ex 4.4.3 | FUS | TTTA | ACCGCUUCACCAUUAGUGAGUGAAGGUGGGCUGCUUGCAUCAGCCUAAUGUCGAGAAGUUCGCCCUCG AAACAAAGAAAGGAAUGCAACGUGGGUAGGUCCAGUUUGGGUUUUAUUUU | 547 |
| Ex 4.4.4 | FUS | TTTA | ACCGCUUCACCAUUAGUGAGUGAAGGUGGGCUGCUUGCAUCAGCCUAAUGUCGAGUUCGCUCGAAAC AAAGAAAGGAAUGCAACGUGGGUAGGUCCAGUUUGGGUUUUAUUUU | 548 |
| Ex 4.5.1 | GAK | TTTA | ACCGCUUCACCAUUAGUGAGUGAAGGUGGGCUGCUUGCAUCAGCCUAAUGUCGAGAAGUGCUCUUCG GAGUAACCCUCGAAACAAAGAAAGGAAUGCAACCAGAGUCCCGGGAACAAGCCUUUUAUUUU | 549 |
| Ex 4.5.2 | GAK | TTTA | ACCGCUUCACCAUUAGUGAGUGAAGGUGGGCUGCUUGCAUCAGCCUAAUGUCGAGAAGUGUUCGUAA CCCUCGAAACAAAGAAAGGAAUGCAACCAGAGUCCCGGGAACAAGCCUUUUAUUUU | 550 |
| Ex 4.6.1 | MLH | TTTA | ACCGCUUCACCAUUAGUGAGUGAAGGUGGGCUGCUUGCAUCAGCCUAAUGUCGAGAAGUGCUCUUCG GAGUAACCCUCGAAACAAAGAAAGGAAUGCAACAGGGAAUGAAAGUGAAGAUGUUUUAUUUU | 551 |

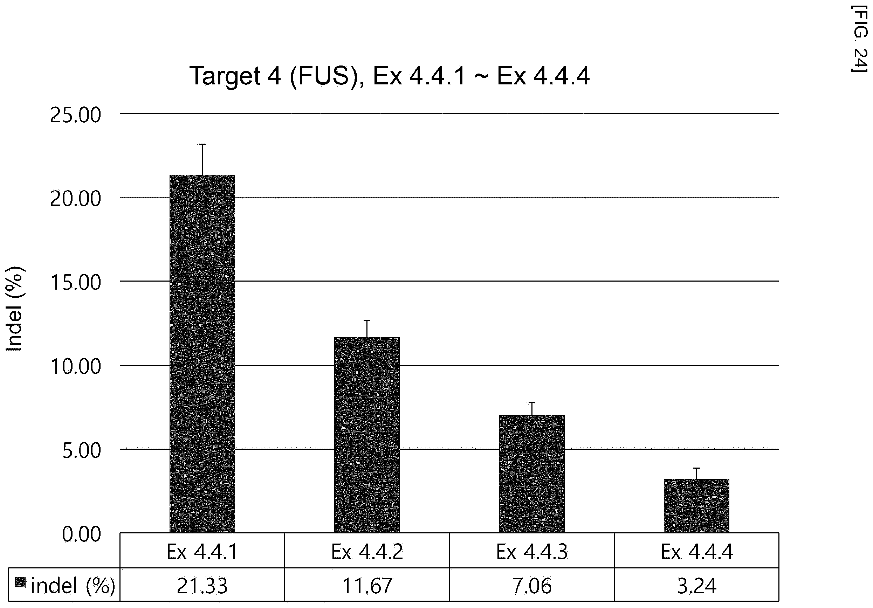

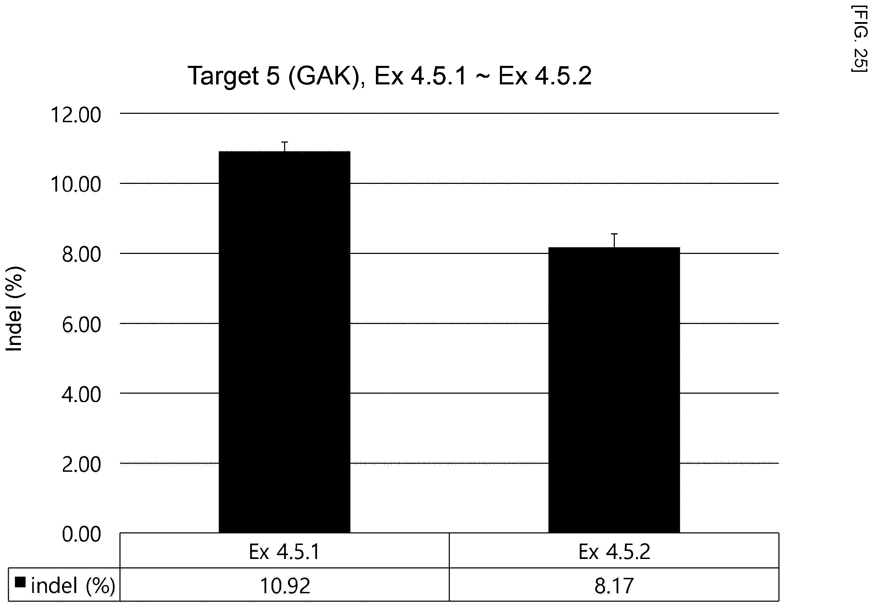

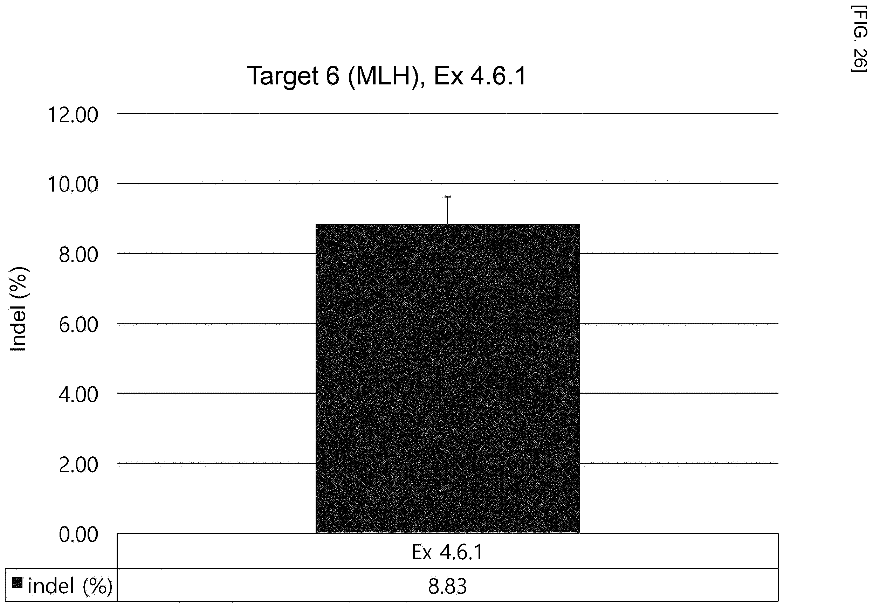

The vector constructed in each Example was transfected into HEK293 T cells according to Experimental Example 1.3, and indel generation efficiency was measured by Experimental Examples 1.4 to 1.5. The results were analyzed by Experimental Example 1.6 and are shown in FIGS. 24 to 26.

### Experimental Example 4.2. Experimental results

From the above experimental results, it is possible to infer that the engineered CRISPR/Cas12f1 system comprising an engineered Cas12f1 guide RNA having a modified third region has higher gene editing efficiency than a CRISPR/Cas12f1 system having a naturally occurring scaffold region, and a CRISPR/Cas12f1 system comprising a Cas12f1 single guide RNA in which a naturally occurring tracrRNA and a naturally occurring crRNA are linked via a linker.

### Experimental Example 5. Large scale validation

Experimental Examples 2 to 4 show the results obtained by measuring indel efficiency only for a few endogenous targets. To supplement the above results, experiments were conducted to see whether the engineered CRISPR/Cas12f1 system is capable of exerting gene editing activity on a wider range of targets.
1) Endogenous targets having 5'-TTTR-N20-NGG-3' were searched in silico, and 88 targets were randomly selected (Tables 20 to 22). Each of the targets is a sequence that can be edited with any of Cas9, Cas12a, and Cas12f1, and thus can be used to compare gene editing efficiency of each CRISPR/Cas system.

**[Table 20]**

| **Target No.** | **Chromosome** | **Location** | **Gene name** | **Protospacer sequence** | **SEQ ID NO** | **Strand** | **Type** |
|---|---|---|---|---|---|---|---|
| **1** | 5 | 359923 | AHRR | CCTTAATAAAGTATAACTTC | 552 | negative | intron |
| 2 | 14 | 20457546 | APEX1 | AAGAAGGAATGGTAGTTGAG | S53 | negative | exon |
| 3 | 22 | 17678603 | BCL2L13 | ATTTCCAAGTCAACCTTATG | 554 | negative | intron |
| 4 | 11 | 3042154 | CARS | CAACAGCCTCACCAGGAACA | 555 | negative | exon |
| 5 | 5 | 202864 | CCDC127 | GGCAAGGGTCTTGATGCATC | 556 | positive | exon |
| 6 | 1 | 25281171 | CLIC4 | CCCTGGCTACCTCCCCTACC | 557 | positive | exon |
| 7 | 3 | 99413340 | COL8A1-1 | GATTCATTCTCAGTGCCATG | 558 | positive | intron |
| 8 | 3 | 99413482 | COL8A1-2 | AGGCAATTGCAACCACTGAA | 559 | positive | intron |
| 9 | 2 | 72933802 | EMX1 | TACTTTGTCCTCCGGTTCTG | 560 | negative | exon |
| 10 | 11 | 22625348 | FANCF-1 | GGTTCTCTCTATAGCCATTG | 561 | positive | exon |
| 11 | 11 | 22625011 | FANCF-2 | ACTTTAGTGACTAGCCGCCA | 562 | negative | exon |
| 12 | 16 | 31193048 | FUS-1 | GTGGGTAGGTCCAGTTTGGG | 563 | positive | exon |
| 13 | 16 | 31193383 | FUS-2 | ACAAAGAAACCAGCAGTGGC | 554 | negative | exon |
| 14 | 22 | 16994935 | GAB4 | CCTGGTGGCTGAGACCAGGG | 565 | negative | exon |
| is | 4 | 888530 | GAK | CAGAGTCCCGGGAACAAGCC | 566 | positive | intron |
| 15 | 11 | 5225683 | HBB | CCAAAGTGATGGGCCAGCAC | 567 | positive | exon |
| 17 | 9 | 112718012 | INIP | AGAGCAGCGA TTGT AAG GAG | 568 | negative | exon |
| is | 5 | 102556075 | intergenic-01 | GAAATATGACTGGAAGTAAA | 569 | negative | intergenic |
| 19 | 5 | 102555078 | intergenic-02 | CTTCCAGTCATATTTCTAAA | 570 | positive | intergenic |
| 20 | 5 | 152068990 | intergenic-03 | CCCTTATTACAATCCTGTGG | 571 | positive | intergenic |
| 21 | 5 | 152058994 | intergenic-04 | CCCCCACAGGATTGTAATAA | 572 | negative | intergenic |
| 22 | 1 | 88052746 | intergenic-05 | ATCTCCATAACAATCTTTGG | 573 | positive | intergenic |
| 23 | 1 | 88052777 | intergenic-06 | CTATCCCCATTTTACAGATG | 574 | positive | intergenic |
| 24 | 3 | 157350012 | intergenic-07 | CTGAGATTTGCGAAGAGTTA | 57.5 | negative | intergenic |
| 25 | 3 | 157350043 | intergenic-08 | ATTAAATAGAGTCTTTTGAA | 576 | negative | intergenic |
| 25 | 3 | 128213929 | intergenic-09 | ATATTAATTG CAAGTTTGGG | 577 | negative | intergenic |
| 27 | 3 | 128213984 | intergenic-10 | GGCCAAGTGCGAAGTCAGAG | 578 | negative | intergenic |
| 28 | 4 | 3634902 | intergenic-11 | GGGGTGAACACCCAAGATCC | S79 | negative | intergenic |
| 29 | 4 | 3634954 | intergenic-12 | GGGTGGGCTCCTGGCAGGGC | 580 | negative | intergenic |

**[Table 21]**

| **Target No.** | **Chromosome** | **Location** | **Gene name** | **Protospacer sequence** | **SEQ ID NO** | **Strand** | **Type** |
|---|---|---|---|---|---|---|---|
| 30 | 6 | 254091 | intergenic-13 | AGAAGCATGCAAAACCGGCA | 581 | positive | intergenic |
| 31 | 6 | 254343 | intergenic-14 | AAGAGGGGAGGTTGACTTTG | 582 | positive | intergenic |
| 32 | 5 | 97245470 | intergenic-15 | GTCAAATAAAGAAAAATACG | 583 | positive | intergenic |
| 33 | 20 | 156154 | intergenic-16 | ATG CATCTCA GTG GTTAACA | 584 | positive | intergenic |
| 34 | 4 | 54520460 | intergenic-17 | CATACAGGGCTCTGTACCCA | 585 | negative | intergenic |
| 35 | 4 | 54520536 | intergenic-18 | CAAAGACACTCACCCTGTTG | 585 | positive | intergenic |
| 36 | 5 | 170399606 | intergenic-19 | AGAACACATACCCCTGGGCC | 587 | negative | intergenic |
| 37 | 5 | 170399701 | intergenic-20 | ATAATAAAAGTATTTCCTCA | 588 | negative | intergenic |
| 38 | 17 | 1919439 | intergenic-21 | AGCCGTGGTCAGTGAGAGGC | 589 | positive | intergenic |
| 39 | 17 | 1919532 | intergenic-22 | GAGCTCATTAGCTTGGGGAG | 590 | positive | intergenic |
| 40 | 9 | 7742784 | intergenic-23 | GAAAATAACTAAACTTCCCA | 591 | negative | intergenic |
| 41 | 15 | 25637364 | intergenic-24 | AATTCTTTAAGTAATTTAAG | 592 | negative | intergenic |
| 42 | 9 | 7742966 | intergenic-25 | CTTAGTAGTCTCAGAACCAA | 593 | positive | intergenic |
| 43 | 15 | 25637516 | intergenic-26 | AAAGGAGCACAAGTACAAAC | 594 | positive | intergenic |
| 44 | 18 | 561716 | intergenic-27 | AATGATGCAGTAATCGTGTA | 595 | positive | intergenic |
| 45 | 5 | 136515295 | intergenic-28 | ATAAAAGGAACTATTTACAA | 596 | positive | intergenic |
| 46 | 2 | 23847019 | KLHL29 | GAGAGACCGCTCAGGCTGGA | 597 | negative | intron |
| 47 | 14 | 28794781 | LINC01551-1 | ATTTTGAAGTGACCGTACGA | 598 | negative | exon |
| 48 | 14 | 28794751 | LINC01551-2 | ATAATACACTCTTTACA CTG | 599 | negative | exon |
| 49 | 19 | 58005513 | LOC100128398-1 | AAGAGTTATTGTCAATAGAA | 600 | negative | exon |
| 50 | 19 | 58005993 | LOC100128398-2 | CAAAGAAATGTACTG CCTTA | 501 | negative | exon |
| 51 | 17 | 943127 | LOC100130876 | AAATAACCGTCGGTTTCTTA | 602 | positive | exon |
| 52 | 3 | 114911114 | LOC101926986 | CAAACAAAATAATTGGCTCA | 503 | positive | intron |
| 53 | 12 | 674075 | LOC105369597 | G CCATG GTG AA G GTG AAATC | 504 | positive | exon |
| 54 | 14 | 19916429 | LOC105370393-1 | GCAGTACACCTGAGGGAACA | 505 | positive | intron |
| 55 | 14 | 19,915499 | LOC105370393-2 | AAGAAAGCTACAGGAAAGCA | 606 | positive | intron |
| 56 | 4 | 42789433 | LOC105374431 | CTTTAAAATG AG GTACTAG G | 607 | negative | intron |
| 57 | 7 | 1596749 | LOC105375122 | CCAACCAG GTACCCTGTG CC | 608 | positive | exon |
| 53 | 1 | 61097826 | LOC105378753 | ATTGAAACATATACGTGGTA | 609 | negative | exon |

**[Table 22]**

| **Target No.** | **Chromosome** | **Location** | **Gene name** | **Protospacer sequence** | **SEQ ID NO** | **Strand** | **Type** |
|---|---|---|---|---|---|---|---|
| 59 | 13 | 19073987 | LOC107984132 | GGAAAGCGCAGAAAAGTAAA. | 610 | negative | exon |
| 60 | 3 | 36995716 | MLH1-1 | AGGGAATGAAAGTGAAGATG | 611 | positive | intron |
| 61 | 3 | 36995868 | MLH1-2 | GATCAATTTACATCAAACTA | 612 | positive | intron |
| 62 | 21 | 2560383S. | MRPL39 | ATTTCACAGGACTTTGTTAA | 613 | positive | exon |
| 63 | 3 | 27160152 | NEK10-1 | AGACAAGCTGTCTTCCTTCA | 514 | negative | intron |
| 64 | 3 | 27160372 | NEK10-Z | ATCTGAAGATCATTGAAACA | 615 | negative | intron |
| 65 | 3 | 173963498 | NLGN1 | GTCTAATAGAAATATAGTAC | 616 | negative | exon |
| 66 | 2 | 32383384 | NLRC4-1 | GAGGGAGACACAAGTTGATA | 617 | negative | intron |
| 67 | 2 | 32383454 | NLRC4-2 | GTCTCAGTCTTCCTTGTGGG | 619 | negative | intron |
| 68 | 3 | 131069719 | NUDT16-1 | GGGGTAGAGGTACTCTACAG | 619 | positive | exon |
| 69 | 3 | 131069756 | NUDT16-2 | GGGGTAGAGGTAGTCTACAG | 520 | positive | exon |
| 70 | 14 | 20117733 | OR4K17 | ACAAGTTCAGAATCACCTTA | 621 | negative | exon |
| 71 | 11 | 3087968 | OSBPLS | GCATTAAGGCCAGCGCTGGG | 522 | positive | exon |
| 72 | 17 | 3669779 | P2RX5-TAX1BP3 | CACATAGGCCATTCAGAAAC | 623 | positive | exon |
| 73 | 19 | 627446 | POLRMT-1 | GAAACTGCCCCAAAACCGGC | 624 | negative | intron |
| 74 | 19 | 627491 | POLRMT-2 | AGGACTATGTGTGGCCAGTG | 525 | negative | intron |
| 75 | 15 | 24987466 | PWAR5 | AACAAATCACTGACTAACCA | 626 | negative | exon |
| 75 | 12 | 97515285 | RMST | ATAATGCCTTTTAGGTGATA | 627 | negative | intron |
| 77 | 17 | 292463 | RPH3AL-1 | ATTTTCAAAACAGCCCTATG | 628 | positive | intron |
| 78 | 17 | 292509 | RPH3AL-2 | CACAAGGGATCTGAGACTTG | 629 | positive | intron |
| 79 | 20 | 964362 | RSPO4-1 | ACTCATACATCACCTCCTCC | 530 | negative | intron |
| 800 | 20 | 964345 | RSPO4-2 | AAGGAAAGGCTTCCTGGAGG | 631 | positive | intron |
| 81 | 1 | 25684228 | RSRP1-1 | ATATAGGATTTAGAAACCAA | 632 | negative | exon |
| 82 | 1 | 25684090 | RSRP1-2 | GCTCTAATGTAAGTATATCC | 533 | negative | exon |
| 83 | 7 | 72574897 | TYW1B | GATCCGATGCAATTTTGGGA | 534 | positive | exon |
| 84 | 7 | 1233674 | UNCX | CCTGAACTCGGGACTCGACC | 635 | negative | exon |
| 85 | 12 | 909294 | WNK1 | GAACCCAGTGAAAAATACCA | 636 | positive | exon |
| 86 | 13 | 20002951 | ZMYM2 | GTAGGCTGCTGTTGGACAGA | 537 | negative | exon |
| 87 | 12 | 133140444 | ZNF10-1 | AATAAGTCTTA CCA CGTGTC | 638 | positive | intron |
| 88 | 12 | 133140502 | ZNF10-2 | ATTCCCACAATAACCCTATG | 639 | positive | intron |

2) Respective components of a CRISPR/SpCas9 system, a CRISPR/AsCas12a system, a naturally occurring CRISPR/Cas12f1 system, or an engineered CRISPR/Cas12f1 system were transfected into HEK293-T cells. Here, the engineered Cas12f1 guide RNAs used in the engineered CRISPR/Cas12f1 system are summarized in Table 23 below.

**[Table 23]**

| **Label** | **guide RNA sequence (5' to 3')** | **SEQ ID No.** |
|---|---|---|
| Canonical Cas12f | CUUCACUGAUAAAGUGGAGAACCGCUUCACCAAAAGCUGUCCCUUAGGGGAUUAGAACUUGAGUGAAGGU GGGCUGCUUGCAUCAGCCUAAUGUCGAGAAGUGCUUUCUUCGGAAAGUAACCCUCGAAACAAAUUCAUUU UUCCUCUCCAAUUCUGCACAAGUUGCAGAACCCGAAUAGACGAAUGAAGGAAUGCAACNNNNNNNNNNN NNNNNNNNN | 640 |
| geCas12f_4.0 | ACCGCUUCACCAAAAGCUGUCCCUUAGGGGAUUAGAACUUGAGUGAAGGUGGGCUGCUUGCAUCAGCCUA AUGUCGAGAAGUGCUUUCUUCGGAAAGUAACCCUCGAAACAAAGAAAGGAAUGCAACNNNNNNNNNNNN NNNNNNNNUUUUAUUUU | 252 |
| geCas12f_4.1 | ACCGCUUCACCAUUAGUGAGUGAAGGUGGGCUGCUUGCAUCAGCCUAAUGUCGAGAAGUGCUUUCUUCGG AAAGUAACCCUCGAAACAAAGAAAGGAAUGCAACNNNNNNNNNNNNNNNNNNNNUUUUAUUUU | 253 |

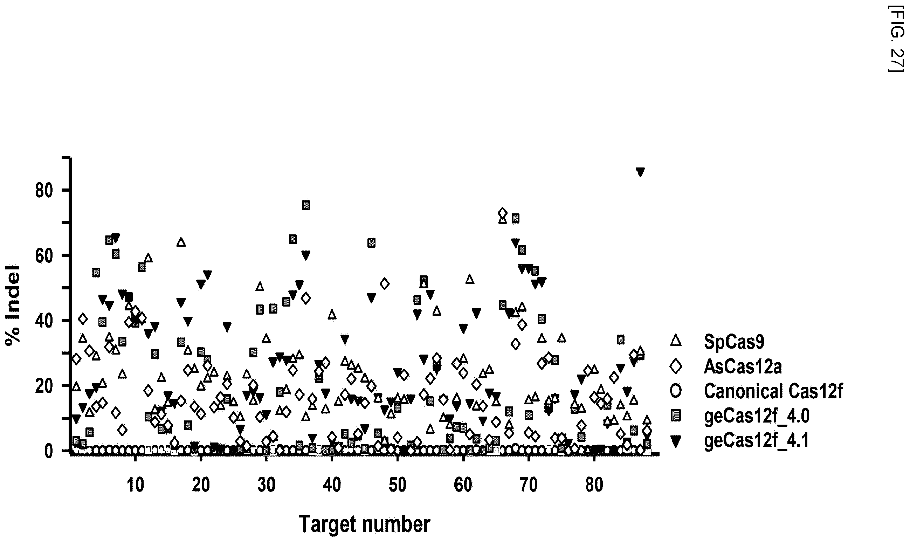

Here, the 5'-(N)₂₀-3' portion in the above sequences, which is a spacer sequence, was designed as a sequence corresponding to each of the protospacer sequences shown in Tables 20 to 22.
3) After transfection, gene editing efficiency for the 88 targets is shown in FIG. 27 and Table 24.

**[Table 24]**

| **Range of %** i**ndel** | **Distribution of % indel (n=88)** | | | |
|---|---|---|---|---|
| | geCas12f_4.0 | geCas12f_4.1 | SpCas9 | AsCas12a |
| ≤1% | 24 | 7 | 3 | 8 |
| 1-10% | 23 | 12 | 11 | 25 |
| 10-20% | 10 | 28 | 32 | 24 |
| 20-30% | 6 | 10 | 19 | 20 |
| 30-50% | 14 | 19 | 17 | 9 |
| ≥ 50% | 11 | 12 | 6 | 2 |

From the experimental results, it can be seen that the engineered CRISPR/Cas12f1 system disclosed herein 1) shows significantly higher gene editing efficiency than a naturally occurring CRISPR/Cas12f1 system, 2) shows gene editing efficiency, which is comparable to the CRISPR/SpCas9 system or the CRISPR/AsCas12a system, for any target in a eukaryotic cell, and 3) show higher gene editing efficiency than the other CRISPR/Cas systems for some targets.

### Experimental Example 6. In vitro cleavage assay

To supplementarily observe the gene cleavage pattern of the engineered CRISPR/Cas12f1 system disclosed herein, an in vitro cleavage assay was performed. The target protospacer sequence used in Experimental Example 6 is 5'-TTTAAGAACACATACCCCTGGGCC-3' (SEQ ID NO: 341, hereinafter Intergenic-22), and a PAM sequence of the target is 5'-TTTA-3'.

The (engineered) Cas12f1 guide RNA used in Experimental Example 6 is shown in Table 25 below.

**[Table 25]**

| **Label** | **Target** | **PAM (5' to 3')** | **Engineered Cas12f1 guide RNA sequence (5' to 3')** | **SEQ ID NOs** |
|---|---|---|---|---|
| Canonical | Intergenic-22 | TTTA | CUUCACUGAUAAAGUGGAGAACCGCUUCACCAAAAGCUGUCCCUUAGGGGAUUAGAACUUGAGUGAAGGU GGGCUGCUUGCAUCAGCCUAAUGUCGAGAAGUGCUUUCUUCGGAAAGUAACCCUCGAAACAAAUUCAUUU UUCCUCUCCAAUUCUGCACAAGUUGCAGAACCCGAAUAGACGAAUGAAGGAAUGCAACAGAACACAUACCCC UGGGCC | 641 |
| MS2/3/4 | Intergenic-22 | TTTA | ACCGCUUCACCAAAAGCUGUCCCUUAGGGGAUUAGAACUUGAGUGAAGGUGGGCUGCUUGCAUCAGCCUA AUGUCGAGAAGUGCUUUCUUCGGAAAGUAACCCUCGAAACAAAGAAAGGAAUGCAACAGAACACAUACCCC UGGGCCUUUUAUUUU | 336 |
| MS2/3/4/5 | Intergenic-22 | TTTA | ACCGCUUCACUUAGAGUGAAGGUGGGCUGCUUGCAUCAGCCUAAUGUCGAGAAGUGCUUUCUUCGGAAA GUAACCCUCGAAACAAAGAAAGGAAUGCAACAGAACACAUACCCCUGGGCCUUUUAUUUU | 649 |

The experimental method is as follows.
1) Recombinant Cas14 (2.5 µg) and each 2 µg of canonical sgRNA, MS2/MS3/MS4 sgRNA, and MS2/MS3/MS4/MS5 sgRNA were incubated to form an RNA complex.
2) An in vitro cleavage assay was performed by allowing the resulting RNA complex to react with a plasmid vector (5 µg) containing a target sequence (Intergenic-22) at 37°C for 3 hours.
3) Cutting with Apal restriction enzyme was performed as a positive control.
4) DNA shearing was carried out on the cleavaged DNA under the following conditions by using Covaris (M220) that is an ultrasonicator.
   Peak incident power: 50 W, duty factor: 20%, cycles per Burst: 200 cpb, treatment time: 110 sec.
5) The fragmented DNA was purified using a DNA purification kit.
6) End-filling of the purified DNA was performed by using a T4 ligase.
7) Then, a DNA library was constructed using a NEBNext^{®} Ultra^{™} II DNA Library Prep Kit for Illumina^{®} (NEB, # E7103) and NEBNext^{®} Multiplex Oligos for Illumina^{®} (Dual Index Primers Set 1) (NEB, # E7600) kit.
8) Quantitative polymerase chain reaction (qPCR) was performed to equally adjust the concentration of each sample.
9) 150-bp paired-end sequencing was performed on the constructed library using Illumina iSeq 100.
10) The analyzed sequences were aligned by using the integrative genomics viewer (IGV).

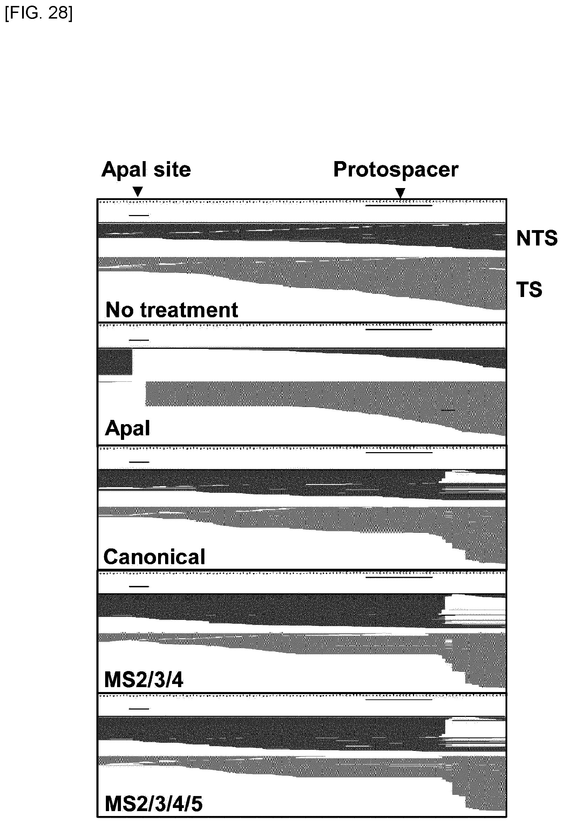

The experimental results are shown in FIG. 28.

From the experimental results, it was found that the engineered CRISPR/Cas12f1 system comprising the engineered guide RNA disclosed herein has higher cleavage activity for a non-target strand (NTS) than a naturally occurring CRISPR/Cas12f1 system. This is considered to be a factor affecting improved gene editing activity of the engineered CRISPR/Cas12f1 system disclosed herein.

### 2. Effect of CRISPR expression regulatory system using engineered Cas12f1 guide RNA

### Experimental Example 1. Dead Cas12f1 protein

A vector expressing Cas12f1 was subjected to mutagenesis so that Cas12f1 was turned into a dead form thereof. The dead form thereof is D326A, E422A, R490A, D510A, R490Q, R490W, R490L, D510L, D510V, or any of its other mutated forms having lost cleavage activity. Primers used for each mutagenesis are shown in Table 26.

**[Table 26]**

| | WT | Mut | Forward (5'-3') | Reverse (5'-3') |
|---|---|---|---|---|
| D360A | GAT | GCT | CGGAGGGATCgctGTGGGGGTCA (SEQ ID NO: 650) | ATGATGCTGGGATCCACGC (SEQ ID NO: 659) |
| E422A | GAG | GCG | AGTGCAGATGgcgAACCTCGAGA (SEQ ID NO: 651) | GTTCCGACCTTGTTCTTAATAAAG (SEQ ID NO: 660) |
| R490A | CGG | GCG | CTTCGAGTACgcgAAGAAGAACAAG (SEQ ID NO: 652) | TTGAAGTAGTTGTTGAGG (SEQ ID NO: 661) |
| R490Q | CGG | CAG | CTTCGAGTACcagAAGAAGAACAAG (SEQ ID NO: 653) | TTGAAGTAGTTGTTGAGG (SEQ ID NO: 662) |
| R490W | CGG | TGG | CTTCGAGTACtggAAGAAGAACAAG (SEQ ID NO: 654) | TTGAAGTAGTTGTTGAGGTG (SEQ ID NO: 663) |
| R490L | CGG | CTG | CTTCGAGTACctgAAGAAGAACAAG (SEQ ID NO: 655) | TTGAAGTAGTTGTTGAGG (SEQ ID NO: 664) |
| D510A | GAT | GCT | GGAGAACGCCgctTACAACGCCG (SEQ ID NO: 656) | TTAAAGTTGCACTTCTCGC (SEQ ID NO: 665) |
| D510L | GAT | CTT | GGAGAACGCCcttTACAACGCCG (SEQ ID NO: 657) | TTAAAGTTGCACTTCTCG (SEQ ID NO: 666) |
| D510V | GAT | GTA | GGAGAACGCCgtaTACAACGCCG (SEQ ID NO: 658) | TTAAAGTTGCACTTCTCGC (SEQ ID NO: 667) |

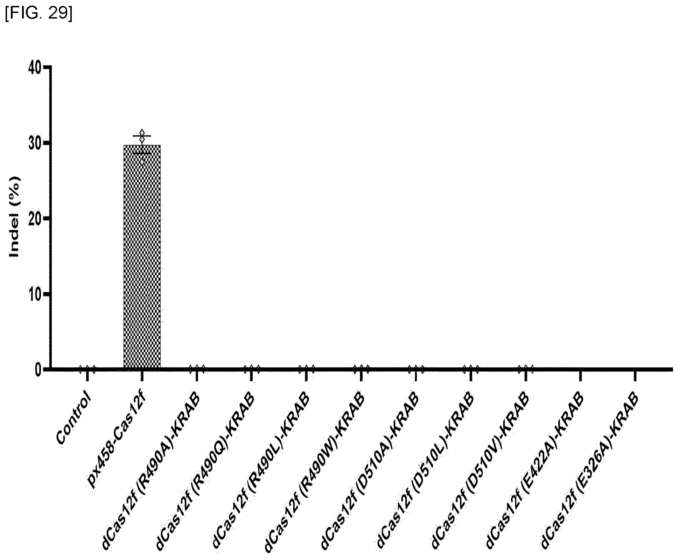

To identify whether the resulting dead Cas12f1 had lost cleavage activity, transfection was performed on HEK293T cells with 5'-CACACACACAGTGGGCTACCATT-3'(SEQ ID NO: 668) as a target. 96 hours after transfection, gDNA was extracted and comparison of indel generation was performed through NGS analysis (FIG. 29).

### Experimental Example 2. CRISPR expression regulatory system using transcriptional inhibitor Cas12f1 fusion protein

### Experimental Example 2.1. Design of transcriptional inhibitor Cas12f1 fusion protein

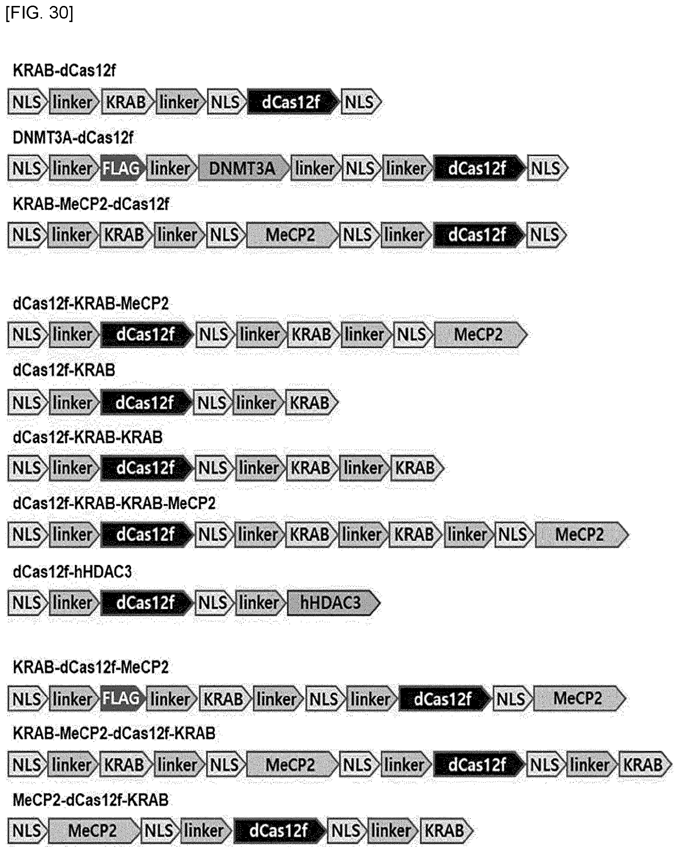

To construct an expression regulatory system module, each or combination of KRAB, MeCP2, and DNMT3A was cloned into the N-terminus or the C-terminus of Cas12f1 (FIG. 30). After the vector and the insert fragment were amplified with KOD-one (TOYOBO) using a template, ligation was performed using Gibson Assembly^{®} Master Mix (NEB) according to the protocol. The template DNA and primers used for construction of each of the vectors and the insert fragments are shown in Tables 27 and 28.

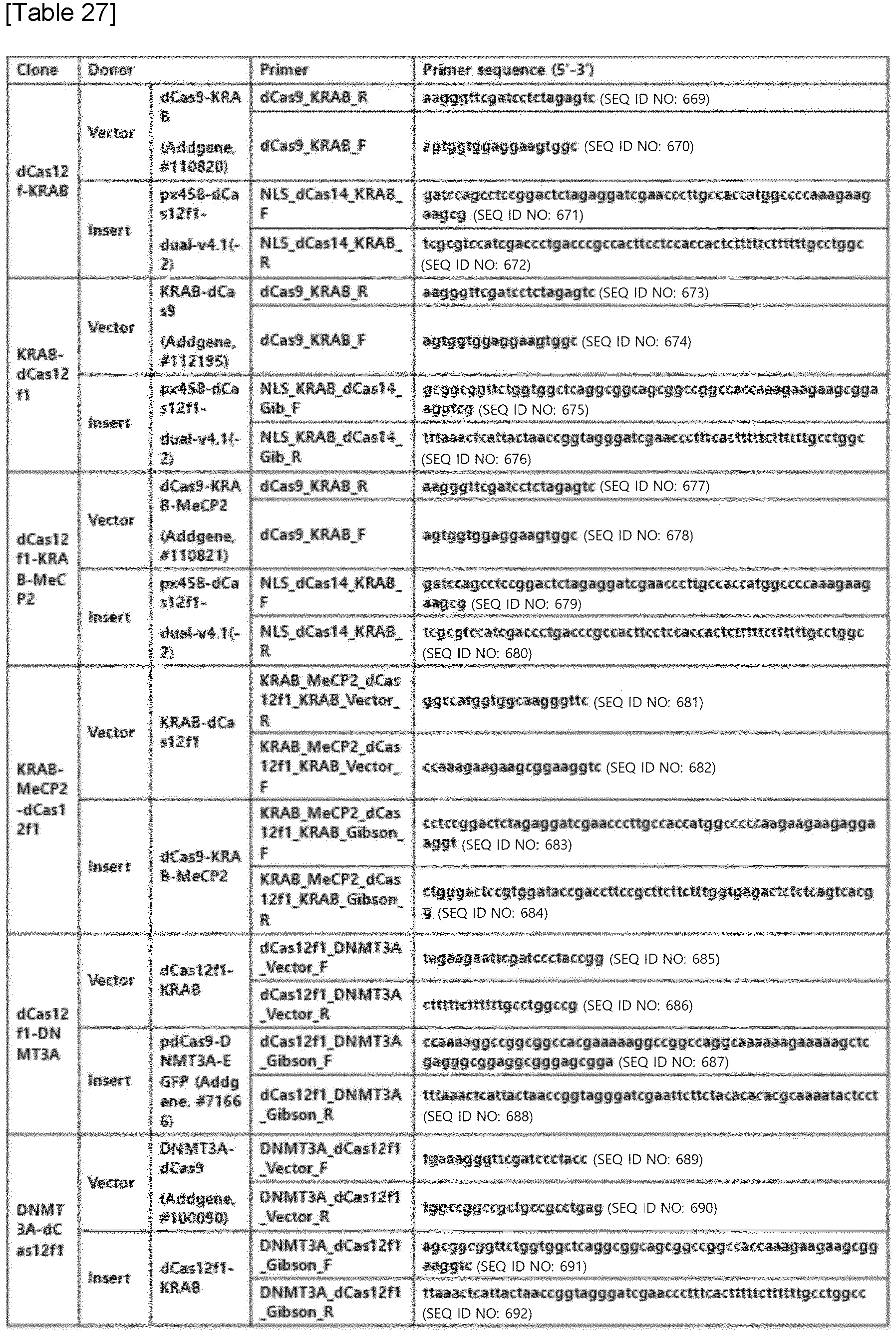

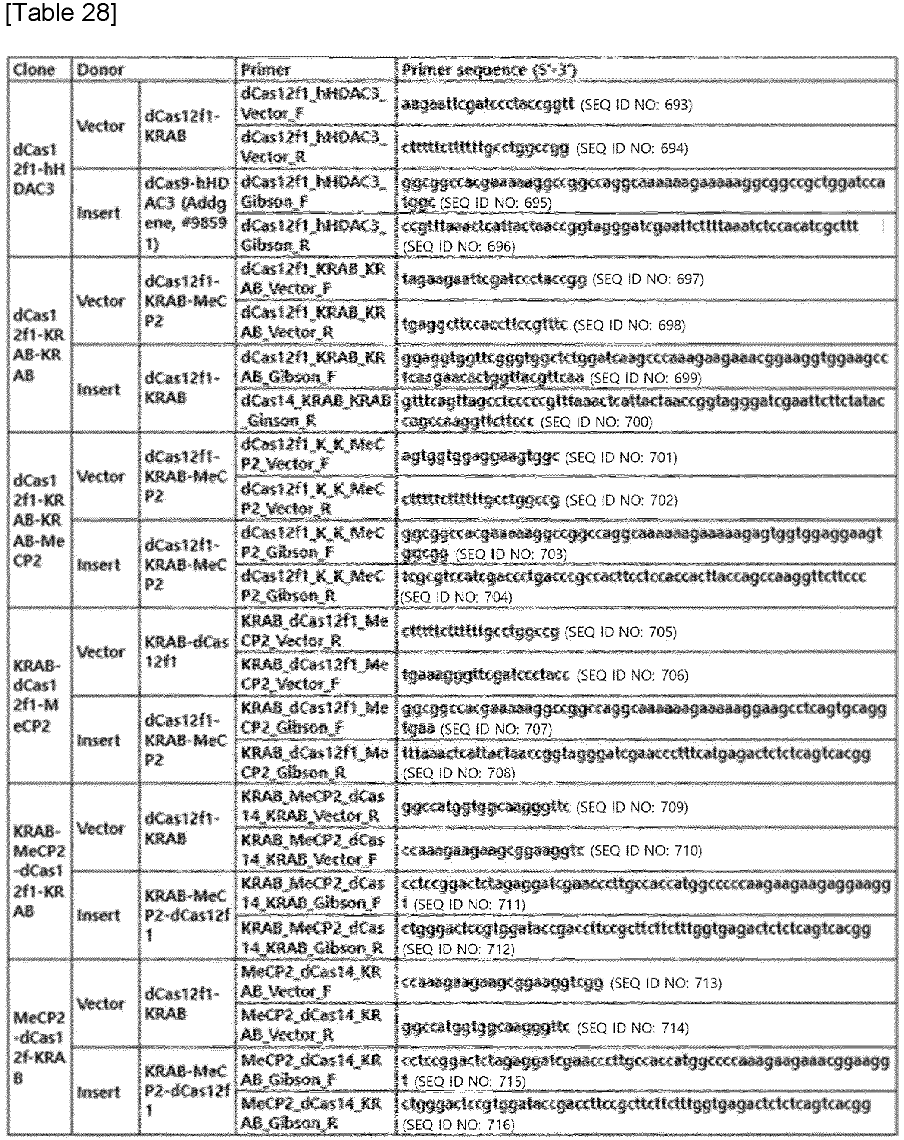

### Experimental Example 2.2. Effect of suppressing (or inhibiting) expression caused by CRISPR interference complex comprising transcriptional inhibitor Cas12f1 fusion protein and engineered Cas12f1 guide RNA

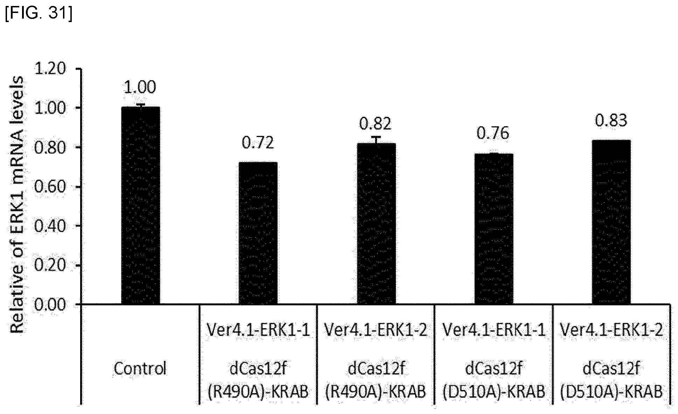

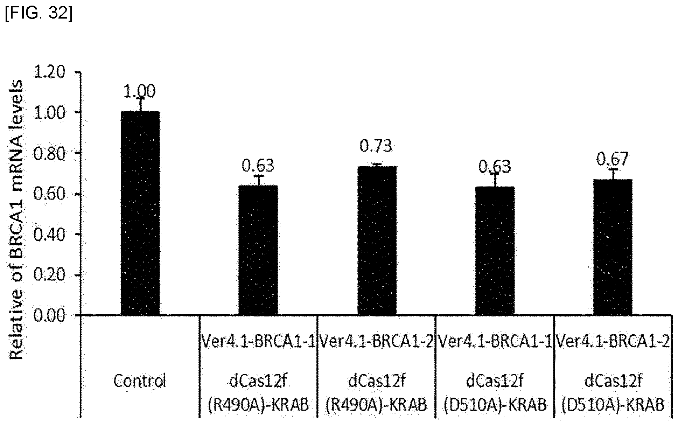

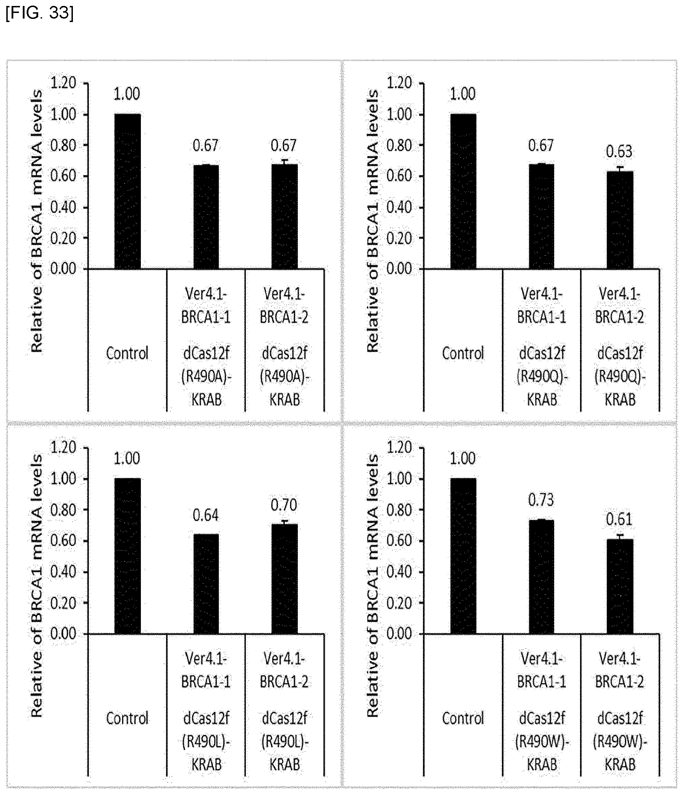

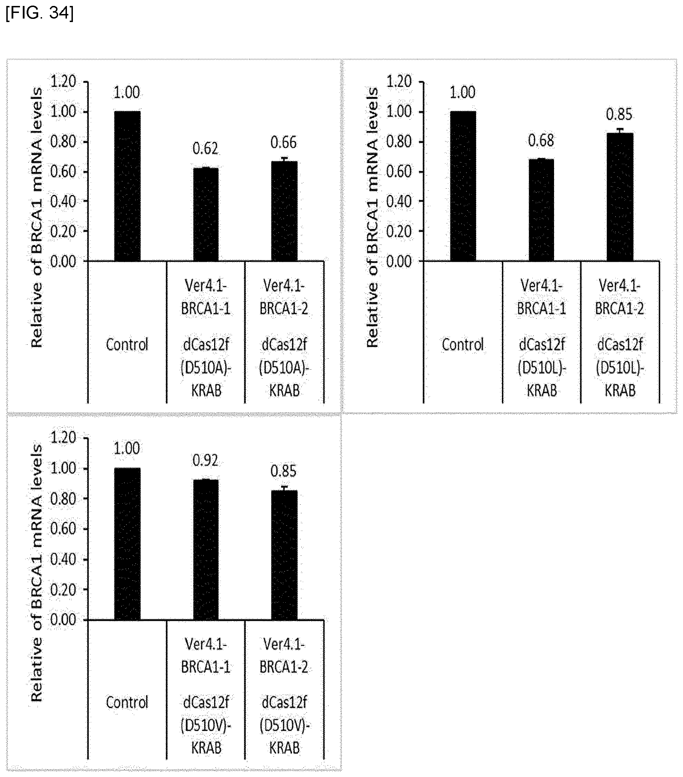

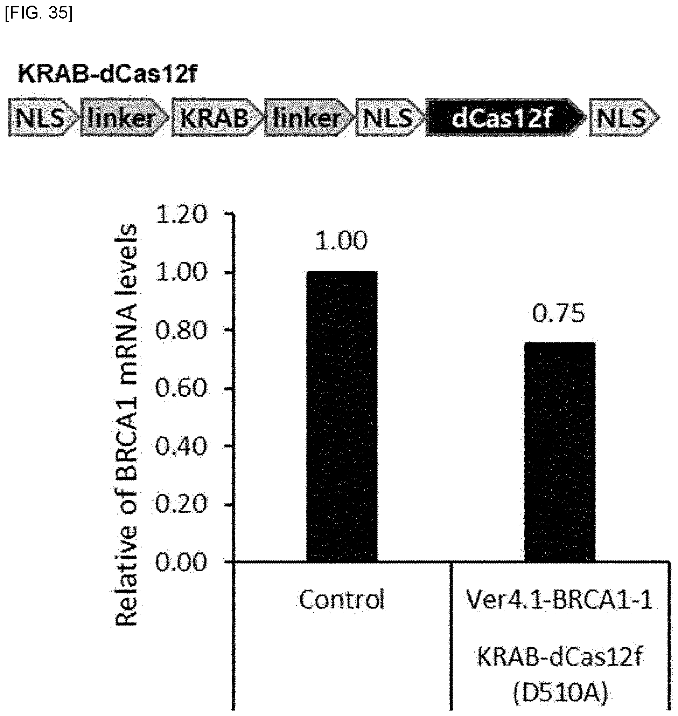

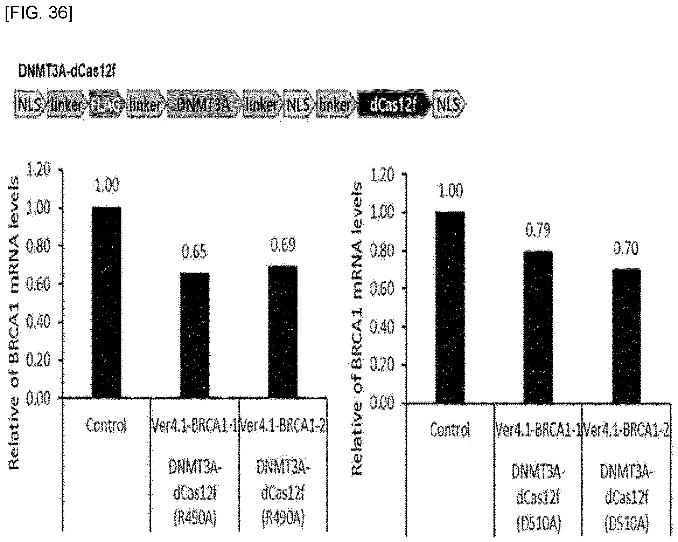

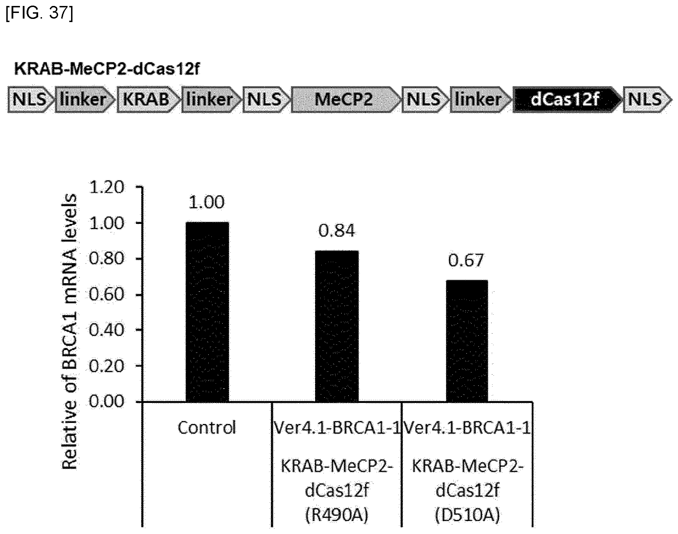

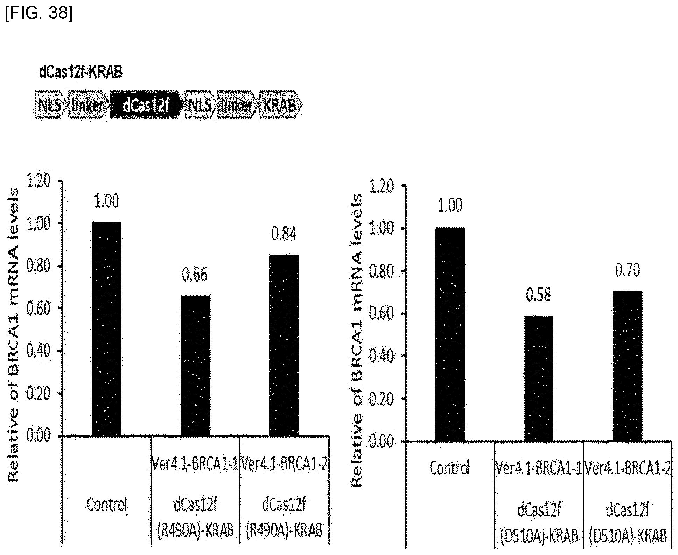

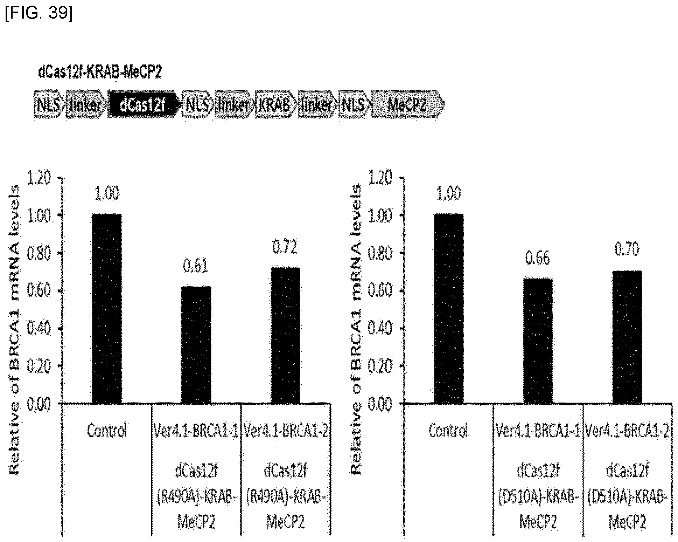

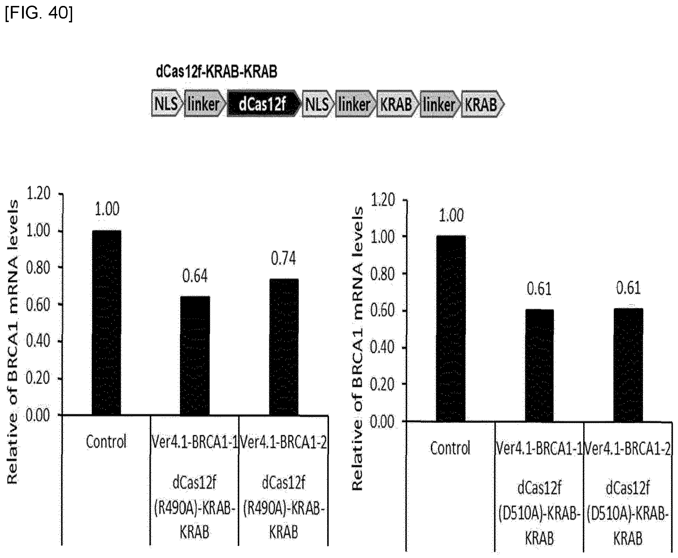

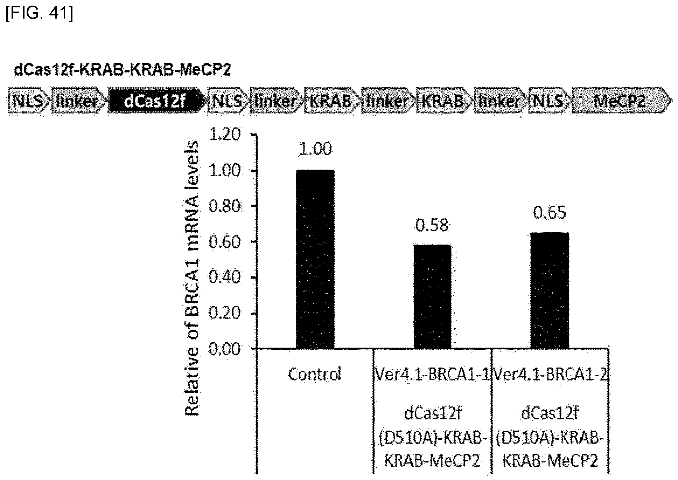

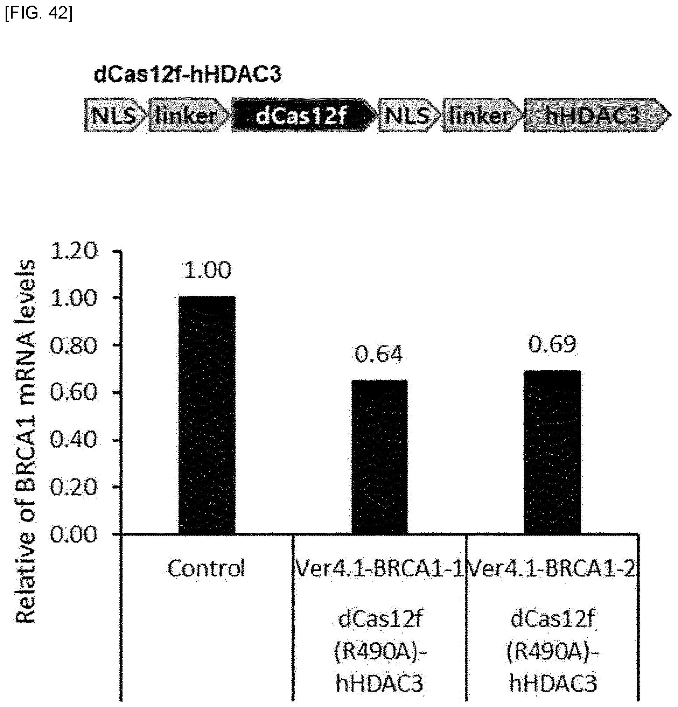

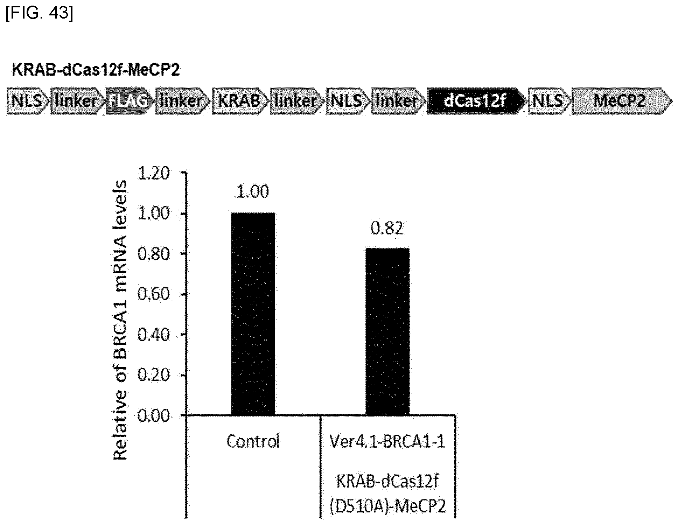

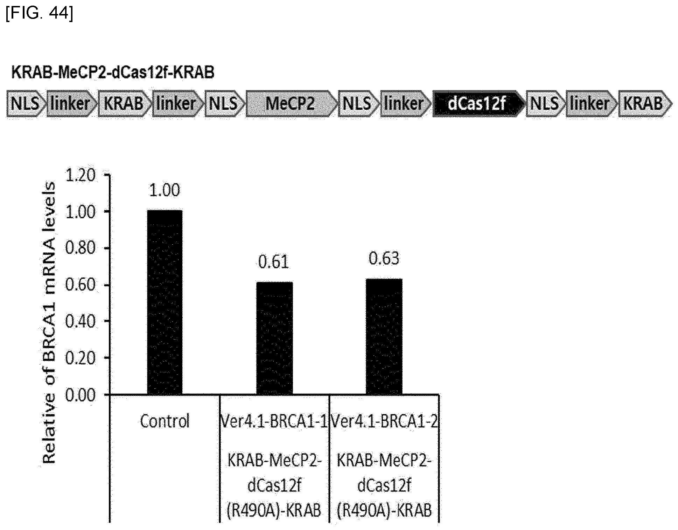

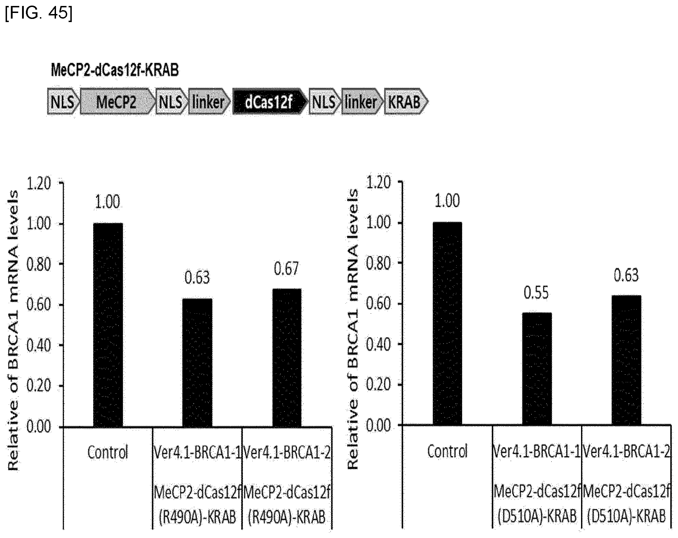

HEK293T cells were transfected with 1.5 µg of each module plasmid and 0.5 µg of a guide RNA cassette for targeting. 96 hours after transfection, the cells were harvested. RNA was extracted therefrom using a maxwell^{®} RSC miRNA Tissue Kit (Promega). cDNA was synthesized from 1 µg of RNA using SuperScript IV Reverse Transcriptase (Invitrogen) according to the protocol. Using the synthesized DNA as a template, an effect of the complex on suppressing expression of each target was identified (FIGS. 31 to 45). 18s was used as a control target, and primers used for qPCR thereof are shown in Table 29 below.

**[Table 29]**

| Name | Sequence (5'-3') |
|---|---|
| 18s_rRNA_F | TCAACTTTCGATGGTAGTCGCC (SEQ ID NO: 717) |
| 18s_rRNA_R | GGCCTCGAAAGAGTCCTGTATTGT (SEQ ID NO: 718) |

### Experimental Example 2.3. Effect of inhibiting expression of PCSK9 gene using CRISPR interference complex

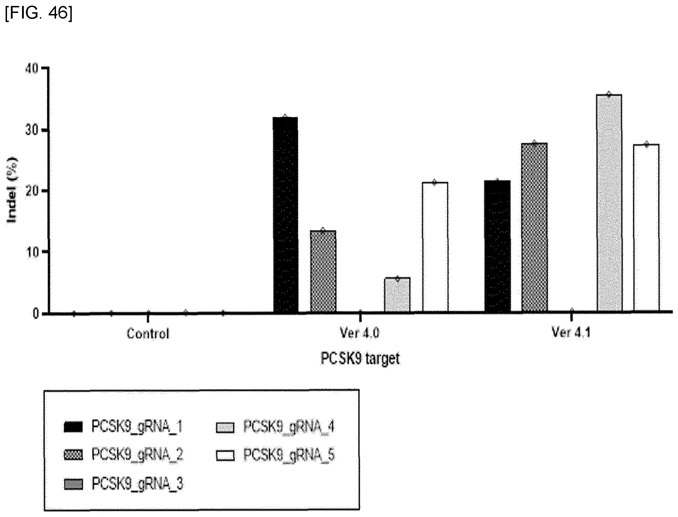

To identify an effect of the complex on inhibiting expression of PCSK9 gene, a target located at the promoter thereof was selected to examine indel efficiency. HEK293T cells were transfected with each 2 µg of vectors encoding Cas12f1 and guide RNA, respectively. 96 hours after transfection, the cells were harvested and indel efficiency was analyzed through NGS (FIG. 46). Here, the gRNA information used is shown in Table 30.

**[Table 30]**

| | **Target** | **Sequence (5'-3')** |
|---|---|---|
| SaCa$9 | gRNA_1 | TCCGTTAATGTTTAATCAGA (SEQ ID NO: 719) |
| | gRNA_2 | GAAACCTGATCCTCCAGTCC (SEQ ID NO: 720) |
| | gRNA_4 | GCCAGGTTAAGGCCAGTGGA (SEQ ID NO: 721) |
| Cas12f | gRNA_1 | ATCAGATAGGATCGTCCGAT (SEQ ID NO: 722) |
| | gRNA_2 | GGGAGGGCGAGGCCGAAACC (SEQ ID NO: 723) |
| | gRNA_3 | GGAGTFFFFRCTTCCCTCTG (SEQ ID NO: 724) |
| | gRNA_4 | GAAGGCTGCCAGGTTAAGGC (SEQ ID NO: 725) |
| | gRNA_5 | TGCACCCTGCACACTGACCT (SEQ ID NO: 726) |

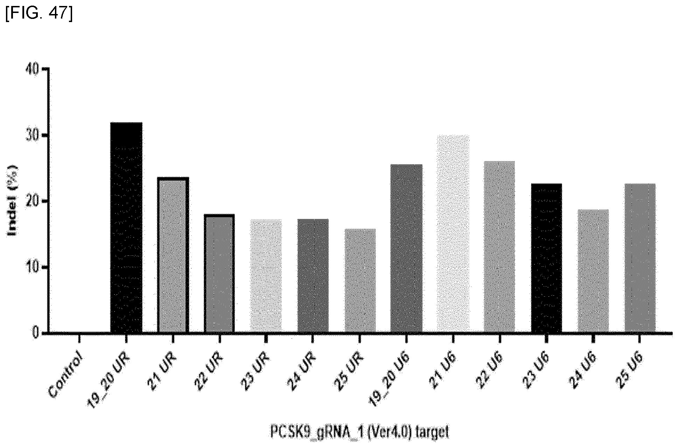

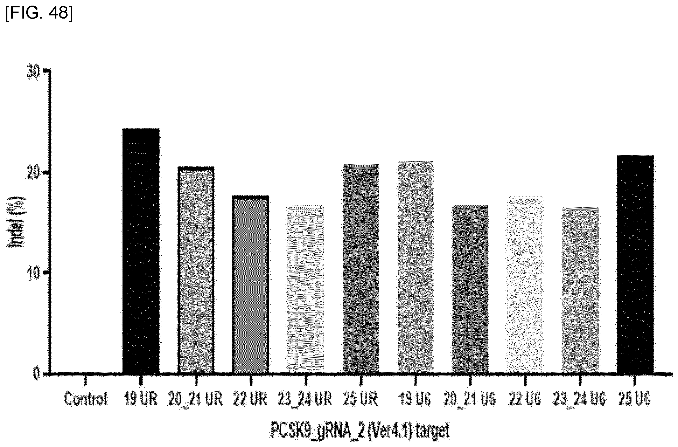

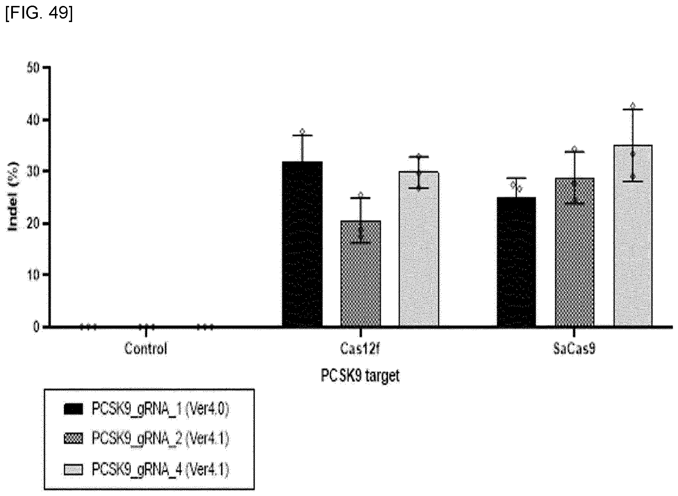

To increase targeting efficiency, guide RNA optimization and spacer optimization were performed to identify the optimal guide RNA form (FIGS. 47 to 49).

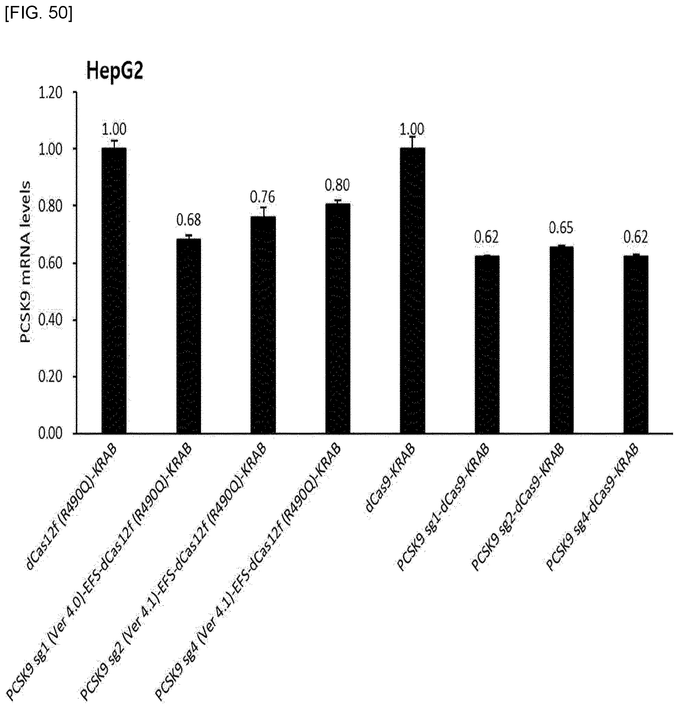

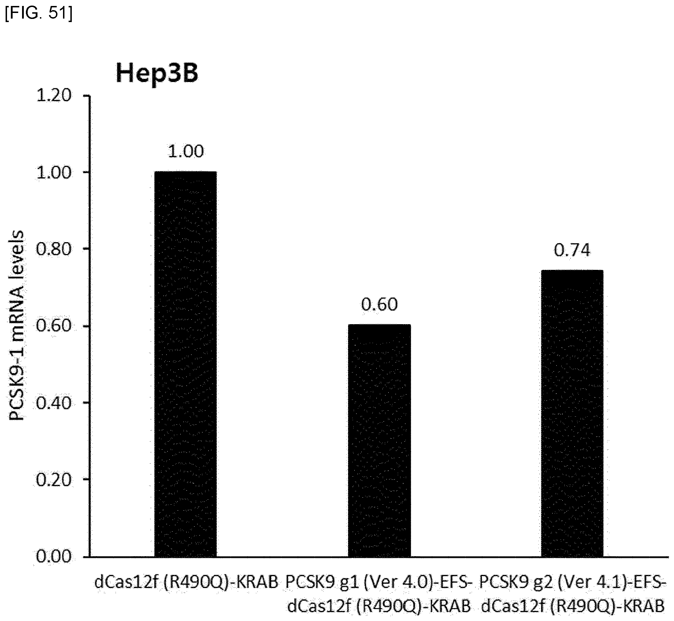

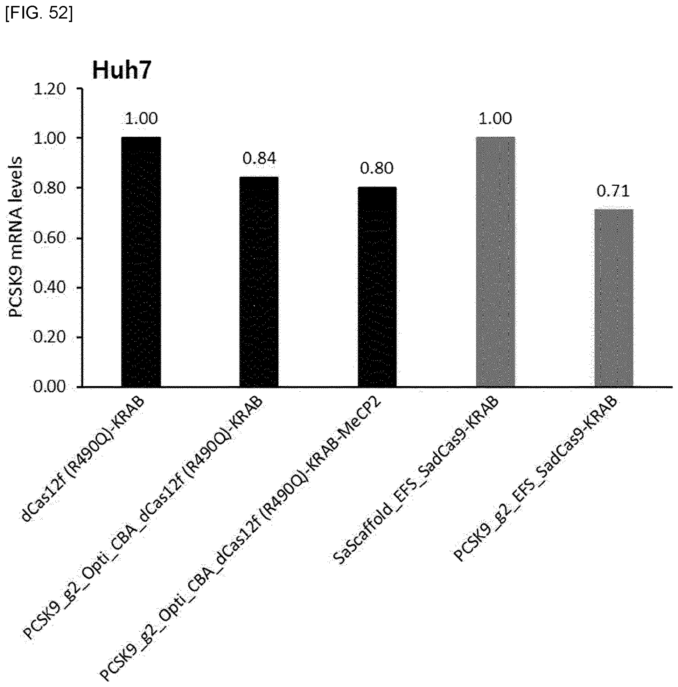

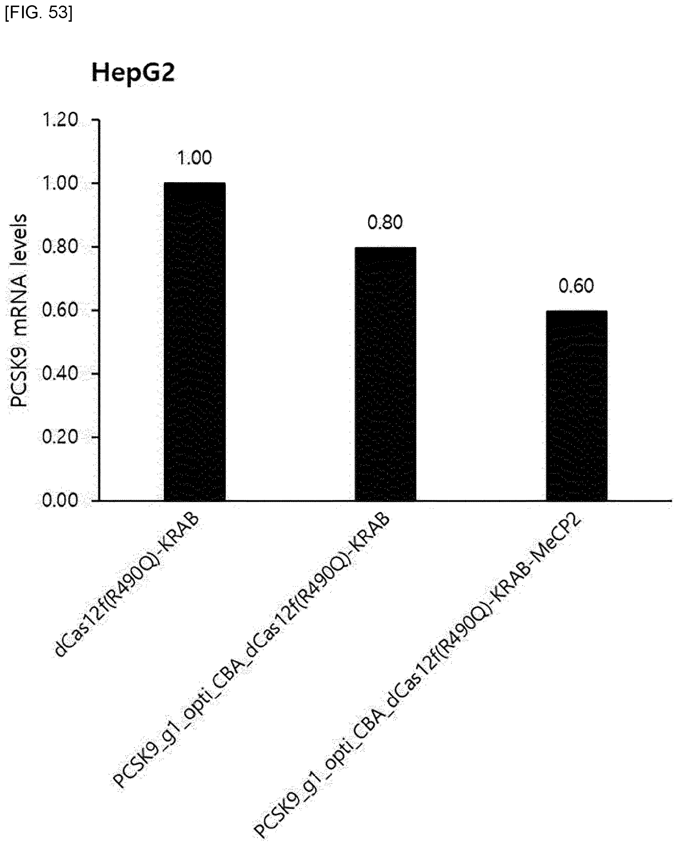

In addition, to identify inhibition of gene expression for a target selected from Huh7, HepG2, and Hep3B cells highly expressing PCSK9, comparison of the mRNA levels thereof was performed for the respective modules (FIGS. 50 to 53).

### Experimental Example 3. CRISPR expression regulatory system using transcriptional activator Cas12f1 fusion protein

### Experimental Example 3.1 Design of transcriptional activator Cas12f1 fusion protein

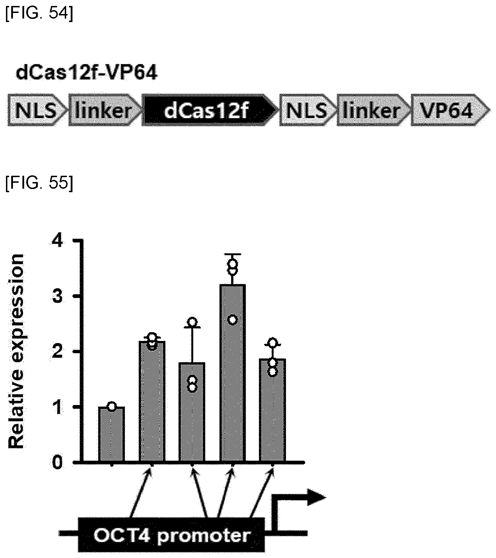

A vector expressing Cas12f1 was transformed into a dead form of Cas12f1 by mutagenesis. The dead form is D326A, E422A, R490A,r D510A, or any of its other mutated forms having lost cleavage activity. VP64, which is a transcriptional activator protein, was fused to the C-terminus of dCas12f1, to produce a transcriptional activator Cas12f1 fusion protein (FIG. 54).

### Experimental Example 3.2. Effect of promoting expression caused by CRISPR activation complex comprising transcriptional activator Cas12f1 fusion protein and engineered Cas12f1 guide RNA

Targets targeting OCT4 gene were selected. The selected targets are shown in Table 31. Vectors encoding each of the produced transcriptional activator Cas12f1 fusion proteins and each of the guide RNAs were transfected into HEK293T cells using a FugeneHD(Promega) reagent. The transfection was performed according to the protocol of the reagent. 72 hours after the transfection, the cells were harvested and RNA was extracted therefrom. cDNA was synthesized from the extracted RNA using a SuperScript IV (Invitrogen) kit. Using cDNA as a template, changes in expression were analyzed by qPCR (FIG. 55).

**[Table 31]**

| PAM | Target (5'-3') |
|---|---|
| TTTG | TTGCCCAGACTGGAGTGCAG (SEQ ID NO: 727) |
| TTTG | GCCCAGTAGATCGAGGCTAC (SEQ ID NO: 728) |
| TTTG | CCTAATGGTGGTGGCAATGG (SEQ ID NO: 729) |
| TTTA | AGACAGGGTCTCACTTTGTTG (SEQ ID NO: 730) |

### INDUSTRIAL APPLICABILITY

In the present disclosure, there is provided a CRISPR expression regulatory system that may be used in the regulation of gene expression. In particular, there is provided a CRISPR expression regulatory system comprising a transcriptional inhibitor Cas12f1 fusion protein and an engineered Cas12f1 guide RNA and/or a CRISPR expression regulatory system comprising a transcriptional activator Cas12f1 fusion protein and an engineered Cas12f1 guide RNA. In a case where the CRISPR expression regulatory system provided herein is used for regulating gene expression, expression of a target gene can be suppressed or promoted.

## Claims

1. A gene expression regulatory composition for inhibiting expression of a target gene, the composition comprising:
a transcriptional inhibitor Cas12f1 fusion protein or a nucleic acid encoding the transcriptional inhibitor Cas12f1 fusion protein, and
an engineered Cas12f1 guide RNA or a nucleic acid encoding the engineered Cas12f1 guide RNA,
wherein the transcriptional inhibitor Cas12f1 fusion protein comprises a dead Cas12f1 (dCas12f1) protein and a transcriptional inhibitor protein,
wherein the dCas12f1 protein is a modified form of a wild-type Cas12f1 protein in which aspartic acid (D) which is the 326^{th} amino acid, glutamic acid (E) which is the 422^{nd} amino acid, arginine (R) which is the 490^{th} amino acid, or aspartic acid (D) which is the 510^{th} amino acid in the amino acid sequence constituting the wild-type Cas12f1 protein is substituted with alanine (A), glutamine (Q), leucine (L), tryptophan (W), or valine (V), and
the transcriptional inhibitor protein is a protein or peptide that inhibits or suppresses transcription of the gene, and
wherein the engineered Cas12f1 guide RNA comprises:
an engineered scaffold region;
a spacer, and
a U-rich tail,
wherein the engineered scaffold region, the spacer, and the U-rich tail are sequentially linked to each other in a 5' to 3' direction,
the spacer comprises 10 to 50 nucleotides, and has a sequence complementary to the target sequence,
a sequence of the U-rich tail is represented by (UₐN)_{b}U_{c}, wherein N is one of adenosine (A), uridine (U), cytidine (C), and guanosine (G), and a, b, c are each an integer, with a being between 1 to 5 inclusive, and b being 0 or greater,
a sequence of the engineered scaffold region is different from 5'-CUUCACUGAUAAAGUGGAGAACCGCUUCACCAAAAGCUGUCCCUUAGGGGAUUAGA ACUUGAGUGAAGGUGGGCUGCUUGCAUCAGCCUAAUGUCGAGAAGUGCUUUUCUU CGGAAAGUAACCCUCGAAACCAAAUUCAUUUGAAAGAAUGAAGGAAUGCAAC-3' (SEQ ID NO: 7), and
the sequence of the engineered scaffold region is such that the following sequences are sequentially linked to each other in a 5' to 3' direction:
a sequence selected from the group consisting of 5'-A-3', 5'-AA-3', 5'-GAA-3', 5'-AGAA-3', 5'-GAGAA-3', 5'-GGAGAA-3', 5'-UGGAGAA-3', 5'-GUGGAGAA-3', 5'-AGUGGAGAA-3', 5'-AAGUGGAGAA-3' (SEQ ID NO: 17), 5'-AAAGUGGAGAA-3' (SEQ ID NO: 18),
5'-UAAAGUGGAGAA-3' (SEQ ID NO: 19),
5'-AUAAAGUGGAGAA-3' (SEQ ID NO: 20),
5'-GAUAAAGUGGAGAA-3' (SEQ ID NO: 21),
5'-UGAUAAAGUGGAGAA-3' (SEQ ID NO: 22),
5'-CUGAUAAAGUGGAGAA-3' (SEQ ID NO: 23),
5'-ACUGAUAAAGUGGAGAA-3' (SEQ ID NO: 24),
5'-CACUGAUAAAGUGGAGAA-3' (SEQ ID NO: 25),
5'-UCACUGAUAAAGUGGAGAA-3' (SEQ ID NO: 26),
5'-UUCACUGAUAAAGUGGAGAA-3' (SEQ ID NO: 27), and
5'-CUUCACUGAUAAAGUGGAGAA-3' (SEQ ID NO: 10);
a sequence selected from the group consisting of 5'-G-3', 5'-UUAGG-3', 5'-CUUAGGG-3', 5'-CUUAGUGG-3', 5'-CCUUAGGUGG-3' (SEQ ID NO: 342), 5'-CCGUUAGGUGG-3' (SEQ ID NO: 343), 5'-CCGCUUAGGGUGG-3' (SEQ ID NO: 344),
5'-CCGCUUUAGAGGUGG-3' (SEQ ID NO: 345),
5'-CCGCUUUUAGAAGGUGG-3' (SEQ ID NO: 346),
5'-CCGCUUCUUAGGAAGGUGG-3' (SEQ ID NO: 347),
5'-CCGCUUCAUUAGUGAAGGUGG-3' (SEQ ID NO: 348),
5'-CCGCUUCACUUAGGUGAAGGUGG-3' (SEQ ID NO: 349),
5'-CCGCUUCACUUAGAGUGAAGGUGG-3' (SEQ ID NO: 350),
5'-CCGCUUCACCUUAGGAGUGAAGGUGG-3' (SEQ ID NO: 351),
5'-CCGCUUCACCAUUAGUGAGUGAAGGUGG-3' (SEQ ID NO: 352),
5'-CCGCUUCACCAAUUAGUUGAGUGAAGGUGG-3' (SEQ ID NO: 353),
5'-CCGCUUCACCAAAUUAGCUUGAGUGAAGGUGG-3' (SEQ ID NO: 354),
5'-CCGCUUCACCAAAAUUAGACUUGAGUGAAGGUGG-3' (SEQ ID NO: 355),
5'-CCGCUUCACCAAAAGUUAGAACUUGAGUGAAGGUGG-3' (SEQ ID NO: 356),
5'-CCGCUUCACCAAAAGCUUAGGAACUUGAGUGAAGGUGG-3' (SEQ ID NO: 357),
5'-CCGCUUCACCAAAAGCUUUAGAGAACUUGAGUGAAGGUGG-3' (SEQ ID NO: 358),
5'-CCGCUUCACCAAAAGCUGUUAGUAGAACUUGAGUGAAGGUGG-3' (SEQ ID NO: 359),
5'-CCGCUUCACCAAAAGCUGUUAGUUAGAACUUGAGUGAAGGUGG-3' (SEQ ID NO: 360),
5'-CCGCUUCACCAAAAGCUGUUUAGAUUAGAACUUGAGUGAAGGUGG-3' (SEQ ID NO: 361),
5'-CCGCUUCACCAAAAGCUGUCUUAGGAUUAGAACUUGAGUGAAGGUGG-3' (SEQ ID NO: 362), and 
5'-
CCGCUUCACCAAAAGCUGUCCCUUAGGGGAUUAGAACUUGAGUGAAGGUGG-3'
(SEQ ID NO: 11);
a sequence selected from the group consisting of 5'-GCUGCUUGCAUCAGCCUAAUGUCGAGAAGUGCUUUCUUCGGAAAGUAACCCUCGA-3' (SEQ ID NO: 434), 
5'-
GCUGCUUGCAUCAGCCUAAUGUCGAGAAGUGCUUUUUCGAAAGUAACCCUCGA-3'
(SEQ ID NO: 435),
 
5'-
GCUGCUUGCAUCAGCCUAAUGUCGAGAAGUGCUUUUCGAAGUAACCCUCGA-3' (SEQ
ID NO: 436),
 
5'-
GCUGCUUGCAUCAGCCUAAUGUCGAGAAGUGCUCUUCGGAGUAACCCUCGA-3'
(SEQ ID NO: 437),
 
5'-GCUGCUUGCAUCAGCCUAAUGUCGAGAAGUGCUUUCGAGUAACCCUCGA-
3' (SEQ ID NO: 438),
 
5'-GCUGCUUGCAUCAGCCUAAUGUCGAGAAGUGCUUCGGUAACCCUCGA-3'

(SEQ ID NO: 439),
 
5'-GCUGCUUGCAUCAGCCUAAUGUCGAGAAGUGUUCGUAACCCUCGA-3'
(SEQ ID NO: 440),
5'-GCUGCUUGCAUCAGCCUAAUGUCGAGAAGUUUCGAACCCUCGA-3' (SEQ ID NO: 441),
5'-GCUGCUUGCAUCAGCCUAAUGUCGAGAAGUUCGACCCUCGA-3' (SEQ ID NO: 442),
5'-GCUGCUUGCAUCAGCCUAAUGUCGAGAAGUUCGCCCUCGA-3' (SEQ ID NO: 443),
5'-GCUGCUUGCAUCAGCCUAAUGUCGAGAAUUCGCCCUCGA-3' (SEQ ID NO: 444),
5'-GCUGCUUGCAUCAGCCUAAUGUCGAGAAUUCGCCUCGA-3' (SEQ ID NO: 445),
5'-GCUGCUUGCAUCAGCCUAAUGUCGAGAUUCGCCUCGA-3' (SEQ ID NO: 446),
5'-GCUGCUUGCAUCAGCCUAAUGUCGAGUUCGCUCGA-3' (SEQ ID NO: 447), and 
5'-
GCUGCUUGCAUCAGCCUAAUGUCGAGAAGUGCUUUCUUCGGAAAGUAACCCUCGA-
3' (SEQ ID NO: 12);
a sequence selected from 5'-AACAAAGAAAGGA-3' (SEQ ID NO: 111),
5'-AACAAAUGAAAAGGA-3' (SEQ ID NO: 112),
5'-AACAAAUUGAAAAAGGA-3' (SEQ ID NO: 113),
5'-AACAAAUUCGAAAGAAGGA-3' (SEQ ID NO: 114),
5'-AACAAAUUCAGAAAUGAAGGA-3' (SEQ ID NO: 115),
5'-AACAAAUUCAUGAAAAUGAAGGA-3' (SEQ ID NO: 116),
5'-AACAAAUUCAUUGAAAAAUGAAGGA-3' (SEQ ID NO: 117), and
5'-AACAAAUUCAUUUGAAAGAAUGAAGGA-3' (SEQ ID NO: 118); and
5'-AUGCAAC-3'.

2. The composition of claim 1, wherein the dCas12f1 protein is a dCas12f1 R490A protein, a dCas12f1 R490Q protein, a dCas12f1 R490L protein, or a dCas12f1 R490W protein.

3. The composition of claim 1, wherein the dCas12f1 protein is a dCas12f1 D510A protein, a dCas12f1 D510L protein, or a dCas12f1 D510V protein.

4. The composition of claim 1, wherein the dCas12f1 protein is a dCas12f1 D326A protein or a dCas12f1 E422A protein.

5. The composition of claim 1, wherein the transcriptional inhibitor protein is a protein or peptide that inhibits or suppresses transcription of the target gene by blocking RNA polymerase from being attached to a promoter of the target gene or inducing a structural change in chromatin of the target gene.

6. The composition of claim 1, wherein the transcriptional inhibitor protein is KRAB, MeCP2, DNMT, LSD, or HDAC.

7. The composition of claim 1, wherein the transcriptional inhibitor Cas12f1 fusion protein further comprises at least one transcriptional inhibitor protein.

8. The composition of claim 7, wherein the transcriptional inhibitor protein is KRAB, MeCP2, DNMT, LSD, or HDAC.

9. The composition of claim 1, wherein the transcriptional inhibitor Cas12f1 fusion protein further comprises at least one NLS or NES.

10. The composition of claim 1, wherein the sequence of the U-rich tail is represented by 5'-UUUURUUUU-3'.

11. The composition of claim 1, wherein the sequence of the scaffold region included in the engineered Cas12f1 guide RNA is such that the following sequences are sequentially linked to each other in a 5' to 3' direction:
5'-A-3'; 
5'-
CCGCUUCACCAAAAGCUGUCCCUUAGGGGAUUAGAACUUGAGUGAAGGUGG-3'
(SEQ ID NO: 11);
 
5'-
GCUGCUUGCAUCAGCCUAAUGUCGAGAAGUGCUUUCUUCGGAAAGUAACCCUCGA-
3' (SEQ ID NO: 12);
5'-AACCAAAGAAAGGA-3' (SEQ ID NO: 111); and
5'-AUGCAAC-3'.

12. The composition of claim 1, wherein a sequence of the scaffold region included in the engineered Cas12f1 guide RNA is such that the following sequences are sequentially linked to each other in a 5' to 3' direction:
5'-A-3';
5'-CCGCUUCACUUAGAGUGAAGGUGG-3' (SEQ ID NO: 350); 
5'-
GCUGCUUGCAUCAGCCUAAUGUCGAGAAGUGCUUUCUUCGGAAAGUAACCCUCGA-
3' (SEQ ID NO: 12);
5'-AACCAAAGAAAGGA-3' (SEQ ID NO: 111); and
5'-AUGCAAC-3'.

13. The composition of claim 1, wherein the composition is in a form of a vector.

14. The composition of claim 13, wherein the composition further comprises promoters for the nucleic acid encoding the transcriptional inhibitor Cas12f1 fusion protein and the nucleic acid encoding the engineered Cas12f1 guide RNA.

15. The composition of claim 13, wherein the vector is at least one selected from the group consisting of a plasmid, a retrovirus, a lentivirus, an adenovirus, an adeno-associated virus, a vaccinia virus, a poxvirus, and a herpes simplex virus.

16. The composition of claim 1, wherein the composition is in a form of a ribonucleoprotein (RNP) in which the engineered Cas12f1 guide RNA and the transcriptional inhibitor Cas12f1 fusion protein are bound to each other.

17. A method of inhibiting expression of a target gene in a cell, the method comprising
delivering, into a cell, a transcriptional inhibitor Cas12f1 fusion protein or a nucleic acid encoding the transcriptional inhibitor Cas12f1 fusion protein, and an engineered Cas12f1 guide RNA or a nucleic acid encoding the engineered Cas12f1 guide RNA,
which allows a CRISPR interference complex to be formed in the cell,
wherein the CRISPR interference complex is capable of suppressing transcription of the target gene,
the transcriptional inhibitor Cas12f1 fusion protein comprises a dead Cas12f1 (dCas12f1) protein and a transcriptional inhibitor protein,
the dCas12f1 protein is a modified form of a wild-type Cas12f1 protein in which aspartic acid (D) which is the 326^{th} amino acid, glutamic acid (E) which is the 422^{nd} amino acid, arginine (R) which is the 490^{th} amino acid, or aspartic acid (D) which is the 510^{th} amino acid in the amino acid sequence constituting the wild-type Cas12f1 protein is substituted with alanine (A), glutamine (Q), leucine (L), tryptophan (W), or valine (V), and
the transcriptional inhibitor protein is a protein or peptide that inhibits or suppresses transcription of the gene, and
wherein the engineered Cas12f1 guide RNA comprises:
an engineered scaffold region;
a spacer, and
a U-rich tail,
wherein the engineered scaffold region, the spacer, and the U-rich tail are sequentially linked to each other in a 5' to 3' direction,
the spacer comprises 10 to 50 nucleotides, and has a sequence complementary to the target sequence,
a sequence of the U-rich tail is represented by (UₐN)_{b}U_{c}, wherein N is one of adenosine (A), uridine (U), cytidine (C), and guanosine (G), and a, b, c are each an integer, with a being between 1 to 5 inclusive, and b being 0 or greater,
a sequence of the engineered scaffold region is different from 5'-CUUCACUGAUAAAGUGGAGAACCGCUUCACCAAAAGCUGUCCCUUAGGGGAUUAGA ACUUGAGUGAAGGUGGGCUGCUUGCAUCAGCCUAAUGUCGAGAAGUGCUUUUCUU CGGAAAGUAACCCUCGAAACCAAAUUCAUUUGAAAGAAUGAAGGAAUGCAAC-3' (SEQ ID NO: 7), and
the sequence of the engineered scaffold region is such that the following sequences are sequentially linked to each other in a 5' to 3' direction:
a sequence selected from the group consisting of 5'-A-3', 5'-AA-3', 5'-GAA-3', 5'-AGAA-3', 5'-GAGAA-3', 5'-GGAGAA-3', 5'-UGGAGAA-3', 5'-GUGGAGAA-3', 5'-AGUGGAGAA-3', 5'-AAGUGGAGAA-3' (SEQ ID NO: 17), 5'-AAAGUGGAGAA-3' (SEQ ID NO: 18),
5'-UAAAGUGGAGAA-3' (SEQ ID NO: 19),
5'-AUAAAGUGGAGAA-3' (SEQ ID NO: 20),
5'-GAUAAAGUGGAGAA-3' (SEQ ID NO: 21),
5'-UGAUAAAGUGGAGAA-3' (SEQ ID NO: 22),
5'-CUGAUAAAGUGGAGAA-3' (SEQ ID NO: 23),
5'-ACUGAUAAAGUGGAGAA-3' (SEQ ID NO: 24),
5'-CACUGAUAAAGUGGAGAA-3' (SEQ ID NO: 25),
5'-UCACUGAUAAAGUGGAGAA-3' (SEQ ID NO: 26),
5'-UUCACUGAUAAAGUGGAGAA-3' (SEQ ID NO: 27), and
5'-CUUCACUGAUAAAGUGGAGAA-3' (SEQ ID NO: 10);
a sequence selected from the group consisting of 5'-G-3', 5'-UUAGG-3', 5'-CUUAGGG-3', 5'-CUUAGUGG-3', 5'-CCUUAGGUGG-3' (SEQ ID NO: 342), 5'-CCGUUAGGUGG-3' (SEQ ID NO: 343), 5'-CCGCUUAGGGUGG-3' (SEQ ID NO: 344),
5'-CCGCUUUAGAGGUGG-3' (SEQ ID NO: 345),
5'-CCGCUUUUAGAAGGUGG-3' (SEQ ID NO: 346),
5'-CCGCUUCUUAGGAAGGUGG-3' (SEQ ID NO: 347),
5'-CCGCUUCAUUAGUGAAGGUGG-3' (SEQ ID NO: 348),
5'-CCGCUUCACUUAGGUGAAGGUGG-3' (SEQ ID NO: 349),
5'-CCGCUUCACUUAGAGUGAAGGUGG-3' (SEQ ID NO: 350),
5'-CCGCUUCACCUUAGGAGUGAAGGUGG-3' (SEQ ID NO: 351),
5'-CCGCUUCACCAUUAGUGAGUGAAGGUGG-3' (SEQ ID NO: 352),
5'-CCGCUUCACCAAUUAGUUGAGUGAAGGUGG-3' (SEQ ID NO: 353),
5'-CCGCUUCACCAAAUUAGCUUGAGUGAAGGUGG-3' (SEQ ID NO: 354),
5'-CCGCUUCACCAAAAUUAGACUUGAGUGAAGGUGG-3' (SEQ ID NO: 355),
5'-CCGCUUCACCAAAAGUUAGAACUUGAGUGAAGGUGG-3' (SEQ ID NO: 356),
5'-CCGCUUCACCAAAAGCUUAGGAACUUGAGUGAAGGUGG-3' (SEQ ID NO: 357),
5'-CCGCUUCACCAAAAGCUUUAGAGAACUUGAGUGAAGGUGG-3' (SEQ ID NO: 358),
5'-CCGCUUCACCAAAAGCUGUUAGUAGAACUUGAGUGAAGGUGG-3' (SEQ ID NO: 359),
5'-CCGCUUCACCAAAAGCUGUUAGUUAGAACUUGAGUGAAGGUGG-3' (SEQ ID NO: 360),
5'-CCGCUUCACCAAAAGCUGUUUAGAUUAGAACUUGAGUGAAGGUGG-3' (SEQ ID NO: 361),
5'-CCGCUUCACCAAAAGCUGUCUUAGGAUUAGAACUUGAGUGAAGGUGG-3' (SEQ ID NO: 362), and 
5'-
CCGCUUCACCAAAAGCUGUCCCUUAGGGGAUUAGAACUUGAGUGAAGGUGG-3'
(SEQ ID NO: 11);
a sequence selected from the group consisting of 5'-GCUGCUUGCAUCAGCCUAAUGUCGAGAAGUGCUUUCUUCGGAAAGUAACCCUCGA-3' (SEQ ID NO: 434), 
5'-
GCUGCUUGCAUCAGCCUAAUGUCGAGAAGUGCUUUUUCGAAAGUAACCCUCGA-3'
(SEQ ID NO: 435),
 
5'-
GCUGCUUGCAUCAGCCUAAUGUCGAGAAGUGCUUUUCGAAGUAACCCUCGA-3' (SEQ
ID NO: 436),
 
5'-
GCUGCUUGCAUCAGCCUAAUGUCGAGAAGUGCUCUUCGGAGUAACCCUCGA-3'
(SEQ ID NO: 437),
 
5'-GCUGCUUGCAUCAGCCUAAUGUCGAGAAGUGCUUUCGAGUAACCCUCGA-
3' (SEQ ID NO: 438),
5'-GCUGCUUGCAUCAGCCUAAUGUCGAGAAGUGCUUCGGUAACCCUCGA-3' (SEQ ID NO: 439),
5'-GCUGCUUGCAUCAGCCUAAUGUCGAGAAGUGUUCGUAACCCUCGA-3' (SEQ ID NO: 440),
5'-GCUGCUUGCAUCAGCCUAAUGUCGAGAAGUUUCGAACCCUCGA-3' (SEQ ID NO: 441),
5'-GCUGCUUGCAUCAGCCUAAUGUCGAGAAGUUCGACCCUCGA-3' (SEQ ID NO: 442),
5'-GCUGCUUGCAUCAGCCUAAUGUCGAGAAGUUCGCCCUCGA-3' (SEQ ID NO: 443),
5'-GCUGCUUGCAUCAGCCUAAUGUCGAGAAUUCGCCCUCGA-3' (SEQ ID NO: 444),
5'-GCUGCUUGCAUCAGCCUAAUGUCGAGAAUUCGCCUCGA-3' (SEQ ID NO: 445),
5'-GCUGCUUGCAUCAGCCUAAUGUCGAGAUUCGCCUCGA-3' (SEQ ID NO: 446),
5'-GCUGCUUGCAUCAGCCUAAUGUCGAGUUCGCUCGA-3' (SEQ ID NO: 447), and 
5'-
GCUGCUUGCAUCAGCCUAAUGUCGAGAAGUGCUUUCUUCGGAAAGUAACCCUCGA-
3' (SEQ ID NO: 12);
a sequence selected from 5'-AACAAAGAAAGGA-3' (SEQ ID NO: 111),
5'-AACAAAUGAAAAGGA-3' (SEQ ID NO: 112),
5'-AACAAAUUGAAAAAGGA-3' (SEQ ID NO: 113),
5'-AACAAAUUCGAAAGAAGGA-3' (SEQ ID NO: 114),
5'-AACAAAUUCAGAAAUGAAGGA-3' (SEQ ID NO: 115),
5'-AACAAAUUCAUGAAAAUGAAGGA-3' (SEQ ID NO: 116),
5'-AACAAAUUCAUUGAAAAAUGAAGGA-3' (SEQ ID NO: 117), and
5'-AACAAAUUCAUUUGAAAGAAUGAAGGA-3' (SEQ ID NO: 118); and
5'-AUGCAAC-3'.

18. The method of claim 17, wherein the delivery is achieved by introducing, into the cell, the transcriptional inhibitor Cas12f1 fusion protein and the engineered Cas12f1 guide RNA as a CRISPR interference complex.

19. The method of claim 17, wherein the delivery is achieved by introducing, into the cell, a vector that comprises the nucleic acid encoding the transcriptional inhibitor Cas12f1 fusion protein and the nucleic acid encoding the engineered Cas12f1 guide RNA.

20. The method of claim 19, wherein the vector is a plasmid vector or a viral vector.

21. The method of claim 20, wherein the viral vector is at least one selected from the group consisting of a retrovirus, a lentivirus, an adenovirus, an adeno-associated virus, a vaccinia virus, a poxvirus, and a herpes simplex virus.

22. The method of claim 17, wherein the cell is a eukaryotic cell.
